# EUROPEAN PATENT SPECIFICATION

(11) **EP 4 013 756 B1**
(45) Date of publication and mention of the grant of the patent: **08.05.2024**
(21) Application number: 20781093.8
(22) Date of filing: 18.09.2020
(51) Int. Cl.: C07D 473/34, C07D 487/04, A61K 31/437, A61K 31/52, A61P 35/00, A61P 35/02

(54) **MLL1 INHIBITORS AND ANTI-CANCER AGENTS**
MLL1-INHIBITOREN UND MITTEL GEGEN KREBS
INHIBITEURS DE MLL1 ET AGENTS ANTICANCÉREUX

(30) Priority: 20.09.2019 WO PCT/CN2019/107010; 12.06.2020 WO PCT/CN2020/095916
(43) Date of publication of application: 22.06.2022
(73) Proprietor: Novartis AG, 4056 Basel (CH)
(72) Inventor: LI, Ming, Shanghai 201204 (CN); LIU, Kevin Kun Chin, Shanghai 201203 (CN); LU, Chunliang, Shanghai 200083 (CN); SUN, Zhuming, Shanghai 201203 (CN); ZHAO, Jichen, Shanghai 200231 (CN); ZHU, Yihui, Warren, New Jersey 07059 (US)
(74) Representative: Laufhütte, Dieter
(86) International application number: PCT/IB2020/058735
(87) International publication number: WO 2021/053617

(56) References cited:
- EP-A1- 1 550 662
- WO-A1-2016/195776
- WO-A1-2018/053267
- WO-A2-2015/002754

## Description

### CROSS-REFERENCE TO RELATED APPLICATIONS

This application claims the benefit of PCT/CN2019/107010 filed 20 September 2019 and PCT/CN2020/095916 filed 12 June 2020.

### FIELD OF THE INVENTION

The present invention relates to compounds, and compositions for inhibiting mixed lineage leukemia 1 (MLL1).

### BACKGROUND OF THE INVENTION

Chromatin is the main repository of genetic information in the eukaryotic nucleus, comprising DNA compacted into structures called nucleosomes by a set of small basic histones and non-histone chromosomal proteins. Histones can be enzymatically modified by the addition of acetyl, methyl, or phosphate groups. Histone-lysine N-methyltransferase 2 (KMT2) family proteins methylate lysine 4 on the histone H3 tail (H3K4) at important regulatory regions in the genome, and thereby impart crucial functions through modulating chromatin structures and DNA accessibility.

KMT2 family mutations are among the most frequent alterations in human cancer (Kandoth et al., Nature 502:333-339 (2013)). The most prominent example is mixed lineage leukemia (MLL or MLL-r), which presents a heterogeneous group of AML (acute myeloid leukemia) and ALL (acute lymphoblastic leukemia) bearing features. The hallmark of this type of disease is the chromosomal rearrangement of MLL1 gene (also known as KMT2A, MLL, ALL-1 and HRX) (Krivtsov et al., Nature Rev. Cancer 7:823-833 (2007); Liedtke et al., Blood 113:6061-6068 (2009).

In MLL1 rearranged leukemia, reciprocal translocation of MLL1 gene results in in-frame fusion of the 5'-end MLL1 with the 3'-end of the fusion partner gene, which may recruit other factors aberrantly such as Dot1L. A common feature of MLL1 abnormality in MLL-r leukemia is the preservation of one wild-type MLL1 allele. It has been reported that MLL1-AF9 induced leukemogenesis requires co-expression of the wild type MLL1 allele, since genetic deletion of MLL1 in MLL1-AF9 murine leukemia cells reduced clonogenic potential and leukemia progression. Mutations in the MLL1 region are also prevalent in a large range of solid tumors, including for example, colon, lung, bladder, endometrial and breast cancers (Ding et al., Nature 455:1069-1075 (2008); Wood et al., Science 318:1108-1113 (2007); Giu et al., Genetics 43:875-878 (2011); Kandoth et al., Nature 497:67-73 (2013); EP1550662; WO2015/002754; WO2016/195776; WO2018/053267).

### MLL1 is a promising therapeutic target for MLL-r AML, ALL and other cancers; and there remains a need for selective inhibitors of MLL1.SUMMARY OF THE INVENTION

The present invention provides novel compounds that inhibit MLL1; and compositions for treating or preventing a disease or condition mediated by MLL1.

In one aspect, the invention provides a compound of Formula (I): 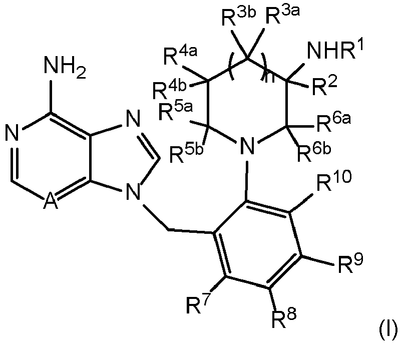 or an enantiomer, an enantiomeric mixture, or a pharmaceutically acceptable salt thereof; wherein:
A is N;
R¹ is H; or
R¹ and R² together with NH forms a 5-8 membered heterocyclyl comprising 1-2 heteroatoms selected from N, O and S as ring members; wherein said 5-8 membered heterocyclyl is unsubstituted or substituted by an oxo substituent;
R² is selected from the group consisting of:
   (i) -C₁₋₆ alkyl, -haloC₁₋₆ alkyl, -hydroxyC₁₋₆ alkyl, -C₁₋₆alkoxyC₁₋₆alkyl or -C₃₋₈ cycloalkoxy(C₁₋₆ alkyl);
   (ii) cyano, -cyanoC₁₋₆ alkyl, -C₁₋₆ alkylthioC₁₋₆alkyl, -C₂₋₆ alkenyl, -haloC₂₋₆ alkenyl, -C₂₋₆ alkynyl, -C₁₋₄ alkyl-S-C₁₋₄alkyl, -C₁₋₄ alkylSO₂C₁₋₄alkyl, -SO₂(₁₋₄ alkyl) or -C(C₁₋₄ alkyl)=N-O(C₁₋₄ alkyl);
   (iii) -C₁₋₄alkylcarbonyl, -(CR^{a}R^{b})ₚ-C(=O)-OR^{10a} or -C(=O)-(CR^{a}R^{b})_{q}R¹¹;
      wherein R¹¹ is C₃₋₇ cycloalkyl, 5-6 membered heterocyclyl or 5-6 membered heteroaryl, each of which is independently unsubstituted or substituted with -C₁₋₆ alkyl or -C₁₋₆ alkoxy; and said 5-6 membered heterocyclyl or 5-6 membered heteroaryl independently comprises 1-3 heteroatoms selected from nitrogen, oxygen and sulfur;
   (iv) -(CR^{a}R^{b})ᵣ-C(=O)-NR¹²R¹³-(CR^{a}R^{b})ₛ-NR¹²R¹³, -(CR^{a}R^{b})₁₋₄-O-(CR^{a}R^{b})₁₋₄-OR^{10a} or -(CR^{a}R⁶)₁₋₄-O-(CR^{a}R⁶)₁₋₄-C(=O)-NR¹²R¹³;
      wherein R¹² is hydrogen or -C₁₋₆ alkyl;
      R¹³ is hydrogen, -C₁₋₆ alkyl, -C₁₋₆alkoxyC₁₋₆alkyl, -cyanoC₁₋₆ alkyl;
         -C₁₋₄ alkylSO₂R^{10b} wherein R^{10b} is C₁₋₆ alkyl or phenyl; C₃₋₁₀ monocylic or bicyclic cycloalkylC₀₋₆alkyl, phenyl, 5-10 membered monocyclic or bicyclic heterocyclic ring or 5-9 membered heteroarylC₀₋₆alkyl;
      said 5-10 membered monocyclic or bicyclic heterocyclic ring or 5-9 membered heteroaryl radical independently comprises 1-4 heteroatoms selected from nitrogen, oxygen and sulfur; and
      said C₃₋₁₀ monocylic or bicyclic cycloalkyl, phenyl, 5-10 membered monocyclic or bicyclic heterocyclic ring or 5-9 membered heteroaryl radical are independently unsubstituted or substituted with 1-2 -C₁₋₄ alkyl, -hydroxyC₁₋₆ alkyl, -C₁₋₄ alkoxy, halo, hydroxyl, phenyl or -S(C₁₋₄ alkyl);
      or R¹² and R¹³ together form a 5-10 membered monocyclic or bicyclic heterocyclic ring comprising 1-4 heteroatoms selected from nitrogen, oxygen and sulfur; and is unsubstituted or substituted with -C₁₋₄ alkyl, hydroxyl, cyano, -cyanoC₁₋₆ alkyl, -SO₂ or -C₂₋₄alkenylcarbonyl;
   (v) 5-6 membered heterocyclylC₀₋₆alkyl or 5-6 membered heterocyclyl(haloC₁₋₄ alkyl)
      wherein each said heterocyclyl radical is unsubstituted or substituted by oxo; and wherein each said heterocyclyl radical comprises 1-3 heteroatoms selected from nitrogen, oxygen and sulfur; and
   (vi) phenyl, 5-9 membered heteroarylC₀₋₆alkyl or 5-9 membered heteroaryl(haloC₁₋₄alkyl),
   wherein each said phenyl or heteroaryl radical is independently unsubstituted or substituted by -C₁₋₄ alkyl, -haloC₁₋₄ alkyl, -hydroxyC₁₋₄ alkyl, -C₁₋₄ alkoxy, -haloC₁₋₄ alkoxy, halo, hydroxy, cyano, oxido, amino, -C₁₋₄ alkylamino, -C₁₋₄ dialkylamino, -aminocarbonylC₀₋₆alkyl, -C₁₋₄alkylaminocarbonylC₀₋₆alkyl, -diC₁₋₄alkylaminocarbonylC₀₋₆alkyl or C₃₋₇ cycloalkyl;
   wherein each said heteroaryl radical comprises 1-4 heteroatoms selected from nitrogen, oxygen and sulfur;
   R^{3a}, R^{3b}, R^{4a}, R^{4b}, R^{5a}, R^{5b}, R^{6a} and R^{6b} are independently hydrogen, halo, cyano, hydroxyl, -C₁₋₆ alkyl, -haloC₁₋₆ alkyl, -hydroxyC₁₋₆ alkyl, -C₁₋₆ alkoxy, -C₁₋₆alkoxyC₁₋₆alkyl, aryl, -C(=O)-OR¹⁴ or-(CR^{a}R^{b})ₛ-C(=O)-NR¹⁵R¹⁶; or
   R^{3a} and R^{3b}, R^{4a} and R^{4b}, R^{5a} and R^{5b} or R^{6a} and R^{6b} forms an oxo substituent;
   R⁷, R⁸, R⁹ and R¹⁰ are independently hydrogen, halo, -C₁₋₄ alkyl, -haloC₁₋₆ alkyl, -hydroxyC₁₋₆ alkyl, cyano, -cyanoC₁₋₆ alkyl, -C₂₋₆ alkenyl, -C₂₋₆ alkynyl, -C₁₋₆ alkylthio, -(CR^{a}R^{b})₁₋₄-NR¹⁷R¹⁸, -(CR^{a}R^{b})₁₋₄NR¹⁷-C(O)-OR¹⁸, -(CR^{a}R^{b})₁₋₄-OR¹⁹, C₃₋₈ cycloalkyl, phenyl or 5-6 membered heteroaryl comprising 1-3 heteroatoms selected from nitrogen, oxygen and sulfur; wherein said C₃₋₈ cycloalkyl, phenyl or 5-6 membered heteroaryl is independently substituted with 1-2 R²⁰; and provided at least one of R⁷, R⁸, R⁹ and R¹⁰ is not hydrogen;
   alternatively, R⁷ and R⁸, R⁸ and R⁹, and R⁹ together with the phenyl ring to which they are attached form a 9-10 membered benzo-fused carbocycle or benzo-fused heterocycle comprising 1-3 heteroatoms selected from nitrogen, oxygen and sulfur; wherein said benzo-fused carbocycle or benzo-fused heterocycle is independently unsubstituted or substituted with 1-2 halo or C₁₋₄ alkyl;
   R^{a}, R^{b}, R^{10a}, R¹⁴, R¹⁵, R¹⁶ and R¹⁷ are independently hydrogen or -C₁₋₄ alkyl;
   R¹⁸ is hydrogen, -C₁₋₄ alkyl, -haloC₁₋₆ alkyl or -C₃₋₆ cycloalkyl;
   alternatively, R¹⁷ and R¹⁸ together with N in the -NR¹⁷R¹⁸ moiety form a 4-10 membered monocyclic or bicyclic heterocyclic ring comprising 1-3 heteroatoms selected from nitrogen, oxygen and sulfur; wherein said 4-10 membered monocyclic or bicyclic heterocyclic ring is unsubstituted or substituted with 1-2 halo or C₁₋₄alkyl;
   R¹⁹ is hydrogen, -C₁₋₄ alkyl, -haloC₁₋₆ alkyl, -C₃₋₆ cycloalkyl, phenyl, 5-6 membered heterocyclyl or 5-6 membered heteroaryl; wherein said 5-6 membered heterocyclyl or 5-6 membered heteroaryl independently comprises 1-3 heteroatoms selected from nitrogen, oxygen and sulfur;
   R²⁰ is -C₁₋₄ alkyl, -halo or -C₃₋₆ cycloalkyl; or two R²⁰ together form a 5-6 membered ring comprising 0-2 heteroatoms selected from nitrogen, oxygen and sulfur;
   n is 0 or 1; and
   p, q, r, and s are independently 0, 1, 2, 3 or 4.

In another aspect, the invention provides a pharmaceutical composition comprising a therapeutically effective amount of a compound of Formula (I) or sub-formulae thereof, or a pharmaceutically acceptable salt thereof; and one or more pharmaceutically acceptable carriers.

In yet another aspect, the invention provides a combination, in particular a pharmaceutical combination, comprising a therapeutically effective amount of a compound of Formula (I) or sub-formulae thereof, or a pharmaceutically acceptable salt thereof; and one or more therapeutically active agent(s).

The compounds of the invention, alone or in combination with one or more therapeutically active agent(s), can be used for treating or preventing a disease or condition mediated by MLL1; and more particularly wherein the disease or condition is characterized by overexpression or undesired upregulation of MLL1.

### DETAILED DESCRIPTION OF THE INVENTION

The present invention provides compositions for treating or preventing a disease or condition mediated by MLL1.

### Definitions

For purposes of interpreting this specification, the following definitions will apply and whenever appropriate, terms used in the singular will also include the plural and vice versa.

As used herein, the term "-C₁₋₆alkyl" refers to a straight or branched hydrocarbon chain radical consisting solely of carbon and hydrogen atoms, containing no unsaturation, having from one to six carbon atoms, and which is attached to the rest of the molecule by a single bond. The term "C₁₋₄alkyl" is to be construed accordingly. Examples of C₁₋₆alkyl include, but are not limited to, methyl, ethyl, *n*-propyl, 1-methylethyl (*iso*-propyl), *n*-butyl, *n*-pentyl and 1,1-dimethylethyl (*t-*butyl).

As used herein, the term "-C₂₋₆alkenyl" refers to a straight or branched hydrocarbon chain radical group consisting solely of carbon and hydrogen atoms, containing at least one double bond, having from two to six carbon atoms, which is attached to the rest of the molecule by a single bond. The term "C₂₋₄alkenyl" is to be construed accordingly. Examples of C₂₋₆alkenyl include, but are not limited to, ethenyl, prop-1-enyl, but-1-enyl, pent-1-enyl, pent-4-enyl and penta-1,4-dienyl.

As used herein, the term "-C₂₋₆alkynyl" refers to a straight or branched hydrocarbon chain radical group consisting solely of carbon and hydrogen atoms, containing at least one triple bond, having from two to six carbon atoms, and which is attached to the rest of the molecule by a single bond. The term "C₂₋₄alkynyl" is to be construed accordingly. Examples of C₂₋₆alkynyl include, but are not limited to, ethynyl, prop-1-ynyl, but-1-ynyl, pent-1-ynyl, pent-4-ynyl and penta-1,4-diynyl.

As used herein, the term "-C₁₋₆alkoxy" refers to a radical of the formula -ORₐ where Rₐ is a C₁₋₆alkyl radical as generally defined above. Examples of C₁₋₆alkoxy include, but are not limited to, methoxy, ethoxy, propoxy, isopropoxy, butoxy, isobutoxy, pentoxy, and hexoxy.

As used herein, the term "-C₁₋₆alkoxyC₁₋₆alkyl " refers to a radical of the formula -Rₐ-O-Rₐ where each Rₐ is independently a C₁₋₆alkyl radical as defined above. The oxygen atom may be bonded to any carbon atom in either alkyl radical. Examples of C₁₋₆alkoxy C₁₋₆alkyl include, but are not limited to, methoxy-methyl, methoxy-ethyl, ethoxy-ethyl, 1-ethoxy-propyl and 2-methoxybutyl.

As used herein, the term "-C₁₋₄alkylcarbonyl" refers to a radical of the formula -C(=O)-Rₐ where Rₐ is a C₁₋₄alkyl radical as defined above.

As used herein, the term "-C₁₋₄alkylthioC₁₋₄alkyl" refers to a radical of the formula -Rₐ-S-Rₐ, where each Rₐ is independently a C₁₋₄ alkyl radical as defined above.

As used herein, the term "-hydroxyC₁₋₆alkyl" refers to a C₁₋₆alkyl radical as defined above, wherein one of the hydrogen atoms of the C₁₋₆alkyl radical is replaced by OH. Examples of hydroxyC₁₋₆alkyl include, but are not limited to, ethan-1-olyl, 2-methylpropan-1-olyl, hydroxymethyl, 2-hydroxy-ethyl, 2-hydroxy-propyl, 3-hydroxy-propyl and 5-hydroxy-pentyl.

As used herein, the term "-aminocarbonylC₀₋₆alkyl" refers to a radical of the formula -Rₐ-C(=O)-NH₂, wherein Rₐ is a single bond or a C₁₋₆alkyl radical as defined above.

As used herein, the term "-C₁₋₄alkylaminocarbonylC₀₋₆alkyl" refers to a radical of the formula -Rₐ₁-C(=O)-NH-Rₐ₂, where Rₐ₁ is a single bond or a C₁₋₆ alkyl radical as defined above; and Rₐ₂ is a C₁₋₆alkyl radical as defined above.

As used herein, the term "-diC₁₋₄alkylaminocarbonylC₀₋₆alkyl" refers to a radical of the formula -Rₐ₁-C(=O)-N(Rₐ₂)-Rₐ₂ where Rₐ₁ is a single bond or a C₁₋₆alkyl radical as defined above; and each Rₐ₂ is a C₁₋₄alkyl radical as defined above, and may be the same or different.

As used herein, the term "-C₃₋₁₀ monocyclic or bicyclic cycloalkylC₀₋₆alkyl" refers to a stable monocyclic or bicyclic saturated hydrocarbon radical consisting solely of carbon and hydrogen atoms, having from three to ten carbon atoms, and which is attached to the rest of the molecule by a single bond (i.e., C₃₋₈cycloalkyl) or by a C₁₋₆alkyl radical as defined above. The terms "C₃₋₇cycloalkyl" and "C₃₋₆cycloalkyl" are to be construed accordingly. Examples include, for example, cyclopropyl, cyclopropyl-methyl, cyclobutyl, cyclobutyl-ethyl, cyclopentyl, cyclopentyl-propyl, cyclohexyl, cyclohepty and cyclooctyl.

As used herein, the term "-C₃₋₈cycloalkoxyC₁₋₆alkyl" refers to a radical of the formula -Rₐ-OR_{b} wherein Rₐ is independently a C₁₋₆alkyl radical as defined above and R_{b} is a C₃₋₈cycloalkyl as defined above. Examples of C₃₋₈cycloalkoxyC₁₋₆alkyl include but are not limited to cyclopropoxymethyl and cyclobutoxymethyl.

"Halo" refers to bromo, chloro, fluoro or iodo.

As used herein, the term "-haloC₁₋₆alkyl" refers to a C₁₋₆alkyl radical, as defined above, substituted by one or more halo radicals, as defined above. Examples of haloC₁₋₆alkyl include, but are not limited to, trifluoromethyl, difluoromethyl, fluoromethyl, trichloromethyl, 2,2,2-trifluoroethyl, 1,3-dibromopropan-2-yl, 3-bromo-2-fluoropropyl and 1,4,4-trifluorobutan-2-yl. The term "haloC₁₋₄alkyl" is to be construed accordingly.

As used herein, the term "-cyanoC₁₋₆ alkyl" refers to a radical of the formula -Rₐ-CN, where Rₐ is a C₁₋₄alkyl radical as defined above.

As used herein, the term "-haloC₂₋₆alkenyl" refers to a C₂₋₆alkenyl radical, as defined above, substituted by one or more halo radicals, as defined above.

As used herein, the term "heterocyclyl" or "heterocyclic" refers to a stable 4-7 membered non-aromatic monocyclic ring radical comprising 1, 2, or 3, heteroatoms individually selected from nitrogen, oxygen and sulfur. The heterocyclyl radical may be bonded via a carbon atom or heteroatom. The term "5-6 membered heterocyclyl" is to be construed accordingly. Examples of heterocyclyl include, but are not limited to, azetidinyl, oxetanyl, pyrrolinyl, pyrrolidyl, tetrahydrofuryl, tetrahydrothienyl, piperidyl, piperazinyl, tetrahydropyranyl or morpholinyl or perhydroazepinyl.

As used herein, the term "heterocyclylC₀₋₆alkyl" refers to a heterocyclic ring as defined above which is attached to the rest of the molecule by a single bond or by a C₁₋₆alkyl radical as defined above.

As used herein, the term "heteroaryl" refers to a 5-9 membered aromatic monocyclic or fused ring radical comprising 1, 2, 3 or 4 heteroatoms individually selected from nitrogen, oxygen and sulfur. The heteroaryl radical may be bonded via a carbon atom or heteroatom. The term "5-6 membered heteroaryl" is to be construed accordingly. Examples of 5-6 membered monocyclic heteroaryls include, but are not limited to, furyl, pyrrolyl, thienyl, pyrazolyl, imidazolyl, thiazolyl, isothiazolyl, oxazolyl, isoxazolyl, triazolyl, tetrazolyl, pyrazinyl, pyridazinyl, pyrimidyl or pyridyl. Examples of fused heteroaryls include but are not limited to 9-membered heteroaryls such as benzofuranyl; 2,3-dihydrobenzofuranyl, 1,3-dihydroisobenzofuranyl; benzo[d][1,3]dioxol-5-yl; imidazo[1,2-a]pyridinyl; pyrazolo[1,5-a]pyridineyl; 1H-indazolyl and 1H-benzo[d]-imidazolyl.

As used herein, the term "heteroarylC₀₋₆alkyl" refers to a heteroaryl ring as defined above which is attached to the rest of the molecule by a single bond or by a C₁₋₆alkyl radical as defined above.

As used herein, the term "heteroaryl(haloC₁₋₄alkyl)" refers to a heteroaryl ring as defined above which is attached to the rest of the molecule by a haloC₁₋₄alkyl as defined above. An illustrative example of an heteroaryl(haloC₁₋₄alkyl) is fluoro(pyridin-2-yl)methyl.

As used herein, the term "IC₅₀" refers to the molar concentration of an inhibitor or modulator that produces 50% inhibition.

As used herein, the term "protected derivatives" refers to derivatives of inhibitors in which a reactive site or sites are blocked with protecting groups. Protected derivatives are useful in the preparation of inhibitors or in themselves may be active as inhibitors. Examples of protected group includes, but are not limited to, acetyl, tetrahydropyran, methoxymethyl ether, β-methoxyethoxymethyl ether, *ρ*-methoxybenzyl, methylthiomethyl ether, pivaloyl, silyl ether, carbobenzyloxy, benzyl, *tert*-butoxycarbonyl, *ρ*-methoxyphenyl, 9-fluorenylmethyloxycarbonyl, acetals, ketals, acylals, dithianes, methylesters, benzyl esters, tert-butyl esters, and silyl esters. A comprehensive list of suitable protecting groups can be found in T.W. Greene, Protecting Groups in Organic Synthesis, 3rd edition, John Wiley & Sons, Inc. 1999.

As used herein, the term "subject" refers to mammals, primates (*e*.*g*., humans, male or female), dogs, rabbits, guinea pigs, pigs, rats and mice. In certain embodiments, the subject is a primate. In yet other embodiments, the subject is a human.

As used herein, the term "inhibit", "inhibition" or "inhibiting" refers to the reduction or suppression of a given condition, symptom, or disorder, or disease, or a significant decrease in the baseline activity of a biological activity or process.

As used herein, the term "treat", "treating" or "treatment" of any disease or disorder refers to alleviating or ameliorating the disease or disorder (i.e., slowing or arresting the development of the disease or at least one of the clinical symptoms thereof); or alleviating or ameliorating at least one physical parameter or biomarker associated with the disease or disorder, including those which may not be discernible to the patient.

As used herein, the term "prevent", "preventing" or "prevention" of any disease or disorder refers to the prophylactic treatment of the disease or disorder; or delaying the onset or progression of the disease or disorder

As used herein, a subject is "in need of" a treatment if such subject would benefit biologically, medically or in quality of life from such treatment.

As used herein, the term "a therapeutically effective amount" of a compound of the present invention refers to an amount of the compound of the present invention that will elicit the biological or medical response of a subject, for example, reduction or inhibition of an enzyme or a protein activity, or ameliorate symptoms, alleviate conditions, slow or delay disease progression, or prevent a disease, etc.

As used herein, the term "anti-cancer agent" or antineoplastic agent, refers to a therapeutic agent that is useful for treating or controlling the growth of cancerous cells.

As used herein, thet term "anti-inflammatory agent" refers to a therapeutic agent that reduces inflammation (redness, swelling and/or pain) in the body. Anti-inflammatory agents block certain substances in the body that cause inflammation.

As used herein, the term "pharmaceutical composition" refers to a compound of the invention, or a pharmaceutically acceptable salt thereof, together with at least one pharmaceutically acceptable carrier, in a form suitable for oral or parenteral administration.

As used herein, the term "pharmaceutically acceptable carrier" refers to a substance useful in the preparation or use of a pharmaceutical composition and includes, for example, suitable diluents, solvents, dispersion media, surfactants, antioxidants, preservatives, isotonic agents, buffering agents, emulsifiers, absorption delaying agents, salts, drug stabilizers, binders, excipients, disintegration agents, lubricants, wetting agents, sweetening agents, flavoring agents, dyes, and combinations thereof, as would be known to those skilled in the art (see, for example, Remington The Science and Practice of Pharmacy, 22nd Ed. Pharmaceutical Press, 2013, pp. 1049-1070).

### Description of Preferred Embodiments

The present invention provides novel compounds that inhibit MLL1; and compositions for treating or preventing a condition mediated by ML1L.

Various enumerated embodiments of the invention are described herein. Features specified in each embodiment may be combined with other specified features to provide further embodiments of the present invention.

Embodiment 1. A compound of Formula (I), or an enantiomer, an enantiomeric mixture, or a pharmaceutically acceptable salt thereof; as described above.

Embodiment 2. A compound according to Embodiment 1, wherein said compound is a compound of Formula (II) 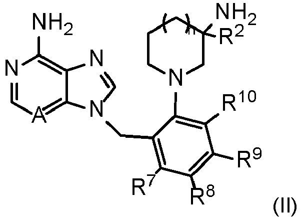 or an enantiomer, an enantiomeric mixture, or a pharmaceutically acceptable salt thereof; wherein:
R² is selected from the group consisting of:
   (i) -hydroxyC₁₋₆ alkyl, -C₁₋₆alkoxyC₁₋₆alkyl or -(CR^{a}R^{b})ₚ-C(=O)-OR^{10a};
   (ii) -(CR^{a}R^{b})ᵣ-C(=O)-NR¹²R¹³ or-(CR^{a}R^{b})₁₋₄-O-(CR^{a}R^{b})₁₋₄-C(=O)-NR¹²R¹³; wherein
      R¹² is hydrogen or -C₁₋₆ alkyl;
      R¹³ is hydrogen, -C₁₋₆ alkyl, -cyanoC₁₋₆ alkyl; -C₁₋₄ alkylSO₂R^{10b} wherein R^{10b} is -C₁₋₆ alkyl or phenyl; C₃₋₁₀ monocylic or bicyclic cycloalkylC₀₋₆alkyl, phenyl, 5-10 membered monocyclic or bicyclic heterocyclic ring or 5-9 membered heteroarylC₀₋₆alkyl; wherein said 5-10 membered monocyclic or bicyclic heterocyclic ring or 5-9 membered heteroaryl radical independently comprises 1-4 heteroatoms selected from nitrogen, oxygen and sulfur; and
         said C₃₋₁₀ monocylic or bicyclic cycloalkyl, phenyl, 5-10 membered monocyclic or bicyclic heterocyclic ring or 5-9 membered heteroaryl radical are independently unsubstituted or substituted with 1-2 -C₁₋₄ alkyl, -hydroxyC₁₋₆ alkyl, -C₁₋₄ alkoxy, halo, hydroxyl, phenyl or -S(C₁₋₄ alkyl); or
      R¹² and R¹³ together form a 5-10 membered monocyclic or bicyclic heterocyclic ring comprising 1-4 heteroatoms selected from nitrogen, oxygen and sulfur; and is unsubstituted or substituted with -C₁₋₄ alkyl, hydroxyl, cyano, -cyanoC₁₋₆ alkyl, -SO₂ or -C₂₋₄alkenylcarbonyl; and
   (iii) phenyl or 5-9 membered heteroarylC₀₋₆alkyl; wherein said heteroaryl radical is unsubstituted or substituted by -C₁₋₄ alkyl, -haloC₁₋₄ alkyl, amino, -C₁₋₄alkylamino or -C₁₋₄ dialkylamino; and said heteroaryl radical comprises 1-4 heteroatoms selected from nitrogen, oxygen and sulfur;
      R⁷, R⁸, R⁹ and R¹⁰ are independently hydrogen, halo, -C₁₋₄ alkyl, -haloC₁₋₆ alkyl, -hydroxyC₁₋₆ alkyl, cyano, -cyanoC₁₋₆ alkyl, -C₂₋₆ alkenyl, -C₂₋₆ alkynyl, -C₁₋₆ alkylthio, -(CR^{a}R^{b})₁₋₄-NR¹⁷R¹⁸, -(CR^{a}R^{b})₁₋₄NR¹⁷-C(O)-OR¹⁸, -(CR^{a}R^{b})₁₋₄-OR¹⁹, -C₃₋₈ cycloalkyl, phenyl or 5-6 membered heteroaryl comprising 1-4 heteroatoms selected from nitrogen, oxygen and sulfur; and
         wherein said C₃₋₈ cycloalkyl, phenyl or 5-6 membered heteroaryl is independently substituted with 1-2 R²⁰; and
         provided at least one of R⁷, R⁸, R⁹ and R¹⁰ is not hydrogen;
      alternatively, R⁷ and R⁸, R⁸ and R⁹, R⁹ and R¹⁰ together with the phenyl ring to which they are attached form a 9-10 membered benzo-fused carbocycle or benzo-fused heterocycle comprising 1-3 heteroatoms selected from nitrogen, oxygen and sulfur; wherein said benzo-fused carbocycle or benzo-fused heterocycle is independently unsubstituted or substituted with 1-2 halo or -C₁₋₄ alkyl;
      R^{a}, R^{b}, R^{10a} and R¹⁷ are independently hydrogen or -C₁₋₄ alkyl;
      R¹⁸ is hydrogen, -C₁₋₄ alkyl, -haloC₁₋₆ alkyl or -C₃₋₆ cycloalkyl;
      alternatively, R¹⁷ and R¹⁸ together with N in the -NR¹⁷R¹⁸ moiety form a 4-10 membered monocyclic or bicyclic heterocyclic ring comprising 1-3 heteroatoms selected from nitrogen, oxygen and sulfur; wherein said heterocyclic ring is unsubstituted or substituted with 1-2 halo or -C₁₋₄ alkyl;
      R¹⁹ is hydrogen, -C₁₋₄ alkyl, -haloC₁₋₆ alkyl, -C₃₋₆ cycloalkyl, phenyl, 5-6 membered heterocyclyl or 5-6 membered heteroaryl; wherein said 5-6 membered heterocyclyl or 5-6 membered heteroaryl independently comprises 1-3 heteroatoms selected from nitrogen, oxygen and sulfur;
      R²⁰ is -C₁₋₄ alkyl, halo or -C₃₋₆ cycloalkyl; or two R²⁰ together form a 5-6 membered ring comprising 0-2 heteroatoms selected from nitrogen, oxygen and sulfur;
      n is 0 or 1; and
      p, q, r and s
      are independently 0, 1, 2, 3 or 4.

Embodiment 3. A compound according to Embodiment 1 or 2, wherein said compound is a compound of Formula (III): 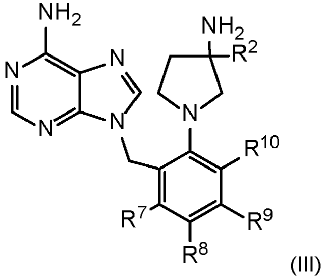 or an enantiomer, an enantiomeric mixture, or a pharmaceutically acceptable salt thereof.

Embodiment 4. A compound according to Embodiment 3, wherein said compound is a compound of Formula (IV): 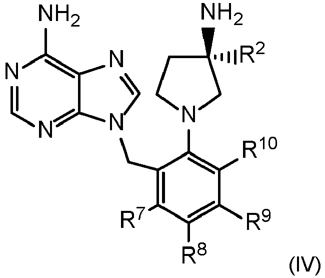 or an enantiomer, an enantiomeric mixture, or a pharmaceutically acceptable salt thereof.

Embodiment 5. A compound according to any one of Embodiments 1-4, or an enantiomer, an enantiomeric mixture, or a pharmaceutically acceptable salt thereof; wherein R² is

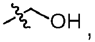

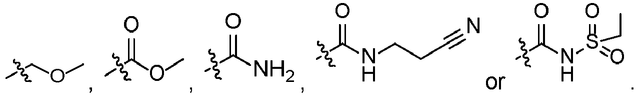

Embodiment 6. A compound according to any one of Embodiments 1-4, or an enantiomer, an enantiomeric mixture, or a pharmaceutically acceptable salt thereof; wherein R² is,

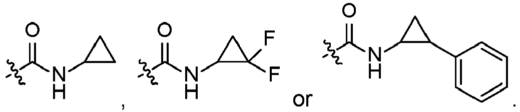

Embodiment 7. A compound according to any one of Embodiments 1-4, or an enantiomer, an enantiomeric mixture, or a pharmaceutically acceptable salt thereof; wherein R² is

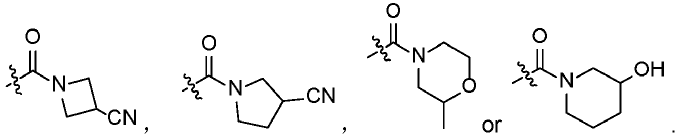

Embodiment 8. A compound according to any one of Embodiments 1-4, or an enantiomer, an enantiomeric mixture, or a pharmaceutically acceptable salt thereof; wherein R² is 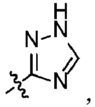 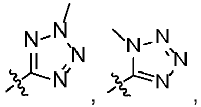 1H-tetrazolyl, 2H-tetrazoly, pyridyl, trifluoromethylpyridyl or phenyl.

Embodiment 9. A compound according to any one of Embodiments 1-4, or an enantiomer, an enantiomeric mixture, or a pharmaceutically acceptable salt thereof; wherein said compound is a compound of Formula (V):

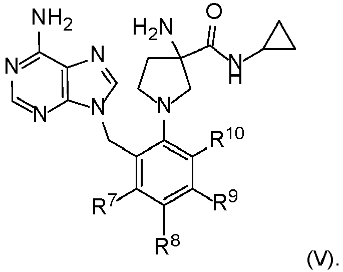

Embodiment 10. A compound according to any one of the above Embodiments or a pharmaceutically acceptable salt thereof; wherein at least two of R⁷, R⁸, R⁹ and R¹⁰ are not hydrogen.

Embodiment 11. A compound according to Embodiment 10, or a pharmaceutically acceptable salt thereof; wherein R⁷, R⁸, R⁹ and R¹⁰ are independently hydrogen, halo, -C₁₋₄alkyl, -haloC₁₋₆ alkyl, -hydroxyC₁₋₆ alkyl, -C₂₋₆ alkenyl, -C₃₋₈ cycloalkyl, -(CR^{a}R^{b})₁₋₄-NR¹⁷R¹⁸, or - (CR^{a}R^{b})₁₋₄-OR¹⁹; provided at least two of R⁷, R⁸, R⁹ and R¹⁰ are not hydrogen;
R^{a}, R^{b} and R¹⁷ are independently hydrogen or -C₁₋₄ alkyl;
R¹⁸ is hydrogen, -C₁₋₄ alkyl, -haloC₁₋₆ alkyl or -C₃₋₆ cycloalkyl;
alternatively, R¹⁷ and R¹⁸ together with N in the -NR¹⁷R¹⁸ moiety form a 4-10 membered monocyclic or bicyclic heterocyclic ring comprising 1-3 heteroatoms selected from nitrogen, oxygen and sulfur; wherein said heterocyclic ring is unsubstituted or substituted with 1-2 halo or -C₁₋₄ alkyl; and
R¹⁹ is hydrogen, -C₁₋₄ alkyl, -haloC₁₋₆ alkyl, -C₃₋₆ cycloalkyl, phenyl, 5-6 membered heterocyclyl or 5-6 membered heteroaryl; wherein said 5-6 membered heterocyclyl or 5-6 membered heteroaryl independently comprises 1-3 heteroatoms selected from nitrogen, oxygen and sulfur.

Embodiment 12. The compound according to Embodiment 1, wherein said compound is selected from the group consisting of:
3-amino-1-(2-((6-amino-9H-purin-9-yl)methyl)-3-bromo-5-chlorophenyl)-N-cyclopropylpyrrolidine-3-carboxamide;
3-amino-1-(2-((6-amino-9H-purin-9-yl)methyl)-5-chloro-3-ethylphenyl)-N-cyclopropylpyrrolidine-3-carboxamide;
3-amino-1-(2-((6-amino-9H-purin-9-yl)methyl)-4-fluoro-3-(trifluoromethyl)phenyl)-N-cyclopropylpyrrolidine-3-carboxamide; and
3-amino-1-(2-((6-amino-9H-purin-9-yl)methyl)-3-bromo-4-(trifluoromethyl)phenyl)-N-(2-cyanoethyl)pyrrolidine-3-carboxamide;
or a pharmaceutically acceptable salt thereof.

Embodiment 13. The compound according to any one of Embodiments 1-12, wherein said compound is in the (R) configuration, (S) configuration, or a mixture thereof.

Embodiment 14. A compound according to Embodiment 1, wherein said compound is a compound from Table 2; or an enantiomer, an enantiomeric mixture, or a pharmaceutically acceptable salt thereof.

Embodiment 15. The compound according to Embodiment 1, wherein said compound is (R)-3-amino-1-(2-((6-amino-9H-purin-9-yl)methyl)-3-bromo-5-chlorophenyl)-N-cyclopropylpyrrolidine-3-carboxamide.

Embodiment 16. The compound according to Embodiment 1, wherein said compound is (R)-3-amino-1-(2-((6-amino-9H-purin-9-yl)methyl)-5-chloro-3-ethylphenyl)-N-cyclopropylpyrrolidine-3-carboxamide.

Embodiment 17. The compound according to Embodiment 1, wherein said compound is (R)-3-amino-1-(2-((6-amino-9H-purin-9-yl)methyl)-4-fluoro-3-(trifluoromethyl)phenyl)-N-cyclopropylpyrrolidine-3-carboxamide.

Embodiment 18. The compound according to Embodiment 1, wherein said compound is (R)-3-amino-1-(2-((6-amino-9*H*-purin-9-yl)methyl)-3-bromo-4-(trifluoromethyl)phenyl)-*N*-(2-cyanoethyl)pyrrolidine-3-carboxamide.

Embodiment 19. A pharmaceutical composition comprising a compound according to any one of Embodiments 1-18 and one or more pharmaceutically acceptable carrier.

Embodiment 20. A combination comprising a compound according to any one of Embodiments 1-18 and one or more additional therapeutically active agent.

Embodiment 21. The combination according to Embodiment 20, wherein said one or more additional therapeutically active agent is an anti-cancer agent, an analgesic, an anti-inflammatory agent, or a combination thereof.

Embodiment 22. A compound according to any one of Embodiments 1-18 and optionally in combination with a second therapeutic agent, for use in treating a disease or condition mediated by mixed lineage leukemia 1 (MLL1).

Embodiment 23. The compound according to Embodiment 22, wherein said second therapeutic agent is an anti-cancer agent, an analgesic, an anti-inflammatory agent or a combination thereof.

Embodiment 24. Use of a compound according to any one of Embodiments 1-18 and optionally in combination with a second therapeutic agent, in the manufacture of a medicament for a disease or condition mediated by MLL1.

Embodiment 25. A compound according to any one of Embodiments 1-18 or a pharmaceutically acceptable salt thereof, for use in a method for treating a disease or condition mediated by mixed lineage leukemia 1 (MLL1).

Embodiment 26. A compound according to any one of Embodiments 1-18 or a pharmaceutically acceptable salt thereof, for use in a method for treating a disease or condition that benefit from or is treatable by inhibition of mixed lineage leukemia 1 (MLL1).

Embodiment 27. The use according to Embodiment 24, or the compound for use according to Embodiment 25 or 26, wherein said disease or condition mediated by MLL1, or said disease or condition that benefit from or is treatable by inhibition of MLL1, is breast cancer, cervical cancer, skin cancer, ovarian cancer, gastric cancer, prostate cancer, pancreatic cancer, lung cancer, hepatocellular carcinoma, head and neck cancer, peripheral nerve sheath tumor, osteosarcoma, myeloma, neuroblastoma, leukemia, lymphoma, or pulmonary arterial hypertension.

Embodiment 28. The use according to Embodiment 27, or the compound for use according to Embodiment 27, wherein said disease or condition mediated by MLL1, or said disease or condition that benefit from or is treatable by inhibition of MLL1, is leukemia.

Embodiment 29. The use according to Embodiment 28, or the compound for use according to Embodiment 28, wherein said leukemia is acute lymphoblastic leukemia (ALL), acute myeloid leukemia (AML), chronic myeloid leukemia (CML) or chronic myelomonocytic leukemia (CMML).

Embodiment 30. The use according to Embodiment 27, or the compound for use according to Embodiment 27, wherein said disease or condition mediated by MLL1, or said disease or condition that benefit from or is treatable by inhibition of MLL1, is breast cancer.

Embodiment 31. The use according to Embodiment 27, or the compound for use according to Embodiment 27, wherein said disease or condition mediated by MLL1, or said disease or condition that benefit from or is treatable by inhibition of MLL1, is lung cancer.

Embodiment 32. The use according to Embodiment 31, or the compound for use according to Embodiment 31, wherein said lung cancer is small cell or non-small cell lung cancer.

Embodiment 33. The use according to Embodiment 27, or the compound for use according to Embodiment 27, wherein said disease or condition mediated by MLL1, or said disease or condition that benefit from or is treatable by inhibition of MLL1, is skin cancer.

Embodiment 34. The use according to Embodiment 33, or the compound for use according to Embodiment 33, wherein said skin cancer is melanoma, basal cell carcinoma or squamous cell carcinoma.

Embodiment 35. The use according to Embodiment 27, or the compound for use according to Embodiment 27, wherein said disease or condition mediated by MLL1, or said disease or condition that benefit from or is treatable by inhibition of MLL1, is lymphoma.

Embodiment 36. The use according to Embodiment 35, or the compound for use according to Embodiment 35, wherein said lymphoma is Hodgkin's lymphoma or non-Hodgkin's lymphoma.

Embodiment 37. The use according to Embodiment 35, or the compound for use according to Embodiment 35, wherein said lymphoma is mantle cell lymphoma or diffuse large B cell lymphoma.

Embodiment 38. The use according to Embodiment 27, or the compound for use according to Embodiment 27, wherein said disease or condition mediated by MLL1, or said disease or condition that benefit from or is treatable by inhibition of MLL1, is multiple myeloma.

Embodiment 39. A compound for use according to any one of Embodiment 25-38, wherein said compound is administered orally.

Unless specified otherwise, the term "compounds of the present invention" or "compound of the present invention" refers to compounds of Formula (I) subformulae thereof, and exemplified compounds, and salts thereof, as well as all stereoisomers (including diastereoisomers and enantiomers), rotamers, tautomers and isotopically labeled compounds (including deuterium substitutions), as well as inherently formed moieties.

Depending on the choice of the starting materials and procedures, the compounds can be present in the form of one of the possible stereoisomers or as mixtures thereof, for example as pure optical isomers, or as stereoisomer mixtures, such as racemates and diastereoisomer mixtures, depending on the number of asymmetric carbon atoms. The present invention is meant to include all such possible stereoisomers, including racemic mixtures, diasteriomeric mixtures and optically pure forms. Optically active (*R*)- and (*S*)- stereoisomers may be prepared using chiral synthons or chiral reagents, or resolved using conventional techniques. Substituents at atoms with unsaturated double bonds may, if possible, be present in *cis-* (*Z*)- or *trans-* (*E*)*-* form. If the compound contains a disubstituted cycloalkyl, the cycloalkyl substituent may have a cis- or trans-configuration.

Any asymmetric atom (e.g., carbon or the like) of the compound(s) of the present invention can be present in racemic or enantiomerically enriched, for example the (*R*)-, (*S*)- or (*R*,*S*)-configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (*R*)- or (*S*)- configuration.

Accordingly, as used herein a compound of the present invention can be in the form of one of the possible stereoisomers, rotamers, atropisomers, tautomers or mixtures thereof, for example, as substantially pure geometric (*cis* or *trans*) stereoisomers, diastereomers, optical isomers (antipodes), racemates or mixtures thereof.

Any resulting mixtures of stereoisomers can be separated on the basis of the physicochemical differences of the constituents, into the pure or substantially pure geometric or optical isomers, diastereomers, racemates, for example, by chromatography and/or fractional crystallization.

Any resulting racemates of compounds of the present invention or of intermediates can be resolved into the optical antipodes by known methods, *e*.*g*., by separation of the diastereomeric salts thereof, obtained with an optically active acid or base, and liberating the optically active acidic or basic compound. In particular, a basic moiety may thus be employed to resolve the compounds of the present invention into their optical antipodes, *e*.*g*., by fractional crystallization of a salt formed with an optically active acid, *e*.*g*., tartaric acid, dibenzoyl tartaric acid, diacetyl tartaric acid, di-*O*,*O*'-*p*-toluoyl tartaric acid, mandelic acid, malic acid or camphor-10-sulfonic acid. Racemic compounds of the present invention or racemic intermediates can also be resolved by chiral chromatography, *e*.*g*., high pressure liquid chromatography (HPLC) using a chiral adsorbent.

Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Isotopes that can be incorporated into compounds of the invention include, for example, isotopes of hydrogen.

Further, incorporation of certain isotopes, particularly deuterium (i.e., ²H or D) may afford certain therapeutic advantages resulting from greater metabolic stability, for example increased in vivo half-life or reduced dosage requirements or an improvement in therapeutic index or tolerability. It is understood that deuterium in this context is regarded as a substituent of a compound of Formula (I) or sub-formulae thereof. The concentration of deuterium, may be defined by the isotopic enrichment factor. The term "isotopic enrichment factor" as used herein means the ratio between the isotopic abundance and the natural abundance of a specified isotope. If a substituent in a compound of this invention is denoted as being deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation). It should be understood that the term "isotopic enrichment factor" can be applied to any isotope in the same manner as described for deuterium.

Other examples of isotopes that can be incorporated into compounds of the invention include isotopes of hydrogen, carbon, nitrogen, oxygen, phosphorous, fluorine, and chlorine, such as ³H, ¹¹C, ¹³C, ¹⁴C, ¹⁵N, ¹⁸F ³¹P, ³²P, ³⁵S, ³⁶Cl, ¹²³I, ¹²⁴I, ¹²⁵I respectively. Accordingly it should be understood that the invention includes compounds that incorporate one or more of any of the aforementioned isotopes, including for example, radioactive isotopes, such as ³H and ¹⁴C, or those into which non-radioactive isotopes, such as ²H and ¹³C are present. Such isotopically labelled compounds are useful in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. In particular, an ¹⁸F or labeled compound may be particularly desirable for PET or SPECT studies. Isotopically-labeled compounds of Formula (I) or sub-formulae thereof can generally be prepared by conventional techniques known to those skilled in the art or by processes analogous to those described in the accompanying examples using an appropriate isotopically-labeled reagents in place of the non-labeled reagent previously employed.

The compounds of the present invention are either obtained in the free form, as a salt thereof. As used herein, the terms "salt" or "salts" refers to an acid addition or base addition salt of a compound of the invention. "Salts" include in particular "pharmaceutical acceptable salts". The term "pharmaceutically acceptable salts" refers to salts that retain the biological effectiveness and properties of the compounds of this invention and, which typically are not biologically or otherwise undesirable. In many cases, the compounds of the present invention are capable of forming acid and/or base salts by virtue of the presence of amino and/or carboxyl groups or groups similar thereto.

Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like.

Pharmaceutically acceptable base addition salts can be formed with inorganic and organic bases. Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

In another aspect, the present invention provides compounds of the present invention in acetate, ascorbate, adipate, aspartate, benzoate, besylate, bromide/hydrobromide, bicarbonate/carbonate, bisulfate/sulfate, camphorsulfonate, caprate, chloride/hydrochloride, chlorotheophyllinate, citrate, ethanedisulfonate, fumarate, gluceptate, gluconate, glucuronate, glutamate, glutarate, glycolate, hippurate, hydroiodide/iodide, isethionate, lactate, lactobionate, laurylsulfate, malate, maleate, malonate, mandelate, mesylate, methylsulphate, mucate, naphthoate, napsylate, nicotinate, nitrate, octadecanoate, oleate, oxalate, palmitate, pamoate, phosphate/hydrogen phosphate/dihydrogen phosphate, polygalacturonate, propionate, sebacate, stearate, succinate, sulfosalicylate, sulfate, tartrate, tosylate trifenatate, trifluoroacetate or xinafoate salt form.

### Processes for Making Compounds of the Invention

All methods described herein can be performed in any suitable order, unless otherwise indicated or otherwise clearly contradicted by context.

Compounds of Formula (I) can be prepared from the correpsonding benzaldehye intermediate (INT-2A) as generally illustrated in Scheme 1 and in the Examples.

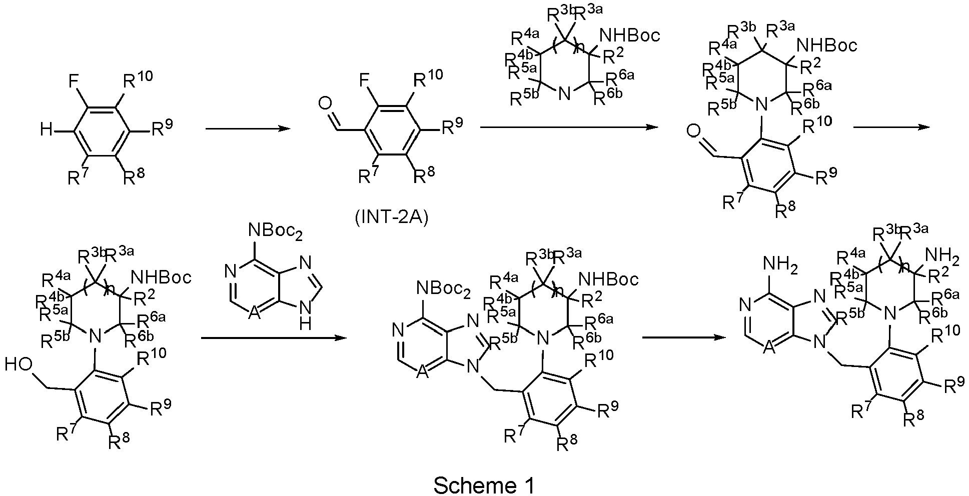

The invention further includes any variant of the present processes; for example, wherein an intermediate product obtainable at any stage thereof is used as starting material and the remaining steps are carried out; wherein starting materials are formed *in situ* under the reaction conditions; or wherein the reaction components are used in the form of their salts or optically pure material. Compounds of the invention and intermediates can also be converted into each other according to methods generally known to those skilled in the art.

### Pharmacology and Utility

In one aspect, the invention provides compounds of Formula (I) or subformulae thereof, or a pharmaceutically acceptable salt thereof, that are useful for therapy; particularly, for treating or preventing a disease or condition that is mediated by MLL1.

In another aspect, the invention provides the use of a compound of Formula (I) or subformulae thereof, or a pharmaceutically acceptable salt thereof, for treating a disease or condition that benefit from or is treatable by inhibition of MLL1; and for the manufacture of a medicament for treating a disease or condition that is treatable by inhibition of MLL1.

Examples of diseases or conditions that are mediated by MLL,1 or that benefit from or are treatable by inhibition of MLL1, include but is not limited to breast cancer, cervical cancer, skin cancer (particularly skin squamous cell carcinoma), ovarian cancer, gastric cancer, prostate cancer, pancreatic cancer, lung cancer, hepatocellular carcinoma, head and neck cancer, peripheral nerve sheath tumor, osteosarcoma, multiple myeloma, neuroblastoma, leukemia (particularly acute lymphoblastic leukemia), non-Hodgkin's lymphoma (particularly mantle cell lymphoma), and pulmonary arterial hypertension.

### Pharmaceutical Compositions, Dosage and Administration

In another aspect, the present invention provides a pharmaceutical composition comprising a compound of the present invention, or a pharmaceutically acceptable salt thereof, and a pharmaceutically acceptable carrier.

In a further embodiment, the composition comprises at least two pharmaceutically acceptable carriers, such as those described herein. The pharmaceutical composition can be formulated for particular routes of administration such as oral administration, parenteral administration (e.g. by injection, infusion, transdermal or topical administration), and rectal administration. Topical administration may also pertain to inhalation or intranasal application. The pharmaceutical compositions of the present invention can be made up in a solid form (including, without limitation, capsules, tablets, pills, granules, powders or suppositories), or in a liquid form (including, without limitation, solutions, suspensions or emulsions). Tablets may be either film coated or enteric coated according to methods known in the art. Typically, the pharmaceutical compositions are tablets or gelatin capsules comprising the active ingredient together with one or more of:
a) diluents, *e*.*g*., lactose, dextrose, sucrose, mannitol, sorbitol, cellulose and/or glycine;
b) lubricants, *e*.*g*., silica, talcum, stearic acid, its magnesium or calcium salt and/or polyethyleneglycol; for tablets also
c) binders, *e*.*g*., magnesium aluminum silicate, starch paste, gelatin, tragacanth, methylcellulose, sodium carboxymethylcellulose and/or polyvinylpyrrolidone; if desired
d) disintegrants, *e*.*g*., starches, agar, alginic acid or its sodium salt, or effervescent mixtures; and
e) absorbents, colorants, flavors and sweeteners.

In another aspect, the compounds of the present invention are combined with other therapeutic agents, such as other anti-cancer agents, anti-allergic agents, anti-nausea agents (or anti-emetics), pain relievers, cytoprotective agents, and combinations thereof.

In one embodiment, the other therapeutic agent is an anti-cancer agent or chemotherapeutic agent. Examples of anit-cancer agents considered for use in combination therapies of the invention include but are not limited erlotinib, bortezomib, fulvestrant, sunitib imatinib mesylate, letrozole, finasunate, platins such as oxaliplatin, carboplatin, and cisplatin, finasunate, fluorouracil, rapamycin, leucovorin, lapatinib, lonafamib, sorafenib, gefitinib,capmtothecin, topotecan, bryostatin, adezelesin, anthracyclin, carzelesin, bizelesin, dolastatin, auristatins, duocarmycin, eleutherobin, taxols such as paclitaxel or docetaxel, cyclophasphamide, doxorubicin, vincristine, prednisone or prednisolone, other alkylating agents such as mechlorethamine, chlorambucil, and ifosfamide, antimetabolites such as azathioprine or mercaptopurine, other microtubule inhibitors (vinca alkaloids like vincristine, vinblastine, vinorelbine and vindesine, as well as taxanes), podophyllotoxins (etoposide, teniposide, etoposide phosphate, and epipodophyllotoxins), topoisomerase inhibitors, other cytotoxins such as actinomycin, daunorubicin, valrubicin, idarubicin, edrecolomab, epirubicin, bleomycin, plicamycin, mitomycin, as well as other anticancer antibodies (cetuximab, bevacizumab, ibritumomab, abagovomab, adecatumumab, afutuzumab, alacizumab, alemtuzumab, anatumomab, apolizumab, bavituximab, belimumab, bivatuzumab mertansine, blinatumomab, brentuximab vedotin, cantuzumab mertansine, catumazomab, cetuximab, citatuzumab bogatox, cixutumumab, clivatuzumab tetraxetan, conatumumab, dacetuzumab, daclizumab, detumomab, ecromeximab, edrecolomab, elotuzumab, epratuzumab, ertumaxomab, etaracizumab, farletuzumab, figitumumab, fresolimumab, galiximab, gembatumumab vedotin, gemtuzumab, ibritumomab tiuxetan, inotuzumab ozogamicin, intetumumab, ipilimumab, iratumumab, labetuzumab, lexatumumab, lintuzumab, lucatumumab, lumilisimab, mapatumumab, matuzumab, milatuzumab, mitumomab, nacolomab tafenatox, naptumomab estafenatox, necitumumab, nimotuzumab, ofatumumab, olaratumab, oportuzumab monatox, oregovomab, panitumumab, pemtumomab, pertuzumab, pintumomab, pritumumab, ramucirumab, rilotumumab, robatumumab, rituximab, sibrotuzumab, tacatuzumab tetraxetan, taplitumomab paptox, tenatumomab, ticilimumab, tigatuzumab, tositumomab or ¹³¹l-tositumomab, trastuzumab, tremelimumab, tuocotuzumab celmoleukin, veltuzumab, visilizumab, volocixumab, votumumab, zalutumumab, zanolimumab, IGN-101, MDX-010,ABX-EGR, EMD72000, ior-t1, MDX-220, MRA, H-11 scFv, huJ591, TriGem, TriAb, R3, MT-201, G-250, ACA-125, Onyvax-105, CD:-960,Cea-Vac, BrevaRexAR54, IMC-1C11, GlioMab-H, ING-1, anti-LCG MAbs, MT-103, KSB-303, Therex, KW2871, anti-HMI.24, Anti-PTHrP, 2C4 antibody, SGN-30, TRAIL-RI MAb, Prostate Cancer antibody, H22xKi-r, ABX-Mai, Imuteran, Monopharm-C), and antibody-drug conjugates comprising any of the above agents (especially auristatins MMAE and MMAF, maytansinoids like DM-1, calicheamycins, or various cytotoxins).

In another embodiment, the compounds of the invention are combined with another therapeutic agent selected from anastrozole (ARIMIDEX^{®}), bicalutamide (CASODEX^{®}), bleomycin sulfate (BLENOXANE^{®}), busulfan (MYLERAN^{®}), busulfan injection (BUSULFEX^{®}), capecitabine (XELODA^{®}), N4-pentoxycarbonyl-5-deoxy-5-fluorocytidine, carboplatin (PARAPLATIN^{®}), carmustine (BiCNU^{®}), chlorambucil (LEUKERAN^{®}), cisplatin (PLATINOL^{®}), cladribine (LEUSTATIN^{®}), cyclophosphamide (CYTOXAN^{®} or NEOSAR^{®}), cytarabine, cytosine arabinoside (CYTOSAR-U^{®}), cytarabine liposome injection (DEPOCYT^{®}), dacarbazine (DTIC-Dome^{®}), dactinomycin (actinomycin D, COSMEGAN^{®}), daunorubicin hydrochloride (CERUBIDINE^{®}), daunorubicin citrate liposome injection (DAUNOXOME^{®}), dexamethasone, docetaxel (TAXOTERE^{®}), doxorubicin hydrochloride (ADRIAMYCIN^{®}, RUBEX^{®}), etoposide (VEPESID^{®}), fludarabine phosphate (FLUDARA^{®}), 5-fluorouracil (ADRUCIL^{®}, EFUDEX^{®}), flutamide (EULEXIN^{®}), tezacitibine, gemcitabine (difluorodeoxycitidine), hydroxyurea (HYDREA^{®}), idarubicin (IDAMYCIN^{®}), ifosfamide (IFEX^{®}), irinotecan (CAMPTOSAR^{®}), L-asparaginase (ELSPAR^{®}), leucovorin calcium, melphalan (ALKERAN^{®}), 6-mercaptopurine (PURINETHOL^{®}), methotrexate (FOLEX^{®}), mitoxantrone (NOVANTRONE^{®}), gemtuzumab ozogamicin (MYLOTARG^{™}), paclitaxel (TAXOL^{®}), nab-paclitaxel (ABRAXANE^{®}), phoenix (Yttrium90/MX-DTPA), pentostatin, polifeprosan 20 with carmustine implant (GLIADEL^{®}), tamoxifen citrate (NOLVADEX^{®}), teniposide (VUMON^{®}), 6-thioguanine, thiotepa, tirapazamine (TIRAZONE^{®}), topotecan hydrochloride for injection (HYCAMPTIN^{®}), vinblastine (VELBAN^{®}), vincristine (ONCOVIN^{®}), and vinorelbine (NAVELBINE^{®}).

In another embodiment, the compounds of the present invention are combined with another therapeutic agent capable of inhibiting BRAF, MEK, CDK4/6, SHP-2, HDAC, EGFR, MET, mTOR, PI3K or AKT, or a combination thereof. In a particular embodiment, the compounds of the present invention are combined with another therapeutic agent selected from vemurafinib, debrafinib, LGX818, trametinib, MEK162, LEE011, PD-0332991, panobinostat, verinostat, romidepsin, cetuximab, gefitinib, erlotinib, lapatinib, panitumumab, vandetanib, INC280, everolimus, simolimus, BMK120, BYL719 or CLR457, or a combination thereof.

In another embodiment, the therapeutic agent for use with the compounds of the present invention is selected based on the disease or condition that is being treated. For example, in the treatment of melanoma, the other therapeutic agent may be selected from aldesleukin (e.g., PROLEUKIN^{®}), dabrafenib (e.g., TAFINLAR^{®}), dacarbazine, recombinant interferon alfa-2b (e.g., INTRON^{®} A), ipilimumab, trametinib (e.g., MEKINIST^{®}), peginterferon alfa-2b (e.g., PEGINTRON^{®}, SYLATRON^{™}), vemurafenib (e.g., ZELBORAF^{®})), and ipilimumab (e.g., YERVOY^{®}).

For the treatment of ovarian cancer, the other therapeutic agent may be selected from doxorubicin hydrochloride (Adriamycin^{®}), carboplatin (PARAPLATIN^{®}), cyclophosphamide (CYTOXAN^{®}, NEOSAR^{®}), cisplatin (PLATINOL^{®}, PLATINOL-AQ^{®}), doxorubicin hydrochloride liposome (DOXIL^{®}, DOX-SL^{®}, EVACET^{®}, LIPODOX^{®}), gemcitabine hydrochloride (GEMZAR^{®}), topotecan hydrochloride (HYCAMTINO), and paclitaxel (TAXOL^{®}).

For the treatment of thyroid cancer, the other therapeutic agent may be selected from doxorubicin hydrochloride (Adriamycin^{®}), cabozantinib-S-malate (COMETRIQO), and vandetanib (CAPRELSA^{®}).

For the treatment of colon cancer, the other therapeutic may be selected from fluorouracil (e.g., ADRUCIL^{®}, EFUDEX^{®}, FLUOROPLEX^{®}), bevacizumab (AVASTINO), irinotecan hydrochloride (CAMPTOSTAR^{®}), capecitabine (XELODA^{®}), cetuximab (ERBITUX^{®}), oxaliplatin (ELOXATIN^{®}), leucovorin calcium (WELLCOVORIN^{®}), regorafenib (STIVARGA^{®}), panitumumab (VECTIBIX^{®}), and ziv-aflibercept (ZALTRAP^{®}).

For the treatment of lung cancer, the other therapeutic may be selected from methotrexate, methotrexate LPF (e.g., FOLEX^{®}, FOLEX PFS^{®}, Abitrexate^{®}, MEXATE^{®}, MEXATE-AQ^{®}), paclitaxel (TAXOL^{®}), paclitaxel albumin-stabilized nanoparticle formulation (ABRAXANE^{®}), afatinib dimaleate (GILOTRIFO), pemetrexed disodium (ALIMTAO), bevacizumab (AVASTINO), carboplatin (PARAPLATIN^{®}), cisplatin (PLATINOL^{®}, PLATINOL-AQ^{®}), crizotinib (XALKORI^{®}), erlotinib hydrochloride (TARCEVA^{®}), gefitinib (IRESSA^{®}) and gemcitabine hydrochloride (GEMZAR^{®}).

For the treatment of pancreatic cancer, the other therapeutic agent may be selected from fluorouracil (ADRUCILO), EFUDEX^{®}, FLUOROPLEX^{®}), erlotinib hydrochloride (TARCEVA^{®}), gemcitabine hydrochloride (GEMZAR^{®}), and mitomycin or mitomycin C (MITOZYTREXTM, MUTAMYCIN^{®}).

For the treatment of cervical cancer, the other therapeutic agent may be selected from bleomycin (BLENOXANE^{®}), cisplatin (PLATINOL^{®}, PLATINOL-AQ^{®}) and topotecan hydrochloride (HYCAMTINO).

For the treatment of head and neck cancer, the other therapeutic agent may be selected from methotrexate, methotrexate LPF (e.g., FOLEX^{®}, FOLEX PFS^{®}, Abitrexate^{®}, MEXATE^{®}, MEXATE-AQ^{®}), fluorouracil (ADRUCILO, EFUDEX^{®}, FLUOROPLEX^{®}), bleomycin (BLENOXANE^{®}), cetuximab (ERBITUX^{®}), cisplatin (PLATINOL^{®}, PLATINOL-AQ^{®}) and docetaxel (TAXOTERE^{®}).

For the treatment of leukemia, including chronic myelomonocytic leukemia (CMML), the other therapeutic agent can be selected from bosutinib (BOSULIFO), cyclophosphamide (CYTOXAN^{®}, NEOSAR^{®}), cytarabine (CYTOSAR-U^{®}, TARABINE PFS^{®}), dasatinib (SPRYCEL^{®}), imatinib mesylate (GLEEVEC^{®}), ponatinib (ICLUSIG^{®}), nilotinib (TASIGNA^{®}) and omacetaxine mepesuccinate (SYNRIBO^{®}).

In another aspect, the present invention provides pharmaceutical compositions comprising at least one compound of the present invention (e.g., a compound of Formula (I) or a sub-formulae theref) or a pharmaceutically acceptable salt thereof, together with a pharmaceutically acceptable carrier suitable for administration to a human or animal subject, either alone or together with other anti-cancer agents.

In combination therapies, compositions will either be formulated together as a combination therapeutic, or as separate compositions. The compound of the invention and the other therapeutic agent may be manufactured and/or formulated by the same or different manufacturers. The structure of therapeutic agents identified by code numbers, generic or trade names may be taken from the actual edition of the standard compendium "The Merck Index" or from databases, e.g. Patents International (e.g. IMS World Publications). The other therapeutic agents, which can be used in combination with a compound of the present invention, can be prepared and administered as described in the art, such as in the documents cited above.

Optionally, the pharmaceutical composition may comprise a pharmaceutically acceptable carrier, as described above. The pharmaceutical composition or combination of the present invention can be in unit dosage of about 0.5-1000 mg of active ingredient(s) for a subject of about 50-70 kg.

In another aspect, the present invention provides methods of treating human or animal subjects suffering from a cellular proliferative disease, such as cancer, comprising administering to the subject a therapeutically effective amount of a compound of the present invention or a pharmaceutically acceptable salt thereof, either alone or in combination with other anti-cancer agents. In combination therapy, the compound of the present invention and other anti-cancer agent(s) may be administered either simultaneously, concurrently or sequentially with no specific time limits, wherein such administration provides therapeutically effective levels of the two compounds in the body of the patient. Moreover, the compound of the invention and the other therapeutic may be brought together into a combination therapy: (i) prior to release of the combination product to physicians (*e*.*g*. in the case of a kit comprising the compound of the invention and the other therapeutic agent); (ii) by the physician themselves (or under the guidance of the physician) shortly before administration; (iii) in the patient themselves, *e*.*g*. during sequential administration of the compound of the invention and the other therapeutic agent.

In one embodiment, the compound of the present invention and the other anti-cancer agent(s) is generally administered sequentially in any order by infusion or orally. The dosing regimen may vary depending upon the stage of the disease, physical fitness of the patient, safety profiles of the individual drugs, and tolerance of the individual drugs, as well as other criteria well-known to the attending physician and medical practitioner(s) administering the combination. The compound of the present invention and other anti-cancer agent(s) may be administered within minutes of each other, hours, days, or even weeks apart depending upon the particular cycle being used for treatment. In addition, the cycle could include administration of one drug more often than the other during the treatment cycle and at different doses per administration of the drug.

In yet another aspect, compounds of the present invention may be combined with other anti-cancer agents, anti-allergic agents, anti-nausea agents (or anti-emetics), pain relievers, cytoprotective agents, and combinations thereof.

In some instances, patients may experience allergic reactions to the compounds of the present invention and/or other anti-cancer agent(s) during or after administration. Therefore, anti-allergic agents may be administered to minimize the risk of an allergic reaction. Suitable anti-allergic agents include corticosteroids, such as dexamethasone (e.g., DECADRON^{®}), beclomethasone (e.g., BECLOVENT^{®}), hydrocortisone (also known as cortisone, hydrocortisone sodium succinate, hydrocortisone sodium phosphate; e.g., ALA-CORT^{®}, hydrocortisone phosphate, Solu-CORTEFO, HYDROCORT Acetate^{®} and LANACORT^{®}), prednisolone (e.g., DELTA-Cortel^{®}, ORAPRED^{®}, PEDIAPRED^{®} and PRELONE^{®}), prednisone (e.g., DELTASONE^{®}, LIQUID REDO, METICORTEN^{®} and ORASONE^{®}), methylprednisolone (also known as 6-methylprednisolone, methylprednisolone acetate, methylprednisolone sodium succinate; e.g., DURALONE^{®}, MEDRALONE^{®}, MEDROL^{®}, M-PREDNISOLO and SOLU-MEDROL^{®}); antihistamines, such as diphenhydramine (e.g., BENADRYL^{®}), hydroxyzine, and cyproheptadine; and bronchodilators, such as the beta-adrenergic receptor agonists, albuterol (e.g., PROVENTILO), and terbutaline (BRETHINE^{®}).

In other instances, patients may experience nausea during and after administration of the compound of the present invention and/or other anti-cancer agent(s). Therefore, anti-emetics may be administered in preventing nausea (upper stomach) and vomiting. Suitable anti-emetics include aprepitant (EMEND^{®}), ondansetron (ZOFRAN^{®}), granisetron HCl (KYTRIL^{®}), lorazepam (ATIVAN^{®}. dexamethasone (DECADRON^{®}), prochlorperazine (COMPAZINEO), casopitant (REZONIC^{®} and Zunrisa^{®}), and combinations thereof.

In yet other instances, medication to alleviate the pain experienced during the treatment period is prescribed to make the patient more comfortable. Common over-the-counter analgesics, such TYLENOL^{®}, are often used. Opioid analgesic drugs such as hydrocodone/paracetamol or hydrocodone/acetaminophen (e.g., VICODIN^{®}), morphine (e.g., ASTRAMORPH^{®} or AVINZA^{®}), oxycodone (e.g., OXYCONTIN^{®} or PERCOCET^{®}), oxymorphone hydrochloride (OPANA^{®}), and fentanyl (e.g., DURAGESIC^{®}) are also useful for moderate or severe pain.

Furthermore, cytoprotective agents (such as neuroprotectants, free-radical scavengers, cardioprotectors, anthracycline extravasation neutralizers, nutrients and the like) may be used as an adjunct therapy to protect normal cells from treatment toxicity and to limit organ toxicities. Suitable cytoprotective agents include amifostine (ETHYOL^{®}), glutamine, dimesna (TAVOCEPT^{®}), mesna (MESNEX^{®}), dexrazoxane (ZINECARD^{®} or TOTECT^{®}), xaliproden (XAPRILAO), and leucovorin (also known as calcium leucovorin, citrovorum factor and folinic acid).

In yet another aspect, a compound of the present invention may be used in combination with known therapeutic processes, for example, with the administration of hormones or in radiation therapy. In certain instances, a compound of the present invention may be used as a radiosensitizer, especially for the treatment of tumors which exhibit poor sensitivity to radiotherapy.

In yet another aspect, the present invention provides kits comprising one or more compounds of the present invention and another therapeutic agent as described above. Representative kits include (a) compound of Formula (I) or sub-formulae thereof or a pharmaceutically acceptable salt thereof; and (b) at least one other therapeutic agent e.g., as indicated above; whereby such kit may further comprise a package insert or other labeling including directions for administration. The kits of the invention may be used for administering different dosage forms, for example, oral and parenteral; for administering two or more separate pharmaceutical compositions at different dosage intervals; or for titrating the separate compositions against one another; wherein at least one pharmaceutical composition comprises a compound a Formula (I) or sub-formulae thereof.

### EXAMPLES

Temperatures are given in degrees Celsius. The structure of final products, intermediates and starting materials is confirmed by standard analytical methods, *e*.*g*., microanalysis and spectroscopic characteristics, *e*.*g*., MS, IR, NMR. Abbreviations used are those conventional in the art.

All starting materials, building blocks, reagents, acids, bases, dehydrating agents, solvents, and catalysts utilized to synthesis the compounds of the present invention are either commercially available or can be produced by organic synthesis methods known to one of ordinary skill in the art (Houben-Weyl 4th Ed. 1952, Methods of Organic Synthesis, Thieme, Volume 21). Unless otherwise specified, starting materials are generally available from commercial sources.

The Examples herein merely illuminate the invention and does not limit the scope of the invention otherwise claimed. Further, the compounds of the present invention can be produced by organic synthesis methods known to one of ordinary skill in the art as shown in the following examples. Where desired, conventional protecting groups are used to protect reactive functional groups in accordance with standard practice, for example, see T.W. Greene and P.G.M. Wuts in "Protecting Groups in Organic Synthesis", John Wiley and Sons, 1991.

### Abbreviations

Abbreviations as used herein, are defined as follows: "1x" for once, "2x" for twice, "3x" for thrice, "°C" for degrees Celsius, "aq" for aqueous, "FCC" for flash column chromatography, "eq" for equivalent or equivalents, "g" for gram or grams, "mg" for milligram or milligrams, "L" for liter or liters, "mL" for milliliter or milliliters, "µL" for microliter or microliters, "N" for normal, "M" for molar, "nM" for nanomolar, "mol" for mole or moles, "mmol" for millimole or millimoles, "min" for minute or minutes, "h" or "hrs" for hour or hours, "RT" for room temperature, "ON" for overnight, "atm" for atmosphere, "psi" for pounds per square inch, "conc." for concentrate, "sat" or "sat'd" for saturated, "MW" for molecular weight, "mw" or "µwave" for microwave, "mp" for melting point, "Wt" for weight, "MS" or "Mass Spec" for mass spectrometry, "ESI" for electrospray ionization mass spectroscopy, "HR" for high resolution, "HRMS" for high resolution mass spectrometry, "LCMS" or "LC-MS" for liquid chromatography mass spectrometry, "HPLC" for high pressure liquid chromatography, "RP HPLC" for reverse phase HPLC, "TLC" or "tic" for thin layer chromatography, "NMR" for nuclear magnetic resonance spectroscopy, "nOe" for nuclear Overhauser effect spectroscopy, "¹H" for proton, "δ" for delta, "s" for singlet, "d" for doublet, "t" for triplet, "q" for quartet, "m" for multiplet, "br" for broad, "Hz" for hertz, "ee" for "enantiomeric excess" and "α", "β", "R", "r", "S", "s", "E", and "Z" are stereochemical designations familiar to one skilled in the art.

The following abbreviations used herein below have the corresponding meanings:
- Δ: heat
- AcOH: acetic acid
- B₂pin₂: bis(pinacolato)diboron
- BINAP: (2,2'-bis(diphenylphosphino)-1,1'bihaphthyl
- Boc: tert-butyloxycarbonyl
- Boc₂O: di-tert butyl dicarbonate
- BSA: bovine serum albumin
- CDCl₃: DASchloroform-d
- CD₃OD: methanol-d₄
- dd: doublet of doublets
- DCE: 1,2-dichloroethane
- DCM: dichloromethane
- DEA: diethanolamine
- DEAD: Diethyl azodicarboxylate
- DIEA: N,N-diisopropyl ethylamine
- DIPEA: N,N-diisopropylethylamine
- DMAP: 4-dimethylaminopyridine
- DMF: dimethylformamide
- DMP: Dess-Martin periodinane
- DMSO: dimethylsulfoxide
- EA: ethyl alcohol
- Et₃N: triethylamine
- EtOAc: ethyl acetate
- EtOH: ethanol
- EtSH: ethanethiol
- FBS: fetal bovine serum
- HATU: Hexafluorophosphate Azabenzotriazole Tetramethyl Uronium)
- Ir[dF(CF₃)ppy]₂: (dtbbpy)PF₆ [4,4'bis(tert-butyl)-2,2'-bipyridine]bis[3,5-difluoro-2-[5-(trifluoromethyl)-2-pyridinyl]phenyl]iridium(III) hexafluorophosphate
- iPrOH: isopropyl alcohol
- ISCO: *in situ* chemical oxidation
- LDA: lithium diisopropylamide
- MeCN: acetonitrile
- MeOH: methanol
- MgSO₄: magnesium sulfate
- MHz: megahertz
- min: minutes
- m/z: mass to charge ratio
- NaBH(OAc)₃: sodium triacetoxyborohydride
- NBS: N-bromosuccinimide
- NMP: N-methyl-2-pyrrolidone
- PBu₃: tributylphosphine
- Pd(dppf)Cl₂: [1,1'-Bis(diphenylphosphino)ferrocene]dichloropalladium (II)
- Pd(dppf)Cl_{2·}DCM: [1,1'-Bis(diphenylphosphino)ferrocene]dichloropalladium (II), complex with dichloromethane
- Pd₂dba₃: tris(dibenzylideneacetone)dipalladium (0)
- Pd[P(t-Bu)₃]₂: bis(tri-tert-butylphosphine)palladium (0)
- PE: petroleum ether
- TMSIppm: parts per million
- *rac*: racemic
- SFC: Supercritical fluid chromatography
- TBAF: tetra-n-butylammonium fluoride
- t-BuOH: tert-butyl alcohol
- TEA: triethylamine
- TFA: trifluoroacetic acid
- THF: tetrahydrofuran
- TMSCF₃: trifluoromethyltrimethylsilane
- TMSI: trimethylsilyl iodide
- UV: ultraviolet

High performance liquid chromatography (HPLC) was performed using an Agilent 1260 HPLC System (Santa Clara, CA). The analytical column was reversed phase Phenomenex Kinetex C18-2.6 µm, 4.6 × 50 mm. A gradient elution was used (flow rate 2.0 mL/min), starting with 5% methanol/ 95% water and progressing to 95% methanol/ 5% water over a period of 10 minutes. All solvents contained 0.1% formic acid (FA). Compounds were detected by ultraviolet light (UV) absorption at 214, 254 and 300 nm. HPLC solvents were purchased from Sigma Aldrich (St. Louis, MO).

Mass spectrometric analysis was performed on an Agilent System (Agilent 1260 HPLC and an Agilent 6130 mass spectrometer detector; Column: Phenomenex Kinetex 2.6 um C18, column size 4.6 × 50 mm; column temperature 40°C; gradient: 5 95% methanol in water with 0.1% FA over a 2 min period; flow rate 2.0 mL/min (or Polar gradient 5-50% over 2.0 min, or Non-Polar gradient 50-95% over 2.0 min); Mass Spectrometer molecular weight scan range 100 1000; or 100-1500; capillary voltage 4000 V. All masses were reported as those of the protonated parent ions, unless otherwise indicated.

Nuclear magnetic resonance (NMR) analysis was performed using a Bruker 400 MHz NMR. The spectral reference was either TMS or the known chemical shift of the solvent.

### Chiral Preparative HPLC Methods Employed in Purification of Examples

SFC chiral screening was carried out on a Thar Insturments Investigator system. The Thar Investigator system consists of:
- ALIAS autosampler
- Thar Fluid Delivery Module (0 to 10mL/min)
- Thar SFC 10 position column oven
- Waters 2998 PDA
- Thar Automated Back Pressure Regulator

All of the Thar components are part of the SuperPure Discovery Series line. The system flowed at 3.0 mL/min and kept at 38 °C. The system back pressure was set to 100 bar. Each sample was screened through a battery of ten 5 µm columns:
- 5µm 4.6x150mm ChiralPak AD
- 5µm 4.6x150mm ChiralCel OD
- 5µm 4.6x150mm ChiralCel OJ
- 5µm 4.6x150mm ChiralPak AS
- 5µm 4.6x250mm ChiralPak AY
- 5µm 4.6x250mm ChiralCel OZ
- 5µm 4.6x150mm ChiralPak IC
- 5µm 4.6x150mm ChiralPak IG
- 5µm 4.6x250mm Regis Whelk-O1
- 5µm 4.6x250mm ChromegaChiral CC4

The system ran a gradient from 5% co-solvent to 50% co-solvent in 9 minutes followed by a 10 minutes hold at 50% co-solvent, a switch back to 5% co-solvent and a 0.5 minute hold at initial condition. In between each gradient there was a 4 minute equilibration method that flows 5% co-solvent through the next column to be screened. The typical solvents screened were, MeOH, EtOH, IPA, MeOH+0.5%NH₃, EtOH+0.5%NH₃, IPA+0.1%NH₃. Once separation was detected using one of the gradient methods, an isocratic method can be developed, and if necessary, scaled up for separation on the Thar Prep 80 system.

### Intermediates

### Intermediate 1: Methyl (R)-3-((tert-butoxycarbonyl)amino)pyrrolidine-3-carboxylate

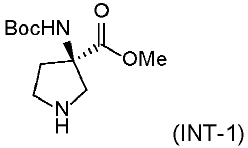

To a solution of methyl 2-((tert-butoxycarbonyl)amino)acrylate (430 g, 2.14 mol) and N-benzyl-1-methoxy-N-((trimethylsilyl)methyl)methanamine (533 g, 2.2 mol) in DCM (5.5 L) was added trifluoroacetic acid (24.4 g, 214 mmol) slowly at 0 °C. The reaction mixture was stirred at 30 °C for 18 hrs. The mixture was quenched with aq.NaHCO₃ (5 L). The crude product was extracted with DCM (2.5 L). The organic layer was washed with brine (3.0 L), dried over Na₂SO₄, filtered and concentrated in vacuo. The residue was purified by column chromatography (SiO₂, PE/EtOAc = 10/1 to 0/1) to afford methyl 1-benzyl-3-((tert-butoxycarbonyl)amino)pyrrolidine-3-carboxylate as a yellow solid (INT-1A). ¹H NMR (400 MHz, CDCl₃) δ 7.35-7.31 (m, 4H), 7.27-7.23 (m, 1H), 5.12 (s, 1H), 3.75 (s, 3H), 3.67-3.61 (m, 2H), 2.95-2.87 (m, 2H), 2.82 (d, J = 9.2 Hz, 1H), 2.67-2.53 (m, 2H), 2.06-1.94 (m, 1H), 1.43 (s, 9H). LC-MS: [M+H]⁺ = 335.1, 336.1. Separation by preparational chiral HPLC using ChiralPak AD, 300×50 mm I.D., 10µm column gave methyl (R)-1-benzyl-3-((tert-butoxycarbonyl)amino)pyrrolidine-3-carboxylate.

To a solution of methyl (R)-1-benzyl-3-((tert-butoxycarbonyl)amino)pyrrolidine-3-carboxylate (141 g, 421.6 mmol) in MeOH (1.4 L) was added Pd/C (25 g, 10% purity, 50% purity of water) under N₂. The mixture was purged with H₂ and was stirred at 30 °C for 20 hrs under H₂ (50 psi). The mixture was filtered through a pad of celite and the filtrate was concentrated to afford methyl (R)-3-((tert-butoxycarbonyl)amino)pyrrolidine-3-carboxylate as yellow gum. ¹H NMR (400 MHz, Methanol-d₄) δ 3.72 (s, 3H), 3.38 (s, 1H), 3.09-2.96 (m, 3H), 2.27-2.17 (m, 1H), 2.07-2.02 (m, 1H), 1.43 (s, 9H).

### General Procedure A for making Benzaldehyde Intermediates

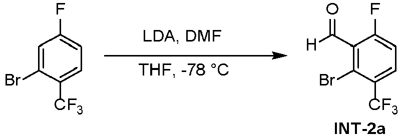

### Intermediate 2A: 2-bromo-6-fluoro-3-(trifluoromethyl)benzaldehyde

To a THF solution (45 mL) of 2-bromo-4-fluoro-1-(trifluoromethyl)benzene (7.0 g, 28.8 mmol) was added 2 M LDA solution (21.6 mL, 43.2 mmol) at -78 °C dropwise under N₂. The reaction mixture was stirred at this temperature for 30 min, followed by addition of DMF (3.35 mL, 43.2 mmol) dropwise. The reaction mixture was stirred for 1 hr at -78 °C. The reaction mixture was then quenched with 0.5 N HCl solution at 0 °C and warm up to rt. The reaction mixture was concentrated under vacuum, and diluted with EA. The organic layer was washed with water, brine, dried over Na₂SO₄ and concentrated under vacuum to give the crude product. The crude product was purified by flash chromatography (0% to 30% EA in Hex) to give 2-bromo-6-fluoro-3-(trifluoromethyl)benzaldehyde (Int-2a) as an off-white solid. ¹H NMR (400 MHz, CDCl₃): δ 10.42 (s, 1H), 7.92 (dd, *J* = 8.93, 5.26 Hz, 1H), 7.33 - 7.25 (m, 1H).

### Intermediate 2: methyl 1-(3-bromo-2-(hydroxymethyl)-4-(trifluoromethyl)phenyl)-3-((tert-butoxycarbonyl)amino)pyrrolidine-3-carboxylate

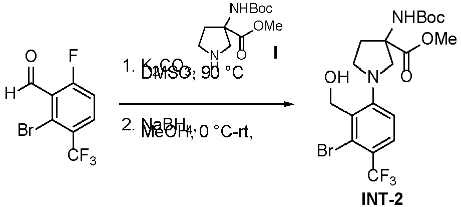

To a DMSO solution (12 mL) of 2-bromo-6-fluoro-3-(trifluoromethyl)benzaldehyde (INT-2a) (2 g, 7.5 mmol) and methyl 1-benzyl-3-((tert-butoxycarbonyl)amino)pyrrolidine-3-carboxylate (INT-1A) (1.5 g, 5.8 mmol) was added K₂CO₃ (1.2 g, 8.7 mmol) at rt. The reaction tube was sealed and submerged to the pre-heated oil bath (90 °C). After stirring overnight, the reaction mixture was diluted with EA, washed with water and brine. The organic layer was dried over Na₂SO₄, and concentrated under vacuum to give the crude product. The crude product was redissolved in MeOH (40 mL) and treated with NaBH₄ (440 mg, 11.62 mmol) in portions at 0 °C. 25 min later, the ice bath was removed. 1.5 hrs later, the solvent was removed under vacuum. The residue was diluted with EA, washed with 0.5 N HCl, water, and brine. The organic layer was dried over Na₂SO₄, and concentrated under vacuum to give the crude product. Purification by flash chromatography (10% to 50% EA in Hex) gave methyl 1-(3-bromo-2-(hydroxymethyl)-4-(trifluoromethyl)phenyl)-3-((*tert*-butoxycarbonyl)amino)pyrrolidine-3-carboxylate as an off-white solid. ¹H NMR (400 MHz, MeOH-d₄): δ 7.54 (d, *J* = 8.80 Hz, 1H), 6.97 (d, *J* = 8.93 Hz, 1H), 4.87 (s, 2 H), 4.16 - 4.27 (m, 2H), 4.06 (br d, *J* = 10.39 Hz, 1H), 3.62 (br d, *J* = 9.90 Hz, 2H), 3.49 (br dd, *J* = 15.34, 7.89 Hz, 1H), 2.41 - 2.54 (m, 1H), 2.24 - 2.34 (m, 1H), 1.44 (s, 9H), 1.27 (br t, *J* = 7.03 Hz, 3H). LC-MS: [M+H]⁺ = 510.7.

### Intermediate 3: methyl (R)-1-(3-bromo-5-chloro-2-(hydroxymethyl)phenyl)-3-((tert-butoxycarbonyl)amino)pyrrolidine-3-carboxylate

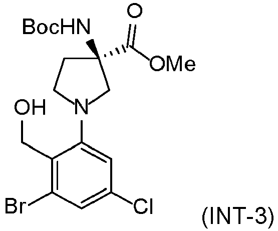

To a solution of methyl (R)-3-((tert-butoxycarbonyl)amino)pyrrolidine-3-carboxylate (INT-1) (55 g, 225.1 mmol) in CH₃CN (440 mL) was added 2-bromo-4-chloro-6-fluorobenzaldehyde (Apollo Scientific) (56.1 g, 236.3 mmol) and N,N-diisopropylethylamine (58.2 g, 450.3 mmol). The mixture was stirred at 80 °C for 18 hours. The mixture was recovered to r.t., water (1.5 L) was added in and the crude product was extracted with EtOAc (1.5 L × 2), washed with brine (1.5 L), dried over Na₂SO₄, filtered and concentrated under vacuum to afford methyl (R)-1-(3-bromo-5-chloro-2-formylphenyl)-3-((tert-butoxycarbonyl)amino)pyrrolidine-3-carboxylate (INT-3a) (229 g, crude). LC-MS: [M+H]⁺ = 463.1, 462.1.

To a solution of methyl (R)-1-(3-bromo-5-chloro-2-formylphenyl)-3-((tert-butoxycarbonyl)amino)pyrrolidine-3-carboxylate (INT-3a) (271.6 g, crude) in THF (1.5 L) was added a solution of NaBH₄ (38.7 g, 1 mol) in H₂O (400 mL) at 0-10 °C. After addition the reaction mixture was stirred at 10-15 °C for 1 hr. The mixture was poured into aq. NH₄Cl (1 L) and H₂O (1 L). The crude product was extracted with EtOAc (2 L × 2). The organic layer was washed with brine (2 L), dried over Na₂SO₄, filtered and concentrated under vacuum. The residue was purified by medium pressure liquid chromatography (25% EtOAc in PE) to afford methyl (R)-1-(3-bromo-5-chloro-2-(hydroxymethyl)phenyl)-3-((tert-butoxycarbonyl)amino) pyrrolidine-3-carboxylate as a yellow solid. ¹H NMR (400 MHz, Methanol-d₄) δ = 7.26 (d, J = 5.6 Hz, 1H), 6.99 (s, 1H), 5.20 (s, 1H), 4.90-4.79 (m, 2H), 3.83 (d, J = 10.4 Hz, 1H), 3.80 (s, 3H), 3.52 - 3.41 (m, 2H), 3.40 - 3.34 (m, 1H), 2.58-2.55 (m, 1H), 2.31-2.18 (m, 1H), 1.45 (s, 9H). ). LC-MS: [M+H]⁺ = 464.9, 462.9.

### Intermediate 4: tert-butyl N-[(tert-butoxy)carbonyl]-N-(9H-purin-6-yl)carbamate

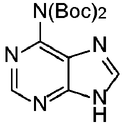

To a solution of 9H-purin-6-amine (200 g, 1.48 mol) and DMAP (18.1 g, 148 mmol) in THF (2.5 L) was added Boc₂O (1292 g, 5.92 mol) slowly at 10-15°C. The mixture was stirred at 25 °C for 18 hrs then the mixture was concentrated under vacuum. The crude intermediate (880 g, crude) as brown oil was dissolved in MeOH (2.8 L) at 5-10 °C. NaHCO₃ (saturated, 800 mL) was added in. The mixture was stirred at 25 °C for 4 hrs. The reaction mixture was diluted with H₂O (1 L) and concentrated to remove MeOH. The reaction was diluted with H₂O (1 L) and a white solid formed. The mixture was filtered and the filter cake was washed with methyl tert-butyl ether (800 mL × 2) to tert-butyl N-[(tert-butoxy)carbonyl]-N-(9H-purin-6-yl)carbamate as a white solid. ¹H NMR (400 MHz, DMSO-*d*₆) δ = 8.79 (s, 1H), 8.61 (s, 1H), 1.36 (s, 18H). LC-MS: [M+H]⁺ = 336.2.

### Intermediate 5: methyl (R)-1-(2-((6-(bis(tert-butoxycarbonyl)amino)-9H-purin-9-yl)methyl)-3-bromo-5-chlorophenyl)-3-((tert-butoxycarbonyl)amino)pyrrolidine-3-carboxylate

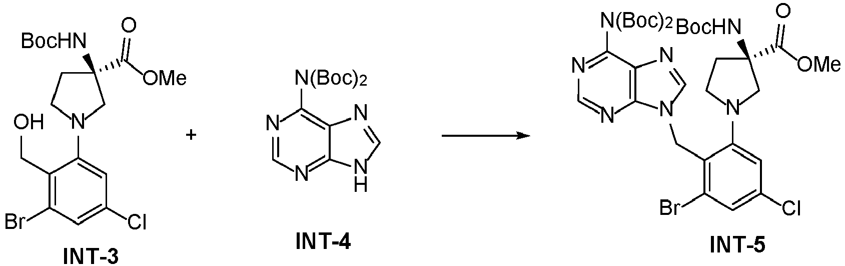

A mixture of methyl (R)-1-(3-bromo-5-chloro-2-(hydroxymethyl)phenyl)-3-((tert-butoxycarbonyl)amino)pyrrolidine-3-carboxylate (INT-3) (190 g, 410 mmol), tert-butyl N-[(tert-butoxy)carbonyl]-N-(9H-purin-6-yl)carbamate (INT-4) (165 g, 492 mmol) and di-tert-butyl azodicarboxylate (378 g, 1.64 mol) in THF (1 L) was added tributylphosphane (332 g, 1.64 mol) under 0-5 °C under N₂. The reaction mixture was stirred at 30 °C for 21 hrs then another portion of tributylphosphane (83 g, 410 mmol) was added in at 10-15 °C. The mixture was stirred at 30 °C for another 1 hr. The reaction mixture was concentrated under reduced pressure. The residue was purified by medium pressure liquid chromatography (25% EtOAc in PE) to afford methyl (R)-1-(2-((6-(bis(tert-butoxycarbonyl)amino)-9H-purin-9-yl)methyl)-3-bromo-5-chlorophenyl)-3-((tert-butoxycarbonyl)amino)pyrrolidine-3-carboxylate as yellow oil ¹H NMR (400 MHz, Methanol-*d*₄) δ 8.86 (s, 1H), 8.23 (s, 1H), 7.42 (d, J = 1.6 Hz, 1H), 7.30 (d, J = 1.6 Hz, 1H), 5.80-5.67 (m, 2H), 3.86 (d, J = 10.0 Hz, 1H), 3.68 (s, 3H), 3.41-3.35 (m, 2H), 3.26-3.16 (m, 1H), 2.47-2.35 (m, 1H), 2.21-2.09 (m, 1H), 1.49-1.34 (m, 27H). LC-MS: [M+H]⁺ = 782.2, 781.2.

### Intermediate 6: tert-butyl (R)-(1-(3-bromo-2-((6-((tert-butoxycarbonyl)amino)-9H-purin-9-yl)methyl)-5-chlorophenyl)-3-(cyclopropylcarbamoyl)pyrrolidin-3-yl)carbamate

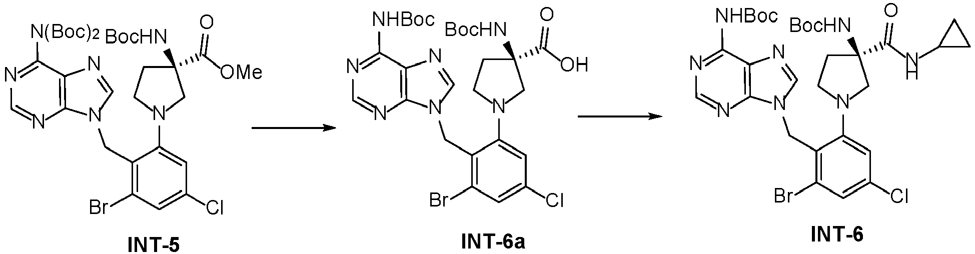

To a solution of methyl (R)-1-(2-((6-(bis(tert-butoxycarbonyl)amino)-9H-purin-9-yl)methyl)-3-bromo-5-chlorophenyl)-3-((tert-butoxycarbonyl)amino)pyrrolidine-3-carboxylate (INT-5) (130 g, 166.4 mmol) in a mixture of THF (650 mL) and MeOH (650 mL) was added aq. LiOH (332.8 mL, 2.5 M, 832 mmol) at 5 °C. The mixture was stirred at 15 °C for 4 hrs. The mixture was concentrated under reduced pressure. The pH of the residue was adjusted to 3-4 using 2 M HCl. The crude product was extracted with EtOAc (1.5 L × 2), washed with brine (1 L), dried over Na₂SO₄, filtered and concentrated to afford (R)-1-(3-bromo-2-((6-((tert-butoxycarbonyl)amino)-9H-purin-9-yl)methyl)-5-chlorophenyl)-3-((tert-butoxycarbonyl)amino)pyrrolidine-3-carboxylic acid (INT-6a). ¹H NMR (400 MHz, Methanol-d₄) δ 8.60 (s, 1H), 7.96 (s, 1H), 7.41 (s, 1H), 7.24 (d, J = 2.0 Hz, 1H), 5.63 (s, 2H), 3.79 (d, J = 9.6 Hz, 1H), 3.35 (s, 1H), 3.29-3.21 (m, 2H), 2.42-2.31 (m, 1H), 2.18-2.15 (m, 1H), 1.61-1.56 (m, 9H), 1.46 (s, 9H). LC-MS: [M+H]⁺ = 668.1, 666.1.

To a solution of (R)-1-(3-bromo-2-((6-((tert-butoxycarbonyl)amino)-9H-purin-9-yl)methyl)-5-chlorophenyl)-3-((tert-butoxycarbonyl)amino)pyrrolidine-3-carboxylic acid (INT-6a) (80 g, 0.12 mol) and cyclopropanamine (10 g, 0.18 mol) in DMF (800 mL) was added HATU (91 g, 0.24 mol) and N,N-Diisopropylethylamine (47 g, 0.36 mol). The mixture was stirred at 25 °C for 2 hours. The mixture was diluted with brine (2 L). The crude product was extracted with EtOAc (3 × 1.5 L). The organic layer was concentrated under vacuum. The residue was purified by silica gel chromatography (PE/EtOAc = 20/1 to 0/1) to afford tert-butyl (R)-(1-(3-bromo-2-((6-((tert-butoxycarbonyl)amino)-9H-purin-9-yl)methyl)-5-chlorophenyl)-3-(cyclopropylcarbamoyl) pyrrolidin-3-yl)carbamate as yellow solid. ¹H NMR (400 MHz, CDCl₃) δ 8.72 (s, 1H), 7.94 (s, 2H), 7.78 (s, 1H), 7.35 (s, 1H), 7.18 (s, 1H), 5.81-5.45 (m, 2H), 3.71-3.55 (m, 2H), 3.40-3.21 (m, 1H), 3.17-3.10 (m, 1H), 2.91-2.86 (m, 1H), 2.63-2.54 (m, 1H), 2.37-3.25 (m, 1H), 1.50 (s, 9H), 1.36 (s, 9H), 0.68-0.60 (m, 2H), 0.52-0.35 (m, 2H) LC-MS: [M+H]⁺ = 707.2.

### Intermediate 7: tert-butyl (R)-(1-(2-((6-((tert-butoxycarbonyl)amino)-9H-purin-9-yl)methyl)-5-chloro-3-ethylphenyl)-3-(cyclopropylcarbamoyl)pyrrolidin-3-yl)carbamate

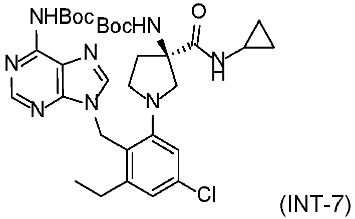

To a solution of tert-butyl (R)-(1-(3-bromo-2-((6-((tert-butoxycarbonyl)amino)-9H-purin-9-yl)methyl)-5-chlorophenyl)-3-(cyclopropylcarbamoyl)pyrrolidin-3-yl)carbamate (INT-6) (65 g, 92.1 mmol) and potassium vinyltrifluoroborate (24.7 g, 184.1 mmol) in dioxane/H₂O (650 mL, 10/1) were added K₂CO₃ (38.2 g, 276.2 mmol) and Pd(dppf)Cl₂·DCM (7.5 g, 9.2 mmol). The mixture was stirred at 90 °C for 4 hours. The mixture was diluted with water (2.0 L). The crude product was extracted with ethyl acetate (3 × 1.5 L). The organic layer was concentrated under vacuum. The residue was purified by silica gel chromatography eluted with PE/EtOAc = 20/1 to 0/1 to afford tert-butyl (R)-(1-(2-((6-((tert-butoxycarbonyl)amino)-9H-purin-9-yl)methyl)-5-chloro-3-vinylphenyl)-3-(cyclopropylcarbamoyl)pyrrolidin-3-yl)carbamate as yellow solid. ¹H NMR (400 MHz, Methanol-*d*₄) δ 8.26 (s, 1H), 7.70 (s, 1H), 7.63-7.57 (m, 1H), 7.53-7.46 (m, 1H), 7.28 (s, 1H), 7.25-7.20 (m, 1H), 7.05-6.88 (m, 1H), 5.79-5.50 (m, 3H), 5.31-5.16 (m, 1H), 3.91-3.75 (m, 1H), 3.26-3.09 (m, 3H), 2.64-2.53 (m, 1H), 2.45-2.31 (m, 1H), 2.14-2.04 (m, 1H), 1.37 (s, 9H), 0.79-0.60 (m, 2H), 0.54-0.40 (m, 2H). LC-MS: [M+H]⁺ = 653.4.

A solution of tert-butyl (R)-(1-(2-((6-((tert-butoxycarbonyl)amino)-9H-purin-9-yl)methyl)-5-chloro-3-vinylphenyl)-3-(cyclopropylcarbamoyl)pyrrolidin-3-yl)carbamate (16 g, purity: 91.2%) in THF (240 mL) was hydrogenated with Pd/C (3.2 g, 10% purity). The mixture was purged by H₂ and the mixture was stirred at 25 °C for 24 hours under H₂ (15 PSI). The mixture was filtered and the filtrate was concentrated under vacuum to afford tert-butyl (R)-(1-(2-((6-((tert-butoxycarbonyl)amino)-9H-purin-9-yl)methyl)-5-chloro-3-ethylphenyl)-3-(cyclopropylcarbamoyl)pyrrolidin-3-yl)carbamate as yellow solid. LC-MS: [M+H]⁺ = 655.4.

### Intermediate 8: 2-(2,3-dihydrobenzofuran-5-yl)-4,4,5,5-tetramethyl-1,3,2-dioxaborolane

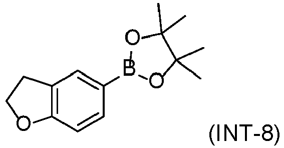

To a solution of 5-bromo-2,3-dihydrobenzofuran (10 g, 50.2 mmol) dissolved in dioxane (100 mL) was added bis(pinacolato)diboron (14 g, 55.27 mmol), potassium acetate (7.1 g, 110.5 mmol) and Pd(dppf)Cl₂DCM (4.1 g, 5 mmol). The mixture was stirred at 90 °C for 16 hours. The mixture was diluted with water (200 mL) and was extracted with EtOAc (3 × 200 mL). The organic layer was dried over Na₂SO₄, filtered and concentrated under vacuum. The residue was purified by silica gel chromatography eluted with PE to afford 2-(2,3-dihydrobenzofuran-5-yl)-4,4,5,5-tetramethyl-1,3,2-dioxaborolane. ¹HNMR: (400 MHz CDCl₃) δ 7.67 (s, 1H), 7.62 (d, *J* = 8.0 Hz, 1H), 6.80 (d, *J* = 8.0 Hz, 1H), 4.58 (t, *J* = 8.8 Hz, 2H), 3.20 (t, *J* = 8.8 Hz, 2H), 1.34 (s, 12H). LC-MS: [M+H]⁺ = 247.3.

### Intermediate 9: methyl (R)-1-(3-bromo-2-((6-((tert-butoxycarbonyl)amino)-9H-purin-9-yl)methyl)-5-chlorophenyl)-3-((tert-butoxycarbonyl)amino)pyrrolidine-3-carboxylate

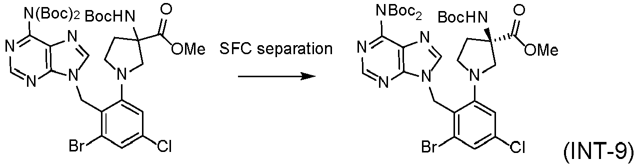

Intermediate methyl 1-(2-((6-(bis(tert-butoxycarbonyl)amino)-9H-purin-9-yl)methyl)-3-bromo-5-chlorophenyl)-3-((tert-butoxycarbonyl)amino)pyrrolidine-3-carboxylate (*rac* INT-5) (5 g, 6.4 mmol) was purified by SFC using ChiralPakAD, 300×50 mm I.D., 10µm column, (0.1% NH₃H₂O in isopropanol, flow rate: 60 mL/min, . Rt = 2.516 min, ee: 99%, the second eluent) to methyl (R)-1-(3-bromo-2-((6-((tert-butoxycarbonyl)amino)-9H-purin-9-yl)methyl)-5-chlorophenyl)-3-((tert-butoxycarbonyl)amino)pyrrolidine-3-carboxylate as a white solid.

### Intermediate 10: tert-butyl (R)-(1-(2-((6-amino-9H-purin-9-yl)methyl)-5-chloro-3-(2,3-dihydrobenzofuran-5-yl)phenyl)-3-(cyclopropylcarbamoyl)pyrrolidin-3-yl)carbamate

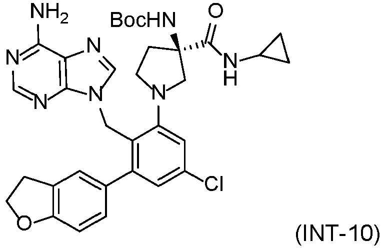

To a mixture of 2-(2,3-dihydrobenzofuran-5-yl)-4,4,5,5-tetramethyl-1,3,2-dioxaborolane (INT-8) (300 mg, 1.22 mmol), methyl (R)-1-(3-bromo-2-((6-((tert-butoxycarbonyl)amino)-9H-purin-9-yl)methyl)-5-chlorophenyl)-3-((tert-butoxycarbonyl)amino)pyrrolidine-3-carboxylate (INT-9) (623 mg, 0.9 mmol) and K₂CO₃ (253 mg, 1.8 mmol) in dioxane/H₂O (7 mL) were added in Pd(dppf)Cl₂DCM (100 mg, 0.12 mmol). The mixture was stirred at 90 °C for 16 hours. The mixture was diluted with water (30 mL) and was extracted with EtOAc (3 × 30 mL). The organic layer was dried over Na₂SO₄, filtered and concentrated under vacuum. The residue was purified by silica gel chromatography eluted with PE/EA = 10/1 to 0/1 to afford the crude, which was further purified by prep-HPLC (column: Phenomenex Synergi C18 150*25*10 µm, gradient 30-50%B (A = water (0.05% HCl), B = acetonitrile, flow rate: 28 mL/min) to afford methyl (R)-1-(2-((6-amino-9H-purin-9-yl)methyl)-5-chloro-3-(2,3-dihydrobenzofuran-5-yl)phenyl)-3-((tert-butoxycarbonyl)amino)pyrrolidine-3-carboxylate. (400 MHz CDCl₃) δ 8.78 (s, 1H), 7.43 (s, 1H), 7.18 (d, *J* = 2.0 Hz, 1H), 7.03 (d, *J* = 2.0 Hz, 1H), 6.93 (d, *J*=7.8 Hz, 1H), 6.91 (s, 1H), 6.76 (d, *J* = 8.2 Hz, 1H), 5.60-5.50 (m, 2H), 4.59-4.56 (m, 2H), 3.75-3.73 (m, 3H), 3.40-3.34 (m, 1H), 3.30-3.25 (m, 2H), 3.17-3.12 (m, 3H), 2.55-2.47 (m, 1H), 1.95-1.92 (m, 1H), 1.44 (s, 9H), 1.25 (s, 18H). LC-MS: [M+H]⁺ = 620.5.

To a solution of methyl (R)-1-(2-((6-amino-9H-purin-9-yl)methyl)-5-chloro-3-(2,3-dihydrobenzofuran-5-yl)phenyl)-3-((tert-butoxycarbonyl)amino)pyrrolidine-3-carboxylate in THF (0.7 mL) and MeOH (0.9 mL) and H₂O (0.7 mL) was added in LiOH·H₂O (101 mg, 2.40 mmol, 10 eq.). The mixture was stirred at 15 °C for 2 hours. The mixture was dissolved in water (20 mL) and was adjust to pH = 4 with 1M HCl. The mixture was extracted with EtOAc (3 × 30 mL). The organic layer was dried over Na₂SO₄, filtered and concentrated under vacuum to afford (R)-1-(2-((6-amino-9H-purin-9-yl)methyl)-5-chloro-3-(2,3-dihydrobenzofuran-5-yl)phenyl)-3-((tert-butoxycarbonyl)amino)pyrrolidine-3-carboxylic acid. LC-MS: [M+H]⁺ = 606.4.

To a solution of (R)-1-(2-((6-amino-9H-purin-9-yl)methyl)-5-chloro-3-(2,3-dihydrobenzofuran-5-yl)phenyl)-3-((tert-butoxycarbonyl)amino)pyrrolidine-3-carboxylic acid (110 mg, 0.18 mmol) and cyclopropylamine (16 mg, 0.27 mmol) dissolved in DMF (1.1 mL) were added in DIPEA (70 mg, 0.54 mmol) and HATU (138 mg, 0.36 mmol). The mixture was stirred at 15 °C for 1 hour. The mixture was diluted with water (20 mL) then was extracted with EtOAc (3 × 20 mL). The organic layer was washed with brine (3 × 20 mL), dried over Na₂SO₄, filtered and concentrated under vacuum to afford tert-butyl (R)-(1-(2-((6-amino-9H-purin-9-yl)methyl)-5-chloro-3-(2,3-dihydrobenzofuran-5-yl)phenyl)-3-(cyclopropylcarbamoyl)pyrrolidin-3-yl)carbamate as a yellow solid. LC-MS: [M+H]⁺ = 645.4.

### Intermediate 11: tert-butyl (9-(2-bromo-6-(3-((tert-butoxycarbonyl)amino)-3-(1H-1,2,4-triazol-3-yl)pyrrolidin-1-yl)-4-chlorobenzyl)-9H-purin-6-yl)carbamate

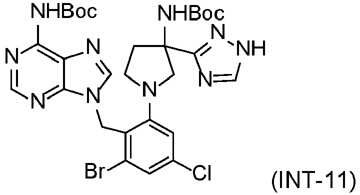

To a solution of 1-(3-bromo-2-((6-((tert-butoxycarbonyl)amino)-9H-purin-9-yl)methyl)-5-chlorophenyl)-3-((tert-butoxycarbonyl)amino)pyrrolidine-3-carboxylic acid (*rac* INT-6a) (200 mg, 0.3 mmol, 1 eq.) dissolved in DMF (10 mL) was added in HATU (171 mg, 0.45 mmol, 1.5 eq.), NH₄Cl (80.2 mg, 1.5 mmol, 5 eq.) and DIPEA (232.5 mg, 1.8 mmol, 6.0 eq.). The mixture was stirred for 30 mins at 26 °C. The mixture was diluted with H₂O then was extracted with EtOAc (10 mL × 3), dried over Na₂SO₄ and evaporated in vacuo to afford tert-butyl (1-(3-bromo-2-((6-((tert-butoxycarbonyl)amino)-9H-purin-9-yl)methyl)-5-chlorophenyl)-3-carbamoylpyrrolidin-3-yl)carbamate as a yellow oil which was used directly. LC-MS: [M+H]⁺ = 667.3.

Tert-butyl (1-(3-bromo-2-((6-((tert-butoxycarbonyl)amino)-9H-purin-9-yl)methyl)-5-chlorophenyl)-3-carbamoylpyrrolidin-3-yl)carbamate (150 mg, 0.23 mmol, 1 eq.) was mixed in DMF-DMA (2 mL). The mixture was stirred for 10 mins at 90 °C. The mixture was evaporated in vacuo to afford tert-butyl (E)-(1-(3-bromo-2-((6-((tert-butoxycarbonyl)amino)-9H-purin-9-yl)methyl)-5-chlorophenyl)-3-(((dimethylamino)methylene)carbamoyl)pyrrolidin-3-yl)carbamate as a yellow oil. LC-MS: [M+H]⁺ = 722.0.

To a solution of tert-butyl (E)-(1-(3-bromo-2-((6-((tert-butoxycarbonyl)amino)-9H-purin-9-yl)methyl)-5-chlorophenyl)-3-(((dimethylamino)methylene)carbamoyl)pyrrolidin-3-yl)carbamate (100 mg, 0.14 mmol, 1 eq.) in HOAc (1 mL) was added hydrazine hydrate (1 mL). The mixture was stirred for 30 mins at 70 °C. The mixture was directly evaporated to give tert-butyl (9-(2-bromo-6-(3-((tert-butoxycarbonyl)amino)-3-(1H-1,2,4-triazol-3-yl)pyrrolidin-1-yl)-4-chlorobenzyl)-9H-purin-6-yl)carbamate as yellow solid. LC-MS: [M+H]⁺ = 691.0.

### Intermediate 12: tert-butyl (1-(3-bromo-2-((6-((tert-butoxycarbonyl)amino)-9H-purin-9-yl)methyl)-5-chlorophenyl)-3-(hydroxymethyl)pyrrolidin-3-yl)carbamate

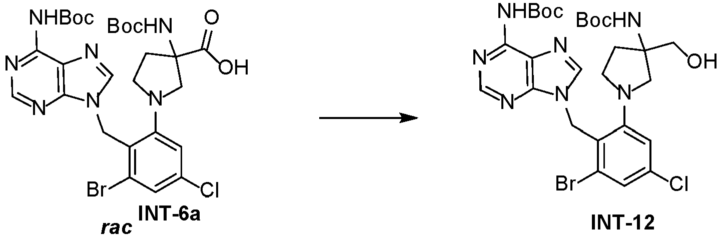

To a solution of 1-(3-bromo-2-((6-((tert-butoxycarbonyl)amino)-9H-purin-9-yl)methyl)-5-chlorophenyl)-3-((tert-butoxycarbonyl)amino)pyrrolidine-3-carboxylic acid (*rac* INT-6a) (300 mg, 0.45 mmol, 1 eq) and Et₃N (91 mg, 0.9 mmol, 2 eq) dissolved in THF (10 mL) were added in dropwisely ethyl carbonochloridate (58.6 mg, 0.54 mmol, 1.2 eq) over 10 minutes at 0 °C. After 10 min, the mixture was filtered off. The filtration was cooled to 0 °C. A solution of NaBH₄ (25.5 mg, 0.67 mmol, 1.5 eq) in 2 mL of water was added dropwisely over 30 min at 0 °C. The mixture was allowed to stir for 2 hours. The mixture was diluted with NH₄Cl (50 mL) and extracted with EtOAc (10 mL × 3). The combined organic layers were dried over Na₂SO₄, filtered off and concentrated to afford tert-butyl (1-(3-bromo-2-((6-((tert-butoxycarbonyl)amino)-9H-purin-9-yl)methyl)-5-chlorophenyl)-3-(hydroxymethyl)pyrrolidin-3-yl)carbamate as a colorless solid. LC-MS: [M+H]⁺ = 653.8.

### Intermediate 13: tert-butyl (1-(3-bromo-5-chloro-2-(hydroxymethyl)phenyl)-3-(methoxymethyl)pyrrolidin-3-yl)carbamate

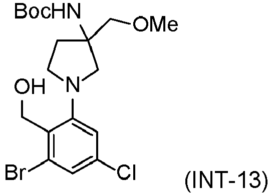

To a solution 1-benzyl 3-methyl 3-((tert-butoxycarbonyl)amino)pyrrolidine-1,3-dicarboxylate (1 g, 2.74 mmol, 1 eq) and N-methylmorpholine (555.2 mg, 5.5 mmol, 2 eq) dissolved in THF (20 mL) was added in a solution of Ethyl chloroformate (312.7 mg, 2.88 mmol, 1 eq) in THF (2 mL) dropwisely at 0 °C. The mixture was stirred for 10 min at 0 °C. The suspension was filtered. The filtrate was added in a solution of NaBH₄ (207.0 mg, 5.5 mmol, 2 eq) suspended in water (1 mL) slowly at 0 °C. The reaction was quenched with water (10 mL) after 0.5 hr. The aqueous phase was extracted with EtOAc. The combined organic layer was washed with brine, dried over Na₂SO₄, filtered and concentrated. The residue was purified by flash chromatograph eluting with 30% EtOAc in petroleum ether to afford benzyl 3-((tert-butoxycarbonyl)amino)-3-(hydroxymethyl)pyrrolidine-1-carboxylate as white solid. 1H NMR (400MHz CDCl₃): δ 7.43-7.29 (m, 5H), 5.14 (s, 2H), 4.90-4.75 (m, 1H), 3.85-3.68 (m, 2H), 3.60-3.50 (m, 4H), 2.26-1.96 (m, 2H), 1.70-1.59 (m, 1H), 1.44 (s, 9 H).

To a solution containing benzyl 3-((tert-butoxycarbonyl)amino)-3-(hydroxymethyl)pyrrolidine-1-carboxylate (490 mg, 1.4 mmol, 1 eq.) and Mel (198 mg, 1.4 mmol, 1 eq.) in DMF (30 mL) was added in NaH (60 % in material oil, 112 mg, 2.8 mmol, 2 eq.) in portion. The mixture was stirred at 27-33 °C for 3 hours. The mixture was treated with water (50 mL) and was extracted with EtOAc. The organic layer was washed with brine, dried over Na₂SO₄, filtered and concentrated under vacuum. The residue was purified by flash column (20% EtOAc in PE) to afford benzyl 3-((tert-butoxycarbonyl)amino)-3-(methoxymethyl)pyrrolidine-1-carboxylate as a colorless gum.¹H NMR (400 MHz, CDCl₃) δ 7.27-7.40 (m, 5H), 5.12 (s, 2H), 4.73 (br s, 1H), 3.58-3.68 (m, 1H), 3.42-3.57 (m, 5H), 3.36 (s, 3H), 2.17-2.44 (m, 1H), 1.89-2.07 (m, 1H), 1.42 (s, 9H).

To a solution of benzyl 3-((tert-butoxycarbonyl)amino)-3-(methoxymethyl)pyrrolidine-1-carboxylate (156 mg, 0.428 mmol) dissolved in MeOH (50 mL) was added in a suspension of Pd/C (wet, 10% Pd, 100 mg) suspended in MeOH (20 mL) under N₂. The mixture was purged with H₂ and was stirred at 27-34 °C for 18 hours under H₂ (50 psi. The mixture was filtered and the filtrate was concentrated under vacuum to give tert-butyl (3-(methoxymethyl)pyrrolidin-3-yl)carbamate as a yellow oil. ¹H NMR (400 MHz, CDCl₃) δ 4.95 (br s, 1H), 3.42 (s, 2H), 3.36 (s, 3H), 3.05-3.17 (m, 2H), 2.75-2.87 (m, 2H), 2.23 (br s, 1H), 1.95-2.04 (m, 1H), 1.78-1.85 (m, 1H), 1.42 (s, 9H).

To a solution of tert-butyl (3-(methoxymethyl)pyrrolidin-3-yl)carbamate (96 mg, 0.417 mmol, 1 eq.) and 2-bromo-4-chloro-6-fluorobenzaldehyde (Apollo Scientific) (99 mg, 0.42 mmol, 1 eq.) dissolved in CH₃CN (10 mL) wa added in K₂CO₃ (116 mg, 0.83 mmol, 2 eq.). The reaction mixture was heated and stirred at 70 °C for 18 hours. The mixture was cooled to room temperature and concentrated under vacuum. The residue was dissolved in CH₂Cl₂ (20 mL), filtered and the filtrate was concentrated to give tert-butyl (1-(3-bromo-5-chloro-2-formylphenyl)-3-(methoxymethyl)pyrrolidin-3-yl)carbamate as a yellow gum which was used for next step directly. LC-MS: [M+H]⁺ = 449.0.

To a solution of tert-butyl (1-(3-bromo-5-chloro-2-formylphenyl)-3-(methoxymethyl)pyrrolidin-3-yl)carbamate (190 mg, crude, 0.42 mmol, 1 eq.) in THF (10 mL) was added slowly in a solution of NaBH₄ (48 mg, 1.2 mmol, 3 eq.) suspended in H₂O (1 mL) under ice-water bath. The reaction mixture was stirred at 0-5 °C for 0.5 hours. The mixture was treated with aq. NH₄Cl (10 mL) and was extracted with EtOAc. The organic layer was dried over Na₂SO₄, filtered and concentrated under vacuum. The residue was purified by flash column (20% EtOAc in PE) to afford tert-butyl (1-(3-bromo-5-chloro-2-(hydroxymethyl)phenyl)-3-(methoxymethyl)pyrrolidin-3-yl)carbamate as colorless gum. LC-MS: [M+H]⁺ = 450.9.

### Intermediate 14: methyl (R)-1-(5-bromo-4-fluoro-6-(hydroxymethyl)-2,3-dihydrobenzofuran-7-yl)-3-((tert-butoxycarbonyl)amino)pyrrolidine-3-carboxylate

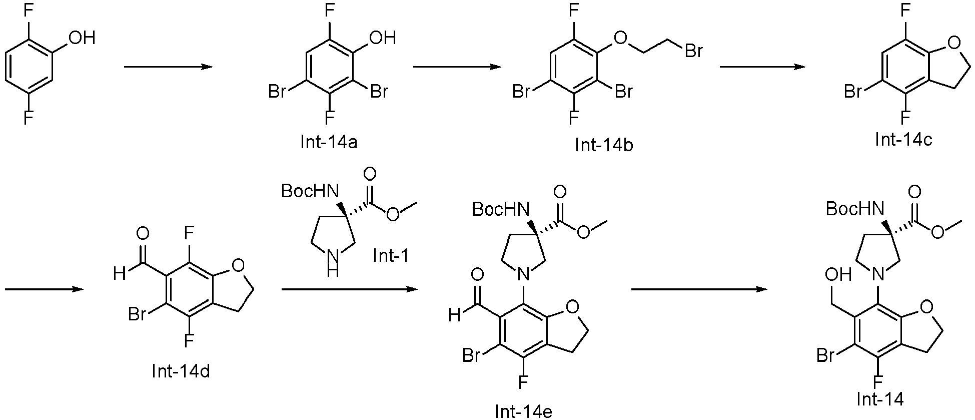

At -40°C, to a solution of 2,5-difluorophenol (2.5 g, 19.2 mmol) and isopropylamine (3.3 mL, 38.4 mmol) in THF (50 mL) was added NBS (7.2 g, 40 mmol). The reaction mixture was warmed up to r.t. and stirred for 2hr. The reaction mixture was diluted with ether, washed with 1N HCl aq. solution and brine, dried, filtered and concentrated. The crude product was purified by flash chromatography (silica gel, 0-30% EtOAc in hexanes) to afford 2,4-dibromo-3,6-difluorophenol (INT-14a) as light brown oil. LC-MS: [M-2/M]⁻ = 284.7/286.7.

A mixture of 2,4-dibromo-3,6-difluorophenol (1 g, 3.47 mmol), 1,2-dibromoethane (0.6 mL, 7 mmol) and potassium carbonate (0.96 g, 7 mmol) in MeCN (10 mL) was stirred at 80°C for 3hr. The reaction mixture was partitioned between EtOAc and water. The organic layer was washed with brine, dried, filtered and concentrated. The crude product was purified by flash chromatography (silica gel, 100% hexanes) to afford 1,3-dibromo-4-(2-bromoethoxy)-2,5-difluorobenzene (INT-14b) as colorless oil. ¹H NMR (400 MHz, DMSO-*d*6) δ 7.95 (dd, *J* = 10.5, 6.7 Hz, 1H), 4.51 - 4.40 (m, 2H), 3.85 - 3.75 (m, 2H).

At -78°C, to a solution of 1,3-dibromo-4-(2-bromoethoxy)-2,5-difluorobenzene (580 mg, 1.469 mmol) in THF (10 mL) was added *n*-butyllithium (2N, 0.88 mL, 1.8 mmol) and the resulting mixture was stirred at -78°C for 2hr. The reaction mixture was warmed up to r.t. and quenched with sat. NH₄Cl aq. solution and extracted with EtOAc. The organic layer was washed with brine, dried, filtered and concentrated. The crude product was purified by flash chromatography (silica gel, 100% hexanes) to afford 5-bromo-4,7-difluoro-2,3-dihydrobenzofuran (INT-14c) as colorless oil. ¹H NMR (400 MHz, DMSO-*d*6) δ 7.58 - 7.48 (m, 1H), 4.75 (t, *J* = 8.7 Hz, 2H), 3.35 (t, *J* = 8.8 Hz, 2H).

At -78°C, to a solution of 5-bromo-4,7-difluoro-2,3-dihydrobenzofuran (100 mg, 0.4 mmol) in THF (3 mL) was added LDA (0.25 mL, 0.5 mmol) and the resulting mixture was stirred at -78^{O}C for 1hr, followed by the addition of DMF (0.05 mL, 0.6 mmol). The reaction mixture was warmed up to r.t. and stirred for 30min. The reaction mixture was quenched with sat. NH₄Cl aq. solution and extracted with EtOAc. The organic layer was washed with brine, dried, filtered and concentrated. The crude product was purified by flash chromatography (silica gel, 0-10% EtOAc in hexanes) to afford 5-bromo-4,7-difluoro-2,3-dihydrobenzofuran-6-carbaldehyde (INT-14d) as white solid. ¹H NMR (400 MHz, DMSO-*d*6) δ 10.14 (d, *J* = 0.9 Hz, 1H), 4.81 (t, *J* = 8.8 Hz, 2H), 3.49 - 3.41 (m, 2H).

A mixture of 5-bromo-4,7-difluoro-2,3-dihydrobenzofuran-6-carbaldehyde (600 mg, 2.281 mmol), (R)-methyl 3-((tert-butoxycarbonyl)amino)pyrrolidine-3-carboxylate (724 mg, 3 mmol) and potassium carbonate (631 mg, 4.56 mmol) in toluene (10 mL) was stirred at 110°C overnight. The reaction mixture was partitioned between EtOAc and water. The organic layer was washed with brine, dried, filtered and concentrated. The crude product was purified by flash chromatography (silica gel, 0-30% EtOAc in hexanes) to afford methyl (*R*)-1-(5-bromo-4-fluoro-6-formyl-2,3-dihydrobenzofuran-7-yl)-3-((*tert*-butoxycarbonyl)amino)pyrrolidine-3-carboxylate (INT-14e) as bright yellow solid. LC-MS: [M/M+2]⁺ = 486.8/488.8.

At 0^{O}C, a mixture of methyl (*R*)-1-(5-bromo-4-fluoro-6-formyl-2,3-dihydrobenzofuran-7-yl)-3-((*tert*-butoxycarbonyl)amino)pyrrolidine-3-carboxylate (400 mg, 0.8 mmol) and sodium borohydride (31 mg, 0.8 mmol) in MeOH (10 mL) was stirred for 30min. The reaction mixture was quenched with sat. NaHCO₃ aq. solution and extracted with EtOAc. The organic layer was washed with brine, dried, filtered and concentrated to give methyl (*R*)-1-(5-bromo-4-fluoro-6-(hydroxymethyl)-2,3-dihydrobenzofuran-7-yl)-3-((*tert*-butoxycarbonyl)amino)pyrrolidine-3-carboxylate. LC-MS: [M/M+2]⁺ = 488.9/490.9.

### Intermediate 15: methyl 1-(3-bromo-2-(hydroxymethyl)-6-methoxyphenyl)-3-((tert-butoxycar bonyl)amino)pyrrolidine-3-carboxylate

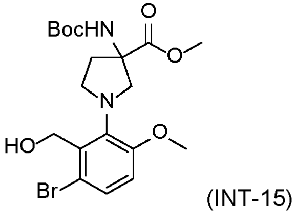

A mixture of 6-bromo-2-fluoro-3-methoxybenzaldehyde (Apollo Scientific) (300 mg, 1.3 mmol), methyl 3-((tert-butoxycarbonyl)amino)pyrrolidine-3-carboxylate (377 mg, 1.5 mmol) and potassium carbonate (356 mg, 2.6 mmol) in DMF (10 mL) was stirred at 85 ^{O}C overnight. The reaction mixture was partitioned between EtOAc and water. The organic layer was washed with brine, dried, filtered and concentrated. The crude product was purified by flash chromatography (silica gel, 0-50% EtOAc in hexanes) to afford methyl 1-(3-bromo-2-formyl-6-methoxyphenyl)-3-((tert-butoxycarbonyl)amino) pyrrolidine-3-carboxylate as a bright yellow oil. LC-MS: [M/M+2]⁺ = 456.9/458.9.

A mixture of methyl 1-(3-bromo-2-formyl-6-methoxyphenyl)-3-((tert-butoxycarbonyl)amino) pyrrolidine-3-carboxylate (210 mg, 0.5 mmol) and sodium borohydride (25 mg, 0.66 mmol) in MeOH (10 mL) was stirred at 0°C for 30min. The reaction mixture was quenched with sat. NaHCO₃ aq. solution and extracted with EtOAc. The organic layer was washed with brine, dried, filtered and concentrated. LC-MS: [M/M+2]⁺ = 458.9.460.9.

The following intermediates were prepared from tert-butyl N-[(tert-butoxy)carbonyl]-N-(9H-purin-6-yl)carbamate (INT-4) and the corresponding starting material, following procedures analogous to Intermediate 5.

| INT | Starting Material | Structure | | |
|---|---|---|---|---|
| INT -16 | 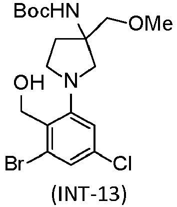 | 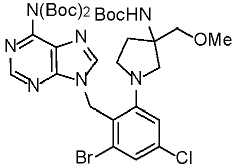 | tert-butyl (9-(2-bromo-6-(3-((tert-butoxycarbonyl)amino)-3-(methoxymethyl)pyrrolidin-1-yl)-4-chlorobenzyl)-9H-purin-6-yl)(tert-butoxycarbonyl)carbamate | LC-MS: |
| | | | | [M+H]⁺ = 790.2. |
| INT -17 | 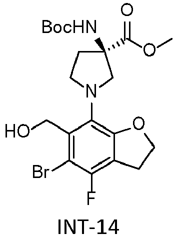 | 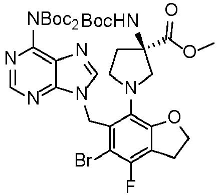 | methyl (R)-1-(6-((6-(bis(*tert-*butoxycarbonyl)amino)-9*H-*purin-9-yl)methyl)-5-bromo-4-fluoro-2,3-dihydrobenzofuran-7-yl)-3-((tert-butoxycarbonyl)amino)pyrr olidine-3-carboxylate | LC-MS: |
| | | | | [M/M+2]⁺ = 805.9/807. 9. |
| INT -18 | 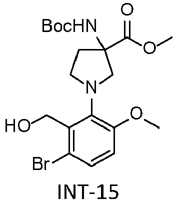 | 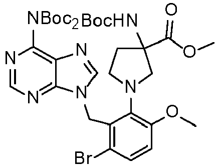 | methyl 1-(2-((6-(bis(*tert-*butoxycarbonyl)amino)-9*H-*purin-9-yl)methyl)-3-bromo-6-methoxyphenyl)-3-((tert-butoxycarbonyl)amino)pyrr olidine-3-carboxylate | LC-MS: |
| | | | | [M/M+2]⁺ = 775.9/777. 9. |

### Intermediate 19: tert-butyl ((3R)-1-(2-((6-((tert-butoxycarbonyl)amino)-9H-purin-9-yl)methyl)-5-chloro-3-(2,2,2-trifluoro-1-hydroxyethyl)phenyl)-3-(cyclopropylcarbamoyl)pyrrolidin-3-yl)carbamate

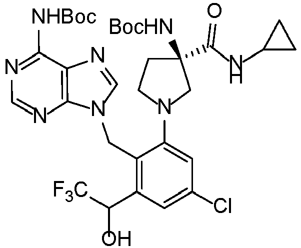

To a solution containing Pd(dppf)Cl₂.DCM (0.46 g, 0.56 mmol), tert-butyl (R)-(1-(3-bromo-2-((6-((tert-butoxycarbonyl)amino)-9H-purin-9-yl)methyl)-5-chlorophenyl)-3-(cyclopropylcarbamoyl)pyrrolidin-3-yl)carbamate (INT-6) (4 g, 5.6 mmol) in toluene (20 ml) and EtOH (20 ml.) was added in Et₃N ( 1.1 g, 11.3 mmol) under N₂ atmosphere. The mixture was heated to 55 °C at 50 psi under CO atmosphere for 36 h. The mixture was filtered and the filtrate was concentrated. The residue was purified by silica gel column (PE/EtOAc/MeOH = 15/15/1) to afford methyl (R)-3-(3-((tert-butoxycarbonyl)amino)-3-(cyclopropylcarbamoyl)pyrrolidin-1-yl)-2-((6-((tert-butoxycarbonyl)amino)-9H-purin-9-yl)methyl)-5-chlorobenzoate as a yellow solid. LC-MS: [M+H]⁺ = 699.3.

To a solution of methyl (R)-3-(3-((tert-butoxycarbonyl)amino)-3-(cyclopropylcarbamoyl)pyrrolidin-1-yl)-2-((6-((tert-butoxycarbonyl)amino)-9H-purin-9-yl)methyl)-5-chlorobenzoate (2.6 g, 3.7 mmol) dissolved in MeOH (50 mL) was added in NaBH₄ (5.6 g, 148.7 mmol) in portion at 20 °C. The mixture was stirred at 20 °C for 5 h. The mixture was diluted with water (150 mL) and was extracted with EtOAc (150 mL × 2). The organic layers were concentrated. The residue was purified by silica gel column (PE/EtOAc/MeOH = 15/15/1) to afford tert-butyl (R)-(1-(2-((6-((tert-butoxycarbonyl)amino)-9H-purin-9-yl)methyl)-5-chloro-3-(hydroxymethyl)phenyl)-3-(cyclopropylcarbamoyl)pyrrolidin-3-yl)carbamate as a yellow solid. LC-MS: [M+H]⁺ = 657.3.

To a solution of tert-butyl (R)-(1-(2-((6-((tert-butoxycarbonyl)amino)-9H-purin-9-yl)methyl)-5-chloro-3-(hydroxymethyl)phenyl)-3-(cyclopropylcarbamoyl)pyrrolidin-3-yl)carbamate (1 g, 1.5 mmol) dissolved in EtOAc (15 mL) was added in 2-iodoxybenzoic acid (1.7 g, 6 mmol) . The mixture was stirred at 40 °C for 12 h. The mixture was filtered and the filtrate was concentrated in vacuo. The residue was purified by medium pressure liquid chromatography (PE/EtOAc/MeOH = 15/15/1) to afford tert-butyl (R)-(1-(2-((6-((tert-butoxycarbonyl)amino)-9H-purin-9-yl)methyl)-5-chloro-3-formylphenyl)-3-(cyclopropylcarbamoyl)pyrrolidin-3-yl)carbamate as a yellow solid. ¹H NMR: (400MHz DMSO-d₆) *δ* 10.32 (s, 1H), 9.98 (s, 1H), 8.51 (s, 1H), 8.18 (s, 1H), 7.63 (m, 1H), 7.59 (m, 1H), 7.49 (m, 1H), 7.24 (m, 1H), 5.81-5.72 (m, 2H), 3.85-3.79 (m, 1H), 3.24-3.21 (m, 1H), 3.08-3.05 (m, 2H), 2.59-2.56 (m, 1H), 2.20-2.19 (m, 1H), 2.11 (m, 1H), 1.45 (s, 9H), 1.35 (s, 9H), 0.59-0.57 (m, 2H), 0.37 (m, 2H). LC-MS: [M+H]⁺ = 655.4.

To a solution containing tert-butyl (R)-(1-(2-((6-((tert-butoxycarbonyl)amino)-9H-purin-9-yl)methyl)-5-chloro-3-formylphenyl)-3-(cyclopropylcarbamoyl)pyrrolidin-3-yl)carbamate (400 mg, 0.6 mmol), trifluoromethyltrimethylsilane (634 mg, 6 mmol) dissolved in THF (4 mL) was added in TBAF (0.018 mL, 0.018 mmol, 1M in THF), the mixture was stirred at 25°C for 2h. The mixture solution was concentrated. The residue was purified together by Pre-HPLC (column: Phenomenex Synergi Max-RP 250*50*10um, condition:water(10mM NH₄HCO₃)-ACN) to give tert-butyl ((3R)-1-(2-((6-((tert-butoxycarbonyl)amino)-9H-purin-9-yl)methyl)-5-chloro-3-(2,2,2-trifluoro-1-hydroxyethyl)phenyl)-3-(cyclopropylcarbamoyl)pyrrolidin-3-yl)carbamate as a white solid. ¹H NMR: (400 MHz, DMSO-*d*₆) *δ* = 8.60-8.59 (m, 1H), 8.20 (s, 1H), 7.69-7.63 (m, 1H), 7.36-7.33 (m, 3H), 7.06-7.05 (m, 1H), 6.09-5.95 (m, 1H), 5.67-5.56 (m, 1H), 5.48-5.41 (m, 1H), 3.25-3.15 (m, 1.75H), 2.99-2.90 (m, 1.75H), 2.30-2.26 (m, 1H), 2.16-2.07 (m, 2.38H), 1.45 (s, 9H), 1.35 (s, 9H), 0.59-0.58 (m, 2H), 0.38 (m, 2H). LC-MS: [M+H]⁺ = 725.4.

### Intermediate 20: tert-butyl (R)-(9-((5-bromo-7-(3-((tert-butoxycarbonyl)amino)-3-(cyclopropyl carbamoyl)pyrrolidin-1-yl)-4-fluoro-2,3-dihydrobenzofuran-6-yl)methyl)-9H-purin-6-yl)(tert-butoxycarbonyl)carbamate

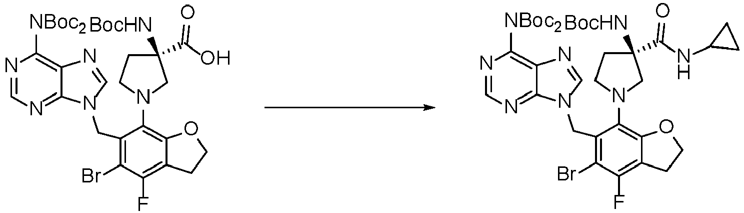

A mixture of methyl (*R*)-1-(6-((6-(bis(tert-butoxycarbonyl)amino)-9*H*-purin-9-yl)methyl)-5-bromo-4-fluoro-2,3-dihydrobenzofuran-7-yl)-3-((tert-butoxycarbonyl)amino)pyrrolidine-3-carboxylate (INT-17) (270 mg, 0.33 mmol) in lithium hydroxide (1N, 4 mL, 4 mmol) and THF (6 mL) was stirred at r.t. overnight. The reaction mixture was acidified to pH 3-4 with 1N HCl and extracted with EtOAc. The organic layer was washed with brine, dried, filtered and concentrated to give (*R*)-1-(6-((6-(bis(*tert*-butoxycarbonyl)amino)-9*H*-purin-9-yl)methyl)-5-bromo-4-fluoro-2,3-dihydrobenzofuran-7-yl)-3-((*tert*-butoxycarbonyl)amino)pyrrolidine-3-carboxylic acid. LC-MS: [M/M+2]⁺ = 791.8/793.8.

A mixture of (R)-1-(6-((6-(bis(tert-butoxycarbonyl)amino)-9H-purin-9-yl)methyl)-5-bromo-4-fluoro-2,3-dihydrobenzofuran-7-yl)-3-((tert-butoxycarbonyl)amino)pyrrolidine-3-carboxylic acid (265 mg, 0.335 mmol), cyclopropylamine (0.035 mL, 0.5 mmol), propanephosphonic acid anhydride (or propylphosphonic anhydride, T3P, 50% solution in EtOAc, 426 mg, 0.7 mmol) and DIEA (0.35 mL, 2 mmol) in EtOAc (6 mL) was stirred at r.t. for 3hr. The reaction mixture was diluted with EtOAc, washed with sat. NaHCO₃ aq. solution, citric acid aq. solution and brine, dried, filtered and concentrated to give *tert*-butyl (*R*)-(9-((5-bromo-7-(3-((tert-butoxycarbonyl)amino)-3-(cyclopropyl carbamoyl)pyrrolidin-1-yl)-4-fluoro-2,3-dihydrobenzofuran-6-yl)methyl)-9*H*-purin-6-yl)(*tert*-butoxycarbonyl)carbamate. LC-MS: [M/M+2]⁺ = 830.8/832.8.

### Intermediate 21: tert-butyl (9-(6-bromo-2-(3-((tert-butoxycarbonyl)amino)-3-(cyclopropylcarba moyl)pyrrolidin-1-yl)-3-methoxybenzyl)-9H-purin-6-yl)carbamate

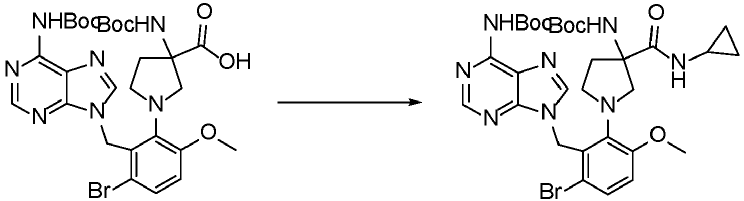

Tert-butyl (9-(6-bromo-2-(3-((*tert*-butoxycarbonyl)amino)-3-(cyclopropylcarbamoyl)pyrrolidin-1-yl)-3-methoxybenzyl)-9*H*-purin-6-yl)carbamate was prepared following procedures analogous to Intermediate 20. LC-MS: [M/M+2]⁺ = 700.8/702.8.

### Intermediate 22: 1-(3-bromo-2-((6-((tert-butoxycarbonyl)amino)-9H-purin-9-yl)methyl)-4-(trifluoromethyl)phenyl)-3-((tert-butoxycarbonyl)amino)pyrrolidine-3-carboxylic acid

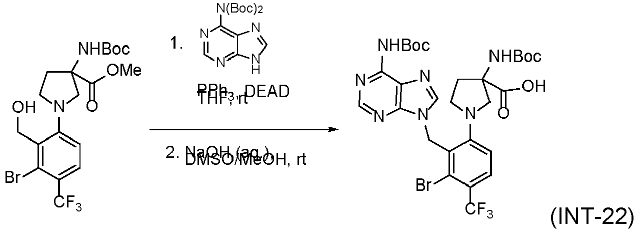

To a THF solution (25 mL) of methyl 1-(3-bromo-2-(hydroxymethyl)-4-(trifluoromethyl)phenyl)-3-((*tert*-butoxycarbonyl)amino)pyrrolidine-3-carboxylate (INT-2) (1 g, 2 mmol), Ph₃P (1.54 g, 5.87 mmol) and tert-butyl N-[(tert-butoxy)carbonyl]-N-(9H-purin-6-yl)carbamate (INT-4) (0.8 g, 2.3 mmol) was added DEAD (929 uL, 5.9 mmol) at rt. 1 hr later, the reaction was concentrated under vaccum to give the crude product. The crude product was purified by combiflash (10% to 50% EA in hexanes) to give a brown syrup, which was then redissolved in DMSO/MeOH (16 mL, 5:3). To the reaction mixture was added 3 N NaOH solution (6.0 mL, 18.1 mmol) at rt. After stirring for 2 hrs, the reaction was acidified with 1 N HCl, diluted with EA, washed with 1 N HCl, water, and brine. The organic layer was dried over Na₂SO₄, and concentrated under vacuum to give a foam like crude product 1-(3-bromo-2-((6-((*tert*-butoxycarbonyl)amino)-9*H*-purin-9-yl)methyl)-4-(trifluoromethyl)phenyl)-3-((*tert-*butoxycarbonyl)amino)pyrrolidine-3-carboxylic acid. LC-MS: [M+H]⁺ = 701.6.

### Intermediate 23: methyl 1-(6-bromo-5-formyl-2,3-dihydro-1H-inden-4-yl)-3-((tert-butoxycarbonyl)amino)pyrrolidine-3-carboxylate

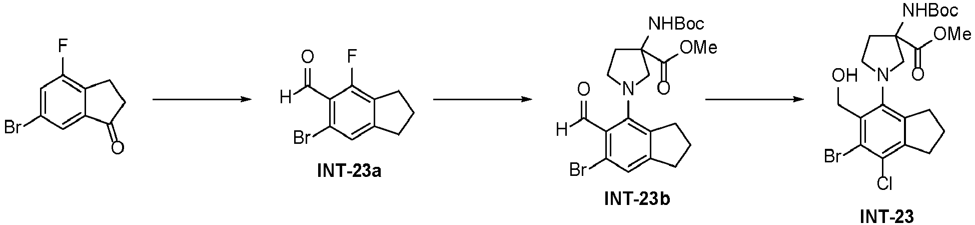

To a TFA solution (14 mL) of 6-bromo-4-fluoro-2,3-dihydro-1H-inden-1-one (800 mg, 3.5 mmol) was added triethylsilane (1.4 mL, 8.7 mmol) at rt. After stirring overnight, the reaction mixture was poured into ice, and extracted with EA. The organic layer was washed with water, saturated NaHCO₃ solution and brine. The organic layer was then dried over Na₂SO₄, and concentrated under vacuum to give the crude product. The crude product was passed through a silica gel column and eluted with Hexane. After concentrated under vacuum, the crude product was redissolved in THF (12 mL) and treated with 2 M LDA solution (2.8 mL, 5.6 mmol) at -78 °C under N₂. The reaction mixture was stirred at this temperature for 30 min, followed by addition of DMF (0.6 mL, 7.4 mmol) dropwise. The reaction mixture was stirred for another 1 hr at -78 °C before warming up to 0 °C. The reaction mixture was then quenched with 0.5 N HCl solution at 0 °C and then warm up to rt. The reaction mixture was diluted with EA, washed with 1N HCl, brine, dried over Na₂SO₄ and concentrated under vacuum to give the crude product. The crude product was purified by flash chromatography (5% to 30% EA in Hex) to give 6-bromo-4-fluoro-2,3-dihydro-1*H*-indene-5-carbaldehyde as an off-white solid. ¹H NMR (400 MHz, DMSO-*d*₆) δ 10.18 (d, *J* = 0.73 Hz, 1H), 7.53 (s, 1H), 2.97 (t, *J* = 7.58 Hz, 2H), 2.88 (t, *J* = 7.52 Hz, 2H), 2.03 - 2.16 (m, 2H).

To a toluene solution (10 mL) of 6-bromo-4-fluoro-2,3-dihydro-1*H*-indene-5-carbaldehyde (INT-23a) (520 mg, 2.1 mmol) and methyl-3-((tert-butoxycarbonyl)amino)pyrrolidine-3-carboxylate (*rac* INT-1) (627 mg, 2.57 mmol) was added K₂CO₃ (443 mg, 3.2 mmol) at rt. The reaction tube was submerged to the pre-heated oil bath (110 °C). The reaction was stirred overnight. The reaction mixture was diluted with EA, washed with water, and brine. The organic layer was dried over Na₂SO₄, concentrated under vacuum to give the crude product. The crude product was purified by combiflash (10% to 50% EA in Hex) to give methyl 1-(6-bromo-5-formyl-2,3-dihydro-1*H*-inden-4-yl)-3-((*tert*-butoxycarbonyl)amino)pyrrolidine-3-carboxylate (INT-23b) as a yellow foam. ¹H NMR (400 MHz, DMSO-*d*₆): δ 10.06 (s, 1H), 7.75 (s, 1H), 7.17 (s, 1H), 3.77 (d, *J* = 10.5 Hz, 1H), 3.63 (s, 3H), 3.48 - 3.57 (m, 1H), 3.40 (td, *J* = 8.6, 4.5 Hz, 1H), 3.31 (s, 1H), 3.01 (br t, *J* = 7.2 Hz, 2H), 2.83 (t, *J* = 7.6 Hz, 2H), 2.15 - 2.34 (m, 2H), 1.94 - 2.07 (m, 2H), 1.37 (s, 9H). LC-MS: [M+H]⁺ = 467.1.

To a CH₃CN (12 mL) solution of methyl 1-(6-bromo-5-formyl-2,3-dihydro-1*H*-inden-4-yl)-3-((*tert*-butoxycarbonyl)amino)pyrrolidine-3-carboxylate (INT-23b) (500 mg, 0.11 mmol) was added Palau'Chlor^{®} (235 mg, 1.12 mmol) at rt. After stirring for 1 hr, the reaction was quenched with water, and extracted with EA. The organic layer was dried over Na₂SO₄, and concentrated under vacuum to give the crude product. The crude product was then dissolved in THF (12 mL), followed by addition of NaBH₄ (61 mg, 1.6 mmol) at 0 °C. The reaction was stirred for 30 min until the yellow color faded. The reaction mixture was diluted with EA, washed with 1 N HCl, water, and brine. The organic layer was then dried over Na₂SO₄, and concentrated under vacuum to give the crude product. The crude product was purified by combiflash (5% to 30% EA in Hex) to give methyl 1-(6-bromo-5-formyl-2,3-dihydro-1*H*-inden-4-yl)-3-((*tert-*butoxycarbonyl)amino)pyrrolidine-3-carboxylate. LC-MS: [M+H]⁺ = 503.1.

### Intermediate 24 and 25: methyl 1-(3-bromo-5,6-dichloro-2-(hydroxymethyl)phenyl)-3-((tert-butoxycarbonyl)amino)pyrrolidine-3-carboxylate (INT-24) and methyl 1-(3-bromo-4,5,6-trichloro-2-(hydroxymethyl)phenyl)-3-((tert-butoxycarbonyl)amino)pyrrolidine-3-carboxylate (INT-25)

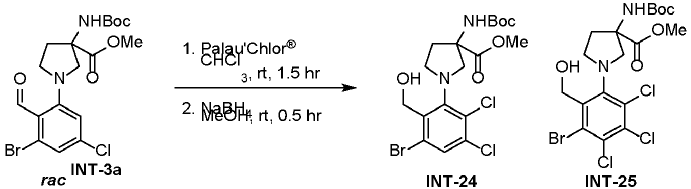

To a solution of methyl-1-(3-bromo-5-chloro-2-formylphenyl)-3-((tert-butoxycarbonyl)amino)pyrrolidine-3-carboxylate (rac INT-3a) (480 mg, 1 mmol) in CHCl₃ (8 mL) was added Palau'Chlor^{®} (233 mg, 1.1 mmol). The mixture was stirred at room temperature for 1.5 hrs. The reaction mixture was quenced with a few drops of water and concentrated under vacuum to give the crude product. The crude product was dissolved in MeOH (15 mL) and treated with NaBH₄ (27 mg, 1.5 mmol) at rt. The mixture was stirred at room temperature for 0.5 hr. The solvent was removed under vacuum. The residue was diluted with EA, washed with 1 N HCl, and brine. The organic layer was dried over Na₂SO₄ and concentrated to give the crude product. The crude product was purified by flash chromatography (5% to 35% EA in Hex) to give methyl 1-(3-bromo-5,6-dichloro-2-(hydroxymethyl)phenyl)-3-((tert-butoxycarbonyl)amino)pyrrolidine-3-carboxylate (INT-24) and methyl 1-(3-bromo-4,5,6-trichloro-2-(hydroxymethyl)phenyl)-3-((tert-butoxycarbonyl)amino)pyrrolidine-3-carboxylate (INT-25) as a mixture. INT-24: LC-MS: [M+H]⁺ = 512.7 (Rt = 4.021 min). INT-25:; LC-MS: [M+H]⁺ = 546.6 (Rt = 4.115 min).

### Intermediate 26: tert-butyl (R)-(9-(6-(3-((tert-butoxycarbonyl)amino)-3-(cyclopropylcarbamoyl)pyrrolidin-1-yl)-2-((dimethylamino)methyl)-3-fluorobenzyl)-9H-purin-6-yl)carbamate

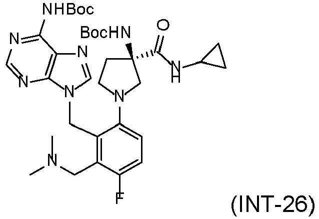

A mixture of methyl (R)-1-(2-((6-(bis(tert-butoxycarbonyl)amino)-9*H*-purin-9-yl)methyl)-3-bromo-4-fluorophenyl)-3-((*tert*-butoxycarbonyl)amino)pyrrolidine-3-carboxylate (prepared following procedures analogous to INT-5) (42 g, 55 mmol), potassium vinyltrifluoroborate (11 g, 82 mmol), Pd(dppf)Cl₂DCM (4.5 g, 5.5 mmol) and K₂CO₃ (22.8 g, 165 mmol) in dioxane/H₂O (420 mL/42 mL) was stirred at 100 _{°}C for 18 hrs under N₂. The reaction mixture was cooled to 25 _{°}C, diluted with H₂O (500 mL) and extracted with EA (1 L). The organic layer was washed with brine (500 mL), dried over Na₂SO₄, filtered and concentrated. The residue was purified by silica gel chromatography (PE/EA = 10/1 to pure EA) to afford methyl (*R*)-3-((tert-butoxycarbonyl)amino)-1-(2-((6-((tert-butoxycarbonyl)amino)-9*H*-purin-9-yl)methyl)-4-fluoro-3-vinylphenyl)pyrrolidine-3-carboxylate (INT-26aa) as yellow gum. ¹H NMR (400 MHz, CDCl₃): δ 8.92 (s, 1H), 7.92 (s, 1H), 7.28-7.24 ( m, 1H), 7.20-7.07 (m, 1H), 6.78-6.70 (m, 1H), 5.70-5.61 (m, 3H), 5.56-5.52 (m, 1H), 3.73 (s, 3H), 3.66 (d, *J* = 10.0 Hz, 1H), 3.21 (d, *J* = 9.2Hz, 1H), 3.140- 2.99 (m, 2H), 2.58- 2.44 (m, 1H), 2.04 -1.99 (m, 2H), 1.51-1.40 (m, 27H). LC-MS: [M+H]⁺ = 712.2.

To the solution of methyl (R)-3-((tert-butoxycarbonyl)amino)-1-(2-((6-((tert-butoxycarbonyl)amino)-9*H*-purin-9-yl)methyl)-4-fluoro-3-vinylphenyl)pyrrolidine-3-carboxylate (17 g, 23.9 mmol) in MeOH/THF (85 mU85 mL) was added aq.LiOH (48 mL, 2.5 M, 120 mmol) at 10 °C. After addition, the reaction mixture was stirred at 25 °C for 4 hrs. The mixture was diluted with H₂O (150 mL), adjusted pH = 3-4 by HCl (1N) and extracted with EA (300 mL × 2). The organic layer was washed with brine (500 mL), dried over Na₂SO₄, filtered and concentrated to give a crude product. To a mixture of the above crude product, cyclopropanamine (3.02 g, 52.8 mmol) and DIPEA (10 g, 79 mmol) in DMF (100 mL) was added HATU (20 g, 53 mmol). The mixture was stirred for 18 hrs at 25 °C. The reaction mixture was diluted with H₂O (200 mL) and extracted with EA (400 mL). The organic layer was washed with brine (600 mL × 2), dried over Na₂SO₄, filtered and concentrated. The residue was purified by silica gel chromatography (PE/EA = 10/1 to pure EA) to afford *tert*-butyl (*R*)-(9-(6-(3-((tert-butoxycarbonyl)amino)-3-(cyclopropylcarbamoyl)pyrrolidin-1-yl)-3-fluoro-2-vinylbenzyl)-9*H-*purin-6-yl)carbamate as a yellow solid. ¹H NMR (400 MHz, DMSO-*d⁶*): δ = 9.98 (s, 1H), 8.57 (s, 1H), 8.10 (s, 1H), 7.57 (s, 1H), 7.34-7.17 (m, 3H), 6.83-6.75(m, 1H), 5.64-5.37 (m, 4H), 3.68 (d, *J* = 9.6 Hz, 1H), 3.10-2.98 (m, 2H), 2.96-2.90 (m, 1H), 2.57 (s, 1H), 2.29-2.18 (m, 1H), 2.04 (s, 1H), 1.46 (s, 9H), 1.32 (s, 9H), 0.55 (s, 2H), 0.35 (s, 2H). LC-MS: [M+H]⁺ = 637.2.

A mixture of *tert*-butyl (*R*)-(9-(6-(3-((tert-butoxycarbonyl)amino)-3-(cyclopropylcarbamoyl)pyrrolidin-1-yl)-3-fluoro-2-vinylbenzyl)-9H-purin-6-yl)carbamate (4.2 g, 6.6 mmol) and OsO₄ (336 mg, 1.32 mmol) in THF/H₂O (40 mL/8 mL) was stirred at 25 °C for 30 mins. NaIO₄ (7 g, 33 mmol) was added to the mixture and stirred at 25 °C for 4 hrs. The reaction mixture was diluted with EA (80 mL) and filtered. The filtrate was concentrated to give tert-butyl (*R*)-(9-(6-(3-((tert-butoxycarbonyl)amino)-3-(cyclopropylcarbamoyl)pyrrolidin-1-yl)-3-fluoro-2-formylbenzyl)-9*H*-purin-6-yl)carbamate (INT-26a) as a yellow solid. LC-MS: [M+H]⁺ = 639.2.

To a mixture of *tert*-butyl (*R*)-(9-(6-(3-((tert-butoxycarbonyl)amino)-3-(cyclopropylcarbamoyl)pyrrolidin-1-yl)-3-fluoro-2-formylbenzyl)-9*H*-purin-6-yl)carbamate (64 mg, 0.100 mmol), dimethylamine hydrochloride (41 mg, 0.5 mmol) in DCM (1 mL) was added triethylamine (0.035 mL, 0.25 mmol). The mixture was stirred at RT for 10 min then sodium triacetoxyborohydride (63.7 mg, 0.3 mmol) was added portionwised. The mixture was stirred for 30 min. More dimethylamine hydrochloride (41 mg, 0.5 mmol) was added followed by sodium triacetoxyborohydride (63.7 mg, 0.3 mmol). The mixture was stirred at RT overnight. MeOH and acetone was added to quench the reaction and the mixture was concentrated. The mixture was diluted with EA and NaHCO₃ (aq.) was added. The mixture was extracted with EA (x2). The combined organic layer was washed with brine, dried over Na₂SO₄, filtered and concentrated to give *tert-butyl (R*)-(9-(6-(3-((tert-butoxycarbonyl)amino)-3-(cyclopropylcarbamoyl)pyrrolidin-1-yl)-2-((dimethylamino)methyl)-3-fluorobenzyl)-9*H*-purin-6-yl)carbamate. LC-MS: [M+H]⁺ = 668.4

### Intermediate 27: tert-butyl (R)-(1-(2-((6-amino-9H-purin-9-yl)methyl)-4-fluoro-3-(((methoxycarbonyl)amino)methyl)phenyl)-3-(cyclopropylcarbamoyl)pyrrolidin-3-yl)carbamate

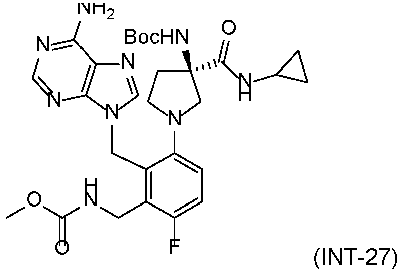

A mixture of *tert*-butyl (*R*)-(9-(6-(3-((tert-butoxycarbonyl)amino)-3-(cyclopropylcarbamoyl) pyrrolidin-1-yl)-3-fluoro-2-formyl benzyl)-9*H*-purin-6-yl)carbamate (INT-26a) (1.2 g, 1.9 mmol), HONH₂•HCl (261 mg, 3.76 mmol) and Py (297 mg, 3.8 mmol) in MeOH (12 mL) was reacted at 30 °C for 4 hrs. The reaction mixture was diluted with H₂O (30 mL) and extracted with EA (50 mL × 2). The organic layer was washed with brine (100 mL × 2), dried over Na₂SO₄, filtered and concentrated under vacuum to give *tert*-butyl *(R*,*E*)-(9-(6-(3-((tert-butoxycarbonyl)amino)-3-(cyclopropylcarbamoyl)pyrrolidin-1-yl)-3-fluoro-2-((hydroxyimino)methyl)benzyl)-9H-purin-6-yl)carbamate as a brown gum.LC-MS: [M+H]⁺ = 654.4.

A mixture of *tert*-butyl *(R*,*E*)-(9-(6-(3-((tert-butoxycarbonyl)amino)-3-(cyclopropylcarbamoyl)pyrrolidin-1-yl)-3-fluoro-2-((hydroxyimino)methyl)benzyl)-9H-purin-6-yl)carbamate (1 g, 1.6 mmol) and Zn (1 g, 1.6 mmol) in AcOH (10 mL) was stirred at 40°C for 18 hrs. The reaction mixture was filtered and concentrated. The residue was diluted with H₂O (20 mL) and adjusted pH = 8 by aq.Na₂CO₃. The mixture was extracted with EA (100 mL). LCMS showed that most of the product is in the aqueous. The aqueous was lyophilized to give a brown solid (2.2 g, crude, contain Na₂CO₃). The crude product (1.7 g, 2.6 mmol) was purified by prep-HPLC (base condition) to give *tert*-butyl (*R*)-(1-(2-((6-amino-9*H*-purin-9-yl)methyl)-3-(aminomethyl)-4-fluorophenyl)-3-(cyclopropylcarbamoyl)pyrrolidin-3-yl)carbamate as a yellow solid. LC-MS: [M+H]⁺ = 540.3.

To a solution of *tert*-butyl (*R*)-(1-(2-((6-amino-9*H*-purin-9-yl)methyl)-3-(aminomethyl)-4-fluorophenyl)-3-(cyclopropylcarbamoyl)pyrrolidin-3-yl)carbamate (150 mg, 0.28 mmol) and TEA (84 mg, 0.82 mmol) in DCM (1.5 mL) was added Methyl chloroformate (24 mg, 0.25 mmol) at 0 °C. After addition the reaction mixture was stirred at 20 °C for 18 hrs. The reaction mixture was concentrated. The residue was purified by prep-HPLC (Column: Phenomenex Synergi C18 150*25*10 um, gradient: 13-43% B (A = water (0.225% FA), B = ACN), flow rate: 25 mL/min) to give *tert*-butyl (*R*)-(1-(2-((6-amino-9H-purin-9-yl)methyl)-4-fluoro-3-(((methoxycarbonyl)amino)methyl)phenyl)-3-(cyclopropylcarbamoyl)pyrrolidin-3-yl)carbamate as a white solid. LC-MS: [M+H]⁺ = 598.4.

### Intermediate 28: (R)-3-((tert-butoxycarbonyl)amino)-1-(2-((6-((tert-butoxycarbonyl)amino)-9H-purin-9-yl)methyl)-4-fluoro-3-(methoxymethyl)phenyl)pyrrolidine-3-carboxylic acid

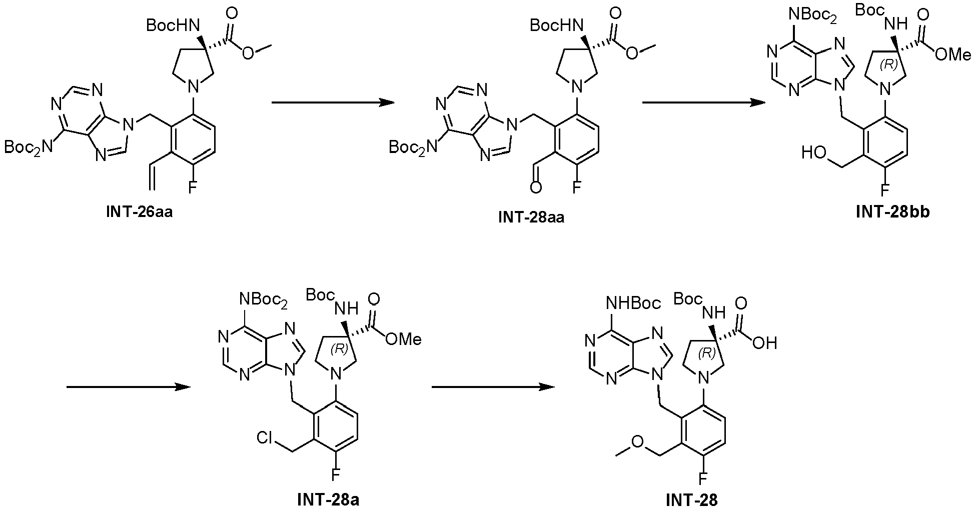

A mixture of methyl (*R*)-3-((tert-butoxycarbonyl)amino)-1-(2-((6-((tert-butoxycarbonyl)amino)-9*H*-purin-9-yl)methyl)-4-fluoro-3-vinylphenyl)pyrrolidine-3-carboxylate (INT-26aa) (2 g, 2.8 mmol) and OsO₄ (142 mg, 0.6 mmol) in THF/H₂O (20 mL/6 mL) was stirred at 25 °C for 30 mins. NaIO₄ (3.0 g, 14 mmol) was added and stirred at 25 °C for 18 hrs. The reaction mixture was diluted with EA (50 mL) and filtered. The filtrate was washed with aq.Na₂SO₃ (100 mL × 2), dried over Na₂SO₄, filtered and concentrated to give methyl (*R*)-1-(2-((6-(bis(tert-butoxycarbonyl)amino)-9*H*-purin-9-yl)methyl)-4-fluoro-3-formylphenyl)-3-((tert-butoxycarbonyl)amino)pyrrolidine-3-carboxylate (INT-28aa) as yellow gum. LC-MS: [M+H]⁺ = 714.2.

To a solution of methyl (*R*)-1-(2-((6-(bis(tert-butoxycarbonyl)amino)-9*H*-purin-9-yl)methyl)-4-fluoro-3-formylphenyl)-3-((tert-butoxycarbonyl)amino)pyrrolidine-3-carboxylate (1.8 g, 2.5 mmol) in THF (20 mL) was added aq.NaBH₄ (2.5 mL, 2 M, 5 mmol) dropwise at 5-10°C. After addition, the reaction mixture was stirred at 10 °C for 1 hr. The reaction mixture was quenched by ap.NH₄Cl (40 mL) and extracted with EA (100 mL). The combined organic layer was washed with brined (50 mL), dried over Na₂SO₄, filtered and concentrated. The residue was purified by COMBI-FLASH^{®} (50% EA in PE) to give methyl (*R*)-1-(2-((6-(bis(tert-butoxycarbonyl)amino)-9*H*-purin-9-yl)methyl)-4-fluoro-3-(hydroxymethyl)phenyl)-3-((tert-butoxycarbonyl)amino)pyrrolidine-3-carboxylate (INT-28bb) as a yellow solid. LC-MS: [M+H]⁺ = 716.3.

To a solution of methyl (*R*)-1-(2-((6-(bis(tert-butoxycarbonyl)amino)-9*H*-purin-9-yl)methyl)-4-fluoro-3-(hydroxymethyl)phenyl)-3-((tert-butoxycarbonyl)amino)pyrrolidine-3-carboxylate (100 mg, 0.07 mmol) in dry DCM (2 mL) under N₂ was added SOCl₂ (11 µ!, 0.15 mmol) at 0 °C. The mixture was stirred at 0 °C to RT for 1h The mixture was concentrated at low temperature to give methyl (*R*)-1-(2-((6-(bis(tert-butoxycarbonyl)amino)-9*H*-purin-9-yl)methyl)-3-(chloromethyl)-4-fluorophenyl)-3-((tert-butoxycarbonyl)amino)pyrrolidine-3-carboxylate (INT-28a) as a yellow oil and was used for the next step without purification. LC-MS: [M+H]⁺ = 734.3, 736.3.

To a suspension of methyl (*R*)-1-(2-((6-(bis(tert-butoxycarbonyl)amino)-9*H*-purin-9-yl)methyl)-3-(chloromethyl)-4-fluorophenyl)-3-((tert-butoxycarbonyl)amino)pyrrolidine-3-carboxylate (INT-28a) (103 mg, 0.14 mmol) in THF (0.9 mL) at 0 °C was added sodium methanolate (5.4 M in MeOH) (0.8 mL, 4.2 mmol). The suspension was stirred at 0 °C to RT overnight. The mixture was cooled to 0 °C and quenched by water (~1 ml). The mixture was then acidified by adding 1M HCl dropwised at 0 °C to pH ~2-3 and the resulting mixture was extracted with EA (X3). The organic layer was washed with water, brine and dried over Na₂SO₄. The mixture was concentrated to afford (*R*)-3-((tert-butoxycarbonyl)amino)-1-(2-((6-((tert-butoxycarbonyl)amino)-9*H*-purin-9-yl)methyl)-4-fluoro-3-(methoxymethyl)phenyl)pyrrolidine-3-carboxylic acid. LC-MS: [M+H]⁺ = 616.3.

### Intermediate 29. methyl (R)-1-(2-((6-(bis(tert-butoxycarbonyl)amino)-9H-purin-9-yl)methyl)-3-cyano-4-fluorophenyl)-3-((tert-butoxycarbonyl)amino)pyrrolidine-3-carboxylate

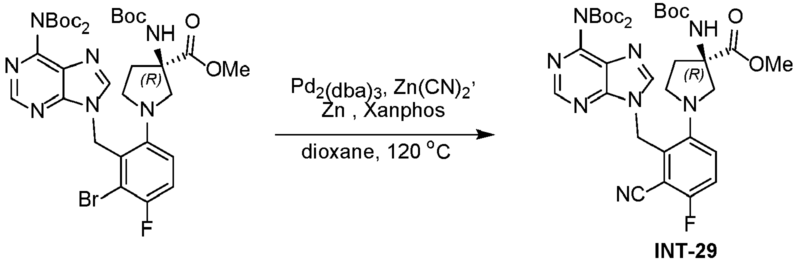

To a mixture of methyl (*R*)-1-(2-((6-(bis(tert-butoxycarbonyl)amino)-9*H*-purin-9-yl)methyl)-3-bromo-4-fluorophenyl)-3-((*tert*-butoxycarbonyl)amino)pyrrolidine-3-carboxylate (prepared following procedures analogous to INT-5) (400 mg, 0.5 mmol) in dioxane (4 mL) was added Zn(CN)₂ (245 mg, 2.1 mmol), Zn (10 mg ,0.1 mmol), Xanphos (61 mg,0.1 mmol) and Pd₂(dba)₃ (96 mg, 0.1 mmol) under N₂. The reaction was stirred at 120 °C for 2 h microwave. The mixture was poured into water (30 mL) and extracted with EA (20 mL× 3) and then dried over Na₂SO₄, filtered and concentrated. The residue was purified by column chromatography (PE/EA = 3/1) to give methyl (R)-1-(2-((6-(bis(tert-butoxycarbonyl)amino)-9H-purin-9-yl)methyl)-3-cyano-4-fluorophenyl)-3-((tert-butoxycarbonyl)amino)pyrrolidine-3-carboxylate as yellow oil. ¹H NMR (400 MHz, Methanol-d₄) δ 8.79 (s, 1H), 8.53 (s, 1H), 7.70 (dd, *J* = 9.2, 5.1 Hz, 1H), 7.35 (t, *J* = 8.8 Hz, 1H), 5.83 (d, *J* = 2.5 Hz, 2H), 3.83 (d, *J* = 10.0 Hz, 1H), 3.73 (s, 3H), 3.27 (d, *J* = 10.1 Hz, 1H), 3.19 - 3.12 (m, 1H), 3.04 (td, *J* = 8.2, 5.2 Hz, 1H), 2.43 (dt, *J* = 13.0, 7.3 Hz, 1H), 2.23 - 2.11 (m, 1H), 1.41 (s, 9H), 1.35 (s, 18H). LC-MS: [M+H]⁺ = 710.7.

### Intermediate 30: (R)-methyl 3-((tert-butoxycarbonyl)amino)-1-(4-fluoro-2-formyl-3-((trimethylsilyl)ethynyl)phenyl) pyrrolidine-3-carboxylate

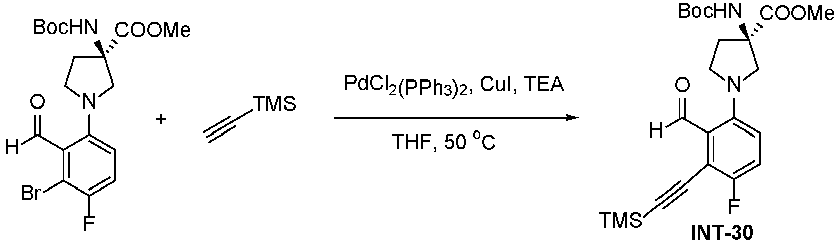

To a 10 mL tube was added methyl (R)-1-(3-bromo-4-fluoro-2-formylphenyl)-3-((tert-butoxycarbonyl)amino)pyrrolidine-3-carboxylate (prepared following procedures analogous to INT-3a) (50 mg, 0.1 mmol), PdCl₂(PPh₃)₃ (7.9 mg, 0.011 mmol) and Cul (4.3 mg, 0.022 mmol). The tube was purged with N₂ for 3 times. Then THF (0.4 mL) was added followed by TEA (0.02 mL, 0.2 mmol) and ethynyltrimethylsilane (0.02 mL, 0.2 mmol). The mixture was stirred at 50 °C under N₂ for 4h. LCMS indicated completion of the reaction.The mixture was filtered by a syringe filter and washed by DCM for 3 times. The mixture was concentrated and was purified by ISCO (0-50% EA in hexane) to give (*R*)-methyl 3-((*tert*-butoxycarbonyl)amino)-1-(4-fluoro-2-formyl-3-((trimethylsilyl)ethynyl)phenyl) pyrrolidine-3-carboxylate as yellow foam. ¹H NMR (400 MHz, Chloroform-d) δ 10.51 (d, *J* = 0.7 Hz, 1H), 7.17 (dd, *J* = 9.4, 7.9 Hz, 1H), 6.83 (ddd, *J* = 9.4, 4.3, 0.8 Hz, 1H), 5.28 (s, 1H), 3.75 (s, 3H), 3.71 - 3.64 (m, 1H), 3.58 (dt, *J* = 9.9, 7.3 Hz, 1H), 3.28 (q, *J* = 8.0, 6.8 Hz, 2H), 2.58 (dt, *J* = 12.8, 7.7 Hz, 1H), 2.37 (dt, *J* = 12.7, 6.2 Hz, 1H), 1.42 (s, 9H), 0.29 (s, 9H). LC-MS: [M+H]⁺ = 463.2.

### Intermediate 31: methyl (R)-1-(2-((6-(bis(tert-butoxycarbonyl)amino)-9H-purin-9-yl)methyl)-4-fluoro-3-(2,2,2-trifluoroethyl)phenyl)-3-((tert-butoxycarbonyl)amino)pyrrolidine-3-carboxylate

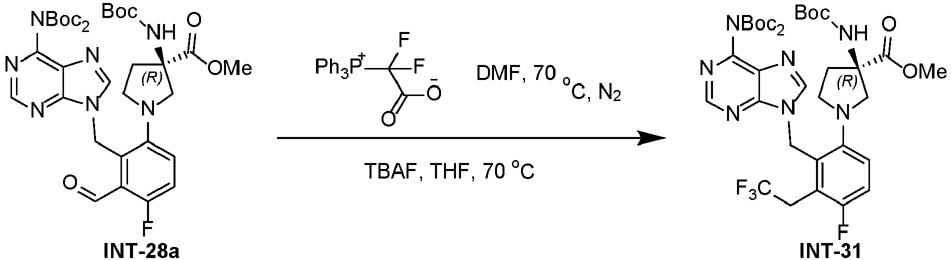

To a 10 mL reaction vial was added methyl (R)-1-(2-((6-(bis(tert-butoxycarbonyl)amino)-9H-purin-9-yl)methyl)-4-fluoro-3-formylphenyl)-3-((tert-butoxycarbonyl)amino)pyrrolidine-3-carboxylate (INT-28aa) (70 mg, 0.1 mmol) and 2,2-difluoro-2-(triphenylphosphonio)acetate (70 mg, 0.2 mmol). The mixture was purged with N₂ and anhydrous DMF was added.The vial was stirred at 70 °C for 1h. LCMS showed consumption of the starting material. Then TBAF(1M in THF, ~5% water) (0.5 mL, 0.5 mmol) was added and the mixture was stirred at 70 °C for 1h. Water was added and the mixture was extracted with EA (x3). The combined organic phase was washed with water, brine and dried over Na₂SO₄. The mixture was concentrated for purification. ISCO purification (0-40% in EA/Hexane) give methyl (*R*)-1-(2-((6-(bis(tert-butoxycarbonyl)amino)-9*H*-purin-9-yl)methyl)-4-fluoro-3-(2,2,2-trifluoroethyl)phenyl)-3-((*tert-*butoxycarbonyl)amino)pyrrolidine-3-carboxylate as a colorless oil. ¹H NMR (400 MHz, Methanol-*d*₄) δ 8.81 (s, 1H), 8.55 (s, 1H), 7.49 (dd, *J* = 8.9, 5.0 Hz, 1H), 7.22 (t, *J* = 9.1 Hz, 1H), 5.70 (d, *J* = 2.1 Hz, 2H), 4.37 - 4.13 (m, 2H), 3.80 - 3.70 (m, 4H), 3.17 (d, *J* = 10.1 Hz, 1H), 3.05 - 2.95 (m, 1H), 2.95 - 2.86 (m, 1H), 2.47 (dt, *J* = 13.0, 7.4 Hz, 1H), 2.21 - 2.11 (m, 1H), 1.44 (s, 9H), 1.35 (s, 18H). ¹⁹F NMR (376 MHz, Methanol-d₄) δ -66.73 (d, *J* = 8.1 Hz, 3H), -117.83 (brs, 1H). LC-MS: [M+H]⁺ = 768.3.

### Intermediate 32. ethyl 3-(1-(3-bromo-5-chloro-2-(hydroxymethyl)phenyl)-3-((tert-butoxycarbonyl)amino)pyrrolidin-3-yl)propanoate

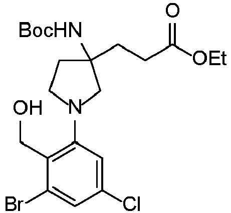

To a THF solution (700 mL) of *tert*-butyl (1-benzyl-3-formylpyrrolidin-3-yl)carbamate (70 g, 230 mmol) was added ethyl 2-(triphenyl-phosphaneylidene)acetate (104 g, 299 mmol) at 25°C under Ar. The sealed tube was then submerged to the preheated oil bath (75 °C) and stirred for 16 h. The reaction mixture was concentrated and filtered through a pad of silica gel column (PE/EA = 4/1) to afford ethyl (*E*)-3-(1-benzyl-3-((tert-butoxycarbonyl)amino)pyrrolidin-3-yl)acrylate as a yellow oil. LC-MS: [M+H]⁺ = 375.2.

To a solution of ethyl (*E*)-3-(1-benzyl-3-((tert-butoxycarbonyl)amino)pyrrolidin-3-yl)acrylate (70 g, 187 mmol) in EtOH (700 mL) was added Pd/C (70 g, wet), the mixture was purged with H₂ three times and stirred under 50 psi of hydrogen atmosphere pressure at 55°C for 16 h. The mixture was filtered through Celite, the filtrate was concentrated to afford ethyl 3-(1-benzyl-3-((tert-butoxycarbonyl)amino)pyrrolidin-3-yl)propanoate as a yellow oil. LC-MS: [M+H]⁺ = 377.5.

Ethyl 3-(1-benzyl-3-((*tert*-butoxycarbonyl)amino)pyrrolidin-3-yl)propanoate (35 g, 93 mmol), Pd/C (35 g, wet) and HCO₂NH₄ (24 g, 374 mmol) in a mixture of EtOH (945 mL) and H₂O (105 mL) was heated at 80 _{°}C for 5 hours. The mixture was filtered through Celite and the filtrate was concentrated. The residue was diluted with water (1 L) and extracted with EtOAc (1 L × 2). The combined organic layer was concentrated to afford ethyl 3-(3-((tert-butoxycarbonyl)amino)pyrrolidin-3-yl)propanoate as a yellow oil. ¹H NMR (400 MHz, CDCl₃) δ 4.70 (s, 1H), 4.16-4.09 (m, 2H), 3.11 ( m, 1H), 3.08 (m, 1H), 2.96 (m, 1H),2.74-2.71 (m, 1H), 2.37-2.33 (m, 2H), 2.23-2.21 (m, 2H), 2.19-2.18 (m, 1H), 1.76 (m, 1H), 1.43 (s, 9H), 1.27-1.24 (t, *J* = 7.2 Hz, 3H). LC-MS: [M+H]⁺ = 287.2.

The mixture of ethyl 3-(3-((*tert*-butoxycarbonyl)amino)pyrrolidin-3-yl)propanoate (53 g, 185 mmol), 2-bromo-4-chloro-6-fluorobenzaldehyde (Apollo Scientific) (44 g, 185 mmol), DIEA (60 g, 463 mmol) and MeCN (530 mL) was stirred at 80 °C for 16 h. The mixture was diluted with water (1.5 L) and extracted with EtOAc (1 L × 3). The combined organic layer was concentrated to afford ethyl 3-(1-(3-bromo-5-chloro-2-formylphenyl)-3-((*tert*-butoxycarbonyl)amino)pyrrolidin-3-yl)propanoate as a yellow solid. ¹H NMR (400 MHz, CDCl₃) δ 10.23 (s, 1H), 6.94 (d, *J* = 1.8 Hz, 1H), 6.72 (d, *J* = 1.2 Hz, 1H), 4.50 (s, 1H), 4.10-4.04 (m, 2H), 3.42- 3.39 (m, 1H), 3.37 (m, 2H), 3.23-3.20 (m, 1H), 2.30-2.25 (m, 3H), 2.18 (m, 2H), 1.91-1.87 (m, 1H), 1.35 (s, 9H), 1.20-1.18 (m, 3H). LC-MS: [M+H]⁺ = 503.1.

To a solution of ethyl 3-(1-(3-bromo-5-chloro-2-formylphenyl)-3-((*tert-*butoxycarbonyl)amino)pyrrolidin-3-yl)propanoate (87 g, 173 mmol) in THF (870 mL) and MeOH (290 mL) was added NaBH₄ (5.9 g, 155 mmol) in portions at 0 °C. The mixture was stirred at 0 °C for 15 min. The mixture was diluted with water (2 L) at 0 °C and extracted with EtOAc (2 L × 3). The combined organic layer was concentrated to afford ethyl 3-(1-(3-bromo-5-chloro-2-(hydroxymethyl)phenyl)-3-((*tert*-butoxycarbonyl)amino)pyrrolidin-3-yl)propanoate as a yellow solid. LC-MS: [M+H]⁺ = 505.0.

### Intermediate 33. ethyl 3-(1-(2-((6-(bis(tert-butoxycarbonyl)amino)-9H-purin-9-yl)methyl)-3-bromo-5-chlorophenyl)-3-((tert-butoxycarbonyl)amino) pyrrolidin-3-yl)propanoate

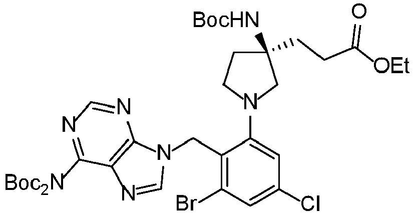

Intermediate 33 was prepared from tert-butyl N-[(tert-butoxy)carbonyl]-N-(9H-purin-6-yl)carbamate (INT-4) and ethyl 3-(1-(3-bromo-5-chloro-2-(hydroxymethyl)phenyl)-3-((*tert-*butoxycarbonyl)amino)pyrrolidin-3-yl)propanoate (INT-32), following procedures analogous to Intermediate 5. ¹H NMR (400 MHz, MeOD) δ 8.89 (s, 1H), 8.25 (s, 1H), 7.37 (d, *J* = 2.0Hz, 1H), 7.23 (d, *J* = 2.0Hz, 1H), 5.71 (q, *J* = 14.6 Hz, 2H), 4.11 (q, *J* =7.1 Hz, 2H), 3.59-3.50 (m, 1H), 3.44 - 3.35 (m, 1H), 3.30 - 3.26 (m, 1H), 3.20 - 3.13 (m, 1H), 2.35 - 2.25 (m, 2H), 2.24 - 2.11 (m, 2H), 2.09 - 1.98 (m, 1H), 1.92 - 1.82 (m, 1H), 1.42 - 1.38 (m, 27H), 1.25 (t, *J* = 7.0 Hz, 3H). LC-MS: [M+H]⁺ = 821.9.

### Intermediate 34. (R)-tert-butyl (9-(2-bromo-6-(3-((tert-butoxycarbonyl)amino)-3-(2-cyanoethyl)pyrrolidin-1-yl)-4-chlorobenzyl)-9H-purin-6-yl)carbamate

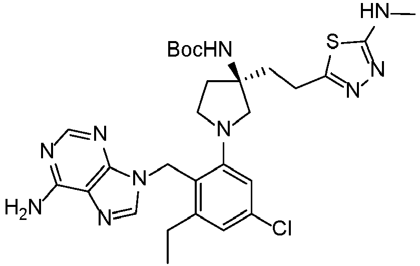

### Step 1. tert-butyl (1-(2-((6-amino-9H-purin-9-yl)methyl)-5-chloro-3-ethylphenyl)-3-(3-hydrazinyl-3-oxopropyl)pyrrolidin-3-yl)carbamate

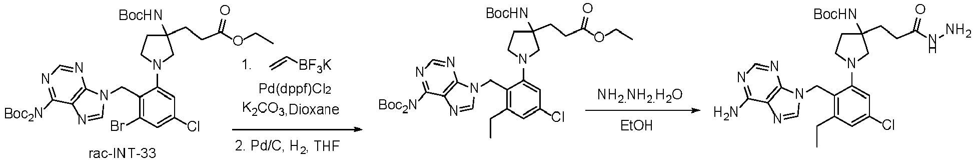

To a solution of ethyl 3-(1-(2-((6-(bis(*tert*-butoxycarbonyl)amino)-9*H*-purin-9-yl)methyl)-3-bromo-5-chlorophenyl)-3-((tert-butoxycarbonyl)amino)pyrrolidin-3-yl)propanoate (*rac*- INT-33) (15 g, 18.2 mmol) and potassium vinyltrifluoroborate (4.9 g, 36 mmol), Pd(dppf)Cl₂DCM (1.5 g, 1.8 mmol) in dioxane (135 mL) and H₂O (15 mL) was added K₂CO₃ (7.5 g, 5 mmol) under N₂. The mixture was stirred at 90 °C for 16 hours. The mixture was diluted with water (500 mL) and extracted with ethyl acetate (3 × 500 mL). The organic layer was combined, dried over anhydrous sodium sulfate, filtered and concentrated under vacuum. The crude residue was dissolved in EtOH (130 mL) and Pd/C (1.3 g, wet) was added. The mixture was purged with H₂ three times and stirred under 15 Psi of hydrogen atmosphere at 25 °C for 16 hours. The mixture was filtered and the filtrate was concentrated to afford ethyl 3-(1-(2-((6-(bis(*tert-*butoxycarbonyl)amino)-9*H*-purin-9-yl)methyl)-5-chloro-3-ethylphenyl)-3-((*tert-*butoxycarbonyl)amino)pyrrolidin-3-yl)propanoate as a yellow solid. LC-MS: [M+H]⁺ =772.4.

A mixture of ethyl 3-(1-(2-((6-(bis(*tert*-butoxycarbonyl)amino)-9*H*-purin-9-yl)methyl)-5-chloro-3-ethylphenyl)-3-((*tert*-butoxycarbonyl)amino)pyrrolidin-3-yl)propanoate (0.5 g, 0.65 mmol), NH₂NH₂•H₂O (0.48 g, 9.7 mmol) and EtOH (5 mL) in a sealed tube was stirred at 25 °C for 48 h. The mixture was concentrated to afford *tert*-butyl (1-(2-((6-amino-9*H*-purin-9-yl)methyl)-5-chloro-3-ethylphenyl)-3-(3-hydrazineyl-3-oxopropyl)pyrrolidin-3-yl)carbamate as a yellow oil. LC-MS: [M+H]⁺ =558.2.

### Step 2. (R)-tert-butyl (9-(2-bromo-6-(3-((tert-butoxycarbonyl)amino)-3-(2-cyanoethyl)pyrrolidin-1-yl)-4-chlorobenzyl)-9H-purin-6-yl)carbamate

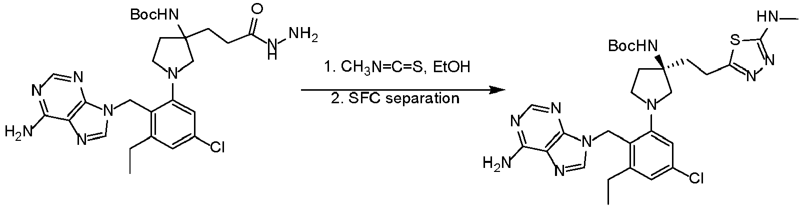

The mixture of *tert*-butyl (1-(2-((6-amino-9*H*-purin-9-yl)methyl)-5-chloro-3-ethylphenyl)-3-(3-hydrazineyl-3-oxopropyl)pyrrolidin-3-yl)carbamate (1.2 g, 2 mmol) and CH₃N=C=S (0.4 g, 5.4 mmol) in EtOH (12 mL) was stirred at 80 °C for 24 h. The mixture was concentrated and the residue was purified by Prep-HPLC (Column: Phenomenex luna C18 250*50mm*10µm. Condition: water (0.1% TFA)-MeCN) to afford a racemic compound. Separation by SFC (AD-3_5CM_IPA (DEA)_40_3ML_7MIN_T35.M) gave (*R*)-*tert*-butyl (9-(2-bromo-6-(3-((tert-butoxycarbonyl)amino)-3-(2-cyanoethyl)pyrrolidin-1-yl)-4-chlorobenzyl)-9H-purin-6-yl)carbamate as a white solid (first peak). ¹H NMR (400 MHz, Methanol-d₄) δ 8.41 (s, 1H), 7.91 (s, 1H), 7.19 (d, *J* = 2.1 Hz, 1H), 7.08 (d, *J* = 1.7 Hz, 1H), 5.64 - 5.47 (m, 2H), 3.50 (s, 3H), 3.41 (s, 1H), 3.28 - 3.21 (m, 1H), 3.16 (d, *J* = 9.7 Hz, 2H), 2.80 - 2.59 (m, 4H), 2.40 - 2.13 (m, 2H), 2.11 - 1.90 (m, 2H), 1.42 (s, 9H), 1.13 (t, *J* = 7.5 Hz, 3H). LC-MS: [M+H]⁺ =613.2 [M+H]⁺.

### Intermediate 35 and 36. tert-butyl (R)-(9-(2-bromo-6-(3-((tert-butoxycarbonyl)amino)-3-(1-methyl-1H-tetrazol-5-yl)pyrrolidin-1-yl)-4-chlorobenzyl)-9H-purin-6-yl)(tert-butoxycarbonyl)carbamate (INT-35) and tert-butyl (R)-(9-(2-bromo-6-(3-((tert-butoxycarbonyl)amino)-3-(2-methyl-2H-tetrazol-5-yl)pyrrolidin-1-yl)-4-chlorobenzyl)-9H-purin-6-yl)(tert-butoxycarbonyl)carbamate (INT-36)

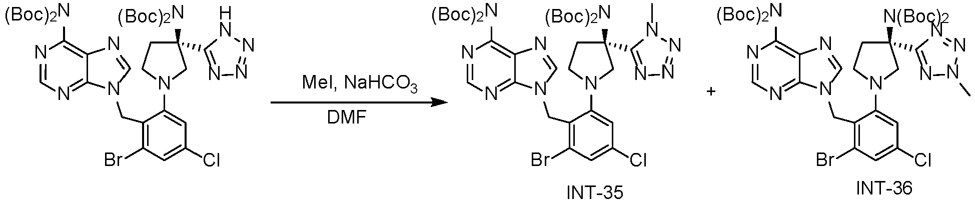

To a solution of *tert*-butyl (*R*)-(9-(2-bromo-6-(3-((*tert*-butoxycarbonyl)amino)-3-(1*H*-tetrazol-5-yl)pyrrolidin-1-yl)-4-chlorobenzyl)-9*H*-purin-6-yl)(*tert*-butoxycarbonyl)carbamate (200 mg, 0.2 mmol) in THF (6 mL) was added CH₃I (16 mg, 0.11 mmol) and NaHCO₃ (38 mg, 0.45 mmol). The mixture was stirred at 20 °C for 16 hours. CH₃I (32 mg, 0.22 mmol) and NaHCO₃ (38 mg, 0.45 mmol) were then added to the above mixture. The mixture was stirred at 20 °C for another 29 hours. The mixture was partitioned between brine (30 mL) and EtOAc (20 mL × 3). The organic layer was dried under vacuum to afford a mixture of *tert*-butyl (*R*)-(9-(2-bromo-6-(3-((*tert*-butoxycarbonyl)amino)-3-(1-methyl-1*H*-tetrazol-5-yl)pyrrolidin-1-yl)-4-chlorobenzyl)-9*H-*purin-6-yl)(*tert*-butoxycarbonyl)carbamate (INT-35) and *tert*-butyl (*R*)-(9-(2-bromo-6-(3-((*tert-*butoxycarbonyl)amino)-3-(2-methyl-2*H*-tetrazol-5-yl)pyrrolidin-1-yl)-4-chlorobenzyl)-9*H*-purin-6-yl)(*tert*-butoxycarbonyl)carbamate (INT-36) as a yellow oil. LC-MS: [M+H]⁺ = 906.4.

### Intermediates 37 and 38: tert-butyl (R)-(tert-butoxycarbonyl)(9-(6-(3-((tert-butoxycarbonyl)amino)-3-(cyclopropylcarbamoyl)pyrrolidin-1-yl)-4-chloro-3-fluoro-2-formylbenzyl)-9H-purin-6-yl)carbamate (INT-37) and tert-butyl (R)-(tert-butoxycarbonyl)(9-(6-(3-((tert-butoxycarbonyl)amino)-3-(cyclopropylcarbamoyl)pyrrolidin-1-yl)-2-chloro-3-fluoro-4-formylbenzyl)-9H-purin-6-yl)carbamate (INT-38)

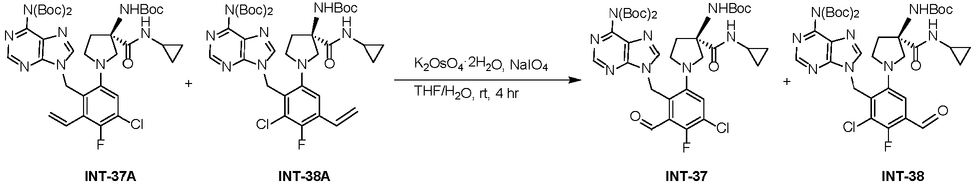

To a THF/Water solution (4 mL, 4:1) of tert-butyl (*R*)-(tert-butoxycarbonyl)(9-(6-(3-((tert-butoxycarbonyl)amino)-3-(cyclopropylcarbamoyl)pyrrolidin-1-yl)-4-chloro-3-fluoro-2-vinylbenzyl)-9H-purin-6-yl)carbamate (INT-37A) and tert-butyl (R)-(tert-butoxycarbonyl)(9-(6-(3-((tert-butoxycarbonyl)amino)-3-(cyclopropylcarbamoyl)pyrrolidin-1-yl)-2-chloro-3-fluoro-4-vinylbenzyl)-9H-purin-6-yl)carbamate (INT38A) (150 mg, 0.19 mmol) was added sodium periodate (125 mg, 0.6 mmol) and potassium osmate(VI) dihydrate (4.3 mg, 12 µmol) in one portion at rt. The reaction was stirred for 2 hr, followed by addition of sodium periodate (125 mg, 0.6 mmol) and potassium osmate(VI) dihydrate (4.3 mg, 12 µmol). The reaction was further stirred for 2 hr. The reaction was diluted with EA, washed with sodium sulfide solution, and brine. The organic layer was dried over Na₂SO₄ and concentrated under vacuum to give the crude product. The crude product was purified by combiflash (10% to 50% EA in DCM) to give a mixture of *tert*-butyl (*R*)-(tert-butoxycarbonyl)(9-(6-(3-((*tert*-butoxycarbonyl)amino)-3-(cyclopropylcarbamoyl)pyrrolidin-1-yl)-4-chloro-3-fluoro-2-formylbenzyl)-9*H*-purin-6-yl)carbamate (INT-37) and tert-butyl (*R*)-(*tert-*butoxycarbonyl)(9-(6-(3-((*tert*-butoxycarbonyl)amino)-3-(cyclopropylcarbamoyl)pyrrolidin-1-yl)-2-chloro-3-fluoro-4-formylbenzyl)-9*H*-purin-6-yl)carbamate (INT-38). LC-MS: [M+H]⁺ = 773.3.

### INT-39: methyl (R)-3-((tert-butoxycarbonyl)amino)-1-(2,2-difluoro-4-formylbenzo[d][1,3]dioxol-5-yl)pyrrolidine-3-carboxylate (for Example 15)

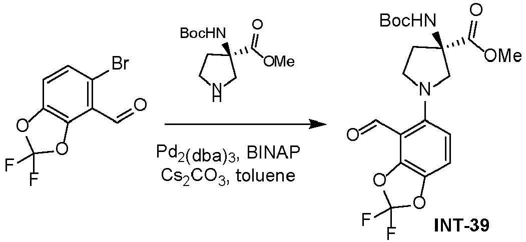

A degassed suspension of 5-bromo-2,2-difluorobenzo[d][1,3]dioxole-4-carbaldehyde (8.7 g, 37 mmol, prepared from 5-bromo-2,2-difluorobenzo[d][1,3]dioxole according to General Procedure A), INT-1 (9.0 g, 37 mmol), Pd₂(dba)₃ (0.64 g, 0.7 mmol), BINAP (0.9 g, 1.4 mmol) and Cs₂C0₃ (14.5 g, 44 mmol) in dry toluene (90 mL) was heated under N₂ to 110 °C for 16 h. The mixture was chilled and the volatiles were removed under reduced pressure. The residue was purified by Combi flash (PE/EA = 100/1 ~ 5/1) to afford compound INT-39 as a yellow solid. LC-MS: [M+H]⁺ = 429.0.

### INT-40: 6-bromo-4-fluoro-2,3-dihydrobenzofuran (For Example 115)

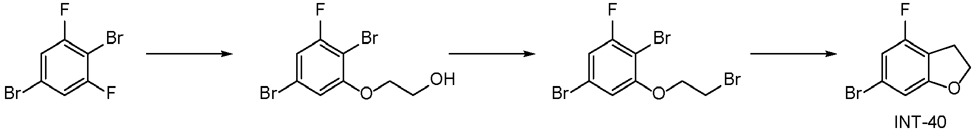

A mixture of 2,5-dibromo-1,3-difluorobenzene (3 g, 11 mmol), ethylene glycol (6 mL, 110 mmol) and potassium carbonate (4.6 g, 33 mmol) in NMP (3 mL) was stirred at 100 °C for 4hr. The reaction mixture was diluted with EtOAc, washed with water and brine, dried, fitlered and concentrated. ISCO (silica gel, 0-30% EtOAc in hexanes) to afford 2-(2,5-dibromo-3-fluorophenoxy)ethanol as white solid. ¹H NMR (400 MHz, DMSO-d6) δ 7.32 (dd, *J* = 8.1, 2.0 Hz, 1H), 7.23 (t, *J* = 1.8 Hz, 1H), 4.93 (s, 1H), 4.15 (dd, *J* = 5.3, 4.4 Hz, 2H), 3.73 (t, *J* = 4.9 Hz, 2H).

To a solution of 2-(2,5-dibromo-3-fluorophenoxy)ethanol (1 g, 3.2 mmol) and triphenylphosphine (0.9 g, 3.5 mmol) in THF (15 mL) was added a solution of carbon tetrabromide (1.2 g, 3.5 mmol) in THF (5 mL). The reaction was stirred at r.t. for 4hr. The reaction mixture was filtered to removed PPhsO and the filtrate was concentrated. ISCO (silica gel, 100% hexanes) to afford 2,5-dibromo-1-(2-bromoethoxy)-3-fluorobenzene. ¹H NMR (400 MHz, DMSO-*d*6) δ 7.36 (dd, *J* = 8.1, 2.0 Hz, 1H), 7.26 (t, *J* = 1.8 Hz, 1H), 4.54 - 4.42 (m, 2H), 3.89 - 3.77 (m, 2H).

At -78 °C, to a solution of 2,5-dibromo-1-(2-bromoethoxy)-3-fluorobenzene (1.6 g, 4.2 mmol) in THF (30 mL) was added n-butyllithium (2.5 mL, 5 mmol) and the resulting mixture was stirred at -78°C for 2hr. The reaction mixture was quenched with sat. NH₄Cl aq. solution and extracted with EtOAc. The organic layer was washed with brine, dried, filtered and concentrated. ISCO (silica gel, 100% hexanes) to afford 6-bromo-4-fluoro-2,3-dihydrobenzofuran as colorless oil. ¹H NMR (400 MHz, DMSO-*d*6) δ 6.97 (dd, *J* = 8.3, 1.5 Hz, 1H), 6.90 (dd, *J* = 1.5, 0.5 Hz, 1H), 4.64 (t, *J* = 8.7 Hz, 2H), 3.23 - 3.15 (m, 2H).

### INT-41: tert-butyl (1-(2-((6-amino-9H-purin-9-yl)methyl)-4-chloro-3-(ethylthio)phenyl)-3-(cyclopropylcarbamoyl)pyrrolidin-3-yl)carbamate (For Example 113)

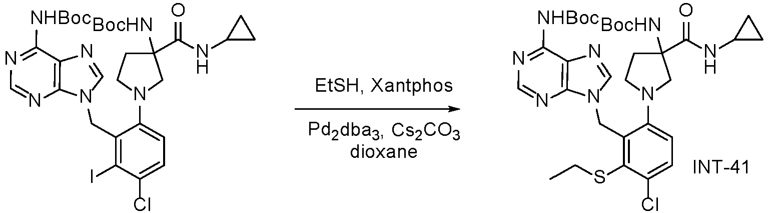

In a sealed tube the reactants *tert*-butyl (1-(2-((6-((tert-butoxycarbonyl)amino)-9*H*-purin-9-yl)methyl)-4-chloro-3-iodophenyl)-3-(cyclopropylcarbamoyl)pyrrolidin-3-yl)carbamate (500 mg, 0.7 mmol, prepared from 1-chloro-4-fluoro-2-iodobenzene), XANTPHOS (77 mg, 0.13 mmol), Pd₂dba₃ (122 mg, 0.13 mmol) and Cs₂CO₃ (433 mg, 1.3 mmol) were mixed in 1,4-dioxane (2 mL) and ethanethiol (2.5 mL, 33 mmol). The mixture was stirred at 80°C for 16 hrs. The reaction was quenched by aq. NH₄Cl, the crude product was extracted by ethyl acetate (3×40 mL). The organic layer was combined and dried with MgSO₄, filtered and concentrated. The crude was purified by prep. HPLC. LC-MS: [M+1]⁺ = 586.9.

### INT-42: tert-butyl (R)-(3-((2-(cyclopropylamino)-2-oxoethoxy)methyl)pyrrolidin-3-yl)carbamate (for Example 30)

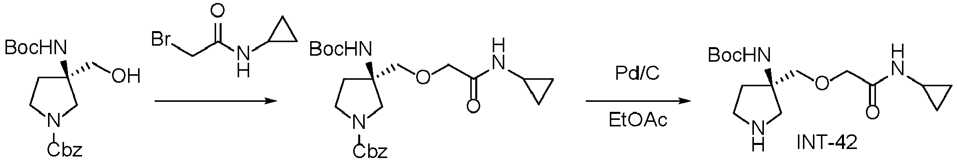

At 0°C, to a solution of benzyl (*R*)-3-((*tert*-butoxycarbonyl)amino)-3-(hydroxymethyl)pyrrolidine-1-carboxylate (100 mg, 0.28 mmol) in THF (5 mL) was added sodium hydride (13.7 mg, 0.57 mmol) and the resulting mixture was stirred at 0°C for 15min before the addition of 2-bromo-*N*-cyclopropylacetamide (102 mg, 0.57 mmol) (in 3mL THF). The reaction mixture was warmed up to r.t. The reaction mixture was quenched with sat. NH₄Cl aq. solution and extracted with EtOAc. The organic layer was washed with brine, dried, fitlered and concentrated. The crude product was purified by ISCO (silica gel, 0-100% EtOAc in hexanes) to afford the product. LC-MS: [M+1]⁺ = 448.1.

A mixture of benzyl (*R*)-3-((*tert*-butoxycarbonyl)amino)-3-((2-(cyclopropylamino)-2-oxoethoxy)methyl)pyrrolidine-1-carboxylate (55 mg, 0.123 mmol) and Pd-C (10%, 26.2 mg, 0.025 mmol) in EtOAc (5 mL) was stirred at r.t. under H₂ atmoshpere overnight. The reaction mixture was filtered and concentrated. The crude product was used in the next step without purification. LC-MS: [M+1]⁺ = 314.2.

### INT-43: methyl (R)-3-((tert-butoxycarbonyl)amino)-1-(2-((6-((tert-butoxycarbonyl)amino)-9H-purin-9-yl)methyl)-5-chloro-3-cyclobutylphenyl)pyrrolidine-3-carboxylate (For Example 29)

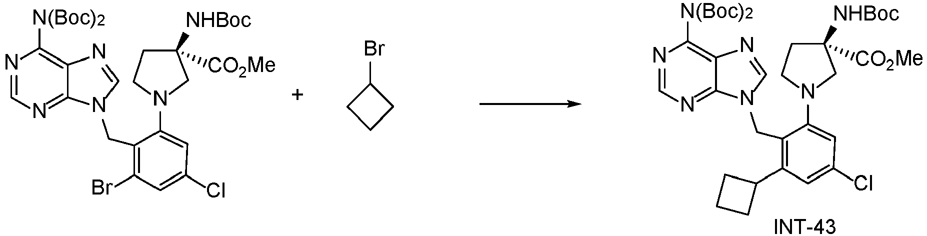

To an 40 mL vial (Thermo scientific 200 series) equipped with a stirring bar was added in photocatalyst Ir[dF(CF₃)ppy]₂(dtbbpy)PF₆ (2.87 mg, 2.56 umol), methyl (R)-1-(2-((6-(bis(tert-butoxycarbonyl)amino)-9H-purin-9-yl)methyl)-3-bromo-5-chlorophenyl)-3-((tert-butoxycarbonyl)amino)pyrrolidine-3-carboxylate (200 mg, 256 umol), bromocyclobutane (52 mg, 384 umol), dichloronickel, 1,2-dimethoxyethane (281 ug, 1.3 umol), 4-*tert*-butyl-2-(4-*tert*-butyl-2-pyridyl)pyridine (412 ug, 1.54 umol), bis(trimethylsilyl)silyl-trimethyl-silane (64 mg, 256 umol) and Na₂CO₃ (54 mg, 512 umol). The vial was sealed and purged with nitrogen (30 min) before 1,2-dimethoxyethane (5 mL) was added in. The mixture was stirred and irradiated with a 34 W blue LED lamp (7 cm away, with cooling fan to keep the reaction temperature at 25 °C) for 16 hours. The mixture was diluted with water (30 mL) and was extracted with EtOAc (30 mL × 3). The organic layer was concentrated under vacuum. The residue was purified by reversed chromatography column (30% MeCN in water, HCl) to afford the title compound as yellow gum. LC-MS: [M+H]⁺ = 756.3.

### INT-44: tert-butyl (9-(2-bromo-6-(3-((tert-butoxycarbonyl)amino)-3-(4-(trifluoromethyl)pyridin-2-yl)pyrrolidin-1-yl)-4-chlorobenzyl)-9H-purin-6-yl)carbamate (for Example 27)

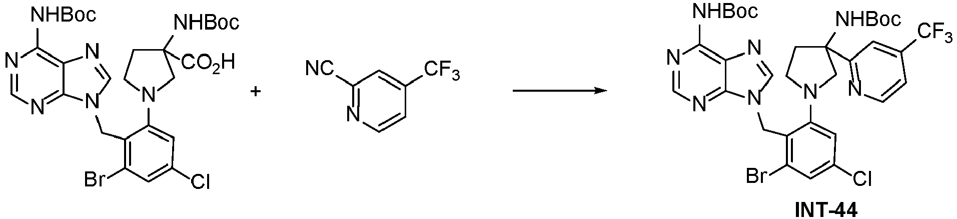

The reactants 1-(3-bromo-2-((6-((tert-butoxycarbonyl)amino)-9H-purin-9-yl)methyl)-5-chlorophenyl)-3-((tert-butoxycarbonyl)amino)pyrrolidine-3-carboxylic acid (400 mg, 0.6 mmol), photocatalyst *fac*-Ir(dfppy)₃ (4.57 mg, 6.00 pmol), 4-(trifluoromethyl)picolinonitrile (0.15 mL, 1.2 mmol) and potassium hydrogen phosphate (209 mg, 1.199 mmol) were mixed in DMSO (12 mL). The mixture was bubbled by N₂ for 30 min then was sealed and put on a 2W blue LED reactor for 4 hrs. The reaction was quenched by brine and water, the crude product was extracted by ethyl acetate (3 × 40 mL). The organic layer was combined and dried with MgSO4, was filtered and was concentrated under vacuum. The residue was purified by flash chromatography column (0% to 100% EtOAc in hexane) to afford the title compound as yellow gum. LC-MS: [M+H]⁺ = 769.2.

### INT-45: tert-butyl (3-(pyridin-2-yl)pyrrolidin-3-yl)carbamate (For Examples 22 & 23)

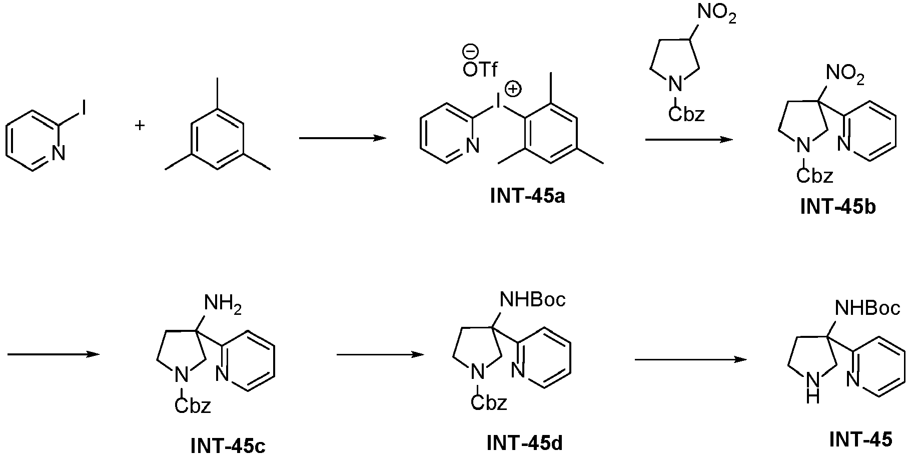

To a solution of 2-iodopyridine (1 mL, 9.8 mmol) in DCM (60 mL) was added *m*-CPBA (3.4 g, 14.6 mmol). The mixture was cooled to 0 °C. TfOH (3.5 mL, 39 mmol) was added in drop-wisely. The mixture was recovered to r.t. and was stirred for 120 min at r.t. then was cooled to 0°C. Mesitylene (1.5 mL, 10.7 mmol) was added in drop-wisely. The mixture was stirred at r.t. for 18 hrs. The mixture was evaporated *in vacuo.* The residue was put on a silica gel plug and was first washed with DCM (400 ml), then washed with5% MeOH in DCM (2000 mL). The eluent was concentrated and the solid was dissolved in Et₂O, the solution was put in -20°C for 18 hrs. The crystals was collected to gave mesityl(pyridin-2-yl)iodonium trifluoromethanesulfonate (INT-45a) as a yellow solid. ¹H NMR (400 MHz, DMSO-*d*₆) δ: 8.55 - 8.46 (m, 1H), 8.20 (d, *J* = 8.0 Hz, 1H), 8.04 - 7.92 (m, 1H), 7.68 (dd, *J* = 7.4, 4.5 Hz, 1H), 7.22 (s, 2H), 2.61 (s, 6H), 2.30 (s, 3H).

To a solution of compound tert-butyl 3-nitropyrrolidine-1-carboxylate (250 mg, 1 mmol), CsF (304 mg, 2 mmol) and t-BuOK (337 mg, 3.00 mmol) in 2-propanol (2 mL), t-BuOH (2 mL) and 1,2-dimethoxyethane (4 mL) was added in mesityl(pyridin-2-yl)iodonium trifluoromethanesulfonate (710 mg, 1.5 mmol). The mixture was stirred at 45 °C for 16 hours. The mixture was diluted with water (100 mL) and extracted with EtOAc (100 mL × 3). The organic layer was concentrated under vacuum. The residue was purified by column chromatography (33% EtOAc in PE) to afford the benzyl 3-nitro-3-(pyridin-2-yl)pyrrolidine-1-carboxylate (INT-45b) as yellow oil. LC-MS: [M+H]⁺ = 328.1. ¹H NMR (400 MHz, CDCl₃) δ: 8.62 (s, 1H), 7.81-7.75 (m, 1H), 7.39-7.36 (m, 7H), 5.18-5.16 (m, 2H), 4.93-4.89 (m, 1H), 4.24-4.13 (m, 1H), 3.81-3.78 (m, 1H), 3.63-3.55 (m, 1H), 3.38-3.25 (m, 1H), 2.66-2.58 (m, 1H).

To a solution of *tert*-butyl 3-nitro-3-(pyridin-2-yl)pyrrolidine-1-carboxylate (1.20 g, 3.67 mmol) in EtOH/H₂O (144 mL, 2:1) was added in iron (4.1 g, 73 mmol) and NH₄Cl (2 g, 36.7 mmol). The mixture was stirred at 60 °C for 3 hours. The mixture was filtered, the filtrate was concentrated under vacuum to afford the benzyl 3-amino-3-(pyridin-2-yl)pyrrolidine-1-carboxylate (INT-45c) as brown oil. LC-MS: [M+H]⁺ = 298.0.

To a solution of benzyl 3-amino-3-(pyridin-2-yl)pyrrolidine-1-carboxylate (900 mg, 3 mmol) in DCM (6.5 mL) was added in DMAP (37 mg, 0.30 mmol) and Boc₂O (2.6 g, 12 mmol). The mixture was stirred at 25-35 °C for 16 hours. The mixture was diluted with water (50 mL) and was extracted with EtOAc (50 mL × 3). The organic layer was concentrated under vacuum. The residue was purified by flash chromatography column (55% EtOAc in PE) to benzyl 3-((tert-butoxycarbonyl)amino)-3-(pyridin-2-yl)pyrrolidine-1-carboxylate (INT-45d) as yellow gum. LC-MS: [M+H]⁺ = 398.1.

To a solution of benzyl 3-((tert-butoxycarbonyl)amino)-3-(pyridin-2-yl)pyrrolidine-1-carboxylate (400 mg, 1 mmol) in MeOH (4 mL) was added in Pd/C (80 mg, 10% purity, 50% purity of water). The mixture was purged with N₂ and H₂. The mixture was stirred at 25-35 °C for 16 hours under H₂ (15 PSI). The mixture was filtered and the filtrate was concentrated to afford *tert*-butyl (3-(pyridin-2-yl)pyrrolidin-3-yl)carbamate as yellow gum. LC-MS: [M+H]⁺ = 264.1.

### INT-46: tert-butyl (9-(6-(3-((tert-butoxycarbonyl)amino)-3-(2-(pyridin-2-yl)ethyl)pyrrolidin-1-yl)-3-fluoro-2-(trifluoromethyl)benzyl)-9H-purin-6-yl)carbamate

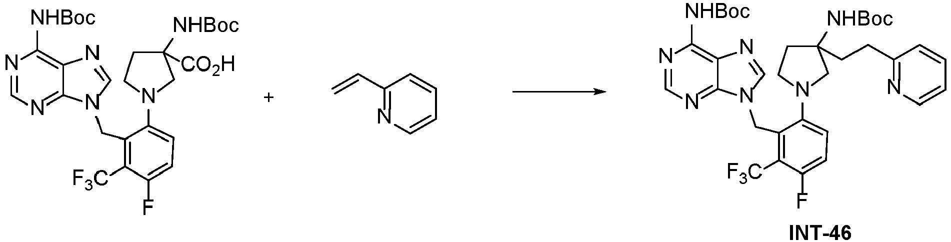

To a solution of 3-((*tert*-butoxycarbonyl)amino)-1-(2-((6-((*tert*-butoxycarbonyl)amino)-9H-purin-9-yl)methyl)-4-fluoro-3-(trifluoromethyl)phenyl)pyrrolidine-3-carboxylic acid (200 mg, 0.3 mmol), potassium hydrogen phosphate (109 mg, 0.6 mmol) and 2-vinylpyridine (0.07 mL, 0.62 mmol) mixed in DMSO (12 mL) was photocatalyst Ir[dF(CF₃)ppy]₂(dtbbpy)PF₆ (7 mg, 6 µmol) added in. The mixture was bubbled by N₂ for 30 min then was sealed and put on a 2W blue LED reactor for 16 hrs. The reaction was quenched by brine and water, the crude product was extracted by ethyl acetate (3 × 40 mL). The organic layer was combined and dried with MgSO4, was filtered and was concentrated under vacuum. The residue was purified by flash chromatography column (0% to 100% EtOAc in hexane) to afford the title compound as yellow gum. LC-MS: [M+H]⁺ = 701.2.

### EXAMPLES

### Example 1. (R)-3-amino-1-(2-((6-amino-9H-purin-9-yl)methyl)-3-bromo-5-chlorophenyl)-N-cyclopropylpyrrolidine-3-carboxamide

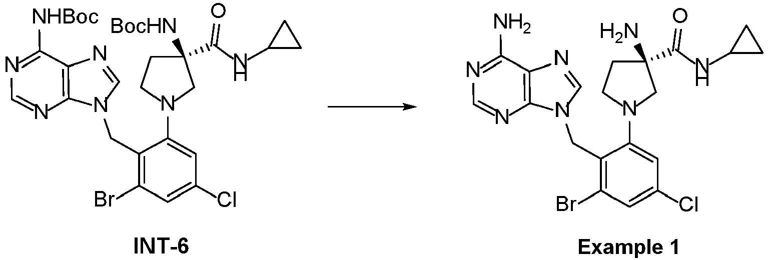

To a solution of tert-butyl (R)-(1-(3-bromo-2-((6-((tert-butoxycarbonyl)amino)-9H-purin-9-yl)methyl)-5-chlorophenyl)-3-(cyclopropylcarbamoyl)pyrrolidin-3-yl)carbamate (INT-6) (25 g, 83.6%purity) in DCM (250 mL) was added HCl/dioxane (75 mL, 4M). The reaction was stirred at 25 °C for 8 hours. The mixture was concentrated under vacuum. The residue was diluted with MeOH (40 mL) and Et₃N (5 mL). The residue was purified by preparational HPLC (column: Phenomenex Gemini C18 250*50mm*10 µm, gradient 20%∼45%B (A = water with 0.05% NH4OH), B = acetonitrile, flow rate: 100 mL/min). The eluent was put on a lyophilizer to afford (R)-3-amino-1-(2-((6-amino-9H-purin-9-yl)methyl)-3-bromo-5-chlorophenyl)-N-cyclopropylpyrrolidine-3-carboxamide as white solid. ¹H NMR (400 MHz, CDCl₃) δ 8.25 (s, 1H), 7.79 (s, 1H), 7.41 (d, J = 2.0 Hz, 1H), 7.25 (d, J = 2.0 Hz, 1H), 5.65-5.47 (m, 2H), 3.67-3.60 (m, 1H), 3.43-3.34 (m, 1H), 3.17-3.08 (m, 1H), 2.94-2.88 (m, 1H), 2.66-2.57 (m, 1H), 2.43-2.33 (m, 1H), 1.82-1.70 (m, 1H), 0.75-0.63 (m, 2H), 0.52-0.41 (m, 2H). LC-MS: [M+H]⁺ = 507.0, 505.0.

### Example 2. (R)-3-amino-1-(2-((6-amino-9H-purin-9-yl)methyl)-5-chloro-3-ethylphenyl)-N-cyclopropylpyrrolidine-3-carboxamide

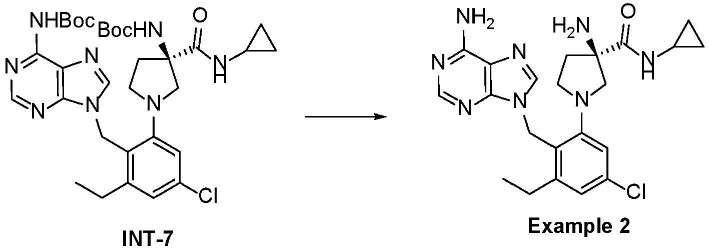

To a mixture of tert-butyl (R)-(1-(2-((6-((tert-butoxycarbonyl)amino)-9H-purin-9-yl)methyl)-5-chloro-3-ethylphenyl)-3-(cyclopropylcarbamoyl)pyrrolidin-3-yl)carbamate (46 g, crude) in DCM (460 mL) was added HCl/dioxane (138 mL, 4 M). The reaction was stirred at 25 °C for 2 hours. The mixture was concentrated under vacuum. The residue was purified by prep-HPLC (column: Phenomenex Gemini C18 250*50 mm*10 um, gradient 25-50% B (A = water (0.05% ammonia hydroxide v/v), B = acetonitrile, flow rate: 80 mL/min) to afford the title compound (7080 mg, purity: 98.8%, ee: 100%) as white solid. ¹H NMR (400 MHz, Methanol-*d*₄) δ 8.26 (s, 1H), 7.71 (s, 1H), 7.21 (d, J = 1.6 Hz, 1H), 7.07 (s, 1H), 5.58-5.44 (m, 2H), 3.60-3.50 (m, 1H), 3.29-3.21 (m, 1H), 3.11-2.99 (m, 1H), 2.88-2.80 (m, 1H), 2.74-2.58 (m, 3H), 2.46-2.34 (m, 1H), 1.81-1.66 (m, 1H), 1.11-1.01 (m, 3H), 0.76-0.64 (m, 2H), 0.52-0.41 (m, 2H). LC-MS: [M+H]⁺ = 455.4.

The following examples were prepared from corresponding intermediates following procedures analogous to Example 1.

| Ex No. | INT | | |
|---|---|---|---|
| 3 | INT-10 | 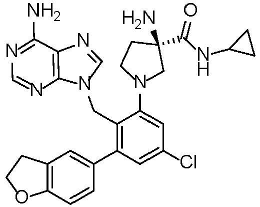 | ¹HNMR (400 MHz, Methanol-*d*₄). δ 8.28 (s, 1H), 7.49 (s, 1H), 7.44 (d, *J* = 2.0 Hz, 1H), 7.00 (d, *J* = 2.0 Hz, 1H), 6.78 (s, 1H), 6.72 (d, *J* = 8.2 Hz, 1H), 6.55 (d, *J* = 8.2 Hz, 1H), 5.79-5.64 (m, 2H), 4.54 (t, *J* = 8.8 Hz, 2H), 3.75 (d, *J* = 10.8 Hz, 1H), 3.57-3.50 (m, 1H), 3.50-3.46 (m, 1H), 3.45-3.38 (m, 1H), 3.18-3.09 (m, 2H), 2.77-2.64 (m, 2H), 2.40-2.31 (m, 1H), 0.81-0.69 (m, 2H), 0.60-0.51 (m, 2H). LC-MS: [M+H]⁺ = 545.2. |
| 4 | INT-29 | 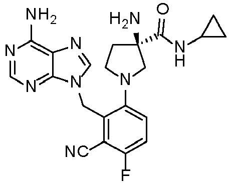 | ¹H NMR (400 MHz, Methanol-d₄) δ 8.19 (s, 1H), 8.08 (s, 1H), 7.67 (dd, *J* = 9.2, 5.1 Hz, 1H), 7.34 (dd, *J* = 9.1, 8.4 Hz, 1H), 5.68 (s, 2H), 3.61 (d, *J* = 9.3 Hz, 1H), 3.29 - 3.23 (m, 1H), 3.02 (td, *J* = 8.6, 5.0 Hz, 1H), 2.87 (dd, *J* = 9.3, 0.7 Hz, 1H), 2.66 (tt, *J* = 7.4, 3.9 Hz, 1H), 2.43 (ddd, *J* = 12.8, 8.5, 6.8 Hz, 1H), 1.80 - 1.72 (m, 1H), 0.75 - 0.67 (m, 2H), 0.52 - 0.46 (m, 2H). LC-MS: [M+H]⁺ = 436.2. |
| 5 | INT-30 | 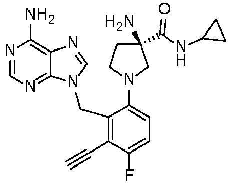 | ¹H NMR (400 MHz, Methanol-d₄) δ 8.25 (s, 1H), 7.84 (s, 1H), 7.43 (dd, J = 9.1, 5.0 Hz, 1H), 7.22 (dd, J = 9.0, 8.6 Hz, 1H), 5.75 - 5.52 (m, 2H), 3.53 - 3.46 (m, 1H), 3.13 - 3.06 (m, 1H), 2.94 (td, J = 8.8, 5.5 Hz, 1H), 2.85 - 2.77 (m, 1H), 2.66 - 2.60 (m, 1H), 2.41 - 2.32 (m, 1H), 1.69 (dddd, J = 13.1, 7.8, 5.4, 0.9 Hz, 1H), 0.75 - 0.61 (m, 2H), 0.56 - 0.43 (m, 2H). LC-MS: [M+H]⁺ = 435.1. |
| 6 | INT-31 | 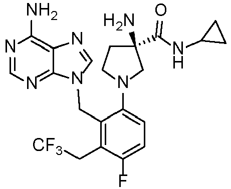 | ¹H NMR (400 MHz, Methanol-d₄) δ 8.22 (s, 1H), 8.15 (s, 1H), 7.51 (dd, *J* = 9.0, 5.1 Hz, 1H), 7.22 (t, *J* = 9.1 Hz, 1H), 5.56 (d, *J* = 1.1 Hz, 2H), 4.19 (dqd, *J* = 15.6, 10.7, 2.1 Hz, 1H), 4.01 (dqd, *J* = 15.6, 10.7, 1.9 Hz, 1H), 3.57 (d, *J* = 9.4 Hz, 1H), 3.09 (dt, *J* = 8.9, 7.1 Hz, 1H), 2.90 (td, *J* = 8.5, 5.0 Hz, 1H), 2.77 (d, *J* = 9.4 Hz, 1H), 2.68 (tt, *J* = 7.4, 3.9 Hz, 1H), 2.45 (ddd, *J* = 12.8, 8.2, 6.9 Hz, 1H), 1.71 (ddd, *J* = 12.5, 7.3, 5.0 Hz, 1H), 0.81 - 0.67 (m, 2H), 0.59 - 0.45 (m, 2H). LC-MS: [M+H]⁺ = 493.2. |
| 7 | | 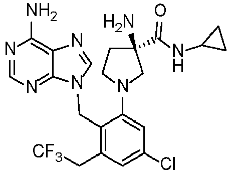 | ¹H NMR (400 MHz, Methanol-d₄) δ 8.22 (s, 1H), 8.00 (s, 1H), 7.39 (d, *J* = 2.1 Hz, 1H), 7.24 - 7.12 (m, 1H), 5.53 (s, 2H), 4.10 - 3.98 (m, 1H), 3.82 (dq, *J* = 15.5, 10.8 Hz, 1H), 3.63 (d, *J* = 9.4 Hz, 1H), 3.28 - 3.23 (m, 1H), 3.01 (td, *J* = 8.5, 4.7 Hz, 1H), 2.83 (d, *J* = 9.4 Hz, 1H), 2.66 (tt, *J* = 7.4, 3.9 Hz, 1H), 2.43 (ddd, *J* = 12.8, 8.2, 7.2 Hz, 1H), 1.75 (ddd, *J* = 12.4, 7.3, 4.7 Hz, 1H), 0.78 - 0.68 (m, 2H), 0.56 - 0.47 (m, 2H). LC-MS: [M+H]⁺ = 509.2/511.2. |
| 8 | | 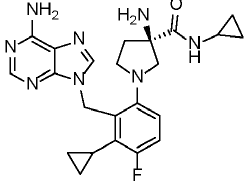 | ¹H NMR (400 MHz, Methanol-d₄) δ 8.24 (s, 1H), 7.75 (s, 1H), 7.30 (ddd, *J* = 9.1, 4.7, 0.7 Hz, 1H), 7.07 (dd, *J* = 10.5, 8.9 Hz, 1H), 5.77 (q, *J* = 13.9 Hz, 2H), 3.49 (d, *J* = 9.3 Hz, 1H), 3.15 - 3.05 (m, 1H), 2.96 (td, *J* = 8.7, 5.4 Hz, 1H), 2.80 (d, *J* = 9.3 Hz, 1H), 2.62 (tt, *J* = 7.2, 3.9 Hz, 1H), 2.41 (ddd, *J* = 12.9, 8.5, 6.2 Hz, 1H), 1.68 (dddd, *J* = 13.0, 7.8, 5.4, 0.7 Hz, 1H), 1.63 - 1.53 (m, 1H), 0.94 - 0.87 (m, 2H), 0.76 - 0.60 (m, 4H), 0.54 - 0.41 (m, 2H). LC-MS: [M+H]⁺ = 451.3. |
| 9 | | 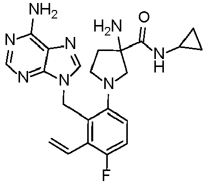 | ¹H NMR (400 MHz, Methanol-d₄) δ 8.26 (s, 1H), 7.77 (s, 1H), 7.39 (dd, *J* = 9.0, 4.7 Hz, 1H), 7.20 (dd, *J* = 10.1, 8.9 Hz, 1H), 6.78 (dd, *J* = 17.8, 11.7 Hz, 1H), 5.70 - 5.53 (m, 4H), 3.51 (d, *J* = 9.8 Hz, 1H), 3.04 - 2.96 (m, 3H), 2.66 (tt, *J* = 7.4, 3.9 Hz, 1H), 2.44 (dt, *J* = 14.4, 7.5 Hz, 1H), 1.90 - 1.80 (m, 1H), 0.78 - 0.65 (m, 2H), 0.55 - 0.44 (m, 2H). LC-MS: [M+H]⁺ = 437.3. |
| 10 | | 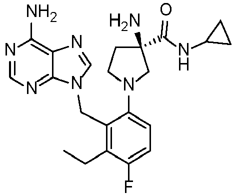 | ¹H NMR (400 MHz, Methanol-d₄) δ 8.27 (s, 1H), 7.74 (s, 1H), 7.33 (dd, J = 8.9, 5.0 Hz, 1H), 7.13 (t, J = 9.2 Hz, 1H), 5.63 - 5.49 (m, 2H), 3.50 (d, J = 9.3 Hz, 1H), 3.13 - 3.04 (m, 1H), 2.97 (td, J = 8.7, 5.4 Hz, 1H), 2.85 - 2.67 (m, 3H), 2.62 (tt, J = 7.4, 3.9 Hz, 1H), 2.41 (dt, *J* = 14.1, 7.4 Hz, 1H), 1.69 (dt, *J* = 13.0, 7.1 Hz, 1H), 0.92 (t, *J* = 7.5 Hz, 3H), 0.69 (tt, *J* = 8.1, 4.5 Hz, 2H), 0.53 - 0.40 (m, 2H). LC-MS: [M+H]⁺ = 439.2. |
| 11 | | 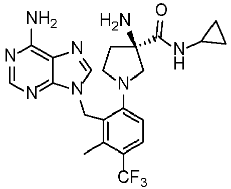 | ¹H NMR (400 MHz, Methanol-d₄) δ = 8.27 (s, 1H), 7.72-7.64 (m, 1H), 7.72-7.64 (m, 1H), 7.25 (d, *J* = 8.8 Hz, 1H), 5.64-5.53 (m, 2H), 3.68 (d, *J* = 9.4 Hz, 1H), 3.47-3.36 (m, 1H), 3.17-3.16 (m, 1H), 2.99-2.974 (m, 1H), 2.70-2.60 (m, 1H), 2.50-2.40 (m, 1H), 2.34 (s, 3H), 1.85-1.83 (m, 1H), 0.77-0.67 (m, 2H), 0.52-0.41 (m, 2H). LC-MS: [M+H]⁺ = 475.2. |
| 12 | | 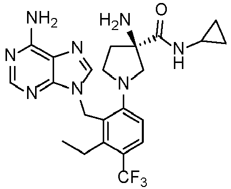 | ¹H NMR (400 MHz, Methanol-d₄) δ = 8.27 (s, 1H), 7.70 (d, *J* = 8.8 Hz, 1H), 7.65 (s, 1H), 7.27 (d, *J* = 8.8 Hz, 1H), 5.64-5.54 (m, 2H), 3.67 (d, *J* = 9.4 Hz, 1H), 3.44-3.36 (m, 1H), 3.11 (dt, *J* = 4.8, 8.7 Hz, 1H), 3.00-2.83 (m, 3H), 2.63 (tt, *J* = 3.8, 7.3 Hz, 1H), 2.44-2.34 (m, 1H), 1.76-1.68 (m, 1H), 1.14 (t, *J* = 7.4 Hz, 3H), 0.74-0.66 (m, 2H), 0.48 (dd, *J* = 1.8, 3.7 Hz, 2H). LC-MS: [M+H]⁺ = 489.2. |
| 13 | | 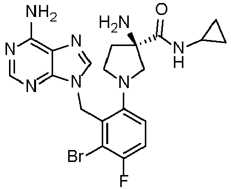 | ¹H NMR (400 MHz, Methanol-d₄) δ 8.25 (s, 1H), 7.78 (s, 1H), 7.44 (dd, *J* = 9.0, 4.8 Hz, 1H), 7.30 (dd, *J* = 9.0, 8.1 Hz, 1H), 5.79 - 5.51 (m, 2H), 3.53 (d, *J* = 9.2 Hz, 1H), 3.19 - 3.09 (m, 1H), 3.00 (td, *J* = 8.7, 5.4 Hz, 1H), 2.85 (d, *J* = 9.2 Hz, 1H), 2.62 (tt, *J* = 7.3, 3.9 Hz, 1H), 2.40 (ddd, *J* = 12.8, 8.6, 6.3 Hz, 1H), 1.71 (ddd, *J* = 12.9, 7.7, 5.5 Hz, 1H), 0.76 - 0.61 (m, 2H), 0.52 - 0.40 (m, 2H). LC-MS: [M+H]⁺ = 489.0/491.0. |
| 14 | | 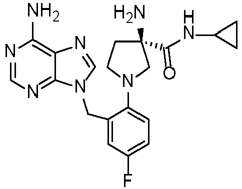 | ¹H NMR (400 MHz, Methanol-d₄) δ 8.21 (s, 1H), 8.15 (s, 1H), 7.29 (dd, J = 8.9, 5.0 Hz, 1H), 7.01 (td, J = 8.5, 3.0 Hz, 1H), 6.71 (dd, J = 9.3, 3.0 Hz, 1H), 5.60 - 5.41 (m, 2H), 3.57 (d, J = 9.3 Hz, 1H), 3.38 - 3.32 (m, 1H), 3.06 (td, J = 8.7, 5.1 Hz, 1H), 2.90 (d, J = 9.3 Hz, 1H), 2.69 (tt, *J* = 7.4, 3.9 Hz, 1H), 2.50 (ddd, *J* = 12.8, 8.5, 6.5 Hz, 1H), 1.81 (ddd, *J* = 12.8, 7.6, 5.1 Hz, 1H), 0.81 - 0.67 (m, 2H), 0.54 (ddd, *J* = 6.9, 4.9, 3.8 Hz, 2H). LC-MS: [M+H]⁺ = 411.2. |
| 15 | INT-39 | 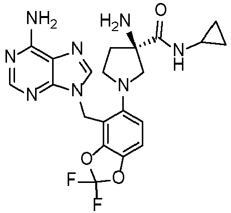 | ¹H NMR (400 MHz, Methanol-d₄) δ 8.25-8.23 (m, 1H), 8.17 (s, 1H), 7.24-7.18 (m, 2H), 5.61 (s, 2H), 3.62-3.60 (d, *J* = 10.4 Hz, 1H), 3.38-3.36 (m, 1H), 3.19-3.16 (t, *J* = 7.0 Hz, 2H), 2.77-2.74 (m, 1H), 2.59-2.57 (m, 1H), 2.29-2.23 (m, 1H), 0.79-0.72 (m, 2H), 0.61-0.58 (m, 2H). LC-MS: [M+H]⁺ = 473.2. |
| 16 | | 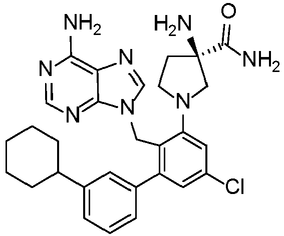 | ¹H NMR (400 MHz, Methanol-*d*₄) δ ppm 8.00 (s, 1 H), 7.25 - 7.39 (m, 2 H), 7.17 (t, *J* = 7.76 Hz, 1 H), 6.88 - 7.05 (m, 3 H), 6.68 (s, 1 H), 5.43 (s, 2 H), 3.72 (d, *J* = 9.41 Hz, 1 H), 3.37 - 3.52 (m, 2 H), 3.22 - 3.29 (m, 2 H), 3.10 (d, *J* = 9.29 Hz, 1 H), 2.45 - 2.54 (m, 1 H), 2.26 - 2.35 (m, 1 H), 1.89 (ddd, *J* = 12.65, 7.52, 5.01 Hz, 1 H), 1.79 (br d, *J* = 12.84 Hz, 2 H), 1.61 - 1.75 (m, 3 H), 1.14 - 1.41 (m, 6 H). LC-MS: [M+H]⁺ = 545.0. |
| 17 | | 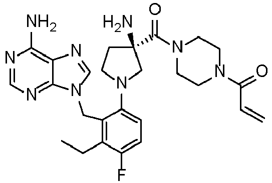 | ¹H NMR (400 MHz, Methanol-d₄) δ 8.32 (s, 1H), 7.69 (s, 1H), 7.47-7.42 (m, 1H), 7.22 (t, J = 8.0 Hz, 1H), 6.66 (s, 1H), 6.23 (d, J = 16.0 Hz, 1H), 5.79 (d, J = 12.0 Hz, 1H), 5.63 - 5.51 (m, 2H), 3.79-3.55 (m, 9H), 3.16-3.09 (m, 3H), 2.77 (s, 2H), 2.60 - 2.54 (m, 1H), 2.06 - 1.98 (m, 1H), 0.96 (t, J = 6.0 Hz, 3H). LC-MS: [M+H]⁺ = 521.9. |

### Example 18. (R)-9-(2-(3-amino-3-(1H-1,2,4-triazol-3-yl)pyrrolidin-1-yl)-6-bromo-4-chlorobenzyl)-9H-purin-6-amine

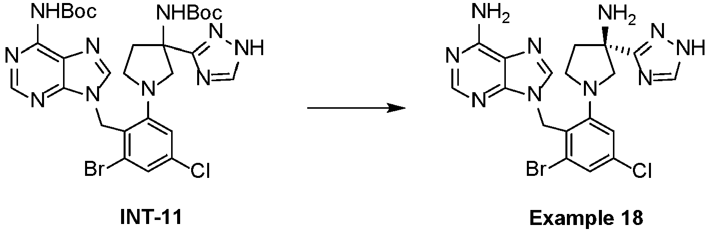

To a solution of tert-butyl (9-(2-bromo-6-(3-((tert-butoxycarbonyl)amino)-3-(1H-1,2,4-triazol-3-yl)pyrrolidin-1-yl)-4-chlorobenzyl)-9H-purin-6-yl)carbamate (INT-11) (75 mg, 0.11 mmol, 1 eq) dissolved in DCM (2 mL) was added in TFA (2 mL). The mixture was stirred for 1.2 hours at 26 °C. The reaction mixture was concentrated in vacuo and the crude compound was purified by prep-HPLC [column: Boston Green ODS 150*30 5u: gradient 15% - 25% B (A: water (0.1%TFA), B (MeCN))] to afford the racemic crude. Separation by SFC using an AD-H chiral column and 0.1% NH₄OH in MeOH gave (R)-9-(2-(3-amino-3-(1H-1,2,4-triazol-3-yl)pyrrolidin-1-yl)-6-bromo-4-chlorobenzyl)-9H-purin-6-amine (second eluent). ¹H NMR (400 MHz Methanol-*d*₄): δ 8.54 (s, 1 H), 8.36 (s, 1 H), 8.07 (s, 1 H),7.57 (d, *J*=1.6 Hz, 1 H), 7.48 (d, *J*=2.0 Hz, 1 H), 5.70 (q, *J*=14.4 Hz, 2 H), 3.80 (d, *J*=10.4 Hz, 1 H), 3.59 (d, *J*=10.4 Hz, 1 H), 3.41 - 3.31 (m, 2 H), 2.90- 2.82 (m, 1 H), 2.44- 2.37 (m, 1 H). LC-MS: [M+H]⁺ = 491.3.

The following examples were prepared from the correspodning intermediates following procedures analogous to Example 18.

| Ex No. | INT | | |
|---|---|---|---|
| 19 | INT-12 | 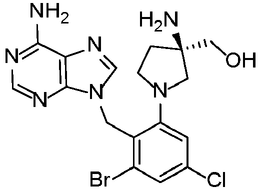 | ¹H NMR (400 MHz, Methanol-d₄) δ 8.26 (br s, 1 H) 7.74 (br s, 1 H). 7.41 (br s, 1 H) 7.23 (br s, 1 H) 5.48 - 5.64 (m, 2 H) 4.59 - 4.80 (m, 1 H) 3.44 (br s, 4 H) 3.15 - 3.24 (m, 2 H) 3.08 (br s, 1 H) 2.86 - 2.98 (m, 1 H) 1.86 - 1.99 (m, 1 H) 1.63 - 1.76 (m, 1 H) 1.29 (br s, 1 H) 0.09 (br s, 1 H) 0.00 (br s, 1 H). LC-MS: [M+H]⁺ = 454.2. |
| 20 | INT-20 | 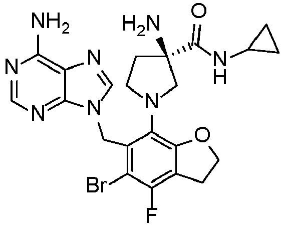 | ¹H NMR (400 MHz, Methanol-*d*4) δ 8.25 (s, 1H), 7.78 (s, 1H), 5.74 - 5.51 (m, 2H), 4.75 (t, *J* = 8.8 Hz, 2H), 3.58 (d, *J* = 9.0 Hz, 1H), 3.37 - 3.31 (m, 2H), 3.07 - 2.98 (m, 1H), 2.93 - 2.85 (m, 1H), 2.81 (d, *J* = 8.9 Hz, 1H), 2.67 - 2.59 (m, 1H), 2.47 - 2.37 (m, 1H), 1.64 - 1.51 (m, 1H), 0.73 - 0.64 (m, 2H), 0.52 - 0.41 (m, 2H). ¹⁹F NMR (376 MHz, Methanol-d4) δ -110.66. LC-MS: [M/M+2]⁺ = 531.0/533.0. |
| 21 | INT-21 | 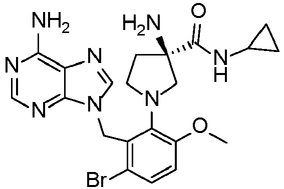 | ¹H NMR (400 MHz, Methanol-*d*4) δ 8.26 (s, 1H), 7.72 (s, 1H), 7.57 (d, *J* = 9.0 Hz, 1H), 7.09 (d, *J* = 9.0 Hz, 1H), 5.63 (dd, *J* = 83.4, 13.3 Hz, 2H), 3.89 (s, 3H), 3.60 (d, *J* = 8.9 Hz, 1H), 3.16 - 2.69 (m, 3H), 2.69 - 2.59 (m, 1H), 2.54 - 2.38 (m, 1H), 1.63 - 1.51 (m, 1H), 0.77 - 0.64 (m, 2H), 0.53 - 0.40 (m, 2H). LC-MS: [M/M+2]⁺ = 500.9/502.9. |
| 22 | INT-45 | 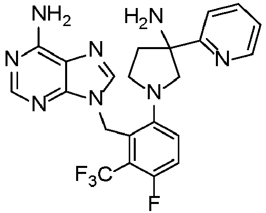 | ¹H NMR (400 MHz, Methanol-d₄) d 8.61 (d, *J* = 4.8 Hz, 1H), 8.30 (s, 1H), 7.96 (dd, *J*₁ = 9.2 Hz, *J*₂ = 4.8 Hz, 1H), 7.82-7.78 (m, 1H), 7.73 (s, 1H), 7.62 (d, *J* = 8.0 Hz, 1H), 7.56-7.47 (m, 1H), 7.38 (dd, *J*₁ = 8.0 Hz, *J*₂ = 4.8 Hz, 1H), 6.01-5.40 (m, 2H), 3.60-3.45 (m, 2H), 3.27-3.22 (m, 1H), 2.93-2.90 (m, 1H), 2.73-2.59 (m, 1H), 2.31-2.16 (m, 1H). LC-MS: [M+H]⁺ = 473.2. |
| 23 | INT-45 | 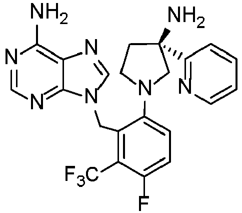 | ¹H NMR (400 MHz, MeOD) δ: 8.44 (d, *J* = 4.8 Hz, 1H), 8.23 (s, 1H), 7.90-7.76 ( m, 1H), 7.70-7.60 (m, 1H), 7.58 (s, 1H), 7.49-7.35(m, 2H), 7.22-7.10 (m, 1H), 5.75 (d, *J* = 14.4 Hz, 1H), 5.50 (d, *J* = 14.4 Hz, 1H), 3.52 (d, *J* = 9.6 Hz, 1H), 3.21-3.04 (m, 3H), 2.55-2.43 (m, 1H), 2.03-1.92 (m, 1H). LC-MS: [M+H]⁺ = 473.2. |
| 24 | | 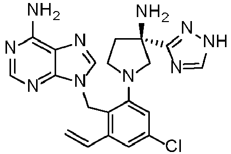 | ¹H NMR (400 MHz, Methanol-d₄) δ 8.59 (s, 1H), 8.43 (s, 1H), 8.05 (s, 1H), 7.45-7.34 (m, 2H), 6.96 (dd, *J* = 10.8, 17.2 Hz, 1H), 5.80-5.70 (m, 2H), 5.60 (d, *J* = 17.2 Hz, 1H), 5.26 (d, *J* = 10.8 Hz, 1H), 3.81 (d, *J* = 10.4 Hz, 1H), 3.57 (d, *J* = 10.4 Hz, 1H), 3.50-3.46 (m, 1H), 3.34-3.32 (m, 1H), 2.90-2.86 (m, 1H), 2.50-2.46 (m, 1H). LC-MS: [M+H]⁺ = 437.2. |
| 25 | | 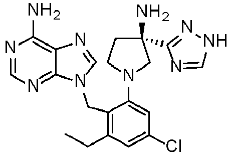 | ¹H NMR (400 MHz, Methanol-d₄) δ 8.25 (s, 1H), 8.24 (s, 1H), 7.60 (s, 1H), 7.22 (s, 1H), 7.07 (s, 1H), 5.55-5.40 (m, 2H), 3.66 (d, *J* = 9.2 Hz, 1H), 3.37-3.32 (m, 1H), 3.24-3.14 (m, 2H), 2.70-2.59 (m, 3H), 2.11-2.06 (m, 1H), 1.06 (t, *J* = 7.6 Hz, 3H). LC-MS: [M+H]⁺ = 439.2 [M+H]⁺. |
| 26 | | 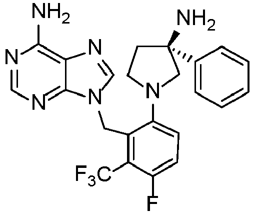 | ¹H NMR (400 MHz, MeOD) δ: 8.23 (s, 1H), 7.82 (dd, *J* = 9.2, 4.6 Hz, 1H), 7.62 (s, 1H), 7.52 - 7.38, m, 1H), 7.38 - 7.29 (m, 2H), 7.29 - 7.12 (m, 3H), 5.77 (dd, *J* = 14.3, 1.5 Hz, 1H), 5.50 (dd, *J* = 14.3, 1.3 Hz, 1H), 3.40 (d, *J* = 9.2 Hz, 1H), 3.27 - 3.10 (m, 2H), 3.04 (td, *J* = 8.9, 4.7 Hz, 1H), 2.36 (ddd, *J* = 12.9, 8.9, 7.0 Hz, 1H), 2.13 - 1.98 (m, 1H). LC-MS: [M+H]⁺ = 472.1. |
| 27 | INT-44 | 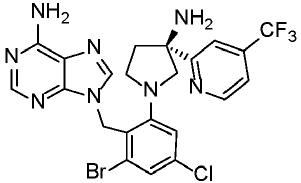 | ¹H NMR (DMSO-*d*₆) δ: 8.87 (s, 2H), 8.73 (d, *J* = 5.0 Hz, 1H), 8.13 (s, 1H), 8.02 (dd, *J* = 1.8, 1.0 Hz, 1H), 7.75 (s, 1H), 7.63 - 7.58 (m, 1H), 7.33 (d, *J* = 2.0 Hz, 1H), 7.20 (s, 2H), 7.12 (d, *J* = 2.0 Hz, 1H), 5.50 (d, *J* = 14.7 Hz, 1H), 5.33 (d, *J* = 14.7 Hz, 1H), 3.86 (d, *J* = 9.2 Hz, 1H), 3.72 (td, *J* = 8.8, 6.9 Hz, 1H), 3.26 - 3.19 (m, 1H), 3.12 (dd, *J* = 9.2, 1.1 Hz, 1H), 2.45 - 2.38 (m, 1H), 1.95 (ddd, *J* = 11.2, 9.3, 5.8 Hz, 1H). LC-MS: [M+H]⁺ = 569.9. |
| 28 | INT-46 | 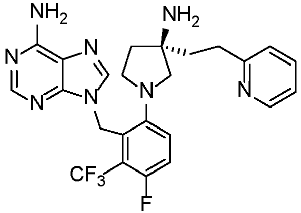 | ¹H NMR: (400 MHz, MeOD) δ: 8.41 (d, *J* = 4.7 Hz, 1H), 8.21 (s, 1H), 7.88 - 7.69 (m, 2H), 7.62 (s, 1H), 7.51 - 7.38 (m, 1H), 7.26 (dd, *J* = 7.7, 3.0 Hz, 2H), 5.77 (d, *J* = 14.2 Hz, 1H), 5.54 (d, *J* = 14.3 Hz, 1H), 3.05 (d, *J* = 9.4 Hz, 1H), 2.99 - 2.85 (m, 3H), 2.83 - 2.70 (m, 2H), 1.98 (dt, *J* = 14.8, 7.7 Hz, 1H), 1.87 (d, *J* = 6.4 Hz, 1H), 1.71 (dt, *J* = 13.1, 6.6 Hz, 1H), 1.48 - 1.19 (m, 1H), 1.04 - 0.82 (m, 1H). LC-MS: [M+H]⁺ = 501.1. |
| 29 | INT-43 | 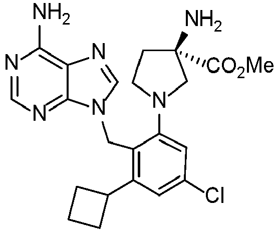 | ¹H NMR: (400 MHz, MeOD) δ: 8.28 (s, 1H), 7.59 (s, 1H), 7.28-7.19 (m, 2H), 5.55-5.40 (m, 2H), 3.68 (d, *J* = 8.8 Hz, 1H), 3.59-3.51 (m, 1H), 3.26-3.19 (m, 1H), 3.10-2.99 (m, 1H), 2.86 (d, *J* = 9.6 Hz, 1H), 2.66-2.58 (m, 1H), 2.46-2.34 (m, 1H), 2.18-2.01 (m, 4H), 1.97-1.85 (m, 1H), 1.82-1.68 (m, 2H), 0.75-0.63 (m, 2H), 0.53-0.38 (m, 2H). LC-MS: [M+H]⁺ = 481.1. |
| 30 | INT-42 | 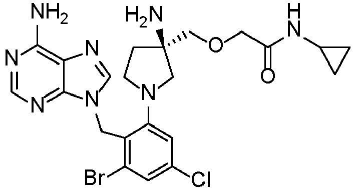 | ¹H NMR (400 MHz, Methanol-*d*4) δ 8.26 (s, 1H), 7.70 (s, 1H), 7.42 (d, *J* = 2.0 Hz, 1H), 7.23 (d, *J* = 2.0 Hz, 1H), 5.61 - 5.46 (m, 2H), 3.87 (d, *J* = 1.6 Hz, 2H), 3.33 (d, *J* = 3.2 Hz, 2H), 3.26 - 3.18 (m, 2H), 3.13 - 3.05 (m, 1H), 2.87 (d, *J* = 9.5 Hz, 1H), 2.70 - 2.61 (m, 1H), 1.92 - 1.82 (m, 1H), 1.75 - 1.65 (m, 1H), 0.75 - 0.65 (m, 2H), 0.54 - 0.48 (m, 2H). LC-MS: [M/M+2]⁺ = 549.0/551.0. |
| 31 | | 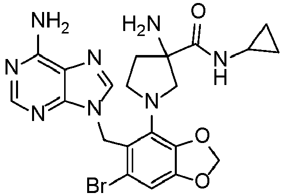 | ¹H NMR (400 MHz, Methanol-d4) δ 8.25 (s, 1H), 7.78 (s, 1H), 7.09 (s, 1H), 6.10 - 6.02 (m, 2H), 5.63 (d, *J* = 13.7 Hz, 1H), 5.49 (d, *J* = 13.7 Hz, 1H), 3.63 (d, *J* = 9.0 Hz, 1H), 3.13 - 3.06 (m, 1H), 3.02 - 2.94 (m, 1H), 2.89 - 2.83 (m, 1H), 2.67 - 2.59 (m, 1H), 2.47 - 2.36 (m, 1H), 1.64 - 1.56 (m, 1H), 0.73 - 0.65 (m, 2H), 0.51 - 0.42 (m, 2H). LC-MS: [M/M+2]⁺ = 514.8/516.8. |

### Example 32. (R)-9-(2-(3-amino-3-(methoxymethyl)pyrrolidin-1-yl)-6-bromo-4-chlorobenzyl)-9H-purin-6-amine

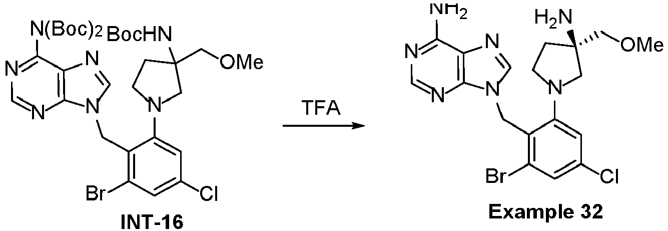

To a solution of tert-butyl (9-(2-bromo-6-(3-((tert-butoxycarbonyl)amino)-3-(methoxymethyl)pyrrolidin-1-yl)-4-chlorobenzyl)-9H-purin-6-yl)(tert-butoxycarbonyl)carbamate (INT-16) (85 mg, 0.11 mmol) dissolved in CH₂Cl₂ (3 mL) was added in TFA (2 mL) slowly. The reaction mixture was stirred at 22-31 °C for 2 hours. The mixture was concentrated in vacuo and the residue was dissolved in MeCN (3 mL). The solution was basified with NH₃.H₂O to pH 8-9 and purified by prep-HPLC [column: Waters Xbridge 150*25mm 5um, gradient: 13%-43% B (A = water/10 mM NH₄HCO₃/ B = MeCN), flow rate: 25 mL/min to afford the racemic crude as a white solid. Separation by SFC using an AD-H chiral column and 0.1% NH₄OH in MeOH gave (R)-9-(2-(3-amino-3-(methoxymethyl)pyrrolidin-1-yl)-6-bromo-4-chlorobenzyl)-9H-purin-6-amine (first eluent). ¹H NMR (400 MHz methanol-*d*₄): δ 8.27 (s, 1H), 7.71 (s, 1H), 7.42 (d, *J*=2.0 Hz, 1H), 7.22 (d, *J*=2.0 Hz, 1H), 5.46-5.63 (m, 2H), 3.27 (s, 3H), 3.22-3.26 (m, 1H), 3.21 (s, 2H), 3.16 (d, *J*=9.2 Hz, 1H), 3.07-3.12 (m, 1H), 2.83 (d, *J*=9.2 Hz, 1H), 1.85-1.93 (m, 1H), 1.64-1.72 (m, 1H). LC-MS: [M+H]⁺ = 467.8.

### Example 33. (R)-3-amino-1-(2-((6-amino-9H-purin-9-yl)methyl)-5-chloro-3-((R)-2,2,2-trifluoro-1-hydroxyethyl)phenyl)-N-cyclopropylpyrrolidine-3-carboxamide

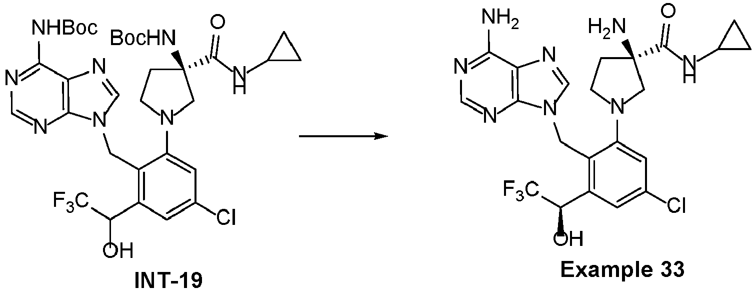

Tert-butyl ((3R)-1-(2-((6-((tert-butoxycarbonyl)amino)-9H-purin-9-yl)methyl)-5-chloro-3-(2,2,2-trifluoro-1-hydroxyethyl)phenyl)-3-(cyclopropylcarbamoyl)pyrrolidin-3-yl)carbamate (INT-19) (65 mg, 0.89 mmol) was mixed with a solution of hydrochloride in EtOAc (0.7 mL, 4M). The mixture was stirred at 25 °C for 12 h. The mixture was concentrated in vacuo and purified by pre-HPLC (Column: Phenomenex Synergi C18 150*25*10um, Condition:water(0.05%HCl)-MeCN) to afford the racemic crude as a white solid. Separation by SFC and purification by pre-HPLC(Column: Phenomenex Synergi C18 150*25*10um, Condition:water(0.05%HCl)-MeCN) gave (R)-3-amino-1-(2-((6-amino-9H-purin-9-yl)methyl)-5-chloro-3-((R)-2,2,2-trifluoro-1-hydroxyethyl)phenyl)-N-cyclopropylpyrrolidine-3-carboxamide as a white solid. ¹H NMR:(400MHz Methanol-*d*₄) δ 8.38 (s, 1H), 7.97 (s, 1H), 7.56-7.56 (m, 1H), 7.44 (s, 1H), 5.77-5.56 (m, 2H), 5.33-5.28 (m, 1H), 3.65-3.62 (m, 1H), 3.26-3.24 (m, 1H), 3.05-3.03 (m, 1H), 2.64-2.60 (m, 1H), 2.49-2.47 (m, 1H), 2.17-2.12 (m, 1H), 0.69-0.60 (m, 2H), 0.51-0.48 (m, 2H).). LC-MS: [M+H]⁺ = 525.1.

The following examples were prepared from corresponding intermediates following procedures analogous to those described in Example 33.

| Ex No. | INT | Structure | |
|---|---|---|---|
| 34 | INT-33 | 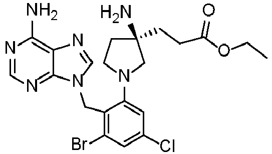 | ¹H NMR (400 MHz, DMSO-d₆) δ 8.71 (s, 3H), 8.23 (s, 1H), 7.78 (s, 1H), 7.54 (s, 1H), 7.34 (s, 3H), 5.75-5.33 (m, 2H), 4.09-4.04 (q, *J* = 6.9 Hz, 2H), 3.36-3.35 (m, 3H), 3.02-3.00 (m, 1H), 2.58-2.55 (m, 2H), 2.08-2.03 (m, 4H), 1.20-1.17 (t, *J* = 7.0Hz, 3H). LC-MS: [M+H]⁺ = 524.1. |
| 35 | INT-34 | 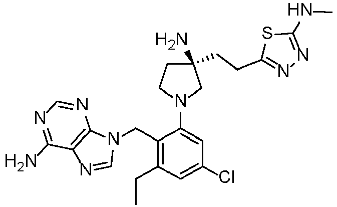 | ¹H NMR (400 MHz, Methanol-d₄) δ 8.49 (s, 1H), 8.29 (s, 1H), 7.68 (s, 1H), 7.33-7.32 (d, *J* = 2.0 Hz, 1H), 7.22 (d, *J* = 1.8Hz, 1H), 5.66-5.42 (m, 2H), 3.51 (s, 3H), 3.28-3.22 (m, 2H), 3.03-3.02 (m, 2H), 2.82-2.71 (m, 4H), 2.27-2.23 (m, 3H), 1.99-1.97 (m, 1H), 1.15-1.11 (t, *J* = 7.6 Hz, 3H). LC-MS: [M+H]⁺ = 513.1. |

### Example 36. (R)-3-(3-amino-1-(2-((6-amino-9H-purin-9-yl)methyl)-3-bromo-5-chlorophenyl)pyrrolidin-3-yl)-N-ethylpropanamide

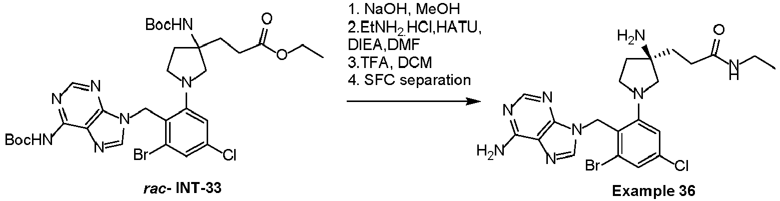

To a solution of ethyl 3-(1-(2-((6-(bis(*tert*-butoxycarbonyl)amino)-9*H*-purin-9-yl)methyl)-3-bromo-5-chlorophenyl)-3-((tert-butoxycarbonyl)amino)pyrrolidin-3-yl)propanoate (rac INT-33) (5 g, 6.9 mmol) in MeOH (50 mL) was added NaOH (966 mg, 24 mmol) in H₂O (13 mL) at 25 °C, the reaction mixture was stirred at 80 °C for 1.6 hrs. Then the reaction mixture was concentrated under vacuum to remove MeOH at 40 °C for 1h. The residue was adjusted to pH = 5 with aq. HCl (2 M). The mixture was extracted with EtOAc (20 mL × 3). The organic phase was concentrated to give a crude acid as yellow oil.

To a solution of the above crude acid (200 mg, 288 umol, 1 eq.), EtNH₂.HCl (130 mg, 2.9 mmol) and HATU (218 mg, 576 umol) in DMF (2 mL) was added DIEA (445 mg, 3.4 mmol) at 25 °C. The reaction mixture was stirred at 25 °C for 4 hrs. The reaction mixture was diluted with H₂O (30 mL) and extracted with EtOAc (5 mL × 3), the combined organic layer was dried over anhydrous sodium sulfate, filtered and concentrated under vacuum to give crude amide (260 mg) as a yellow oil, which was dissolved in DCM (2 mL) was added TFA (1 mL) at 25 °C. The reaction mixture was stirred at 25 °C for 3 hrs. The reaction mixture was concentrated and purified by prep-HPLC [column: Phenomenex Gemini 150 × 25 mm × 10 um, gradient 30 %-60 % B (A = water (10mM NH₄HCO₃), B = MeCN), flow rate: 25 mL/min] to afford the racemic compound as a white solid. Separation by SFC [Column: Chiralpak IC-3 50×4.6mm I.D., 3um Mobile phase: Phase A for CO₂, and Phase B for MeOH (0.05%DEA);Gradient elution: 40% MeOH (0.05% DEA) in CO₂ Flow rate: 3mL/min; Wavelength: 220nm] gave (*R*)-3-(3-amino-1-(2-((6-amino-9H-purin-9-yl)methyl)-3-bromo-5-chlorophenyl)pyrrolidin-3-yl)-*N*-ethylpropanamide as a yellow solid (first peak). ¹H NMR (400 MHz, Methanol-d₄) δ = 8.28 (s, 1H), 7.77 (s, 1H), 7.41 (s, 1H), 7.22 (s, 1H), 5.67-5.48 (m, 2H), 3.24-3.14 (m, 4H), 3.07-3.04 (m, 1H), 2.93 (d, *J* = 8.8 Hz, 1H), 2.29-2.18 (m, 2H), 1.95-1.72 (m, 4H), 1.12 (t, *J =* 7.2 Hz, 3H). LC-MS: [M+H]⁺ = 523.0.

The following examples were prepared from corresponding intermediates following procedures analogous to those described in Example 36.

| Ex No. | | |
|---|---|---|
| 37 | 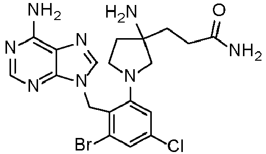 | ¹H NMR (400 MHz, Methanol-d₄) δ = 8.29 (s, 1H), 7.78 (s, 1H), 7.41 (d, *J* = 2.0 Hz, 1H), 7.22 (d, *J* = 2.0 Hz, 1H), 5.67-5.50 (m, 2H), 3.44-3.38 (m, 1H), 3.21-3.15 (m, 1H), 3.08-3.05 (m, 1H), 2.94 (d, *J* = 9.2 Hz, 1H), 2.35-2.20 (m, 2H), 1.98-1.71(m, 4H). LC-MS: [M+H]⁺ = 495.0. |
| 38 | 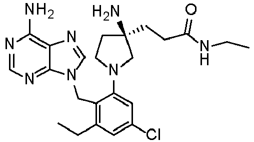 ( | ¹H NMR (400 MHz, Methanol-d₄) δ = 8.30-8.25 (m, 1H), 7.60 (s, 1H), 7.16 (d, *J* = 2.0 Hz, 1H), 7.06 (d, *J* = 2.0 Hz, 1H), 5.56-5.40 (m, 2H), 3.24-3.14 (m, 3H), 3.06 (d, *J* = 9.2 Hz, 1H), 2.87-2.85 (m, 1H), 2.65 (q, *J* = 7.6 Hz, 2H), 2.27-2.16 (m, 2H), 1.95-1.79 (m, 3H), 1.75-1.63 (m, 1H), 1.69-1.67 (m, 1H), 1.09 (q, *J* = 7 .6 Hz, 6H). LC-MS: [M+H]⁺ = 471.3. |

### Example 39. (R)-9-(2-(3-(2-(2H-tetrazol-5-yl)ethyl)-3-aminopyrrolidin-1-yl)-6-bromo-4-chlorobenzyl)-9H-purin-6-amine

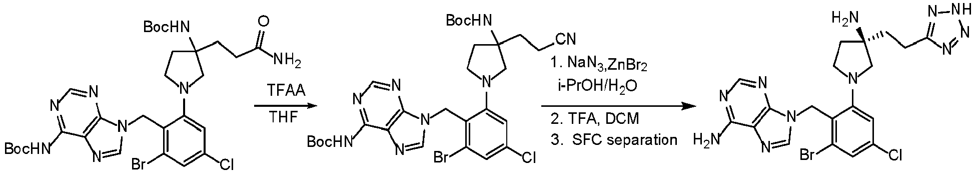

To a solution of tert-butyl (9-(2-(3-(3-amino-3-oxopropyl)-3-((tert-butoxycarbonyl)amino)pyrrolidin-1-yl)-6-bromo-4-chlorobenzyl)-9H-purin-6-yl)carbamate (1.8 g, 2.6 mmol) in THF (18 mL) was added trifluoroacetic anhydride (1.36 g, 6.5 mmol) and TEA (1.4 g, 14.3 mmol) at 15 °C. The reaction mixture was stirred at 15 °C for 15 min. The reaction mixture was diluted with H₂O (100 mL) and extracted with EtOAc (20 mL × 3), the combined organic layer dried over anhydrous sodium sulfate, filtered and concentrated under vacuum to give *tert*-butyl (9-(2-bromo-6-(3-((tert-butoxycarbonyl)amino)-3-(2-cyanoethyl)pyrrolidin-1-yl)-4-chlorobenzyl)-9H-purin-6-yl)carbamate as a yellow oil. LC-MS: [M+H]⁺ = 677.4.

To a solution of *tert*-butyl (9-(2-bromo-6-(3-((tert-butoxycarbonyl)amino)-3-(2-cyanoethyl)pyrrolidin-1-yl)-4-chlorobenzyl)-9H-purin-6-yl)carbamate (2 g, 2.9 mmol) in i-PrOH/H₂O (13 mL/7 mL) was added NaN₃ (383 mg, 5.9 mmol) and ZnBr₂ (663 mg, 2.9 mmol) at 15 °C. The reaction mixture was stirred at 120 °C for 16 hrs. The reaction mixture was diluted with H₂O (20 mL) and concentrated to remove i-PrOH, then extracted with EtOAc (15 mL × 3). The combined organic layer was dried over anhydrous sodium sulfate, filtered and concentrated. The crude residue was diluted with DCM (4 mL) and TFA (2 mL) was added at 15 °C. The reaction mixture was stirred at 15 °C for 2 hrs. The reaction mixture was concentrated and purified by prep-HPLC [column: Phenomenex Gemini 150 × 25 mm × 10 um, gradient 30 %-60 % B (A = water (10mM NH₄HCO₃), B = MeCN), flow rate: 25 mL/min] to afford racemic compound. Separation by SFC [Column: Chiralpak IC-3 50×4.6mm I.D., 3um Mobile phase: Phase A for CO₂, and Phase B for MeOH (0.05% DEA); Gradient elution: 40% MeOH (0.05% DEA) in CO₂ Flow rate: 3mL/min; Wavelength: 220nm] gave (*R*)-9-(2-(3-(2-(2*H*-tetrazol-5-yl)ethyl)-3-aminopyrrolidin-1-yl)-6-bromo-4-chlorobenzyl)-9*H*-purin-6-amine as a white solid (first peak). ¹H NMR (400 MHz, Methanol-d₄) δ 8.31 (s, 1H), 7.98 (s, 1H), 7.60 (d, *J* = 1.8 Hz, 1H), 7.46 (d, *J* = 2.0 Hz, 1H), 5.79-5.48 (m, 2H), 3.31-2.28 (m, 2H), 3.10-3.00 (m, 4H), 2.31-2.19 (m, 3H), 2.06-1.98 (m, 1H). LC-MS: [M+H]⁺ = 520.1.

### Example 40 was prepared from the corresponding intermediate following procedures analogous to those described in Example 39.

| Ex No. | | |
|---|---|---|
| 40 | 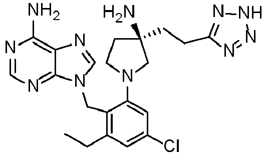 | ¹H NMR (400 MHz, Methanol-d₄) δ 8.27 (s, 1H), 7.62 (s, 1H), 7.16 (d, *J* = 2.0 Hz, 1H), 7.04 (d, *J* = 2.0 Hz, 1H), 5.60-5.41 (m, 2H), 3.28-3.21 (m, 1H), 3.11 (d, *J* = 9.4 Hz, 1H), 3.01-2.87 (m, 4H), 2.63 (q, *J* = 7.6 Hz, 2H), 2.04-1.93 (m, 3H), 1.77-1.68 (m, 1H), 1.06 (t, *J* = 7.6 Hz, 3H). LC-MS: [M+H]⁺ = 468.2 [M+H]⁺. |

### Example 41. (R)-3-amino-1-(2-((6-amino-9H-purin-9-yl)methyl)-3-bromo-4-(trifluoromethyl)phenyl)-N-cyclopropylpyrrolidine-3-carboxamide

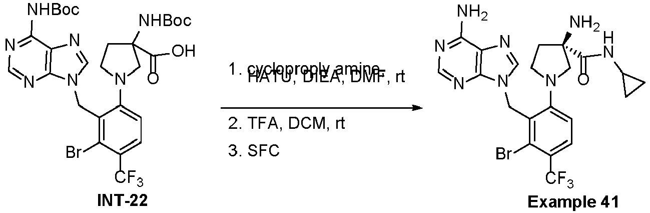

To a DMF solution (3 mL) of 1-(3-bromo-2-((6-((*tert*-butoxycarbonyl)amino)-9*H*-purin-9-yl)methyl)-4-(trifluoromethyl)phenyl)-3-((*tert*-butoxycarbonyl)amino)pyrrolidine-3-carboxylic acid (INT-22) (110 mg, 0.16 mmol), DIPEA (69 uL, 0.4 mmol) and cyclopropanamine (18 mg, 0.3 mmol) was added HATU (119 mg, 0.3 mmol) at rt. After stirring for 1 hr, the reaction mixture was diluted with EA, washed with water and brine. The organic layer was dried over Na₂SO₄, and concentrated under vacuum to give the crude product.

The crude product was dissolved in DCM (3 mL) and treated with TFA (2 mL) at rt. The reaction was stirred for 1 hr, then concentrated under vacuum to give the crude product. Purification by prep-HPLC (Column: XBridge 30*150mm 5um, gradient: 30-100% B (A = water (0.05% NH₄OH), B = acetonitrile (0.05% NH₄OH)), flow rate: 30 mL/min) gave a white powder, which was separated by SFC to give (R)-3-amino-1-(2-((6-amino-9*H*-purin-9-yl)methyl)-3-bromo-4-(trifluoromethyl)phenyl)-N-cyclopropylpyrrolidine-3-carboxamide. ¹H NMR (400 MHz, MeOH-*d*₄): δ 8.26 (s, 1H), 7.78 (s, 1H), 7.73 (d, *J* = 8.9 Hz, 1H), 7.24 (d, *J* = 8.8 Hz, 1H), 5.66 - 5.73 (m, 1H), 5.55 - 5.62 (m, 1H), 3.77 (d, *J* = 9.5 Hz, 1H), 3.59 (q, *J* = 7.9 Hz, 1H), 3.23 - 3.29 (m, 1H), 3.00 (d, *J* = 9.5 Hz, 1H), 2.62 (tt, *J* = 7.3, 3.8 Hz, 1H), 2.40 (dt, *J* = 12.6, 8.3 Hz, 1H), 1.75 - 1.84 (m, 1H), 0.66 - 0.75 (m, 2H), 0.43 - 0.51 (m, 2H). LC-MS: [M+H]⁺ = 540.7.

The following examples were prepared from corresponding intermediates following procedures analogous to those described in Example 41.

| Ex No. | INT | | |
|---|---|---|---|
| 42 | INT-23 | 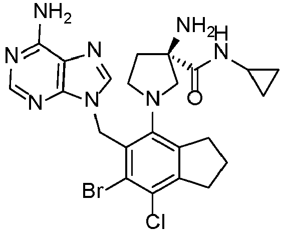 | ¹H NMR (400 MHz, MeOH-d₄): δ 8.26 (s, 1H), 7.73 (s, 1H), 5.80 (d, *J* = 13.4 Hz, 1H), 5.61 (d, *J* = 13.4 Hz, 1H), 3.62 (d, *J* = 8.8 Hz, 1H), 3.07 - 3.18 (m, 3H), 3.03 (t, *J* = 7.5 Hz, 2H), 2.92 (q, *J* = 7.7 Hz, 1H), 2.83 (d, *J* = 8.8 Hz, 1H), 2.62 (tt, *J* = 7.3, 3.8 Hz, 1H), 2.43 (ddd, *J* = 12.7, 9.0, 7.0 Hz, 1H), 2.17 (quin, *J* = 7.4 Hz, 2H), 1.62 (ddd, *J* = 12.6, 7.6, 4.7 Hz, 1H), 0.67 - 0.75 (m, 2H), 0.44 - 0.51 (m, 2H). LC-MS: [M+H]⁺ = 547.0. |
| 43 | INT-24 | 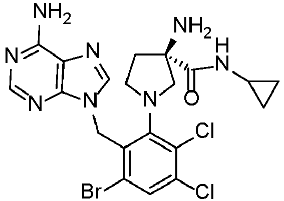 | ¹H NMR (400 MHz, MeOH-*d*₄) δ 8.23 (s, 1H), 7.90 (s, 1H), 7.86 (s, 1H), 5.75 (br d, *J* = 13.45 Hz, 1H), 5.55 (brd, *J* = 13.69 Hz, 1H), 3.82 (br s, 1H), 2.91 (br s, 1H), 2.49 - 2.69 (m, 2H), 1.69 (br s, 1H), 0.70 (br d, *J* = 6.85 Hz, 2H), 0.48 (br s, 2H). LC-MS: [M+H]⁺ = 540.8; |
| 44 | INT-25 | 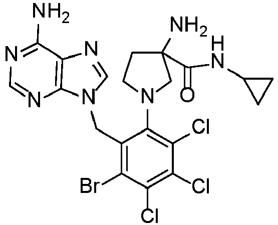 | ¹H NMR (400 MHz, MeOH-*d*₄) δ 8.23 (s, 1H), 7.89 (s, 1 H), 5.83 (br d, *J* = 13.82 Hz, 1H), 5.66 (d, *J* = 13.69 Hz, 1H), 3.82 (br s, 1H), 2.71 - 3.11 (m, 1H), 2.43 - 2.63 (m, 2H), 1.58 - 1.81 (m, 1H), 0.69 (br d, *J* = 6.48 Hz, 2H), 0.47 (br s, 2H). LC-MS: [M+H]⁺ = 574.7. |

### Example 45. (R)-3-amino-1-(2-((6-amino-9H-purin-9-yl)methyl)-3-((dimethylamino)methyl)-4-fluorophenyl)-N-cyclopropylpyrrolidine-3-carboxamide

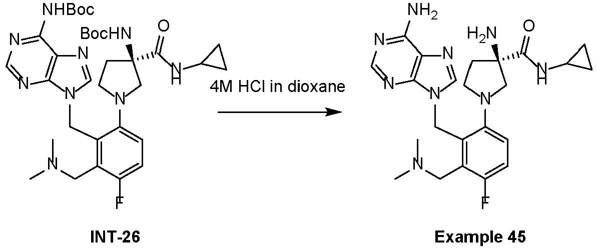

A solution of *tert-butyl (R*)-(9-(6-(3-((tert-butoxycarbonyl)amino)-3-(cyclopropylcarbamoyl)pyrrolidin-1-yl)-2-((dimethylamino)methyl)-3-fluorobenzyl)-9*H*-purin-6-yl)carbamate (INT-26) in 4M HCl in dioxane (2 mL) was reacted at RT for 3 hrs. The reaction mixture was concentrated. The residue was adjusted to pH = 8 with NH₃·H₂O and was purified by prep-HPLC [Column: Waters Xbridge 150*25*5um; Condition: water(10mM NH₄HCO₃)-MeCN; Flow rate: 25 mL/min] to give (*R*)-3-amino-1-(2-((6-amino-9*H*-purin-9-yl)methyl)-3-((dimethylamino)methyl)-4-fluorophenyl)-*N*-cyclopropylpyrrolidine-3-carboxamide as an off-white solid. ¹H NMR (400 MHz, Methanol-d₄) δ 8.24 (s, 1H), 7.92 (s, 1H), 7.42 (dd, *J* = 9.0, 4.9 Hz, 1H), 7.16 (t, *J* = 9.2 Hz, 1H), 5.69 - 5.54 (m, 2H), 3.73 (dd, *J* = 12.9, 2.5 Hz, 1H), 3.60 (dd, *J* = 12.9, 2.3 Hz, 1H), 3.49 (d, *J* = 9.3 Hz, 1H), 2.97 (td, *J* = 8.1, 6.3 Hz, 1H), 2.86 (td, *J* = 8.6, 5.6 Hz, 1H), 2.75 (d, *J* = 9.3 Hz, 1H), 2.64 (tt, *J* = 7.3, 3.8 Hz, 1H), 2.37 (ddd, *J* = 12.9, 8.4, 6.2 Hz, 1H), 2.23 (s, 6H), 1.60 (ddd, *J* = 13.0, 7.5, 5.6 Hz, 1H), 0.77 - 0.65 (m, 2H), 0.54 - 0.44 (m, 2H). LC-MS: [M+H]⁺ =468.2.

The following examples were prepared from corresponding intermediates following procedures analogous to Example 45.

| Ex No. | | |
|---|---|---|
| 46 | 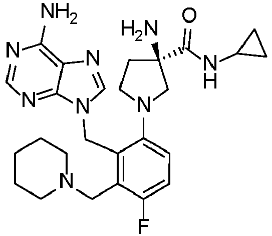 | ¹H NMR (400 MHz, Methanol-d₄) δ 8.26 (s, 1H), 7.85 (s, 1H), 7.43 (dd, *J* = 9.0, 5.0 Hz, 1H), 7.17 (t, *J* = 9.1 Hz, 1H), 5.69 - 5.50 (m, 2H), 3.67 - 3.53 (m, 2H), 3.47 (d, *J* = 9.2 Hz, 1H), 3.00 - 2.83 (m, 2H), 2.77 (d, *J* = 9.2 Hz, 1H), 2.62 (tq, *J* = 7.3, 3.8 Hz, 1H), 2.44 - 2.28 (m, 5H), 1.58 (ddd, *J* = 13.1, 7.7, 5.7 Hz, 1H), 1.40 - 1.28 (m, 6H), 0.73 - 0.63 (m, 2H), 0.52 - 0.42 (m, 2H). LC-MS: [M+H]⁺ = 508.3. |
| 47 | 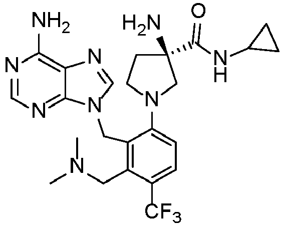 | ¹H NMR: (400MHz, Methanol-*d₄*) δ = 8.23 (s, 1H), 7.71-7.66 (m, 2H), 7.31 (d, *J* = 8.8 Hz, 1H), 5.87-5.67 (m, 2H), 3.81 (d, *J* = 13.8 Hz, 1H), 3.70 (d, *J* = 9.6 Hz, 1H), 3.63 (br d, *J* = 14.4 Hz, 1H), 3.49-3.39 (m, 1H), 3.12-3.11 (m, 1H), 2.89 (d, *J* = 9.0 Hz, 1H), 2.64 (tt, *J* = 3.8, 7.3 Hz, 1H), 2.39 (td, *J* = 8.0, 12.3 Hz, 1H), 2.16 (s, 6H), 1.73-1.65 (m, 1H), 0.75-0.69 (m, 2H), 0.52-0.46 (m, 2H). LC-MS: [M+H]⁺ = 518.4 |
| 48 | 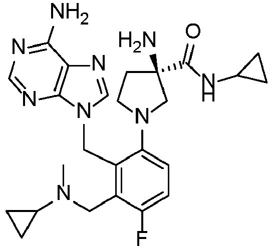 | ¹H NMR (400 MHz, DMSO-*d₆*) δ 8.15 (s, 1H), 7.86 (s, 1H), 7.83 (s, 1H), 7.34-7.29 (m, 1H), 7.22-7.08 (m, 3H), 5.52-5.31 (m, 2H), 3.94-3.86 (m, 1H), 3.84-3.73 (m, 1H), 3.02 (q, *J*=7.4 Hz, 1H), 3.07-2.81 (m, 1H), 2.70 (d, *J*=9.0 Hz, 1H), 2.65-2.61 (m, 1H), 2.30-2.21 (m, 1H), 2.16 (s, 4H), 1.74-1.62 (m, 1H), 1.60-1.48 (m, 1H), 0.66-0.54 (m, 2H), 0.49-0.38 (m, 2H), 0.32-0.27 (m, 2H), 0.13-0.02 (m, 2H). LC-MS: [M+H]⁺ = 494.4. |
| 49 | 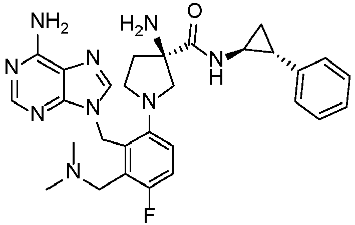 | ¹H NMR: (400MHz, Methanol-*d₄*) δ = 8.26 (s, 1H), 7.95 (s, 1H), 7.47-7.45 (m, 1H), 7.28-7.10 (m, 6H), 5.73-5.57 (m, 2H), 3.75 (d, *J* = 12 Hz, 1H), 3.63 (d, *J* = 12 Hz, 1H), 3.54 (d, *J* = 9.4 Hz, 1H), 3.04-2.96 (m, 1H), 2.92-2.85 (m, 2H), 2.82 (d, *J* = 9.4 Hz, 1H), 2.47-2.37 (m, 1H), 2.27 (s, 6H), 2.12-2.02 (m, 1H), 1.71-1.60 (m, 1H), 1.25-1.16(m, 2H). LC-MS: [M+H]⁺ = 544.3. |
| 50 | 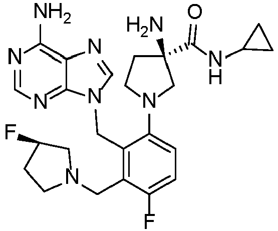 | ¹H NMR (400 MHz, Methanol-d₄) δ 8.25 (s, 1H), 7.94 (s, 1H), 7.44 (dd, *J* = 9.0, 5.0 Hz, 1H), 7.18 (t, *J* = 9.1 Hz, 1H), 5.72 (d, *J* = 13.9 Hz, 1H), 5.54 (d, *J* = 13.9 Hz, 1H), 5.13 - 4.96 (m, 1H), 3.91 (dd, *J* = 12.9, 2.9 Hz, 1H), 3.76 (dd, *J* = 12.8, 2.4 Hz, 1H), 3.47 (d, *J* = 9.3 Hz, 1H), 2.93 - 2.72 (m, 5H), 2.71 - 2.55 (m, 2H), 2.42 - 2.30 (m, 2H), 2.16 - 1.95 (m, 1H), 1.94 - 1.74 (m, 1H), 1.55 (dt, *J* = 13.1, 6.7 Hz, 1H), 0.77 - 0.62 (m, 2H), 0.57 - 0.42 (m, 2H). LC-MS: [M+H]⁺ = 512.3 |
| 51 | 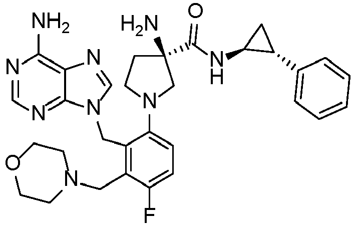 | ¹H NMR (400 MHz, Methanol-*d*₄) δ = 8.27 (s, 1H), 7.82 (s, 1H), 7.49-7.47 (m, 1H), 7.29-7.07(m, 6H), 5.73-5.57 (m, 2H), 3.73-3.59 (m, 2H), 3.55-3.54 (m, 1H), 3.38 (s, 4H), 3.14-3.06 (m, 1H), 2.00-2.98 (m, 1H), 2.90-2.80 (m, 2H), 2.48-2.33 (m, 5H), 2.06-1.99 (m, 1H), 1.75-1.66 (m, 1H), 1.18-1.13 (m, 2H). LC-MS: [M+H]⁺ = 586.3. |
| 52 | 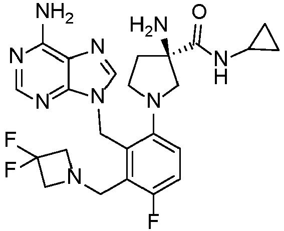 | ¹H NMR (400 MHz, Methanol-*d*₄) δ 8.24 (s, 1H), 7.94 (s, 1H), 7.44 (dd, *J* = 9.0, 5.1 Hz, 1H), 7.19 (t, *J* = 9.2 Hz, 1H), 5.68 (d, *J* = 14.1 Hz, 1H), 5.60 (d, *J* = 14.1 Hz, 1H), 4.05 - 3.97 (m, 1H), 3.96 - 3.88 (m, 1H), 3.58 - 3.48 (m, 5H), 3.00 (ddd, *J* = 9.0, 7.6, 6.2 Hz, 1H), 2.89 (td, *J* = 8.7, 5.6 Hz, 1H), 2.77 (d, *J* = 9.3 Hz, 1H), 2.64 (td, *J* = 7.2, 3.7 Hz, 1H), 2.39 (ddd, *J* = 12.8, 8.4, 6.2 Hz, 1H), 1.63 (ddd, *J* = 12.9, 7.6, 5.5 Hz, 1H), 0.78 - 0.64 (m, 2H), 0.57 - 0.43 (m, 2H). LC-MS: [M+H]⁺ = 516.2. |
| 53 | 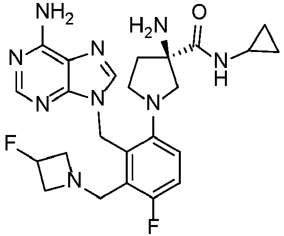 | ¹H NMR (400 MHz, Methanol-*d*₄) δ 8.24 (s, 1H), 7.94 (s, 1H), 7.42 (dd, *J* = 9.0, 5.1 Hz, 1H), 7.17 (t, *J* = 9.1 Hz, 1H), 5.65 (d, *J* = 14.0 Hz, 1H), 5.56 (d, *J* = 14.1 Hz, 1H), 5.09 - 4.88 (m, 1H), 4.03 - 3.76 (m, 2H), 3.59 - 3.45 (m, 3H), 3.28 - 3.14 (m, 2H), 2.98 (ddd, *J* = 9.0, 7.7, 6.2 Hz, 1H), 2.87 (td, *J* = 8.7, 5.5 Hz, 1H), 2.77 (d, *J* = 9.3 Hz, 1H), 2.64 (tt, *J* = 7.4, 3.9 Hz, 1H), 2.38 (ddd, *J* = 12.9, 8.5, 6.2 Hz, 1H), 1.62 (ddd, *J* = 13.0, 7.6, 5.5 Hz, 1H), 0.78 - 0.63 (m, 2H), 0.57 - 0.43 (m, 2H). LC-MS: [M+H]⁺ = 498.1. |
| 54 | 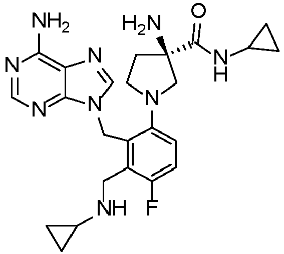 | ¹H NMR (400 MHz, Methanol-*d*₄) δ = 8.48 (s, 1H), 8.21 (s, 1H), 7.80-7.77 ( m, 1H), 7.43-7.38 (m, 1H), 5.96-5.81 (m, 2H), 4.56 (s, 2H), 3.67 (d, *J* = 10.8 Hz, 1H), 3.42-3.34 (m, 1H), 3.25-3.09 (m, 2H), 2.94-2.85 (m, 1H), 2.76-2.73 (m, 1H), 2.69-2.59 (m, 1H), 2.33-2.20 (m, 1H), 1.09-0.92 (m, 4H), 0.84-0.69 (m, 2H), 0.66-0.55 (m, 2H). LC-MS: [M+H]⁺ = 480.3. |
| 55 | 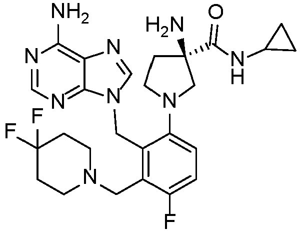 | ¹H NMR (400 MHz, Methanol-d₄) δ 8.36 (s, 1H), 8.03 (s, 1H), 7.61 (dd, *J* = 9.0, 5.0 Hz, 1H), 7.32 (t, *J* = 9.1 Hz, 1H), 5.79 - 5.60 (m, 2H), 3.89 (s, 2H), 3.56 (d, *J* = 10.6 Hz, 1H), 3.03 (dd, *J* = 9.3, 5.2 Hz, 1H), 2.90 (td, *J* = 8.7, 5.8 Hz, 1H), 2.80 - 2.63 (m, 5H), 2.47 (ddd, *J* = 15.0, 9.3, 5.9 Hz, 1H), 2.07 (ddd, *J* = 14.1, 8.3, 5.2 Hz, 1H), 1.84 (s, 4H), 0.93 - 0.82 (m, 1H), 0.79 - 0.72 (m, 1H), 0.68 (dddd, *J* = 10.4, 7.1, 4.8, 2.9 Hz, 1H), 0.57 - 0.43 (m, 2H). LC-MS: [M+H]⁺ = 544.3. |
| 56 | 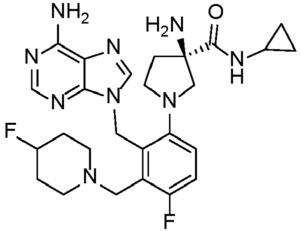 | ¹H NMR (400 MHz, Methanol-d₄) δ 8.28 (s, 1H), 8.04 (s, 1H), 7.49 (dd, *J* = 9.0, 5.0 Hz, 1H), 7.22 (t, *J* = 9.1 Hz, 1H), 5.70 - 5.48 (m, 2H), 4.65 - 4.45 (m, 1H), 3.76 - 3.59 (m, 2H), 3.49 (d, *J* = 10.0 Hz, 1H), 3.02 (d, *J* = 9.9 Hz, 1H), 2.93 (td, *J* = 9.0, 5.5 Hz, 1H), 2.81 (q, *J* = 8.2 Hz, 1H), 2.68 - 2.53 (m, 3H), 2.38 (ddd, *J* = 14.3, 8.9, 5.8 Hz, 3H), 1.78 (ddd, *J* = 13.7, 7.9, 5.5 Hz, 1H), 1.65 (d, *J=* 44.8 Hz, 4H), 0.76 - 0.63 (m, 2H), 0.52 - 0.42 (m, 2H). LC-MS: [M+H]⁺ = 526.3. |
| 57 | 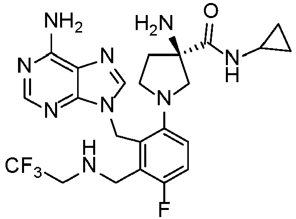 | ¹H NMR (400 MHz, Methanol-d₄) δ = 8.23 (s, 1H), 8.11 (s, 1H), 7.42 (dd, *J* = 5.2, 8.8 Hz, 1H), 7.18 (t, *J* = 9.2 Hz, 1H), 5.69-5.55 (m, 2H), 4.17-4.06 (m, 2H), 3.47 (d, *J* = 9.2 Hz, 1H), 3.29-3.22 (m, 2H), 2.94-2.86 (m, 1H), 2.78 (dt, *J* = 5.6, 8.8 Hz, 1H), 2.71 (d, *J* = 9.2 Hz, 1H), 2.68-2.64 (m, 1H), 2.42-2.32 (m, 1H), 1.64-1.54 (m, 1H), 0.77-0.68 (m, 2H), 0.56-0.45 (m, 2H). LC-MS: [M+H]⁺ = 522.2. |
| 58 | 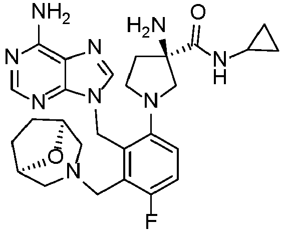 | ¹H NMR (400 MHz, Methanol-d₄) δ 8.24 (s, 1H), 7.91 (s, 1H), 7.40 (dd, *J* = 9.0, 5.0 Hz, 1H), 7.14 (t, *J* = 9.1 Hz, 1H), 5.79 - 5.62 (m, 2H), 4.27 - 4.18 (m, 2H), 3.72 (dd, *J* = 12.9, 2.5 Hz, 1H), 3.60 (dd, *J* = 12.8, 2.4 Hz, 1H), 3.51 (d, *J* = 9.2 Hz, 1H), 3.07 (ddd, *J* = 9.0, 7.6, 6.4 Hz, 1H), 2.91 (td, *J* = 8.6, 5.4 Hz, 1H), 2.77 (d, *J* = 9.2 Hz, 1H), 2.69 - 2.56 (m, 2H), 2.52 (d, *J* = 10.9 Hz, 1H), 2.38 (tt, *J* = 11.5, 7.9 Hz, 3H), 1.80 - 1.70 (m, 4H), 1.64 (ddd, *J* = 12.9, 7.6, 5.4 Hz, 1H), 0.77 - 0.63 (m, 2H), 0.57 - 0.42 (m, 2H). LC-MS: [M+H]⁺ = 536. |
| 59 | 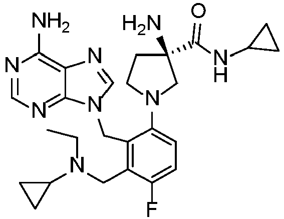 | ¹H NMR (400 MHz, Methanol-*d*₄) δ = 8.25 (s, 1H), 7.80 (s, 1H), 7.41 (dd, *J* = 5.2, 9.2 Hz, 1H), 7.16 (t, *J* = 9.2 Hz, 1H), 5.67-5.48 (m, 1H), 5.67-5.48 (m, 1H), 4.03-3.86 (m, 2H), 3.46 (d, *J* = 9.2 Hz, 1H), 2.99-2.91 (m, 1H), 2.86 (dt, *J* = 5.6, 8.8 Hz, 1H), 2.74 (d, *J* = 9.2 Hz, 1H), 2.66-2.55 (m, 3H), 2.41-2.30 (m, 1H), 1.82-1.74 (m, 1H), 1.64-1.54 (m, 1H), 1.04 (t, *J* = 7.2 Hz, 3H), 0.74-0.65 (m, 2H), 0.52-0.43 (m, 2H), 0.33 (d, *J* = 6.6 Hz, 2H), 0.09-0.02 (m, 2H). LC-MS: [M+H]⁺ = 508.3. |
| 60 | 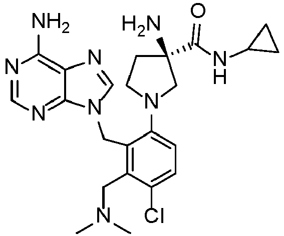 | ¹H NMR (400 MHz, Methanol-d₄) δ = 8.25 (s, 1H), 7.83 (s, 1H), 7.46 (d, *J* = 8.8 Hz, 1H), 7.35 (d, *J* = 8.8 Hz, 1H), 5.75-5.58 (m, 2H), 3.92-3.71 (m, 2H), 3.54 (d, *J* = 9.2 Hz, 1H), 3.16-3.05 (m, 1H), 2.93 (dt, *J* = 5.4, 8.8 Hz, 1H), 2.79 (d, *J* = 9.2 Hz, 1H), 2.64 (tt, *J*=4.0, 7.2 Hz, 1H), 2.40-2.34 (m, 1H), 2.26 (s, 6H), 1.65-1.61 (m, 1H), 0.79-0.66 (m, 2H), 0.54-0.43 (m, 2H). LC-MS: [M+H]⁺ = 484.2. |
| 61 | 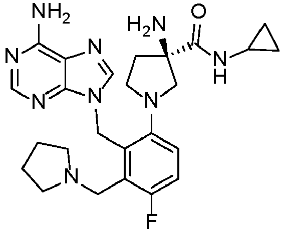 | ¹H NMR (400 MHz, Methanol-d₄) δ 8.24 (s, 1H), 7.85 (s, 1H), 7.42 (dd, *J* = 9.0, 5.0 Hz, 1H), 7.16 (t, *J* = 9.2 Hz, 1H), 5.65 (d, *J* = 13.9 Hz, 1H), 5.57 (d, *J* = 13.9 Hz, 1H), 3.86 (dd, *J* = 12.9, 2.7 Hz, 1H), 3.74 (dd, *J* = 12.9, 2.6 Hz, 1H), 3.47 (s, 1H), 2.98 (ddd, *J* = 9.0, 7.7, 6.1 Hz, 1H), 2.89 (td, *J* = 8.7, 5.6 Hz, 1H), 2.77 (d, *J* = 9.2 Hz, 1H), 2.62 (tt, *J* = 7.4, 3.9 Hz, 1H), 2.46 (h, *J* = 4.6, 3.9 Hz, 4H), 2.37 (ddd, *J* = 12.8, 8.4, 6.1 Hz, 1H), 1.69 - 1.55 (m, 5H), 0.77 - 0.60 (m, 2H), 0.56 - 0.40 (m, 2H). LC-MS: [M+H]⁺ = 494.3. |
| 62 | 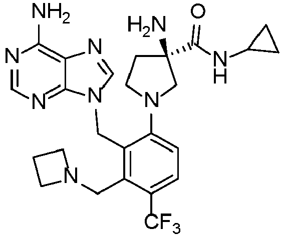 | ¹H NMR (400 MHz, Methanol-d₄) δ 8.23 (s, 1H), 7.74 (s, 1H), 7.66 (d, *J* = 8.8 Hz, 1H), 7.29 (d, *J* = 8.7 Hz, 1H), 5.86 - 5.62 (m, 2H), 3.88 (d, *J* = 14.4 Hz, 1H), 3.76 (d, *J* = 14.2 Hz, 1H), 3.70 (d, *J* = 9.4 Hz, 1H), 3.42 (dt, *J* = 9.0, 7.3 Hz, 1H), 3.20 (t, *J* = 7.0 Hz, 4H), 3.10 (dd, *J* = 8.8, 4.7 Hz, 1H), 2.93 - 2.86 (m, 1H), 2.64 (tt, *J* = 7.4, 3.9 Hz, 1H), 2.39 (ddd, *J* = 12.7, 8.4, 7.3 Hz, 1H), 1.90 (p, *J* = 6.9 Hz, 2H), 1.70 (ddd, *J* = 12.3, 7.6, 4.8 Hz, 1H), 0.78 - 0.65 (m, 2H), 0.55 - 0.43 (m, 2H). LC-MS: [M+H]⁺ = 530.3. |
| 63 | 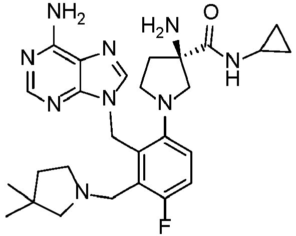 | ¹H NMR (400 MHz, Methanol-d₄) δ = 8.26 (s, 1H), 7.89 (s, 1H), 7.42 (dd, *J* = 5.2, 9.2 Hz, 1H), 7.17 (t, *J* = 9.2 Hz, 1H), 5.72-5.55 (m, 2H), 3.88-3.65 (m, 2H), 3.46 (d, *J* = 9.2 Hz, 1H), 2.98-2.82 (m, 2H), 2.76 (d, *J* = 9.2 Hz, 1H), 2.67-2.53 (m, 3H), 2.41-2.27 (m, 3H), 1.63-1.53 (m, 1H), 1.47 (t, *J* = 6.8 Hz, 2H), 0.97 (d, *J* = 1.6 Hz, 6H), 0.76-0.64 (m, 2H), 0.53-0.43 (m, 2H). LC-MS: [M+H]⁺ = 522.3 [M+H]⁺. |
| 64 | 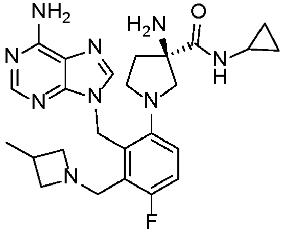 | ¹H NMR (400 MHz, Methanol-d₄) δ 8.63 (s, 1H), 8.28 (s, 1H), 7.59 (dd, J = 9.0, 5.2 Hz, 1H), 7.28 (t, J = 9.2 Hz, 1H), 5.58 (d, J = 2.4 Hz, 2H), 4.57 (s, 2H), 4.22 - 4.04 (m, 2H), 3.73 (s, 1H), 3.59 (d, J = 9.6 Hz, 1H), 2.97 (dq, J = 15.8, 7.6 Hz, 2H), 2.80 - 2.61 (m, 3H), 2.46 (dt, J = 13.0, 7.9 Hz, 1H), 1.81 (ddd, J = 13.0, 7.1, 4.3 Hz, 1H), 1.30 (d, J = 6.7 Hz, 4H), 0.81 - 0.74 (m, 2H), 0.60 - 0.53 (m, 2H). LC-MS: [M+H]⁺ = 494.3. |
| 65 | 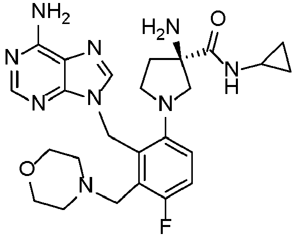 | ¹H NMR (400 MHz, Methanol-d₄) δ 8.24 (s, 1H), 7.80 (s, 1H), 7.45 (dd, *J* = 9.0, 5.0 Hz, 1H), 7.18 (t, *J* = 9.2 Hz, 1H), 5.70 - 5.55 (m, 2H), 3.69 - 3.58 (m, 2H), 3.50 (d, *J* = 9.3 Hz, 1H), 3.37 (s, 4H), 3.10 - 3.02 (m, 1H), 2.99 - 2.92 (m, 1H), 2.80 (d, *J* = 9.3 Hz, 1H), 2.61 (tt, *J* = 7.3, 3.9 Hz, 1H), 2.42 - 2.31 (m, 5H), 1.68 - 1.59 (m, 1H), 0.69 (dtd, *J* = 8.7, 7.0, 6.4, 4.7 Hz, 2H), 0.51 - 0.41 (m, 2H). LC-MS: [M+H]⁺ = 510.3. |
| 66 | 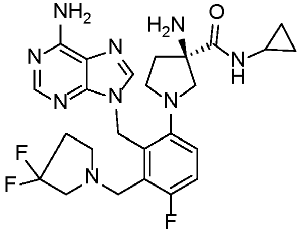 | ¹H NMR (400 MHz, Methanol-d₄) δ 8.25 (s, 1H), 7.85 (s, 1H), 7.46 (dd, *J* = 9.0, 5.0 Hz, 1H), 7.19 (t, *J* = 9.2 Hz, 1H), 5.72 - 5.55 (m, 2H), 3.91 - 3.73 (m, 2H), 3.51 (d, *J* = 9.3 Hz, 1H), 3.02 (q, *J* = 7.7 Hz, 1H), 2.92 (td, *J* = 8.7, 5.6 Hz, 1H), 2.85 - 2.75 (m, 3H), 2.71 - 2.59 (m, 3H), 2.38 (ddd, *J* = 12.8, 8.4, 6.1 Hz, 1H), 2.06 (tt, *J* = 14.5, 6.9 Hz, 2H), 1.63 (ddd, *J* = 13.1, 7.7, 5.6 Hz, 1H), 0.70 (hd, *J* = 7.7, 3.6 Hz, 2H), 0.55 - 0.40 (m, 2H). LC-MS: [M+H]⁺ = 530.2. |
| 67 | 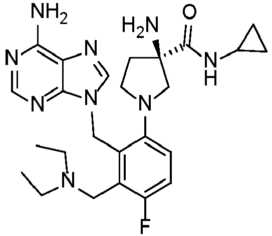 | ¹H NMR (400 MHz, Methanol-d₄) δ 8.24 (s, 1H), 7.89 (s, 1H), 7.41 (dd, *J* = 9.0, 5.0 Hz, 1H), 7.17 (t, *J* = 9.2 Hz, 1H), 5.75 - 5.50 (m, 2H), 3.88 (d, *J* = 13.2 Hz, 1H), 3.77 (d, *J* = 13.2 Hz, 1H), 3.45 (d, *J* = 9.3 Hz, 1H), 2.91 (td, *J* = 8.2, 7.7, 6.3 Hz, 1H), 2.82 (td, *J* = 8.6, 5.6 Hz, 1H), 2.71 (d, *J* = 9.3 Hz, 1H), 2.63 (tt, *J* = 7.3, 3.9 Hz, 1H), 2.59 - 2.47 (m, 4H), 2.35 (ddd, *J* = 12.8, 8.4, 6.1 Hz, 1H), 1.61 - 1.50 (m, 1H), 0.97 (t, *J* = 7.1 Hz, 6H), 0.77 - 0.62 (m, 2H), 0.57 - 0.42 (m, 2H). LC-MS: [M+H]⁺ = 496.3. |
| 68 | 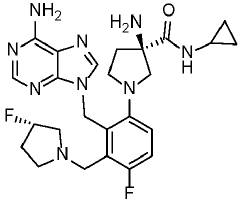 | ¹H NMR (400 MHz, Methanol-d₄) δ 8.25 (s, 1H), 7.92 (s, 1H), 7.43 (dd, *J* = 9.0, 5.0 Hz, 1H), 7.18 (t, *J* = 9.2 Hz, 1H), 5.75 - 5.56 (m, 2H), 5.13 - 4.90 (m, 1H), 3.93 - 3.74 (m, 2H), 3.50 (s, 1H), 2.99 (ddd, *J* = 9.0, 7.7, 6.1 Hz, 1H), 2.91 - 2.72 (m, 4H), 2.72 - 2.56 (m, 2H), 2.36 (tt, *J* = 8.6, 5.4 Hz, 2H), 2.15 - 1.95 (m, 1H), 1.83 (ddt, *J* = 29.7, 14.4, 7.2 Hz, 1H), 1.61 (ddd, *J* = 13.1, 7.7, 5.7 Hz, 1H), 0.78 - 0.61 (m, 2H), 0.58 - 0.42 (m, 2H). LC-MS: [M+H]⁺ = 512.3. |
| 69 | 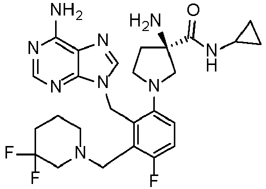 | ¹H NMR (400 MHz, Methanol-d₄) δ = 8.26 (s, 1H), 7.96 (s, 1H), 7.45 (dd, *J* = 5.2, 8.8 Hz, 1H), 7.19 (t, *J* = 9.2 Hz, 1H), 5.70-5.52 (m, 2H), 3.89-3.69 (m, 2H), 3.47 (d, *J* = 9.2 Hz, 1H), 2.93-2.81 (m, 2H), 2.75 (d, *J* = 9.2 Hz, 1H), 2.70-2.60 (m, 3H), 2.49 (s, 2H), 2.39-2.29 (m, 1H), 1.83 (dt, *J* = 6.8, 13.2 Hz, 2H), 1.65-1.52 (m, 3H), 0.75-0.66 (m, 2H), 0.54-0.45 (m, 2H). LC-MS: [M+H]⁺ = 544.3. |
| 70 | 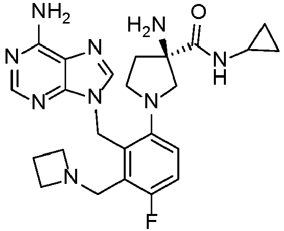 | ¹H NMR (400 MHz, Methanol-d₄) δ 8.24 (s, 1H), 7.99 (s, 1H), 7.41 (dd, *J* = 8.9, 5.0 Hz, 1H), 7.16 (t, *J* = 9.1 Hz, 1H), 5.59 (q, *J* = 14.0 Hz, 2H), 3.95 - 3.72 (m, 2H), 3.49 (d, *J* = 9.3 Hz, 1H), 3.29 - 3.21 (m, 4H), 2.97 (ddd, *J* = 8.9, 7.6, 6.2 Hz, 1H), 2.85 (td, *J* = 8.6, 5.5 Hz, 1H), 2.75 (d, *J* = 9.3 Hz, 1H), 2.65 (tt, *J* = 7.3, 3.9 Hz, 1H), 2.38 (ddd, *J* = 12.9, 8.4, 6.3 Hz, 1H), 2.00 (p, *J* = 7.0 Hz, 2H), 1.62 (ddd, *J* = 13.0, 7.6, 5.5 Hz, 1H), 0.78 - 0.64 (m, 2H), 0.58 - 0.43 (m, 2H). LC-MS: [M+H]⁺ = 480.3 |

### Example 71. methyl (R)-(3-(3-amino-3-(cyclopropylcarbamoyl)pyrrolidin-1-yl)-2-((6-amino-9H-purin-9-yl)methyl)-6-fluorobenzyl)carbamate

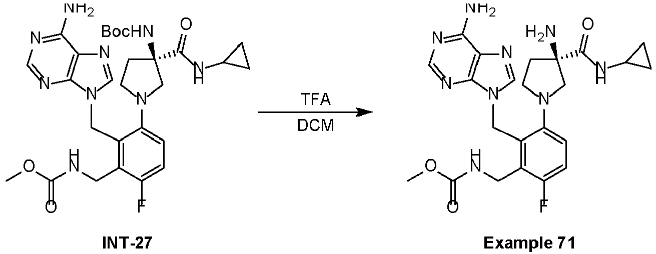

A mixture of *tert*-butyl (*R*)-(1-(2-((6-amino-9H-purin-9-yl)methyl)-4-fluoro-3-(((methoxycarbonyl)amino)methyl)phenyl)-3-(cyclopropylcarbamoyl)pyrrolidin-3-yl)carbamate (INT-27) (32 mg, 0.054 mmol) and TFA (0.35 mL) in DCM (0.7 mL) was reacted at 20 °C for 2 hrs. The reaction was concentrated under vacuum. The residue was purified by prep-HPLC [Column: Phenomenex Synergi C18 150*25*10 um, gradient: 0-24% B (A = water (0.225% FA), B = MeCN), flow rate: 25 mL/min] to afford methyl (*R*)-(3-(3-amino-3-(cyclopropylcarbamoyl)pyrrolidin-1-yl)-2-((6-amino-9*H*-purin-9-yl)methyl)-6-fluorobenzyl)carbamate as a white solid. ¹H NMR (400 MHz, DMSO-*d*₆) δ = 8.19-8.12 (m, 2H), 8.01 (s, 2H), 7.88 (s, 1H), 7.40-7.31 (m, 1H), 7.25-7.15 (m, 3H), 5.56-5.42 (m, 2H), 4.56-4.40 (m, 2H), 3.50 (s, 3H), 3.41 (d, *J* = 9.2 Hz, 1H), 2.88 (s, 1H), 2.78-2.69 (m, 3H), 2.31-2.22 (m, 1H), 1.65-1.61 (m, 1H), 0.63 (d, *J* = 5.6 Hz, 2H), 0.51-0.42 (m, 2H). LC-MS: [M+H]⁺ = 498.4.

### Example 72. (R)-3-amino-1-(2-((6-amino-9H-purin-9-yl)methyl)-6-chloro-3-((dimethylamino)methyl)-4-fluorophenyl)-N-cyclopropylpyrrolidine-3-carboxamide

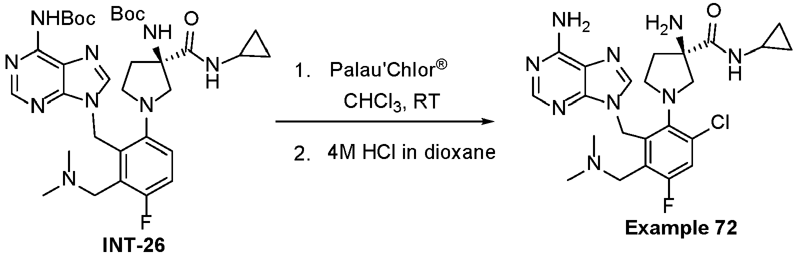

To a CHCl₃ solution (2 mL) of *tert*-butyl (R)-(9-(6-(3-((tert-butoxycarbonyl)amino)-3-(cyclopropylcarbamoyl)pyrrolidin-1-yl)-2-((dimethylamino)methyl)-3-fluorobenzyl)-9*H*-purin-6-yl)carbamate (INT-26) (50 mg, 0.075 mmol) was added Palau'Chlor^{®} (17.26 mg, 0.082 mmol) at rt. The reaction was stirred for 1 hr. More Palau'Chlor^{®} (17.26 mg, 0.082 mmol) was added. After 1h, LCMS showed 50% conversion. The mixture was stirred overnight. NaHCO₃ (aq.) was added and the mixture was extracted with EA (x3). The combined organic layer was washed with brine and dried over Na₂SO₄.

To the above crude material was added HCl (4M) (0.2 ml, 0.800 mmol) and the mixture was stirred at RT for 2h. The reaction was concentrated. Several drops of NH3 in MeOH (7M) was added and the mixture was concentrated. The residue was purified purified by prep-HPLC [Column: Waters Xbridge 150*25*5um; Condition: water(10mM NH₄HCO₃)-MeCN; Flow rate: 25 mL/min] to afford (*R*)-3-amino-1-(2-((6-amino-9*H*-purin-9-yl)methyl)-6-chloro-3-((dimethylamino)methyl)-4-fluorophenyl)-*N*-cyclopropylpyrrolidine-3-carboxamide as a white solid. ¹H NMR (400 MHz, Methanol-*d*₄) δ 8.24 (s, 1H), 7.85 (brs, 1H), 7.36 (d, *J* = 9.5 Hz, 1H), 5.73 - 5.29 (m, 2H), 3.97 - 3.51 (m, 3H), 2.74 - 2.35 (m, 4H), 2.23 (s, 7H), 1.66 - 1.31 (m, 1H), 0.72 (td, *J* = 7.1, 5.0 Hz, 2H), 0.55 - 0.45 (m, 2H). LC-MS: [M+H]⁺ = 502.1.

The following examples were prepared from corresponding intermediates following procedures analogous to Example 72.

| Ex No. | | |
|---|---|---|
| 73 | 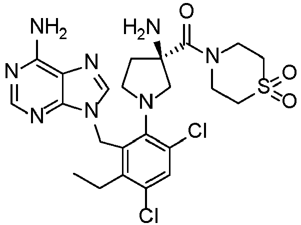 | ¹H NMR (400 MHz, Methanol-*d*₄) δ = 8.26 (s, 1H), 7.62 (d, *J* = 13.6 Hz, 2H), 5.54-5.50 (m, 2H), 4.07-4.03 (m, 4H), 3.37-3.33 (m, 1H), 3.18-2.31 (m, 10H), 1.92-1.71 (m, 1H), 1.15-0.90 (m, 3H). LC-MS: [M+H]⁺ = 567.2. |
| 74 | 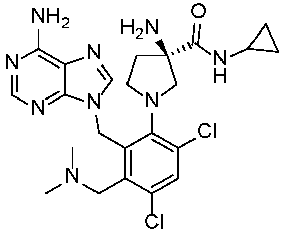 | ¹H NMR (400 MHz, Methanol-*d*₄) δ = 8.33 (s, 1H), 8.24 (s, 1H), 7.88 (s, 1H), 6.13-5.42 (m, 2H), 4.07-3.34 (m, 3H), 3.31-2.83 (m, 8H), 2.80-2.86 (m, 1H), 2.74-2.63 (m, 1H), 2.56-2.52 (m, 1H), 2.24-2.20 (m, 1H), 0.83-0.79 (m, 2H), 0.67-0.63 (m, 2H). LC-MS: [M+H]⁺ = 518.2. |
| 75 | 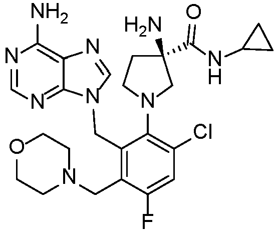 | ¹H NMR (400 MHz, Methanol-d₄) δ 8.24 (s, 1H), 7.82 (s, 1H), 7.38 (d, J = 9.4 Hz, 1H), 5.77 - 5.39 (m, 2H), 3.95 - 3.78 (m, 1H), 3.76 - 3.57 (m, 2H), 3.52 - 3.32 (m, 6H), 2.78 - 2.51 (m, 3H), 2.52 - 2.21 (m, 4H), 1.85 - 1.41 (m, 1H), 0.82 - 0.56 (m, 2H), 0.55 - 0.37 (m, 2H). LC-MS: [M+H]⁺ = 544.2. |

### Example 76. (R)-3-amino-1-(2-((6-amino-9H-purin-9-yl)methyl)-4-fluoro-3-(hydroxymethyl) phenyl)-N-cyclopropylpyrrolidine-3-carboxamide

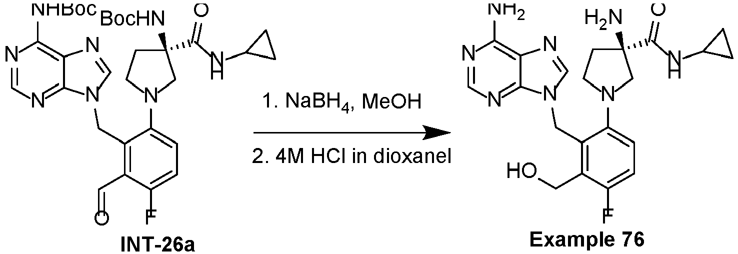

To a solution of *tert*-butyl (*R*)-(9-(6-(3-((tert-butoxycarbonyl)amino)-3-(cyclopropylcarbamoyl) pyrrolidin-1-yl)-3-fluoro-2-formyl benzyl)-9*H*-purin-6-yl)carbamate (INT-26a) (50 mg, 0.078 mmol) in methanol (1 mL) was added NaBH₄ (3 mg, 0.078 mmol). The mixture was stirred at 0 °C for 1h. The reaction was quenched with NH₄Cl (aq.). The mixture was extracted with EA (X2) and washed with brine, dried over Na₂SO₄ and concentrated. The crude material was used for the next step. To the above crude material was added HCl (4M) (0.2 ml, 0.800 mmol) and the mixture was stirred at RT. LC MS showed the reaction was complete. The reaction was concentrated. Several drops of NH₃ in MeOH (7M) was added and the mixture was concentrated for purification. HPLC separation gave (*R*)-3-amino-1-(2-((6-amino-9H-purin-9-yl)methyl)-4-fluoro-3-(hydroxymethyl) phenyl)-*N*-cyclopropylpyrrolidine-3-carboxamide as a white solid. ¹H NMR (400 MHz, Methanol-d₄) δ 8.19 (s, 1H), 8.19 (s, 1H), 7.43 (dd, *J* = 9.0, 5.1 Hz, 1H), 7.17 (t, *J* = 9.1 Hz, 1H), 5.67 (d, *J* = 14.2 Hz, 1H), 5.59 (d, *J* = 14.2 Hz, 1H), 4.91 (d, *J* = 2.2 Hz, 2H), 3.49 (d, *J* = 9.4 Hz, 1H), 2.91 (dt, *J* = 8.8, 7.1 Hz, 1H), 2.80 - 2.62 (m, 3H), 2.39 (ddd, *J* = 12.8, 8.3, 6.5 Hz, 1H), 1.61 (ddd, *J* = 12.7, 7.5, 5.3 Hz, 1H), 0.80 - 0.67 (m, 2H), 0.58 - 0.46 (m, 2H). LC-MS: [M+H]⁺ = 441.2.

### Example 77. (R)-3-amino-1-(2-((6-amino-9H-purin-9-yl)methyl)-4-fluoro-3-(methoxymethyl) phenyl)-N-cyclopropylpyrrolidine-3-carboxamide

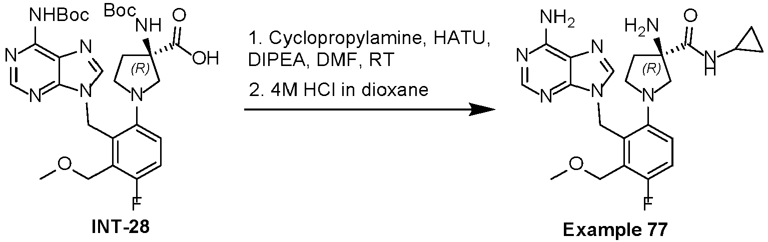

To a solution of crude (*R*)-3-((tert-butoxycarbonyl)amino)-1-(2-((6-((tert-butoxycarbonyl)amino)-9*H*-purin-9-yl)methyl)-4-fluoro-3-(methoxymethyl)phenyl)pyrrolidine-3-carboxylic acid (INT-28) (86 mg, 0.14 mmol), cyclopropanamine (0.02 mL, 0.3 mmol) and DIPEA (0.12 mL, 0.7 mmol) in DMF (2 mL) was added HATU (160 mg, 0.4 mmol) at RT. The mixture was stirred at RT for 30 min and was diluted with EA and water. The mixture was extracted with EA (x2). The combined organic phase was washed 1M HCl, NaHCO₃ (aq.), brine and dried over Na₂SO₄. The mixture was concentrated and the crude product was treated with HCl(4M) (0.2 ml, 0.8 mmol). The mixture was stirred at RT overnight. The reaction was concentrated. Several drops of NH₃ in MeOH (7M) was added and the mixture was concentrated. The residue was purified by prep-HPLC [Column: Waters Xbridge 150*25*5um; Condition: water(10 mM NH₄HCO₃)-MeCN; Flow rate: 25 mL/min] to afford (*R*)-3-amino-1-(2-((6-amino-9*H*-purin-9-yl)methyl)-4-fluoro-3-(methoxymethyl) phenyl)-N-cyclopropylpyrrolidine-3-carboxamide as a white solid. ¹H NMR (400 MHz, Methanol-*d*₄) δ 8.25 (s, 1H), 7.88 (s, 1H), 7.46 (dd, *J* = 9.0, 5.1 Hz, 1H), 7.19 (t, *J* = 9.1 Hz, 1H), 5.63 (d, *J* = 14.0 Hz, 1H), 5.53 (d, *J* = 14.0 Hz, 1H), 4.66 (td, *J* = 11.1, 2.3 Hz, 2H), 3.46 (s, 1H), 3.31 (s, 3H, overlapped with MeOD-d4), 2.96 - 2.80 (m, 2H), 2.74 (d, *J* = 9.2 Hz, 1H), 2.64 (tt, *J* = 7.4, 3.9 Hz, 1H), 2.36 (ddd, *J* = 12.8, 8.4, 6.1 Hz, 1H), 1.58 (ddd, *J* = 13.1, 7.9, 5.7 Hz, 1H), 0.78 - 0.63 (m, 2H), 0.57 - 0.43 (m, 2H). LC-MS: [M+H]⁺ = 455.2.

The following examples were prepared from corresponding intermediates following procedures analogous to Example 77.

| Ex No. | | |
|---|---|---|
| 78 | 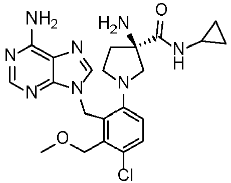 | ¹H NMR (400 MHz, Methanol-*d*4) δ = 8.26 (s, 1H), 7.82 (s, 1H), 7.51-7.45 (m, 1H), 7.38 (d, *J* = 8.8 Hz, 1H), 5.68-5.55 (m, 2H), 4.78-4.68 (m, 2H), 3.52 (d, *J* = 9.2 Hz, 1H), 3.29 (s, 3H), 3.11-3.05 (m, 1H), 2.93 (dt, *J* = 5.2, 8.8 Hz, 1H), 2.79 (d, *J* = 9.2 Hz, 1H), 2.66-2.62 (m, 1H), 2.40-2.34 (m, 1H), 1.69-1.59 (m, 1H), 0.76-0.67 (m, 2H), 0.55-0.44 (m, 2H). LC-MS: [M+H]⁺ = 471.2. |
| 79 | 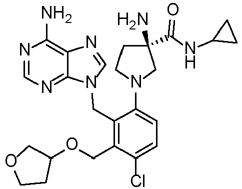 | ¹H NMR (400 MHz, Methanol-d₄) δ 8.26 (s, 1H), 7.78 (d, *J* = 2.7 Hz, 1H), 7.49 (d, *J* = 8.8 Hz, 1H), 7.39 (dd, *J* = 8.8, 1.2 Hz, 1H), 5.70 - 5.55 (m, 2H), 4.81 - 4.70 (m, 2H), 4.11 - 4.04 (m, 1H), 3.77 - 3.57 (m, 4H), 3.53 (dd, *J* = 9.3, 5.3 Hz, 1H), 3.19 - 3.07 (m, 1H), 2.97 (tdd, *J* = 8.9, 5.4, 3.9 Hz, 1H), 2.81 (dd, *J* = 9.2, 4.6 Hz, 1H), 2.64 (tdt, *J* = 7.2, 3.9, 1.9 Hz, 1H), 2.39 (ddd, *J* = 12.8, 8.5, 6.3 Hz, 1H), 1.98 - 1.84 (m, 2H), 1.67 (dt, *J* = 13.2, 6.5 Hz, 1H), 0.77 - 0.64 (m, 2H), 0.49 (tp, *J* = 4.0, 1.4 Hz, 2H). LC-MS: [M+H]⁺ =527.2. |
| 80 | 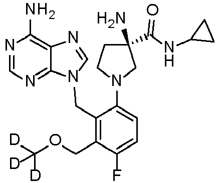 | ¹H NMR (400 MHz, Methanol-d₄) δ 8.25 (s, 1H), 7.88 (s, 1H), 7.46 (dd, *J* = 9.0, 5.0 Hz, 1H), 7.19 (t, *J* = 9.1 Hz, 1H), 5.63 (d, *J* = 14.0 Hz, 1H), 5.53 (d, *J* = 14.1 Hz, 1H), 4.68 (dd, *J* = 10.8, 2.2 Hz, 2H), 3.47 (d, *J* = 9.2 Hz, 1H), 2.92 (td, *J* = 8.2, 7.8, 6.2 Hz, 1H), 2.84 (td, *J* = 8.7, 5.7 Hz, 1H), 2.74 (d, *J* = 9.2 Hz, 1H), 2.64 (tt, *J* = 7.3, 3.9 Hz, 1H), 2.36 (ddd, *J* = 12.8, 8.5, 6.1 Hz, 1H), 1.64 - 1.53 (m, 1H), 0.78 - 0.65 (m, 2H), 0.56 - 0.43 (m, 2H). LC-MS: [M+H]⁺ =458.3. |
| 81 | 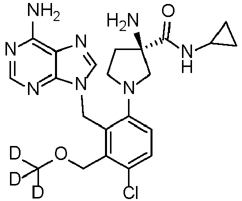 | ¹H NMR (400 MHz, Methanol-*d*4) δ 8.26 (s, 1H), 7.83 (s, 1H), 7.48 (d, *J* = 8.8 Hz, 1H), 7.38 (d, *J* = 8.8 Hz, 1H), 5.65 (d, *J* = 14.3 Hz, 1H), 5.57 (d, *J* = 14.3 Hz, 1H), 4.80 - 4.69 (m, 2H), 3.52 (d, *J* = 9.3 Hz, 1H), 3.06 (ddd, *J* = 9.0, 7.6, 6.3 Hz, 1H), 2.92 (td, *J* = 8.6, 5.3 Hz, 1H), 2.80 (d, *J* = 9.3 Hz, 1H), 2.64 (tt, *J* = 7.3, 3.9 Hz, 1H), 2.38 (ddd, *J* = 12.9, 8.5, 6.3 Hz, 1H), 1.64 (ddd, *J* = 13.0, 7.7, 5.5 Hz, 1H), 0.79 - 0.63 (m, 2H), 0.58 - 0.43 (m, 2H). LC-MS: [M+H]⁺ = 474.3. |

### Example 82. (R)-3-amino-1-(2-((6-amino-9H-purin-9-yl)methyl)-4-fluoro-3-((S)-2,2,2-trifluoro-1-hydroxyethyl)phenyl)-N-cyclopropylpyrrolidine-3-carboxamide

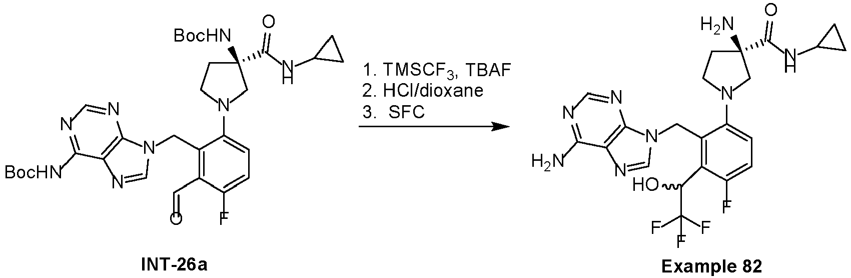

A mixture of *tert*-butyl (*R*)-(9-(6-(3-((tert-butoxycarbonyl)amino)-3-(cyclopropylcarbamoyl) pyrrolidin-1-yl)-3-fluoro-2-formyl benzyl)-9*H*-purin-6-yl)carbamate (INT-26a) (300 mg, 0.47 mmol) and TMSCF₃ (668 mg, 4.7 mmol) in THF (1 mL) was added TBAF (47 uL, 0.05 mmol) at 25 °C. The mixture was stirred at 30 °C for 22 hrs. More TMSCF₃ (223 mg, 1.6 mmol) and TBAF (16 uL, 0.016 mmol) was added and the mixture was stirred at 30 °C for 4 hrs. The reaction mixture was concentrated. The residue was treated with in 4M HCl in dioxane (0.4 mL). The mixture was stirred at 30 °C for 18 hrs. The reaction mixture was concentrated. The residue was purified by prep-HPLC [Column: Waters Xbridge 150*25*5 um; gradient: 20-50% B (A = water(10 mM NH₄HCO₃), B = MeCN); Flow rate: 25 mL/min] to give a white solid. The first peak from SFC chiral separation was designated as (*R*)-3-amino-1-(2-((6-amino-9*H*-purin-9-yl)methyl)-4-fluoro-3-((*S*)-2,2,2-trifluoro-1-hydroxyethyl)phenyl)-*N-*cyclopropylpyrrolidine-3-carboxamide (absolute stereochemistry not determined). ¹H NMR (400 MHz, Methanol-d₄) δ = 8.22 (s, 1H), 7.93 (s, 1H), 7.57 (dd, *J* = 5.2, 8.8 Hz, 1H), 7.26 (t, *J* = 9.6 Hz, 1H), 5.87-5.70 (m, 3H), 3.49 (d, *J* = 9.2 Hz, 1H), 3.24-3.15 (m, 1H), 2.67 (tt, *J* = 3.6, 7.6 Hz, 1H), 2.56-2.53 (m, 1H), 2.37 (d, *J* = 9.2Hz, 1H), 2.32-2.28(m, 1H), 1.65-1.62 (m, 1H), 0.80-0.68 (m, 2H), 0.57-0.49 (m, 2H). LC-MS: [M+H]⁺ = 509.2.

### Example 83. (R)-9-(2-(3-amino-3-(1H-tetrazol-5-yl)pyrrolidin-1-yl)-6-bromo-4-chlorobenzyl)-9H-purin-6-amine

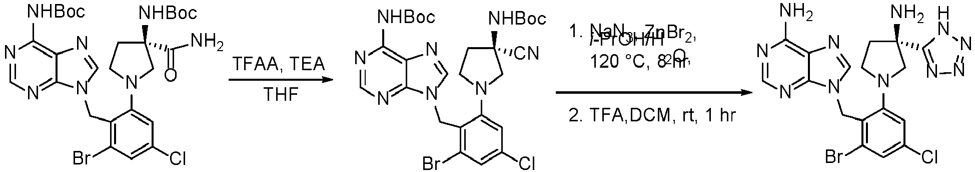

To a solution of tert-butyl (R)-(9-(2-bromo-6-(3-((tert-butoxycarbonyl)amino)-3-carbamoylpyrrolidin-1-yl)-4-chlorobenzyl)-9H-purin-6-yl)carbamate (1 g, 1.5 mmol) in THF (10 mL) were added trifluoroacetic anhydride (788 mg, 3.7 mmol) and TEA (836 mg, 8.3 mmol). The reaction mixture was stirred at 90 °C for 0.5 hours. The mixture was diluted with water (30 mL) and extracted with EtOAc (3 × 30 mL). The organic layer was washed with brine (3 × 50 mL), dried over anhydrous sodium sulfate, filtered and concentrated under vacuum. The residue was purified by silica gel chromatography eluted with PE: EtOAc = 10:1 to 3:1 to afford tert-butyl (R)-(9-(2-bromo-6-(3-((tert-butoxycarbonyl)amino)-3-cyanopyrrolidin-1-yl)-4-chlorobenzyl)-9H-purin-6-yl)carbamate as a yellow solid. LC-MS: [M+H]⁺ = 649.4.

To a solution of tert-butyl (R)-(9-(2-bromo-6-(3-((tert-butoxycarbonyl)amino)-3-cyanopyrrolidin-1-yl)-4-chlorobenzyl)-9H-purin-6-yl)carbamate (800 mg, 1.2 mmol) in *i-*PrOH/H₂O (8 mL, 2:1) were added NaN₃ (161 mg, 2.5 mmol) and ZnBr₂ (278 mg, 1.2 mmol). The mixture was stirred at 120 °C for 8 hours. The mixture was then diluted with water (20 mL) and extracted with EtOAc (20 mL). The mixture was filtered and the filter cake was washed with EtOAc (10 mL), dried under vacuum to afford the crude product (680 mg, crude). To a solution of the crude product (670 mg, crude) in DCM (13.4 mL) was added TFA (6.7 mL). The reaction was stirred at 15 °C for 1 hour. The reaction mixture was then concentrated under vacuum. The crude product was diluted with water (10 mL) and EtOAc (10 mL). The mixture was adjusted to pH 9 with 1N sodium hydroxide solution. The mixture was filtered and the filter cake was washed with EtOAc (5 mL), dried under vacuum to afford the crude product (500 mg, crude). Some of the crude product (100 mg, crude) was purified by prep-HPLC (column: Phenomenex synergi C18 150*25mm*10 µm, gradient 14∼34%B (A = water (0.05% hydrogen chloride v/v), B = acetonitrile, flow rate: 28 mL/min) to afford (*R*)-9-(2-(3-amino-3-(1*H*-tetrazol-5-yl)pyrrolidin-1-yl)-6-bromo-4-chlorobenzyl)-9*H*-purin-6-amine. ¹H NMR (400 MHz, Methanol-d₄) δ ppm 8.38 (s, 1H), 8.10 (s, 1H), 7.57 (d, *J* = 1.6 Hz, 1H), 7.50 (d, *J* = 1.9 Hz, 1H), 5.78 - 5.69 (m, 2H), 3.86 - 3.83 (m, 1H), 3.72 - 3.69 (m, 1H), 3.58 - 3.50 (m, 1H), 3.49 - 3.39 (m, 1H), 2.92 - 2.90 (m, 1H), 2.63 - 2.53 (m, 1H). LC-MS: [M+H]⁺ = 492.0.

The following examples were prepared from corresponding intermediates following procedures analogous to Example 83.

| Ex No. | | |
|---|---|---|
| 84 | 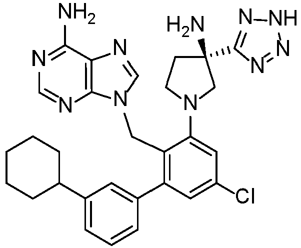 | ¹H NMR: (400 MHz, Methanol-d₄) δ ppm 8.15 (s, 1H), 7.49 - 7.45 (m, 2H), 7.20 - 7.15 (m, 1H), 7.05 (d, *J* = 8.0 Hz, 1H), 7.01 (d, *J* = 2.0 Hz, 1H), 6.96 (d, *J* = 7.6 Hz, 1H), 6.69 (s, 1H), 5.72 - 5.61 (m, 2H), 3.90 (d, *J* = 10.4 Hz, 1H), 3.78 (d, *J* = 10.4 Hz, 1H), 3.71 - 3.63 (m, 1H), 3.56 - 3.49 (m, 1H), 3.02 - 2.92 (m, 1H), 2.69 - 2.58 (m, 1H), 2.41 - 2.30 (m, 1H), 1.88 - 1.79 (m, 2H), 1.78 - 1.66 (m, 3H), 1.46 - 1.34 (m, 2H), 1.33 - 1.23 (m, 3H). LC-MS: [M+H]⁺ = 570.2. |
| 85 | 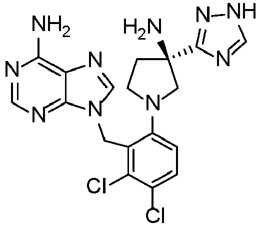 | ¹H NMR: (400 MHz, Methanol-*d*₄) δ ppm 8.27 (s, 1 H), 8.25 (s, 1 H), 7.76 (s, 1 H), 7.55 (d, *J* = 8.8 Hz, 1 H), 7.27 (d, *J* = 9.2 Hz, 1 H), 5.63 (q, *J* = 14.4 Hz, 2 H), 3.71 (d, *J* = 9.2 Hz, 1 H), 3.44 - 3.40 (m, 1 H), 3.38 - 3.19 (m, 3 H), 2.66 - 2.61 (m, 1 H), 2.15 - 2.08 (m, 1 H). LC-MS: [M+H]⁺ = 445.0. |
| 86 | 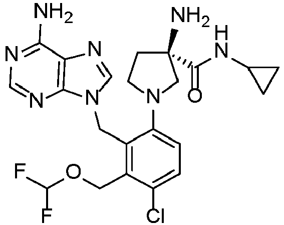 | ¹H NMR (400 MHz, Methanol-*d*₄) δ ppm 8.24 (s, 1H), 7.89 (s, 1H), 7.51 (d, *J* = 8.8 Hz, 1H), 7.43 (d, *J* = 8.8 Hz, 1H), 6.36 (t, *J* = 74.8 Hz, 1H), 5.69 - 5.56 (m, 2H), 5.35 (d, *J* = 11.4 Hz, 1H), 5.27 (d, *J* = 11.4 Hz, 1H), 3.56 (d, *J* = 9.4 Hz, 1H), 3.14 (dt, *J* = 8.9, 7.1 Hz, 1H), 2.95 (td, *J* = 8.6, 5.2 Hz, 1H), 2.80 (d, *J* = 9.3 Hz, 1H), 2.65 (tt, *J* = 7.4, 3.9 Hz, 1H), 2.41 (ddd, *J* = 12.8, 8.4, 6.6 Hz, 1H), 1.68 (ddd, *J* = 12.7, 7.5, 5.1 Hz, 1H), 0.78 - 0.65 (m, 2H), 0.50 (pd, *J* = 5.0, 4.6, 2.0 Hz, 2H). LC-MS: [M+H]⁺ = 507.2. |
| 87 | 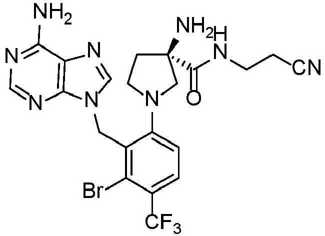 | ¹H NMR (400 MHz, Methanol-d₄) δ ppm 8.24 (s, 1 H), 7.84 (s, 1 H), 7.82 (s, 1 H), 5.83 (br d, *J* = 13.69 Hz, 1 H), 5.64 (d, *J* = 13.57 Hz, 1 H), 3.81 (br s, 1 H), 2.46 - 3.11 (m, 4 H), 1.69 (br s, 1 H), 0.70 (br d, *J* = 6.72 Hz, 2 H), 0.47 (br s, 2 H). LC-MS: [M+H]⁺ = 541.0. |

### Examples 88 and 89. (R)-3-amino-1-(2-((6-amino-9H-purin-9-yl)methyl)-4,5-dichloro-3-((dimethylamino)methyl)phenyl)-N-cyclopropylpyrrolidine-3-carboxamide and (R)-3-amino-1-(2-((6-amino-9H-purin-9-yl)methyl)-5,6-dichloro-3-((dimethylamino)methyl)phenyl)-N-cyclopropylpyrrolidine-3-carboxamide

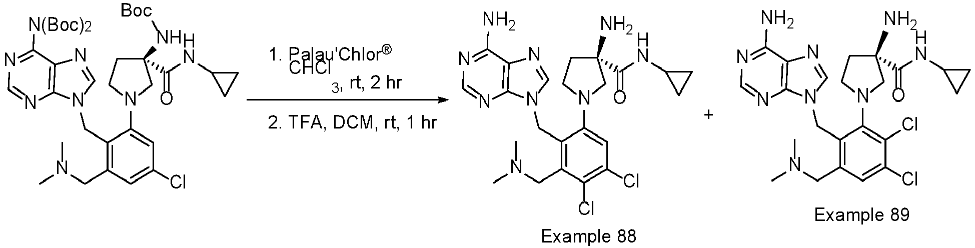

To a CHCl₃ solution (2 mL) of tert-butyl (R)-(tert-butoxycarbonyl)(9-(2-(3-((tert-butoxycarbonyl)amino)-3-(cyclopropylcarbamoyl)pyrrolidin-1-yl)-4-chloro-6-((dimethylamino)methyl)benzyl)-9H-purin-6-yl)carbamate (70 mg, 0.09 mmol) was added Palau'Chlor^{®} (37 mg, 0.18 mmol) at rt. The reaction was stirred for 2 hr, and 2 drops of AcOH was added to the reaction. 45 min later, LC-MS showed clean conversion. The reaction was quenched with 2-methyl-2-butene and then diluted with EA. The reaction mixture was washed with water and brine. The organic layer was dried over Na₂SO₄ and concentrated under vacuum to give the intermediate, which was redissolved in DCM (2 mL) and treated with TFA (1 mL). The reaction was stirred for 1 hr, and then directly concentrated to give the crude product. Purification by Prep-HPLC (Column: XBridge 30*150mm 5um, gradient: 30-100% B (A = water (0.05% NH₄OH), B = acetonitrile (0.05% NH₄OH)), flow rate: 30 mL/min) gave (*R*)-3-amino-1-(2-((6-amino-9*H*-purin-9-yl)methyl)-4,5-dichloro-3-((dimethylamino)methyl)phenyl)-N-cyclopropylpyrrolidine-3-carboxamide and (*R*)-3-amino-1-(2-((6-amino-9*H*-purin-9-yl)methyl)-5,6-dichloro-3-((dimethylamino)methyl)phenyl)-*N*-cyclopropylpyrrolidine-3-carboxamide.

Example 88 (fast eluent):¹H NMR (400 MHz, Methanol-d₄) δ ppm 8.24 (s, 1H), 7.88 (s, 1H), 7.52 (s, 1H), 5.67 (d, *J* = 14.4 Hz, 1H), 5.61 (d, *J* = 14.4 Hz, 1H), 3.95 (d, *J* = 13.2 Hz, 1H), 3.83 (d, *J* = 13.2 Hz, 1H), 3.58 (d, *J* = 9.4 Hz, 1H), 3.18 (dt, *J* = 9.0, 7.2 Hz, 1H), 2.97 (td, *J* = 8.6, 5.1 Hz, 1H), 2.82 (d, *J* = 9.4 Hz, 1H), 2.64 (tt, *J* = 7.3, 3.9 Hz, 1H), 2.37 (ddd, *J* = 12.8, 8.4, 6.8 Hz, 1H), 2.26 (s, 6H), 1.67 (ddd, *J* = 12.7, 7.4, 5.1 Hz, 1H), 0.78 - 0.64 (m, 2H), 0.57 - 0.41 (m, 2H). LC-MS: [M+H]⁺ = 518.2.

Example 89 (slow eluent): ¹H NMR (400 MHz, Methanol-d₄) δ ppm 8.24 (s, 1H), 7.81 (s, 1H), 7.53 (s, 1H), 5.63 (d, *J* = 13.6 Hz, 1H), 5.48 (s, 1H), 3.98 - 3.70 (m, 1H), 3.67 - 3.44 (m, 2H), 2.63 (s, 4H), 2.20 (s, 6H), 1.55 (s, 1H), 0.89 (d, *J* = 9.2 Hz, 1H), 0.71 (d, *J* = 6.4 Hz, 2H), 0.49 (s, 2H). LC-MS: [M+H]⁺ = 518.2.

### Examples 90. (R)-9-(2-(3-amino-3-(1-methyl-1H-tetrazol-5-yl)pyrrolidin-1-yl)-6-bromo-4-chlorobenzyl)-9H-purin-6-amine and 91 (KRH358) (R)-9-(2-(3-amino-3-(2-methyl-2H-tetrazol-5-yl)pyrrolidin-1-yl)-6-bromo-4-chlorobenzyl)-9H-purin-6-amine

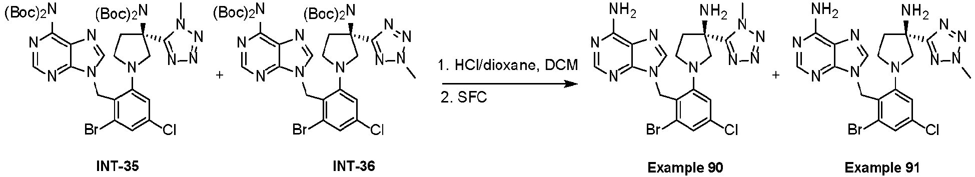

To a mixture oftert-butyl (*R*)-(9-(2-bromo-6-(3-((*tert*-butoxycarbonyl)amino)-3-(1-methyl-1*H-*tetrazol-5-yl)pyrrolidin-1-yl)-4-chlorobenzyl)-9*H*-purin-6-yl)(*tert*-butoxycarbonyl)carbamate (INT-35) and *tert*-butyl (*R*)-(9-(2-bromo-6-(3-((*tert*-butoxycarbonyl)amino)-3-(2-methyl-2*H*-tetrazol-5-yl)pyrrolidin-1-yl)-4-chlorobenzyl)-9*H*-purin-6-yl)(*tert*-butoxycarbonyl)carbamate (INT-36) (260 mg, crude) in DCM (5 mL) was added 4M HCl in dioxane solution (10 mL). The mixture was stirred at 20 °C for 16 hours.

The mixture was concentrated under vacuum to give the crude product. The residue was purified by prep-HPLC (column: Phenomenex Kromasil C18 150*25*10 µm, gradient 10-40%B (A = water (0.225% HCOOH), B = acetonitrile, flow rate: 25 mL/min) to afford the crude product. The crude product was purified by SFC (AS-3C_3_5_40_3ML; column: AS (250 mm*30 mm, 10 µm), Mobile phase: MeOH (0.1% NH₃.H₂O) in CO₂ from 40% to 40%; Flow rate: 70 mL/min Wave length: 220nm) to afford two fractions, which was further separately purified by prep-HPLC (column: Phenomenex Gemini C18 250*25 mm*10 um, gradient 25-55% B (A = water (0.05% NH₃.H₂O), B = acetonitrile, flow rate: 25 mL/min) to give (*R*)-9-(2-(3-amino-3-(1-methyl-1H-tetrazol-5-yl)pyrrolidin-1-yl)-6-bromo-4-chlorobenzyl)-9*H*-purin-6-amine (Example 90) and (*R*)-9-(2-(3-amino-3-(2-methyl-2*H*-tetrazol-5-yl)pyrrolidin-1-yl)-6-bromo-4-chlorobenzyl)-9*H-*purin-6-amine (Example 91).

Example 90 (slow eluent): ¹H NMR: (400 MHz, Methanol-d₄) δ ppm 8.24 - 8.22 (m, 1H), 7.68 (s, 1H), 7.41 (d, *J* = 2.0 Hz, 1H), 7.25 (d, *J* = 2.0 Hz, 1H), 5.61 - 5.45 (m, 2H), 4.28 (s, 3H), 3.73 - 3.69 (m 1H), 3.49 - 3.42 (m, 1H), 3.35 - 3.32 (m, 1H), 3.28 - 3.25 (m, 1H), 2.64 - 2.56 (m, 1H), 2.22 - 2.11 (m, 1H). LC-MS: [M+H]⁺ = 506.0.

Example 91 (fast eluent.: ¹H NMR: (400 MHz, Methanol-*d*₄) δ ppm 8.24 - 8.21 (m, 1H), 7.78 (s, 1H), 7.45 (d, *J* = 2.0 Hz, 1H), 7.29 (d, *J* = 2.0 Hz, 1H), 5.64 - 5.49 (m, 2H), 4.19 - 4.15 (m, 3H), 3.88 - 3.84 (m, 1H), 3.50 - 3.41 (m, 1H), 3.40 - 3.35 (m, 1H), 3.21 - 3.12 (m, 1H), 2.65 - 2.55 (m, 1H), 2.28 - 2.20 (m, 1H). LC-MS: [M+H]⁺ = 506.0.

### Example 92. (R)-3-amino-1-(4-((6-amino-9H-purin-9-yl)methyl)-5-ethyl-2'-fluoro-[1,1'-biphenyl]-3-yl)-N-cyclopropylpyrrolidine-3-carboxamide

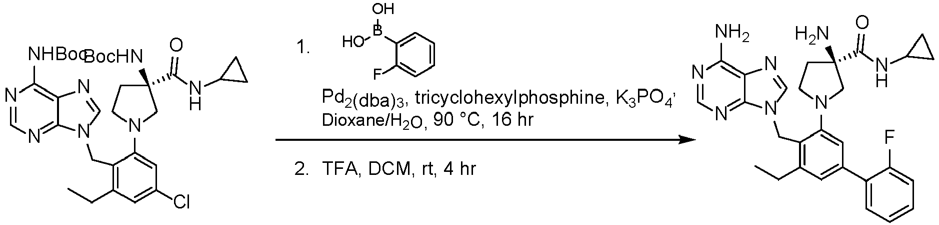

To a solution of tert-butyl (R)-(9-(2-(3-((tert-butoxycarbonyl)amino)-3-(cyclopropylcarbamoyl)pyrrolidin-1-yl)-4-chloro-6-ethylbenzyl)-9H-purin-6-yl)carbamate (120 mg, 183 µmol), 2-fluorophenylboronic acid (128 mg, 915 µmol) in dioxane/H₂O (1 mL/0.1 mL) was added Pd₂(dba)₃ (17 mg, 18.3 pmol), tricyclohexylphosphine (5.1 mg, 18.3 pmol), and K₃PO₄ (116 mg, 549 µmol) at 25 °C. The mixture was stirred at 110 °C under N₂ for 16 hrs. The mixture was diluted with H₂O (50 mL) and extracted with ethyl acetate (3 × 10 mL). The organic layer was combined, dried over anhydrous sodium sulfate, filtered and concentrated under vacuum to afford the crude product (210 mg, crude) as a yellow oil. The crude product was then redissolved in DCM (2 mL) and treated with TFA (0.5 mL), the mixture was stirred at 25 °C for 4 hours. The mixture was then concentrated under vacuum. The residue was purified by prep-HPLC [column: Phenomenex Gemini 150 × 25 mm × 10 um, gradient 30 %-60 % B (A = water (10mM NH₄HCO₃), B = MeCN), flow rate: 25 mL/min] to afford (*R*)-3-amino-1-(4-((6-amino-9H-purin-9-yl)methyl)-5-ethyl-2'-fluoro-[1,1'-biphenyl]-3-yl)-*N*-cyclopropylpyrrolidine-3-carboxamide as a white solid. ¹H NMR: (400 MHz, Methanol-d₄) δ ppm 8.31 (s, 1H), 7.75 (s, 1H), 7.53 - 7.50 (m, 1H), 7.45 - 7.36 (m, 2H), 7.33 - 7.17 (m, 3H), 5.69 - 5.54 (m, 2H), 3.61 (d, *J* = 9.4 Hz, 1H), 3.27 - 3.22 (m, 1H), 3.10 - 3.08 (m, 1H), 2.92 (d, *J* = 9.2 Hz, 1H), 2.83 - 2.72 (m, 2H), 2.66 - 2.64 (m, 1H), 2.50 - 2.39 (m, 1H), 1.74 - 1.72 (m, 1H), 1.13 (t, *J* = 7.6 Hz, 3H), 0.76 - 0.66 (m, 2H), 0.53 - 0.44 (m, 2H). LC-MS: [M+H]⁺ = 515.2.

The following examples were prepared by coupling the corresponding intermediate and boronic acid following procedures analogous to Example 92.

| Ex No. | Boronic Agent | | |
|---|---|---|---|
| 93 | 2-thiophene boronic acid | 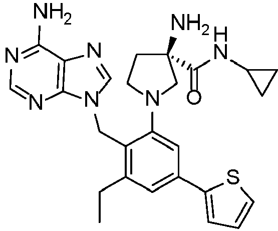 | ¹H NMR (400 MHz, Methanol-d₄) δ ppm 8.55 (s, 1 H), 8.28 (s, 1 H), 7.71 (s, 1 H), 7.48 (d, *J* = 1.75 Hz, 1 H), 7.45 (dd, *J* = 3.63, 1.13 Hz, 1 H), 7.40 (dd, *J* = 5.07, 1.06 Hz, 1 H), 7.35 (d, *J* = 1.75 Hz, 1 H), 7.11 (dd, *J* = 5.13, 3.63 Hz, 1 H), 5.49 - 5.61 (m, 2 H), 3.61 (d, *J* = 9.26 Hz, 1 H), 3.21 - 3.28 (m, 1 H), 3.08 (td, *J* = 8.72, 5.32 Hz, 1 H), 2.89 (d, *J* = 9.26 Hz, 1 H), 2.72 (qd, *J* = 7.46, 2.50 Hz, 2 H), 2.60 - 2.68 (m, 1 H), 2.44 (ddd, *J* = 12.85, 8.54, 6.50 Hz, 1 H), 1.73 (ddd, *J* = 12.88, 7.50, 5.38 Hz, 1 H), 1.11 (t, *J* = 7.57 Hz, 3 H), 0.66 - 0.76 (m, 2 H), 0.43 - 0.52 (m, 2 H). LC-MS: [M+H]⁺ = 503.3. |
| 94 | 2-thiophene boronic acid | 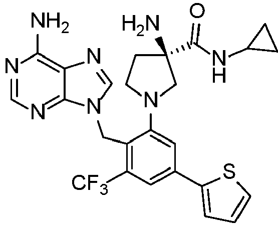 | ¹H NMR (400 MHz, Methanol-d₄) δ ppm 8.28 (s, 1H), 7.88 (s, 1H), 7.80 (s, 1H), 7.63 (s, 1H), 7.60 (d, *J* = 4.0 Hz, 1H), 7.52 (d, *J* = 5.2 Hz, 1H), 7.21 - 7.13 (m, 1H), 5.73 - 5.52 (m, 2H), 3.68 - 3.57 (m, 1H), 3.21 - 3.11 (m, 1H), 3.09 - 2.99 (m, 1H), 2.86 (d, *J* = 9.6 Hz, 1H), 2.65 - 2.62 (m, 1H), 2.43 - 2.27 (m, 1H), 1.67 - 1.51 (m, 1H), 0.72 - 0.70 (m, 2H), 0.50 - 0.47 (m, 2H). LC-MS: [M+H]⁺ = 543.3. |
| 95 | 3-pyridyl boronic acid | 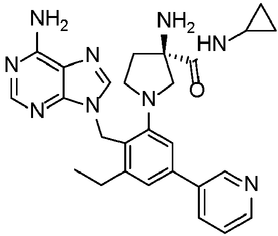 | ¹H NMR (400 MHz, DMSO-d₆) δ ppm 8.84 (d, *J* = 2.0 Hz, 1H), 8.54 (dd, *J =* 1.6, 4.8 Hz, 1H), 8.28 (s, 1H), 8.16 - 8.12 (m , 1H), 7.74 (s, 1H), 7.57 - 7.48 (m, 2H), 7.38 (d, *J* = 1.2 Hz, 1H), 7.40 - 7.35 (m, 1H), 5.67 - 5.53 (m, 2H), 3.64 (d, *J* = 9.2 Hz, 1H), 3.33 (d, *J* = 1.6 Hz, 1H), 3.12 - 3.07 (m, 1H), 2.93 (d, *J* = 9.2 Hz, 1H), 2.85 - 2.74 (m, 2H), 2.66 - 2.62 (m, 1H), 2.47 - 2.43 (m, 1H), 1.77 - 1.73 (m, 1H), 1.15 - 1.11 (m, 3H), 0.74 - 0.67 (m, 2H), 0.52 - 0.44 (m, 1H), 0.52 - 0.44 (m, 2H). LC-MS: [M+H]⁺ = 498.5. |
| 96 | 1-methyl-4-(4,4,5,5-tetramethyl-1,3,2-dioxaborolan-2-yl)pyrazole | 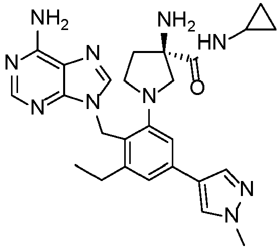 | ¹H NMR (400 MHz, Methanol-d₄) δ ppm 8.28 (s, 1H), 8.01 (s, 1H), 7.86 (s, 1H), 7.67 (s, 1H), 7.41 (d, *J* = 1.6 Hz, 1H), 7.28 (s, 1H), 5.59 - 5.48 (m, 2H), 3.93 (s, 3H), 3.58 (d, *J* = 9.2 Hz, 1H), 3.24 - 3.20 (m, 1H), 3.08 - 3.04 (m, 1H), 2.88 (d, *J* = 9.2 Hz, 1H), 2.73 - 2.61 (m, 3H), 2.43 (m, 1H), 1.77 - 1.67 (m, 1H), 1.12 - 1.08 (m, 3H), 0.75 - 0.64 (m, 2H), 0.51 - 0.43 (m, 2H). LC-MS: [M+H]⁺ = 501.1. |
| 97 | cyclopropyl boronic acid | 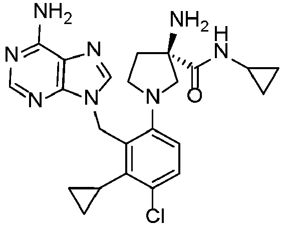 | ¹H NMR (400 MHz, Methanol-*d*₄) δ ppm 8.22 (s, 1H), 7.70 (s, 1H), 7.37 (d, *J* = 8.7 Hz, 1H), 7.21 (d, *J* = 8.8 Hz, 1H), 5.87 (d, *J* = 14.4 Hz, 1H), 5.78 (d, *J* = 14.3 Hz, 1H), 3.55 (d, *J* = 9.4 Hz, 1H), 3.25 (dd, *J* = 8.9, 7.1 Hz, 3H), 3.01 (td, *J* = 8.7, 5.1 Hz, 1H), 2.83 (d, *J* = 9.3 Hz, 1H), 2.63 (tt, *J* = 7.3, 3.9 Hz, 1H), 2.41 (ddd, *J* = 12.8, 8.5, 6.6 Hz, 1H), 1.70 (ddd, *J* = 12.7, 7.5, 5.1 Hz, 1H), 1.54 (ddd, *J* = 14.2, 8.4, 5.9 Hz, 1H), 1.13-1.02 (m, 2H), 0.78 - 0.59 (m, 4H), 0.53 - 0.40 (m, 2H). LC-MS: [M+H]⁺ = 466.9. |

### Example 98. (R)-3-amino-1-(2-((6-amino-9H-purin-9-yl)methyl)-3-ethyl-5-(thiazol-4-yl)phenyl)-N-cyclopropylpyrrolidine-3-carboxamide

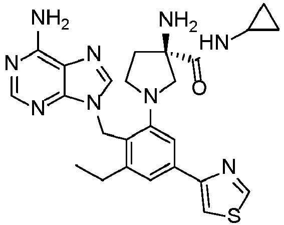

### Step 1: tert-butyl (R)-(1-(2-((6-amino-9H-purin-9-yl)methyl)-3-ethyl-5-(thiazol-4-yl)phenyl)-3-(cyclopropylcarbamoyl)pyrrolidin-3-yl)carbamate (15-1)

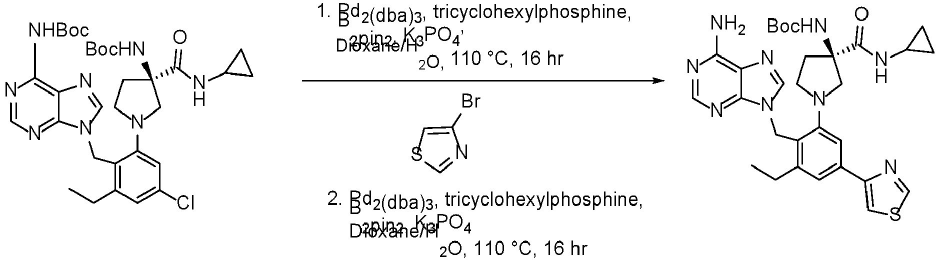

To a solution of tert-butyl (R)-(9-(2-(3-((tert-butoxycarbonyl)amino)-3-(cyclopropylcarbamoyl)pyrrolidin-1-yl)-4-chloro-6-ethylbenzyl)-9H-purin-6-yl)carbamate (350 mg, 0.5 mmol), B₂pin₂ (540 mg, 2.13 mmol) and tricyclohexylphosphine (49 mg, 0.2 mmol) in dioxane (3.5 mL) and H₂O (0.35 mL) was added K₃PO₄ (224 mg, 1 mmol) and Pd₂(dba)₃ (49 mg, 0.06 mmol). The mixture was stirred at 110 °C for 16 hrs under N₂. The mixture was poured into 10 mL of water and extracted with EA (10 mL × 2). The combined organic phase was dried over anhydrous Na₂SO₄, concentrated under vacuum to give the crude product as a yellow oil (700 mg, crude). To a solution of the crude product (700 mg, 0.94 mmol), 4-bromothiazole (616 mg, 3.7 mmol) and tricyclohexylphosphine (77 mg, 0.3 mmol) in dioxane (7 mL) and H₂O (0.7 mL) was added K₃PO₄ (399 mg, 1.9 mmol) and Pd₂(dba)₃ (63.7 mg, 0.07 mmol). The mixture was stirred at 110 °C for 16 hrs under N₂. The mixture was poured into 20 mL of water and extracted with EA (20 mL × 2). The combined organic phase was dried over anhydrous Na₂SO₄ and concentrated under vacuum to give the crude product. The residue was purified by reversed-phase chromatography to give *tert*-butyl (*R*)-(1-(2-((6-amino-9*H*-purin-9-yl)methyl)-3-ethyl-5-(thiazol-4-yl)phenyl)-3-(cyclopropylcarbamoyl)pyrrolidin-3-yl)carbamate as a white solid. LC-MS: [M+H]⁺ = 604.2.

### Step 2: (R)-3-amino-1-(2-((6-amino-9H-purin-9-yl)methyl)-3-ethyl-5-(thiazol-4-yl)phenyl)-N-cyclopropylpyrrolidine-3-carboxamide

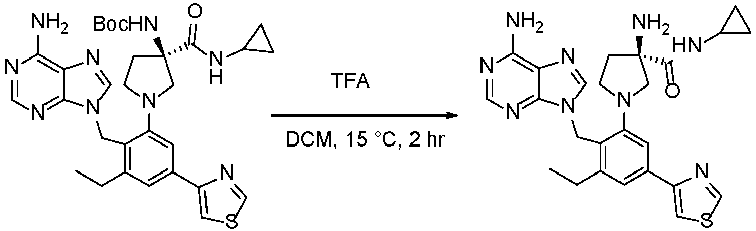

To the solution of *tert*-butyl (*R*)-(1-(2-((6-amino-9*H*-purin-9-yl)methyl)-3-ethyl-5-(thiazol-4-yl)phenyl)-3-(cyclopropylcarbamoyl)pyrrolidin-3-yl)carbamate (100 mg, 0.14 mmol) in DCM (1 mL) was added TFA (1 mL). The mixture was stirred at 15 °C for 2 hours. The mixture was concentrated in vacuum and purified by prep-HPLC (Phenomenex Synergi C18 150*25*10 um, water (0.1 %TFA)-MeCN) to give (R)-3-amino-1-(2-((6-amino-9H-purin-9-yl)methyl)-3-ethyl-5-(thiazol-4-yl)phenyl)-*N*-cyclopropylpyrrolidine-3-carboxamide (as an off-white solid. ¹H NMR (400 MHz, Methanol-*d*₄) δ ppm 9.11 (d, *J* = 2.0 Hz, 1H), 8.43 (s, 1H), 8.02 (d, *J* = 2.0 Hz, 1H), 7.95 (d, *J* = 1.6 Hz, 1H), 7.93 (s, 1H), 7.76 (d, *J* = 1.6 Hz, 1H), 5.75 - 5.65 (m, 2H), 3.71 (d, *J* = 10.8 Hz, 1H), 3.41 (d, *J =* 10.8 Hz, 1H), 3.23 (t, *J =* 7.2 Hz, 2H), 2.73 - 2.66 (m, 3H), 2.56 (td, *J* = 7.6, 14.8 Hz, 1H), 2.26 - 2.16 (m, 1H), 1.10 (t, *J =* 7.6 Hz, 3H), 0.80 - 0.66 (m, 2H), 0.57 - 0.47 (m, 2H). LC-MS: [M+H]⁺ = 504.3.

### Example 99. (R)-3-amino-1-(2-((6-amino-9H-purin-9-yl)methyl)-3-ethyl-5-(pyridin-2-yl)phenyl)-N-cyclopropylpyrrolidine-3-carboxamide

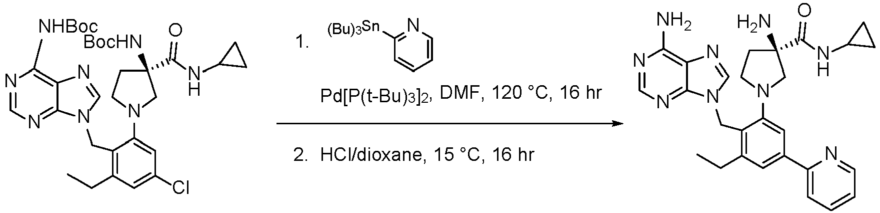

To a solution of tert-butyl (R)-(9-(2-(3-((tert-butoxycarbonyl)amino)-3-(cyclopropylcarbamoyl)pyrrolidin-1-yl)-4-chloro-6-ethylbenzyl)-9H-purin-6-yl)carbamate (100 mg, 0.15 mmol), 2-(tributylstannyl)pyridine (66 mg, 0.18 mmol) in DMF (1 mL) was added Pd[P(t-Bu)₃]₂ (16 mg, 0.304 mmol). The mixture was stirred at 120 °C for 16 hrs under N₂. The mixture was poured into 10 mL of water and extracted with EA (10 mL × 2). The combined organic phase was dried over anhydrous Na₂SO₄ and concentrated under vacuum to give the crude product (170 mg, crude) as a yellow solid. The crude product was then treated with 4 M HCI in dioxane solution (2 mL) and stirred at 15 °C for 16 hours. The mixture was concentrated in vacuum to give the crude product. The residue was purified by prep-HPLC (Phenomenex Gemini 150*25mm*10um, water(0.04% NH₃H_{z}O+10mM NH₄HCO₃)-MeCN) to give (*R*)-3-amino-1-(2-((6-amino-9*H*-purin-9-yl)methyl)-3-ethyl-5-(pyridin-2-yl)phenyl)-*N*-cyclopropylpyrrolidine-3-carboxamide as a yellow solid. ¹H NMR (400 MHz, Methanol-*d*₄) δ ppm 8.63 (d, *J =* 4.4 Hz, 1H), 8.28 (s, 1H), 7.96 - 7.87 (m, 2H), 7.82 (d, *J* = 1.6 Hz, 1H), 7.74 (s, 1H), 7.66 (d, *J* = 1.6 Hz, 1H), 7.40 - 7.36 (m, 1H), 5.67 - 5.54 (m, 2H), 3.65 (d, *J =* 9.2 Hz, 1H), 3.29 - 3.27 (m, 1H), 3.15 - 3.09 (m, 1H), 2.94 (d, *J =* 9.4 Hz, 1H), 2.83 - 2.75 (m, 2H), 2.66 - 2.62 (m, 1H), 2.52 - 2.41 (m, 1H), 2.01 (s, 1H), 1.80 - 1.71 (m, 1H), 1.14 (t, *J =* 7.6 Hz, 3H), 0.76 - 0.64 (m, 2H), 0.52 - 0.43 (m, 2H). LC-MS: [M+H]⁺ = 498.1.

The following example was prepared following the general procedure for Example 99 by coupling with 2-(tributylstannyl)pyrazine.

### Example 100. (R)-3-amino-1-(2-((6-amino-9H-purin-9-yl)methyl)-3-ethyl-5-(pyrazin-2-yl)phenyl)-H-cyclopropylpyrrolidine-3-carboxamide

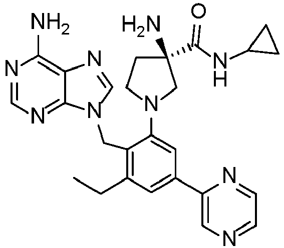

¹H NMR (400 MHz, Methanol-*d*₄) δ ppm 9.17 (d, *J* = 1.6 Hz, 1H), 8.75 - 8.66 (m, 1H), 8.58 (d, *J* = 2.4 Hz, 1H), 8.30 (s, 1H), 7.97 (d, *J* = 1.6 Hz, 1H), 7.82 (d, *J* = 1.6 Hz, 1H), 7.77 (s, 1H), 5.71 - 5.57 (m, 2H), 3.68 (d, *J* = 9.4 Hz, 1H), 3.30 - 3.25 (m, 1H), 3.14 - 3.13(m, 1H), 2.96 (d, *J* = 9.4 Hz, 1H), 2.88 - 2.75 (m, 2H), 2.68 - 2.66 (m, 1H), 2.48 - 2.46 (m, 1H), 1.85 - 1.71 (m, 1H), 1.17 (t, *J* = 7.5 Hz, 3H), 0.78 - 0.67 (m, 2H), 0.56 - 0.45 (m, 2H). LC-MS: [M+H]⁺ = 499.2.

### Example 101. (R)-3-amino-1-(2-((6-amino-9H-purin-9-yl)methyl)-5-chloro-4-cyano-3-ethylphenyl)-N-cyclopropylpyrrolidine-3-carboxamide

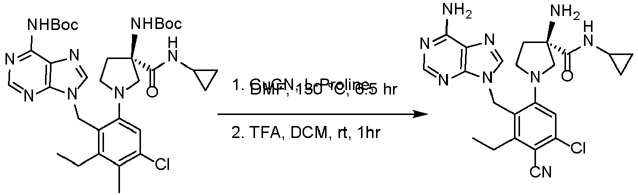

To a DMF solution (3 mL) of tert-butyl (R)-(1-(2-((6-((tert-butoxycarbonyl)amino)-9H-purin-9-yl)methyl)-5-chloro-3-ethyl-4-iodophenyl)-3-(cyclopropylcarbamoyl)pyrrolidin-3-yl)carbamate (200 mg, 0.26 mmol) was added CuCN (46 mg, 0.51 mmol) and L-proline (30 mg, 0.26 mmol). The reaction tube was sealed and placed into the pre-heated heating block (130 °C). The reaction was stirred for 6.5 hr. After cooling down to rt, the reaction was diluted with MeOH and filtered through a syringe filter. The filtrate was extracted with EA, washed with saturated NaHCO₃ solution, water and brine. The organic layer was then dried over Na₂SO₄ and concentrated under vacuum to give the crude product. The crude product was purified by column chromatography (0 to 10% MeOH in DCM) to give the intermediate, which was redissolved in DCM (2 mL) and treated with TFA (1 mL). The reaction was stirred at rt for 1 hr, and then directly concentrated to give the crude product. Purification by Prep-HPLC (Column: XBridge 30*150mm 5um, gradient: 30-100% B (A = water (0.05% NH₄OH), B = acetonitrile (0.05% NH₄OH)), flow rate: 30 mL/min) to give (*R*)-3-amino-1-(2-((6-amino-9*H*-purin-9-yl)methyl)-5-chloro-4-cyano-3-ethylphenyl)*-N*-cyclopropyl pyrrolidine-3-carboxamide.¹H NMR (400 MHz, Methanol-*d*₄) δ ppm 8.26 (s, 1H), 7.78 (s, 1H), 7.12 (s, 1H), 5.51 (d, *J* = 15.4 Hz, 1H), 5.44 (d, *J =* 15.3 Hz, 1H), 3.80 (d, *J =* 9.6 Hz, 1H), 3.64 (td, *J* = 8.9, 7.1 Hz, 1H), 3.02 (dd, *J* = 9.5, 1.2 Hz, 1H), 2.96 - 2.79 (m, 2H), 2.62 (tt, *J* = 7.4, 3.9 Hz, 1H), 2.36 (dt, *J* = 12.6, 8.4 Hz, 1H), 1.78 (dddd, *J* = 12.3, 7.1, 3.8, 1.1 Hz, 1H), 1.10 (t, *J =* 7.5 Hz, 3H), 0.78 - 0.64 (m, 2H), 0.52 - 0.43 (m, 2H). LC-MS: [M+H]⁺ = 480.2.

### Examples 102 and 103. (R)-3-amino-1-(2-((6-amino-9H-purin-9-yl)methyl)-5-chloro-4-fluoro-3-(morpholinomethyl)phenyl)-N-cyclopropylpyrrolidine-3-carboxamide and (R)-3-amino-1-(2-((6-amino-9H-purin-9-yl)methyl)-3-chloro-4-fluoro-5-(morpholinomethyl)phenyl)-N-cyclopropylpyrrolidine-3-carboxamide

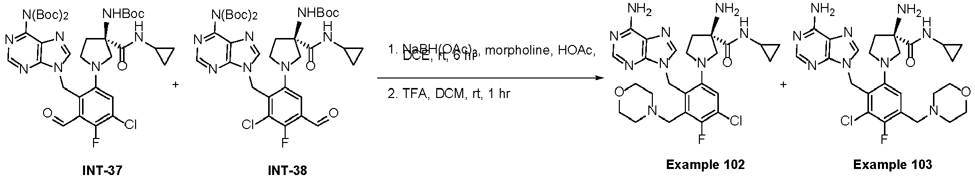

To a DCE solution (2 mL) of *tert*-butyl (*R*)-(tert-butoxycarbonyl)(9-(6-(3-((*tert-*butoxycarbonyl)amino)-3-(cyclopropylcarbamoyl)pyrrolidin-1-yl)-4-chloro-3-fluoro-2-formylbenzyl)-9*H*-purin-6-yl)carbamate (INT-37) and tert-butyl (*R*)-(*tert*-butoxycarbonyl)(9-(6-(3-((*tert*-butoxycarbonyl)amino)-3-(cyclopropylcarbamoyl)pyrrolidin-1-yl)-2-chloro-3-fluoro-4-formylbenzyl)9*H*-purin-6-yl)carbamate (INT-38) (100 mg, 0.13 mmol) and morpholine (34 uL, 0.4 mmol) was added 3 drops of AcOH. The solution was stirred for 15 min followed by addition of sodium triacetoxyborohydride (55 mg, 0.26 mmol). The reaction was stirred for 2 hr, LC-MS showed about 50% conversion. Sodium triacetoxyborohydride (55 mg, 0.26 mmol) was added to the reaction, and the reaction was stirred for another 2 hr. The reaction was diluted with EA, washed with saturated NaHCO₃ solution and brine. The organic layer was dried over Na₂SO₄ and concentrated under vacuum to give the intermediate, which was redissolved in DCM (2 mL) and treated with TFA (1 mL) at rt. The reaction was stirred for 1 hr, and then directly concentrated to give the crude product. Purification by prep-HPLC (Column: XBridge 30*150mm 5um, gradient: 30-100% B (A = water (0.05% NH₄OH), B = acetonitrile (0.05% NH₄OH)), flow rate: 30 mL/min) gave (*R*)-3-amino-1-(2-((6-amino-9*H*-purin-9-yl)methyl)-5-chloro-4-fluoro-3-(morpholinomethyl)phenyl)-*N*-cyclopropylpyrrolidine-3-carboxamide (Example 102) and (*R*)-3-amino-1-(2-((6-amino-9*H*-purin-9-yl)methyl)-3-chloro-4-fluoro-5-(morpholinomethyl)phenyl)-*N-*cyclopropylpyrrolidine-3-carboxamide (Example 103) as white solids.

Example 102 (slow eluent): ¹H NMR (400 MHz, Methanol-*d*₄) δ ppm 8.24 (s, 1H), 7.87 (s, 1H), 7.54 (d, *J =* 6.9 Hz, 1H), 5.64 (d, *J =* 14.1 Hz, 1H), 5.57 (d, *J =* 14.1 Hz, 1H), 3.73 (dd, *J* = 13.0, 2.9 Hz, 1H), 3.64 (dd, *J* = 13.0, 2.8 Hz, 1H), 3.54 (d, *J =* 9.3 Hz, 1H), 3.39 (s, 4H), 3.18 - 3.10 (m, 1H), 2.99 (td, *J* = 8.6, 5.3 Hz, 1H), 2.82 (d, *J =* 9.3 Hz, 1H), 2.63 (tt, *J =* 7.3, 3.9 Hz, 1H), 2.43 - 2.31 (m, 5H), 1.68 (tt, *J* = 7.4, 5.5 Hz, 1H). LC-MS: [M+H]⁺ = 544.2.

Example 103 (fast eluent): ¹H NMR (400 MHz, Methanol-*d*₄) δ ppm 8.24 (s, 1H), 7.86 (s, 1H), 7.41 (d, *J =* 6.3 Hz, 1H), 5.68 (d, *J =* 14.1 Hz, 1H), 5.59 (d, *J =* 14.1 Hz, 1H), 3.73 - 3.67 (m, 4H), 3.63 (d, *J =* 1.6 Hz, 2H), 3.56 (d, *J =* 9.3 Hz, 1H), 3.20 (ddd, J = 8.8, 7.7, 6.4 Hz, 1H), 3.02 (td, *J* = 8.8, 5.4 Hz, 1H), 2.88 (d, *J =* 9.2 Hz, 1H), 2.64 (tt, *J* = 7.2, 3.9 Hz, 1H), 2.51 (t, *J* = 4.7 Hz, 4H), 2.42 (ddd, *J* = 12.9, 8.6, 6.4 Hz, 1H), 1.74 (ddd, *J* = 12.9, 7.6, 5.4 Hz, 1H), 0.71 (dddd, *J* = 8.4, 4.9, 4.1, 2.3 Hz, 2H), 0.52 - 0.42 (m, 2H). LC-MS: [M+H]⁺ = 544.2.

The following examples were prepared from corresponding intermediates following procedures analogous to those described in the examples.

| Ex No. | | |
|---|---|---|
| 104 | 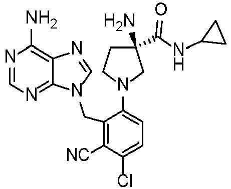 | ¹H NMR (400 MHz, Methanol-d₄): δ = 8.39 (s, 1H), 8.34 (s, 1H), 7.78 (d, *J=* 8.8 Hz, 1H), 7.68 (d, *J=* 8.8 Hz, 1H), 5.96-5.79 (m, 2H), 3.77-3.74 (m, 1H), 3.48-3.43 (m, 2H), 3.33-3.31 (m, 1H), 2.74-2.64 (m, 2H), 2.34-2.32 (m, 1H), 0.77-0.71 (m, 2H), 0.57-0.56 (m, 2H). LC-MS: [M+H]⁺ = 452.1. |
| 105 | 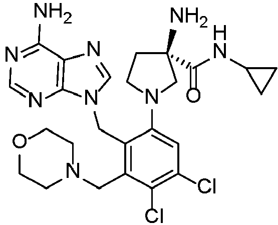 | ¹H NMR (400 MHz, Methanol-*d*₄) δ ppm 8.25 (s, 1H), 7.78 (s, 1H), 7.54 (s, 1H), 5.74 - 5.54 (m, 2H), 3.94 (d, *J* = 13.2 Hz, 1H), 3.86 (d, *J* = 13.1 Hz, 1H), 3.59 (d, *J* = 9.4 Hz, 1H), 3.38 (d, *J* = 5.3 Hz, 4H), 3.29 - 3.22 (m, 1H), 3.06 (td, J = 8.6, 5.0 Hz, 1H), 2.85 (dd, *J* = 9.3, 0.8 Hz, 1H), 2.63 (tt, *J* = 7.4, 3.9 Hz, 1H), 2.43 (t, *J* = 4.7 Hz, 4H), 2.40 - 2.33 (m, 1H), 1.69 (ddd, *J* = 12.6, 7.3, 4.9 Hz, 1H), 0.77 - 0.61 (m, 2H), 0.56 - 0.39 (m, 2H). LC-MS: [M+H]⁺ = 560.2. |
| 106 | 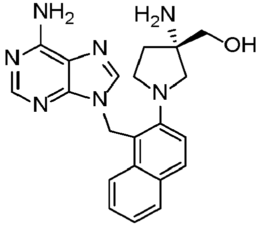 | ¹H NMR (400 MHz, Methanol-*d*4) δ = 8.35 (s, 1H), 7.96 (d, *J* = 9.2 Hz, 1H), 7.90-7.83 (m, 2H), 7.61 (d, *J* = 9.2 Hz, 1H), 7.46 (s, 1H), 7.46-7.37 (m, 2H), 5.97-5.85 (m, 2H), 3.59-3.49 (m, 2H), 3.37-3.33 (m, 1H), 3.30-3.26 (m, 1H), 3.20-3.12 (m, 1H), 3.00 (d, *J=* 9.2 Hz, 1H), 2.05-1.95 (m, 1H), 1.80-1.70 (m, 1H). LC-MS: [M+1]⁺ = 390.3. |
| 107 | 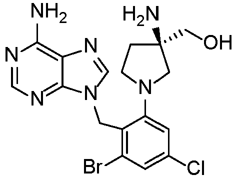 | ¹H NMR (400 MHz, Methanol-*d*4) δ = 0.00 (br s, 1 H) 0.09 (br s, 1 H) 1.29 (br s, 1 H) 1.63 - 1.76 (m, 1 H) 1.86 - 1.99 (m, 1 H) 2.86 - 2.98 (m, 1 H) 3.08 (br s, 1 H) 3.15 - 3.24 (m, 2 H) 3.44 (br s, 4 H) 4.59 - 4.80 (m, 1 H) 5.48 - 5.64 (m, 2 H) 7.23 (br s, 1 H) 7.41 (br s, 1 H) 7.74 (br s, 1 H) 8.26 (br s, 1 H). LC-MS: [M+1]⁺ = 454.1. |
| 108 | 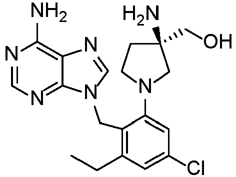 | ¹H NMR (400 MHz, Methanol-*d*4) δ = 8.30 (s, 1H), 7.62 (s, 1H), 7.21 (d, *J* =2.0 Hz, 1H), 7.10 (d, *J=2.0* Hz, 1H), 5.56- 5.45 (m, 2H), 3.46 (m, 3H), 3.17-3.11 (m, 3H), 3.05-3.00 (m, 1H), 2.88-2.85 (m, 1H), 2.67-2.63 (m, 2H), 1.09 (t, *J* = 7.6 Hz, 3H). LC-MS: [M+1]⁺ = 402.4. |
| 109 | 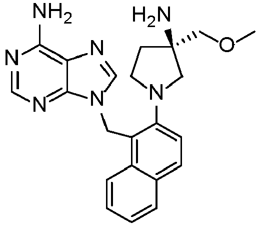 | ¹H NMR (400 MHz, Methanol-*d*4) δ = 8.34 (s, 1H), 7.96-7.81 (m, 3H), 7.55 (d, *J* = 8.8 Hz, 1H), 7.46 (s, 1H), 7.44-7.33 (m, 2H), 5.90 (s, 2H), 5.86-5.73 (m, 2H), 3.46-3.37 (m, 1H), 3.31-3.22 (m, 6H), 3.21-3.13 (m, 1H), 2.86 (d, *J* = 8.8 Hz, 1H), 1.91-1.87 (m, 1H), 1.70-1.60 (m, 1H). LC-MS: [M+1]⁺ = 404.4. |
| 110 | 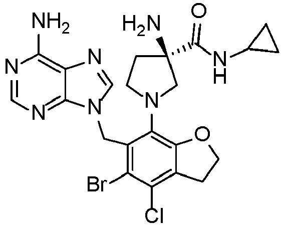 | ¹H NMR (400 MHz, Methanol-*d*4) δ 8.25 (s, 1H), 7.75 (s, 1H), 5.75 (d, *J* = 13.4 Hz, 1H), 5.60 (d, *J* = 13.5 Hz, 1H), 4.74 (t, *J* = 8.9 Hz, 2H), 3.59 (d, *J* = 9.0 Hz, 1H), 3.35 (t, *J=* 8.8 Hz, 2H), 3.09 - 3.02 (m, 1H), 2.99 - 2.90 (m, 1H), 2.82 (d, *J =* 9.1 Hz, 1H), 2.66 - 2.57 (m, 1H), 2.48 - 2.37 (m, 1H), 1.63 - 1.54 (m, 1H), 0.76 - 0.63 (m, 2H), 0.50 - 0.40 (m, 2H). LC-MS: [M/M+2]⁺ = 546.8/548.8. |
| 111 | 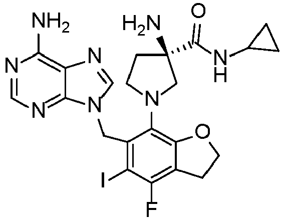 | ¹H NMR (400 MHz, Methanol-*d*4) δ 8.26 (s, 1H), 7.69 (s, 1H), 5.79 - 5.54 (m, 2H), 4.79 - 4.73 (m, 2H), 3.56 (d, *J* = 9.0 Hz, 1H), 3.37 - 3.32 (m, 2H), 3.06 - 2.99 (m, 1H), 2.93 - 2.83 (m, 1H), 2.78 (d, *J* = 9.0 Hz, 1H), 2.66 - 2.58 (m, 1H), 2.47 - 2.35 (m, 1H), 1.60 - 1.50 (m, 1H), 0.73 - 0.65 (m, 2H), 0.49 - 0.42 (m, 2H). LC-MS: [M+1]⁺ = 578.8. |
| 112 | 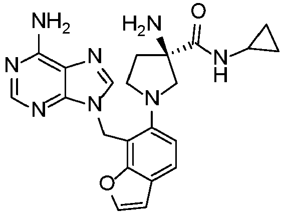 | ¹H NMR: (MeOD 400 MHz) : δ = 8.25 (s, 1H), 7.96 (s, 1H), 7.71 (d, *J*=2.4 Hz, 1H), 7.60 (d, *J*=8.4 Hz, 1H), 7.24 (d, *J*=8.4 Hz, 1H), 6.82 (d, *J*=2.4 Hz, 1H), 5.85 - 5.72 (m, 2H), 3.63 (d, *J*=9.2 Hz, 1H), 3.17 - 3.06 (m, 2H), 2.98 (d, *J*=9.2 Hz, 1H), 2.71-2.65 (m, 1H), 2.53-2.45 (m, 1H), 1.89 - 1.75 (m, 1H), 0.77 - 0.67 (m, 2H), 0.55 - 0.48 (m, 2H). LC-MS: [M+1]⁺ = 433.4. |
| 113 | 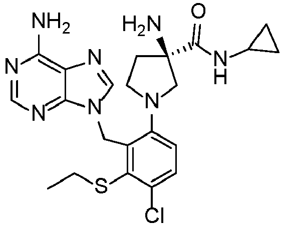 | ¹H NMR (400 MHz, METHANOL-*d*4) δ ppm 0.40 - 0.49 (m, 2 H) 0.63 - 0.73 (m, 2 H) 1.07 (t, *J* = 7.40 Hz, 3 H) 1.28 (s, 1 H) 1.71 (ddd, *J*=12.72, 7.46, 5.26 Hz, 1 H) 2.39 (ddd, *J*=12.84, 8.56, 6.72 Hz, 1 H) 2.61 (tt, *J*=7.34, 3.79 Hz, 1 H) 2.73 - 2.88 (m, 3 H) 3.02 (td, *J*=8.74, 5.14 Hz, 1 H) 3.18 - 3.26 (m, 1 H) 3.56 (d, *J*=9.29 Hz, 1 H) 5.76 (d, *J*=13.57 Hz, 1 H) 5.86 (d, *J*=13.57 Hz, 1 H) 7.35 (d, *J*=8.80 Hz, 1 H) 7.55 (d, J=8.80 Hz, 1 H) 7.65 (s, 1 H) 8.25 (s, 1 H). LC-MS: [M+1]⁺ = 486.9. |
| 114 | 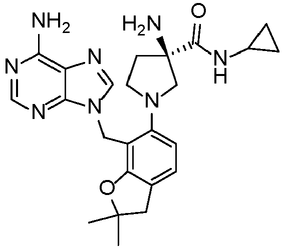 | ¹H NMR: (MeOD 400MHz) *δ* = 8.24 (s, 1H), 7.97 (s, 1H), 7.13-1.11 (m, 1H), 6.71-6.69 (m, 1H), 5.46-5.38 (m, 2H), 3.51-3.48 (m, 1H), 3.27-3.25 (m, 1H), 3.02-2.99 (m, 3H), 2.92-2.90 (m, 1H), 2.69-2.67 (m, 1H), 2.44 (m, 1H), 1.83-1.81 (m, 1H), 1.41-1.39 (m, 6H), 0.74-0.71 (m, 2H), 0.52-0.52 (m, 2H). HPLC. LC-MS: [M+1]⁺ = 463.2. |
| 115 | 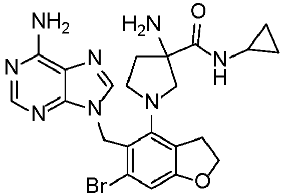 | ¹H NMR (400 MHz, Methanol-*d*4) δ 8.26 (s, 1H), 7.73 (s, 1H), 6.99 (s, 1H), 5.66 (d, *J=* 13.7 Hz, 1H), 5.49 (d, *J* = 13.6 Hz, 1H), 4.61 (t, *J* = 8.7 Hz, 2H), 3.59 (d, *J* = 8.9 Hz, 1H), 3.38 - 3.32 (m, 2H), 3.13 - 3.06 (m, 1H), 2.95 - 2.86 (m, 1H), 2.81 (d, *J* = 8.9 Hz, 1H), 2.66 - 2.58 (m, 1H), 2.42 - 2.32 (m, 1H), 0.75 - 0.63 (m, 2H), 0.51 - 0.42 (m, 2H). HPLC. LC-MS: [M/M+2]⁺ = 512.8/514.8. |

### Example 116. (R)-3-amino-1-(2-((6-amino-9H-purin-9-yl)methyl)-4-fluoro-3-(trifluoromethyl)phenyl)-N-cyclopropylpyrrolidine-3-carboxamide

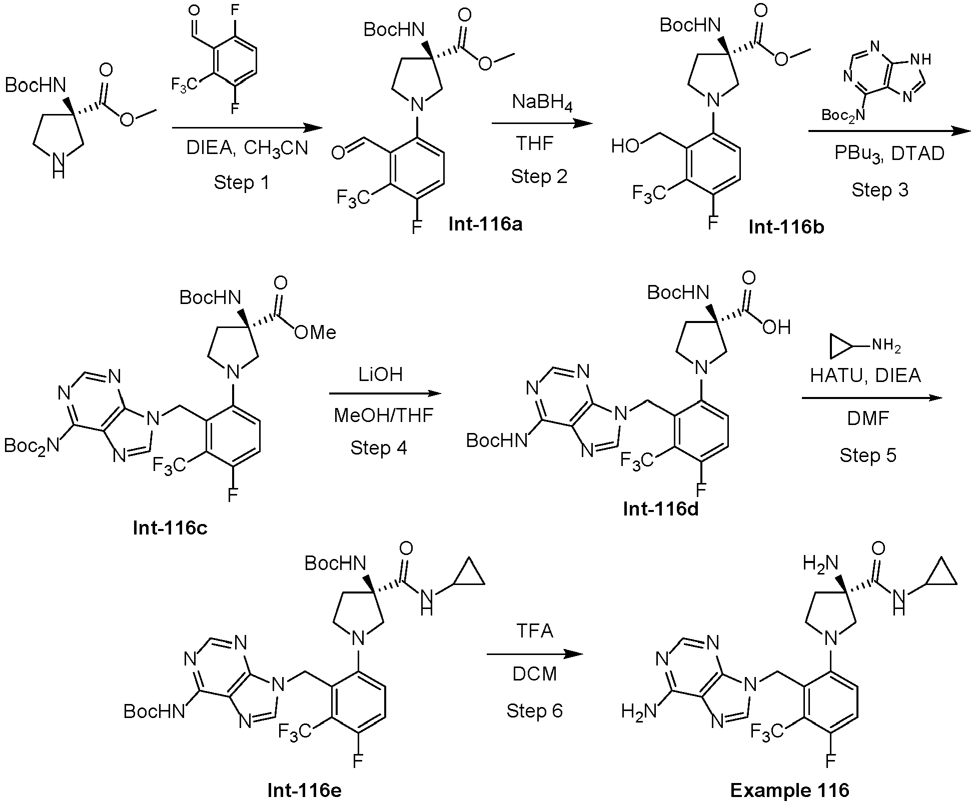

Step 1. To a solution of compound methyl (*R*)-3-((tert-butoxycarbonyl)amino)pyrrolidine-3-carboxylate (8.43 g, 0.035 mol), 3,6-difluoro-2-(trifluoromethyl)benzaldehyde (8.00 g, 0.035 mol, prepared according to General Procedure A from 1,4-difluoro-2-(trifluoromethyl)benzene) in MeCN (84 mL) was added DIEA (13.4 g, 0.1 mol). The solution was stirred at 80 °C for 16 hours. The mixture was concentrated under vacuum. The residue was purified by silica gel column (50%EtOAc in PE) to afford methyl (*R*)-3-((tert-butoxycarbonyl)amino)-1-(4-fluoro-2-formyl-3-(trifluoromethyl)phenyl)pyrrolidine-3-carboxylate (Int-116a) as a yellow oil. ¹H NMR (400MHz, CDCl₃) δ 10.31 (q, *J* = 2.8 Hz, 1H), 7.24 (t, *J =* 10.0 Hz, 1H), 7.16-7.11 (m, 1H), 5.07 (br s, 1H), 3.77 (s, 3H), 3.69 (d, *J*=11.2 Hz, 1H), 3.54-3.44 (m, 1H), 3.33-3.22 (m, 2H), 2.56-2.54 (m, 1H), 2.38-2.27 (m, 1H), 1.44 (s, 9H). LC-MS: [M+H]⁺ = 435.0.

Step 2. To a solution of methyl (*R*)-3-((tert-butoxycarbonyl)amino)-1-(4-fluoro-2-formyl-3-(trifluoromethyl)phenyl)pyrrolidine-3-carboxylate (Int-116a) (6.00 g. 13.81 mmol) in THF (60 mL) was added NaBH₄ (1 g, 27.63 mmol) dropwise. The mixture was stirred at 20-26 °C for 0.5 hour. The mixture was quenched with aq.NH₄Cl (50 mL) and extracted with EtOAc (50 mL × 2). The organic layer was concentrated under vacuum. The residue was purified by COMBI-FLASH^{®} (35% EtOAc in PE) to afford methyl (*R*)-3-((tert-butoxycarbonyl)amino)-1-(4-fluoro-2-(hydroxymethyl)-3-(trifluoromethyl)phenyl)pyrrolidine-3-carboxylate (Int-116b) as a yellow gum. ¹H NMR (400MHz, CDCl₃) δ 7.48-7.44 (m, 1H), 7.16-7.06 (m, 1H), 5.30 (br s, 1H), 4.90 (s, 2H), 3.82 (s, 3H), 3.78-3.71 (m, 1H), 3.47-3.44 (m, 1H), 3.38-3.25 (m, 2H), 2.64-2.51 (m, 1H), 2.24-2.20 (m, 1H), 1.45 (s, 9H).

Step 3. To a mixture of methyl (*R*)-3-((tert-butoxycarbonyl)amino)-1-(4-fluoro-2-(hydroxymethyl)-3-(trifluoromethyl)phenyl)pyrrolidine-3-carboxylate (Int-116b) (7 g, 16 mmol), tert-butyl (tert-butoxycarbonyl)(9H-purin-6-yl)carbamate (5.38 g, 16.04 mmol) and DTAD (11 g, 48 mmol) in THF (70 mL) was added PBu₃ (9.7 g, 48 mmol) dropwise under N₂. The mixture was stirred at 20~30°C for 16hours. The mixture was concentrated under vacuum. The residue was purified by COMBI-FLASHO (40% of EtOAc in PE) to afford methyl (R)-1-(2-((6-(bis(tert-butoxycarbonyl)amino)-9*H*-purin-9-yl)methyl)-4-fluoro-3-(trifluoromethyl)phenyl)-3-((tert-butoxycarbonyl)amino)pyrrolidine-3-carboxylate (Int-116c) as yellow solid. ¹H NMR (400MHz, CDCl₃) *δ* 8.91 (s, 1H), 7.76 (s, 1H), 7.65 (dd, *J*₁ = 9.2 Hz, *J*₂ = 4.4 Hz, 1H), 7.31 (t, *J* = 9.4 Hz, 1H), 5.87-5.68 (m, 2H), 5.42 (br s, 1H), 3.71 (s, 3H), 3.61 (d, *J* = 10.0 Hz, 1H), 3.20 (d, *J* = 10.0 Hz, 1H), 2.99-2.84 (m, 2H), 2.45-2.35 (m, 1H), 1.92-1.86 (m, 1H), 1.45 (s, 9H), 1.42 (s, 18H). LC-MS: [M+H]⁺ = 754.1.

Step 4. To a solution of methyl (*R*)-1-(2-((6-(bis(tert-butoxycarbonyl)amino)-9*H*-purin-9-yl)methyl)-4-fluoro-3-(trifluoromethyl)phenyl)-3-((tert-butoxycarbonyl)amino)pyrrolidine-3-carboxylate (Int-116c) (4.5 g, 6 mmol) in THF/MeOH/H₂O (45 mL, 4/4/2) was added aq.LiOH (1 mL, 24 mmol). The reaction mixture was stirred at 20-35 °C for 1 hour. The mixture was diluted with brine (50 mL) and adjusted pH = 2-3 with aq.HCI (1N). The mixture was extracted with EtOAc (50 mL × 3). The organic layer was concentrated under vacuum to afford (*R*)-3-((tert-butoxycarbonyl)amino)-1-(2-((6-((tert-butoxycarbonyl)amino)-9*H*-purin-9-yl)methyl)-4-fluoro-3-(trifluoromethyl)phenyl)pyrrolidine-3-carboxylic acid (Int-116d) as yellow gum. LC-MS: [M+H]⁺ =640.1.

Step 5. To a solution of (*R*)-3-((tert-butoxycarbonyl)amino)-1-(2-((6-((tert-butoxycarbonyl)amino)-9*H*-purin-9-yl)methyl)-4-fluoro-3-(trifluoromethyl)phenyl)pyrrolidine-3-carboxylic acid (Int-116d) (4 g, 6.2 mmol), HATU (4.76 g, 12.51 mmol) in DMF (40 mL) was added cyclopropanamine (0.71 g, 12.5 mmol) and DIEA (2.42 g, 18.76 mmol). The solution was stirred at 20-35 °C for 1 hour. The mixture was diluted with EtOAc (300 mL) and washed with brine (100 mL × 2). The organic layer was concentrated under vacuum. The residue was purified by COMBI-FLASHO (100%EtOAc) to afford *tert*-butyl (*R*)-(9-(6-(3-((tert-butoxycarbonyl)amino)-3-(cyclopropylcarbamoyl)pyrrolidin-1-yl)-3-fluoro-2-(trifluoromethyl)benzyl)-9*H*-purin-6-yl)carbamate (Int-116e) as yellow solid. LC-MS: [M+H]⁺ = 679.2.

Step 6. To a solution of *tert*-butyl (*R*)-(9-(6-(3-((tert-butoxycarbonyl)amino)-3-(cyclopropylcarbamoyl)pyrrolidin-1-yl)-3-fluoro-2-(trifluoromethyl)benzyl)-9*H*-purin-6-yl)carbamate (Int-116e) (1 g, 1.5 mol) in DCM (10 mL) was added TFA (6 mL). The mixture was stirred at RT for 1 hour. The mixture was concentrated under vacuum. The mixture was diluted with water (30 mL) and adjust to pH = 10-12 with aq.NH₃/H₂O. The mixture was extracted with EtOAc (50 mL × 3). The organic layer was concentrated under vacuum. The residue was purified by Prep-HPLC (column: Kromasil 250*50*10 µm. Gradient: 10-40%B, A = water (0.225% FA V/V), B = MeCN, flow rate: 100 mL/min). The product was concentrated and dried by lyophilization to afford (*R*)-3-amino-1-(2-((6-amino-9*H*-purin-9-yl)methyl)-4-fluoro-3-(trifluoromethyl)phenyl)-*N*-cyclopropylpyrrolidine-3-carboxamide as a white solid. ¹H NMR (400MHz, MeOH*-d₄*) δ 8.24 (s, 1H), 7.79 (dd, *J* = 9.2 Hz, 4.8 Hz, 1H), 7.68 (s, 1H), 7.47-7.38 (m, 1H), 5.78-5.58 (m, 2H), 3.50 (d, *J =* 9.2 Hz, 1H), 3.03-2.91 (m, 2H), 2.79 (d, *J =* 9.2 Hz, 1H), 2.64-2.60 (m, 1H), 2.38-2.32 (m, 1H), 1.63-1.58 (m, 1H), 0.74-0.64 (m, 2H), 0.51-0.40 (m, 2H). LC-MS: [M+H]⁺ = 479.0.

The following examples were prepared from corresponding intermediates following procedures analogous to those described in the examples.

| Ex No. | | |
|---|---|---|
| 117 | 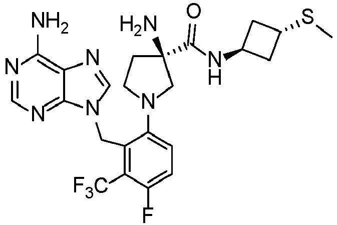 | ¹H NMR (400 MHz, Methanol-d4) δ 8.24 (s, 1H), 7.78 (dd, *J* = 9.2, 4.7 Hz, 1H), 7.67 (s, 1H), 7.42 (dd, *J* = 11.1, 9.1 Hz, 1H), 5.78 - 5.59 (m, 2H), 4.49 - 4.40 (m, 1H), 3.50 - 3.46 (m, 1H), 3.20-3.30 (m, 1H), 3.02 - 2.92 (m, 2H), 2.79 (d, *J* = 9.3 Hz, 1H), 2.41 - 2.27 (m, 3H), 2.26 - 2.18 (m, 2H), 2.03 (s, 3H), 1.65 - 1.57 (m, 1H). LC-MS: [M+1]⁺ = 539.2. |
| 118 | 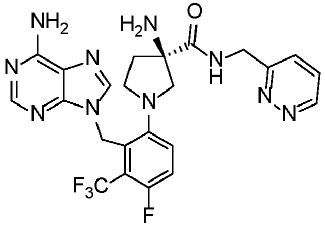 | ¹H NMR (400 MHz, Methanol-*d*4) δ 9.03 (dd, *J* = 4.8, 1.8 Hz, 1H), 8.19 (s, 1H), 7.79 (dd, *J* = 9.1, 4.7 Hz, 1H), 7.70 (s, 1H), 7.66 - 7.54 (m, 2H), 7.46 - 7.38 (m, 1H), 5.80 - 5.59 (m, 2H), 4.72 - 4.54 (m, 2H), 3.56 (d, *J* = 9.4 Hz, 1H), 3.04 - 2.90 (m, 3H), 2.48 - 2.39 (m, 1H), 1.79 - 1.68 (m, 1H). LC-MS: [M+1]⁺ = 531.2. |
| 119 | 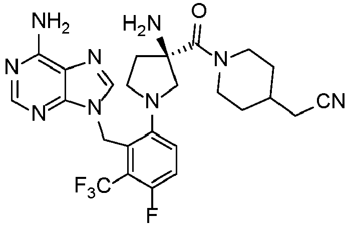 | ¹H NMR (400 MHz, Methanol-*d*4) δ 8.24 (s, 1H), 7.79 (dd, *J* = 9.2, 4.7 Hz, 1H), 7.64 (s, 1H), 7.44 (dd, *J* = 11.1, 9.1 Hz, 1H), 5.73 - 5.55 (m, 2H), 4.49 - 4.31 (m, 2H), 3.54 - 3.45 (m, 1H), 3.07 - 3.00 (m, 1H), 2.97 - 2.68 (m, 4H), 2.46 - 2.37 (m, 3H), 1.93 - 1.83 (m, 1H), 1.83 - 1.69 (m, 3H), 1.25 - 1.11 (m, 2H). LC-MS: [M+1]⁺ = 546.3. |
| 120 | 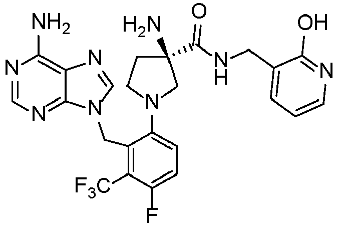 | ¹H NMR (400 MHz, Methanol-*d*4) δ 8.21 (s, 1H), 7.79 (dd, *J* = 9.1, 4.6 Hz, 1H), 7.69 (s, 1H), 7.45 - 7.38 (m, 2H), 7.31 (dd, *J* = 6.5, 2.0 Hz, 1H), 6.31 (t, *J* = 6.7 Hz, 1H), 5.79 - 5.58 (m, 2H), 4.28 - 4.13 (m, 2H), 3.53 (d, *J* = 9.4 Hz, 1H), 3.35 - 3.32 (m, 1H), 3.01 - 2.94 (m, 2H), 2.88 (d, *J* = 9.4 Hz, 1H), 2.43 - 2.32 (m, 1H), 1.73 - 1.60 (m, 1H). LC-MS: [M+1]⁺ = 546.2. |
| 121 | 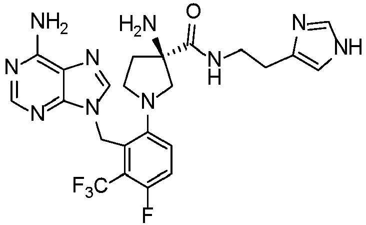 | ¹H NMR (400 MHz, Methanol-*d*4) δ 8.22 (s, 1H), 7.77 (dd, *J* = 9.2, 4.7 Hz, 1H), 7.68 (s, 1H), 7.58 - 7.55 (m, 1H), 7.42 (dd, *J =* 11.1, 9.1 Hz, 1H), 6.82 (s, 1H), 5.74 - 5.56 (m, 2H), 3.48 - 3.40 (m, 3H), 2.99 - 2.87 (m, 2H), 2.81 - 2.73 (m, 3H), 2.39 - 2.30 (m, 1H), 1.65 - 1.56 (m, 1H). LC-MS: [M+1]⁺ = 533.3. |
| 122 | 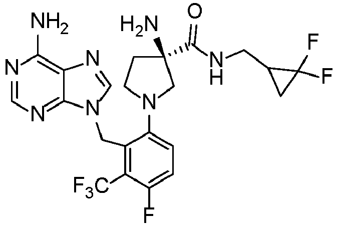 | ¹H NMR (400 MHz, Methanol-*d*4) δ 8.24 (s, 1H), 7.79 (dd, *J* = 9.1, 4.7 Hz, 1H), 7.68 (s, 1H), 7.43 (dd, *J* = 11.1, 9.1 Hz, 1H), 5.81 - 5.55 (m, 2H), 3.52 - 3.45 (m, 1H), 3.38 - 3.33 (m, 1H), 3.27 - 3.20 (m, 1H), 3.03 - 2.90 (m, 2H), 2.83 - 2.75 (m, 1H), 2.42 - 2.32 (m, 1H), 1.89 - 1.76 (m, 1H), 1.66 - 1.56 (m, 1H), 1.47 - 1.36 (m, 1H), 1.20 - 1.10 (m, 1H). LC-MS: [M+1]⁺ = 529.2. |
| 123 | 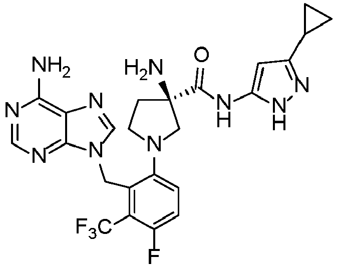 | ¹H NMR (400 MHz, Methanol-*d*4) δ 8.22 (s, 1H), 7.81 (dd, *J* = 9.2, 4.7 Hz, 1H), 7.69 (s, 1H), 7.43 (dd, *J* = 11.1, 9.1 Hz, 1H), 6.18 (s, 1H), 5.79 - 5.62 (m, 2H), 3.60 (d, *J* = 9.3 Hz, 1H), 3.08 - 2.98 (m, 2H), 2.89 (d, *J* = 9.3 Hz, 1H), 2.50 - 2.41 (m, 1H), 1.89 - 1.83 (m, 1H), 1.74 - 1.67 (m, 1H), 0.98 - 0.93 (m, 2H), 0.72 - 0.66 (m, 2H). LC-MS: [M+1]⁺ = 545.3. |
| 124 | 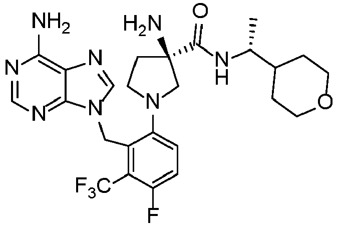 | ¹H NMR (400 MHz, Methanol-*d*4) δ 8.24 (s, 1H), 7.79 (dd, *J* = 9.2, 4.6 Hz, 1H), 7.68 (s, 1H), 7.43 (dd, *J* = 11.1, 9.1 Hz, 1H), 5.79 - 5.59 (m, 2H), 3.95 - 3.87 (m, 2H), 3.73 - 3.65 (m, 1H), 3.52 (d, *J* = 9.2 Hz, 1H), 3.39 - 3.33 (m, 2H), 3.01 - 2.96 (m, 2H), 2.79 (d, *J* = 9.3 Hz, 1H), 2.43 - 2.32 (m, 1H), 1.66 - 1.52 (m, 4H), 1.31 - 1.23 (m, 2H), 1.07 (d, *J* = 6.7 Hz, 3H). LC-MS: [M+1]⁺ = 551.3. |
| 125 | 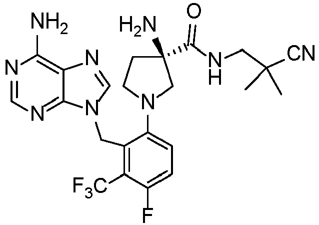 | ¹H NMR (400 MHz, Methanol-*d*4) δ 8.24 (s, 1H), 7.79 (dd, *J* = 9.1, 4.6 Hz, 1H), 7.69 (s, 1H), 7.42 (dd, *J* = 11.1, 9.1 Hz, 1H), 5.78 - 5.57 (m, 2H), 3.53 (d, *J* = 9.3 Hz, 1H), 3.35 (d, *J* = 7.1 Hz, 2H), 3.04 - 2.94 (m, 2H), 2.86 (d, *J* = 9.3 Hz, 1H), 2.46 - 2.37 (m, 1H), 1.73 - 1.62 (m, 1H), 1.30 (d, *J* = 4.5 Hz, 6H). LC-MS: [M+1]⁺ = 520.3. |
| 126 | 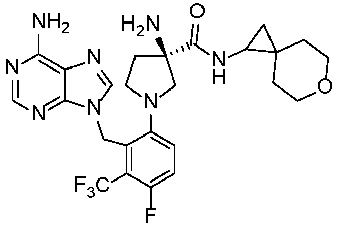 | ¹H NMR (400 MHz, Methanol-*d*4) δ 8.24 (d, *J* = 2.1 Hz, 1H), 7.80 (dt, *J* = 8.8, 4.3 Hz, 1H), 7.68 (d, *J* = 1.9 Hz, 1H), 7.43 (dd, *J* = 11.1, 9.1 Hz, 1H), 5.79 - 5.56 (m, 2H), 3.83 - 3.75 (m, 1H), 3.69 - 3.57 (m, 3H), 3.54 - 3.45 (m, 1H), 3.02 - 2.93 (m, 2H), 2.81 (t, *J* = 8.5 Hz, 1H), 2.54 - 2.50 (m, 1H), 2.44 - 2.29 (m, 1H), 1.69 - 1.58 (m, 1H), 1.45 - 1.39 (m, 3H), 0.82 - 0.75 (m, 1H), 0.55 - 0.45 (m, 1H). LC-MS: [M+1]⁺ = 549.3. |
| 127 | 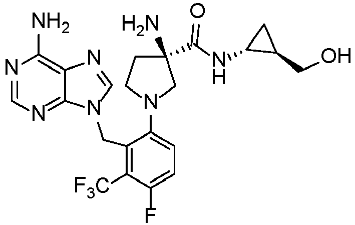 | ¹H NMR (400 MHz, Methanol-*d*4) δ 8.24 (s, 1H), 7.80 (dd, *J* = 9.1, 4.6 Hz, 1H), 7.70 (d, *J* = 1.9 Hz, 1H), 7.43 (dd, *J* = 11.1, 9.1 Hz, 1H), 5.78 - 5.57 (m, 2H), 3.52 - 3.40 (m, 3H), 2.98 - 2.91 (m, 2H), 2.91 - 2.84 (m, 1H), 2.56 - 2.48 (m, 1H), 2.40 - 2.30 (m, 1H), 1.71 - 1.62 (m, 1H), 1.19-1.10(m, 1H), 0.75-0.67 (m, 2H). LC-MS: [M+1]⁺ = 509.3. |
| 128 | 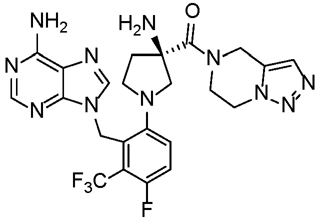 | ¹H NMR (400 MHz, Methanol-*d*4) δ 8.23 (s, 1H), 7.78 (dd, *J* = 9.2, 4.6 Hz, 1H), 7.64 (s, 1H), 7.58 (s, 1H), 7.43 (dd, *J* = 11.1, 9.1 Hz, 1H), 5.77 - 5.55 (m, 2H), 4.43 (t, *J* = 5.5 Hz, 2H), 4.15 (s, 2H), 3.59 (d, *J* = 9.5 Hz, 1H), 3.06 - 2.96 (m, 1H), 2.93 - 2.79 (m, 2H), 2.47 (dt, *J* = 13.8, 7.4 Hz, 1H), 1.93 (s, 2H), 1.82 (ddd, *J* = 13.0, 7.6, 5.5 Hz, 1H). LC-MS: [M+1]⁺ = 546.3. |
| 129 | 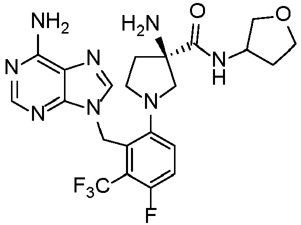 | ¹H NMR (400 MHz, Methanol-*d*4) δ 8.25 (d, *J* = 1.6 Hz, 1H), 7.80 (dd, *J* = 9.2, 4.7 Hz, 1H), 7.69 (s, 1H), 7.44 (dt, *J* = 11.1, 7.0 Hz, 1H), 5.79 - 5.57 (m, 2H), 4.41 - 4.26 (m, 1H), 3.94 - 3.71 (m, 3H), 3.62 - 3.47 (m, 2H), 2.98 - 2.84 (m, 3H), 2.47 - 2.34 (m, 1H), 2.25 - 2.16 (m, 1H), 1.86-1.78(m, 1H), 1.71 -1.61 (m, 1H). LC-MS: [M+1]⁺ = 509.3. |
| 130 | 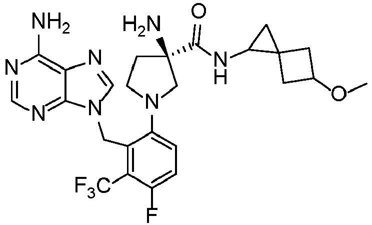 | ¹H NMR (400 MHz, Methanol-*d*4) δ 8.25 - 8.21 (m, 1H), 7.80 (dd, *J* = 9.2, 4.6 Hz, 1H), 7.69 (d, *J* = 1.9 Hz, 1H), 7.43 (t, *J* = 10.1 Hz, 1H), 5.77 - 5.57 (m, 2H), 4.13 - 4.00 (m, 1H), 3.56 - 3.49 (m, 1H), 3.21 (d, *J* = 1.1 Hz, 3H), 3.01 - 2.93 (m, 2H), 2.86 (t, *J* = 8.9 Hz, 1H), 2.55 - 2.45 (m, 1H), 2.45 - 2.33 (m, 1H), 2.31 - 2.18 (m, 1H), 2.18 - 1.91 (m, 3H), 1.72 - 1.59 (m, 1H), 0.95 - 0.77 (m, 1H), 0.62 - 0.41 (m, 1H). LC-MS: [M+1]⁺ = 549.3. |
| 131 | 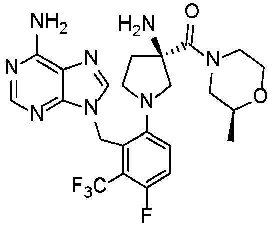 | ¹H NMR (400 MHz, Methanol-*d*4) δ 8.23 (s, 1H), 7.78 (dd, *J* = 9.1, 4.6 Hz, 1H), 7.64 (s, 1H), 7.43 (dd, *J* = 11.1, 9.1 Hz, 1H), 5.75 - 5.55 (m, 2H), 4.35 - 4.17 (m, 2H), 3.79 (d, *J* = 11.6 Hz, 1H), 3.55 - 3.40 (m, 3H), 3.05 - 2.96 (m, 1H), 2.96 - 2.76 (m, 3H), 2.66 - 2.29 (m, 2H), 1.80 - 1.70 (m, 1H), 1.09 (d, *J* = 6.2 Hz, 3H). LC-MS: [M+1]⁺ = 523.3 |
| 132 | 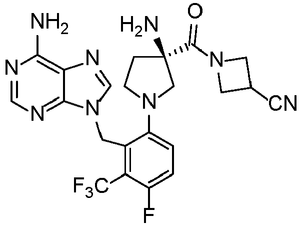 | ¹H NMR (400 MHz, Methanol-*d*4) δ 8.24 (s, 1H), 7.78 (dd, *J* = 9.1, 4.7 Hz, 1H), 7.65 (s, 1H), 7.42 (dd, *J* = 11.1, 9.1 Hz, 1H), 5.75 - 5.55 (m, 2H), 4.74 - 4.48 (m, 2H), 4.31 - 3.98 (m, 2H), 3.68 - 3.60 (m, 1H), 3.55 - 3.34 (m, 2H), 3.00 - 2.88 (m, 2H), 2.46 - 2.25 (m, 1H), 1.73 - 1.62 (m, 1H). LC-MS: [M+1]⁺ = 504.2. |
| 133 | 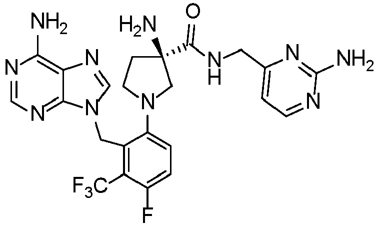 | ¹H NMR (400 MHz, Methanol-*d*4) δ 8.19 (s, 1H), 8.08 (d, *J* = 5.2 Hz, 1H), 7.80 (dd, *J* = 9.1, 4.7 Hz, 1H), 7.72 (s, 1H), 7.43 (dd, *J* = 11.0, 9.1 Hz, 1H), 6.45 (d, *J* = 5.2 Hz, 1H), 5.81 - 5.59 (m, 2H), 4.37 - 4.11 (m, 2H), 3.60 (d, *J* = 9.5 Hz, 1H), 3.08 - 2.95 (m, 3H), 2.54 - 2.44 (m, 1H), 1.84 - 1.74 (m, 1H). LC-MS: [M+1]⁺ = 546.3. |
| 134 | 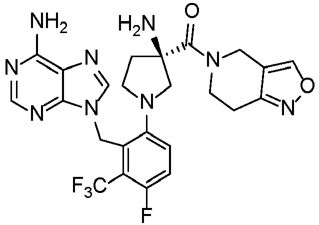 | ¹H NMR (400 MHz, Methanol-*d*4) δ 8.43 (s, 1H), 8.23 (s, 1H), 7.78 (dd, *J* = 9.1, 4.7 Hz, 1H), 7.64 (d, *J* = 3.5 Hz, 1H), 7.47 - 7.41 (m, 1H), 5.74 - 5.59 (m, 2H), 4.77 - 4.51 (m, 2H), 3.97 - 3.76 (m, 2H), 3.56 (d, *J* = 9.5 Hz, 1H), 3.05 - 2.96 (m, 1H), 2.93 - 2.80 (m, 4H), 2.48 - 2.36 (m, 1H), 1.86 - 1.74 (m, 1H). LC-MS: [M+1]⁺ = 546.2. |
| 135 | 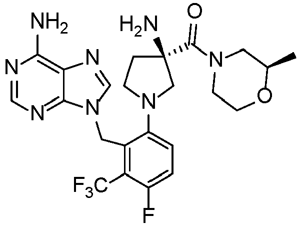 | ¹H NMR (400 MHz, Methanol-*d*4) δ 8.23 (s, 1H), 7.77 (dd, *J* = 9.2, 4.6 Hz, 1H), 7.64 (s, 1H), 7.43 (dd, *J* = 11.1, 9.1 Hz, 1H), 5.75 - 5.55 (m, 2H), 4.31 - 4.19 (m, 2H), 3.77 (d, *J* = 11.6 Hz, 1H), 3.58 - 3.39 (m, 3H), 3.04 - 2.79 (m, 4H), 2.60 - 2.32 (m, 2H), 1.84 - 1.71 (m, 1H), 1.11 (d, *J* = 6.2 Hz, 3H). LC-MS: [M+1]⁺ = 523.3. |
| 136 | 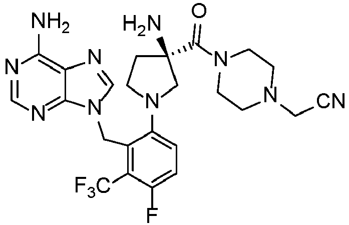 | ¹H NMR (400 MHz, Methanol-*d*4) δ 8.25 (s, 1H), 7.80 (dd, *J* = 9.1, 4.7 Hz, 1H), 7.65 (s, 1H), 7.45 (dd, *J* = 11.1, 9.1 Hz, 1H), 5.77 - 5.54 (m, 2H), 3.84 - 3.58 (m, 6H), 3.56 (d, *J* = 9.8 Hz, 1H), 3.06 - 2.90 (m, 3H), 2.58 - 2.39 (m, 5H), 1.87 - 1.76 (m, 1H). LC-MS: [M+1]⁺ = 547.3. |
| 137 | 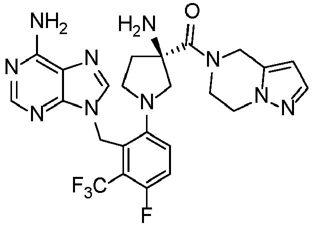 | ¹H NMR (400 MHz, Methanol-*d*4) δ 8.22 (s, 1H), 7.78 (dd, *J* = 9.1, 4.7 Hz, 1H), 7.64 (s, 1H), 7.47 - 7.38 (m, 2H), 6.10 (s, 1H), 5.76 - 5.60 (m, 2H), 4.27 - 4.02 (m, 4H), 3.59 (d, *J* = 9.5 Hz, 1H), 3.03 - 2.94 (m, 1H), 2.92 - 2.81 (m, 2H), 2.50 - 2.41 (m, 1H), 2.18 - 2.15 (m, 2H), 1.84 - 1.77 (m, 1H). LC-MS: [M+1]⁺ = 545.2. |
| 138 | 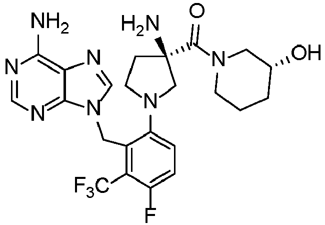 | ¹H NMR (400 MHz, Methanol-*d*4) δ 8.23 (s, 1H), 7.79 (dd, *J* = 9.2, 4.6 Hz, 1H), 7.65 (s, 1H), 7.43 (dd, *J* = 11.1, 9.1 Hz, 1H), 5.75 - 5.55 (m, 2H), 3.99 - 3.78 (m, 1H), 3.73 - 3.51 (m, 2H), 3.29 - 3.20 (m, 2H), 3.09 - 2.95 (m, 2H), 2.93 - 2.83 (m, 2H), 2.54 - 2.37 (m, 1H), 1.93 - 1.82 (m, 1H), 1.82 - 1.67 (m, 2H), 1.57 - 1.34 (m, 2H). LC-MS: [M+1]⁺ = 523.3. |
| 139 | 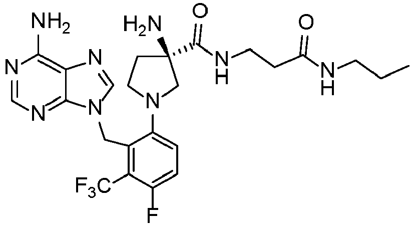 | ¹H NMR (400 MHz, Methanol-*d*4) δ 8.23 (s, 1H), 7.79 (dd, *J* = 9.1, 4.6 Hz, 1H), 7.69 (s, 1H), 7.43 (dd, *J* = 11.1, 9.0 Hz, 1H), 5.79 - 5.61 (m, 2H), 3.52 - 3.42 (m, 3H), 3.12 - 3.04 (m, 2H), 3.00 - 2.91 (m, 2H), 2.84 (d, *J* = 9.4 Hz, 1H), 2.40 - 2.31 (m, 3H), 1.67 - 1.57 (m, 1H), 1.53 - 1.41 (m, 2H), 0.89 (t, *J* = 7.4 Hz, 3H). LC-MS: [M+1]⁺ = 552.3. |
| 140 | 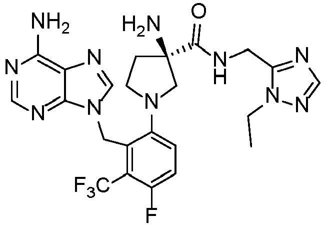 | ¹H NMR (400 MHz, Methanol-*d*4) δ 8.21 (s, 1H), 7.83 - 7.75 (m, 2H), 7.69 (s, 1H), 7.44 - 7.38 (m, 1H), 5.79 - 5.62 (m, 2H), 4.60 - 4.45 (m, 2H), 4.24 - 4.19 (m, 2H), 3.51 (d, *J* = 9.4 Hz, 1H), 3.03 - 2.97 (m, 2H), 2.86 (d, *J* = 9.3 Hz, 1H), 2.43 - 2.32 (m, 1H), 1.71 - 1.63 (m, 1H), 1.40 (t, *J* = 7.3 Hz, 3H). LC-MS: [M+1]⁺ = 548.2. |
| 141 | 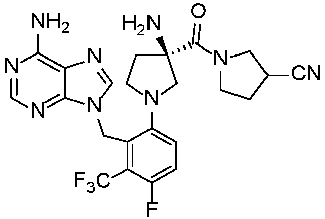 | ¹H NMR (400 MHz, Methanol-*d*4) δ 8.27 - 8.21 (m, 1H), 7.78 (dt, *J* = 8.8, 4.0 Hz, 1H), 7.65 (s, 1H), 7.47 - 7.39 (m, 1H), 5.78 - 5.52 (m, 2H), 4.08 - 3.39 (m, 6H), 3.07 - 2.96 (m, 1H), 2.94 - 2.79 (m, 2H), 2.49 - 2.36 (m, 1H), 2.30 - 2.09 (m, 2H), 1.80 - 1.70 (m, 1H). LC-MS: [M+1]⁺ = 518.2. |
| 142 | 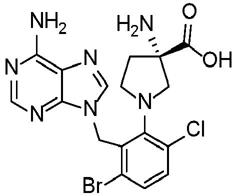 | ¹H NMR (400 MHz, Methanol-d4) δ 8.37 (s, 1H), 8.20 (s, 1H), 7.61 (d, J = 8.4 Hz, 1H), 7.48 (d, J = 8.8 Hz, 1H), 5.91-5.79 (m, 2H), 4.30-4.10 (m, 1H), 3.75-3.49 (m, 3H), 2.81-2.65 (m, 1H),2.30-2.23 (m, 1H). LC-MS: [M+H]+ = 466.0. |
| 143 | 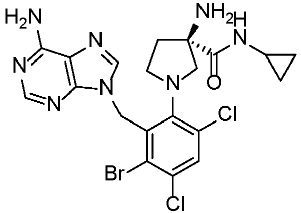 | ¹H NMR (400 MHz, Methanol-d4) δ ppm 8.24 (s, 1 H), 7.84 (s, 1 H), 7.82 (s, 1 H), 5.83 (br d, J = 13.69 Hz, 1 H), 5.64 (d, J = 13.57 Hz, 1 H), 3.81 (br s, 1 H), 2.46 - 3.11 (m, 5H), 1.69 (br s, 1 H), 0.70 (br d, J = 6.72 Hz, 2 H), 0.47 (br s, 2 H). LC-MS: [M+H]+ = 541.0. |
| 144 | 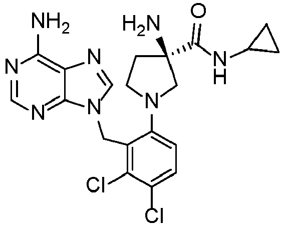 | ¹H NMR (400 MHz, Methanol-d4) δ 8.37-8.44 (m, 1H) 8.01 (br s, 1H) 7.40 (dd, J = 9.66, 1.96 Hz, 2H) 6.95 (br dd, J = 17.12, 11.00 Hz, 1H) 5.66-5.77 (m, 2H) 5.58 (d, J = 17.12 Hz, 1H) 5.25 (d, J = 11.49 Hz, 1H) 4.76-4.84 (m, 1H) 3.51-3.64 (m, 1H) 3.48 (dt, J = 3.27, 1.60 Hz, 1H) 3.34-3.39 (m, 1H) 3.10-3.26 (m, 5H) 3.00 (s, 1H) 2.77-2.96 (m, 2H) 2.19-2.33 (m, 1H) 1.79-2.06 (m, 3H) 1.37 (dd, J = 6.60, 3.79 Hz, 1H) 1.30 (br s, 1H) 0.03-0.01 (m, 1H). LC-MS: [M+H]⁺ = 461.2 |
| 145 | 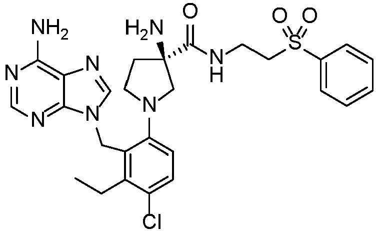 | ¹H NMR (400 MHz, Methanol-d4) δ 8.26 (s, 1H), 7.90 - 7.87 (m, 2H), 7.74 - 7.69 (m, 2H), 7.64 - 7.59 (m, 2H), 7.44 (d, J = 8.7 Hz, 1H), 7.26 (d, J = 8.7 Hz, 1H), 5.64 - 5.55 (m, 2H), 3.61 - 3.46 (m, 3H), 3.45 - 3.37 (m, 2H), 3.17 - 3.10 (m, 1H), 3.04 - 2.98 (m, 1H), 2.90 - 2.79 (m, 3H), 2.40 - 2.33 (m, 1H), 0.91 (t, J = 7.5 Hz, 3H). LC-MS: [M+H]⁺ = 583.2 |
| 146 | 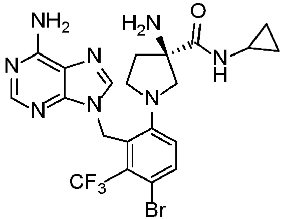 | ¹H NMR (400 MHz, Methanol-d4) δ 8.22 (s, 1H), 7.85 (d, J=8.8 Hz, 1H), 7.66 (s, 1H), 7.51 (d, J=8.8 Hz, 1H), 5.79-5.63 (m, 2H), 3.59 (d, J=9.6 Hz, 1H), 3.26-3.19 (m, 1H), 3.10-3.07 (m, 1H), 2.88 (d, J=9.2 Hz, 1H), 2.65-2.60 (m, 1H), 2.43-2.38 (m, 1H), 1.75-1.68 (m, 1H), 0.74 - 0.66 (m, 2H), 0.51 - 0.41 (m, 2H). LC-MS: [M+H]+ = 539.3 |
| 147 | 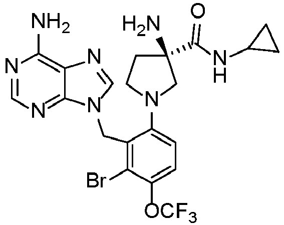 | ¹H NMR (400 MHz, Methanol-d4) δ 8.28 (s, 1H), 7.78 (s, 1H), 7.54-7.36 (m, 2H), 5.79-5.59 (m, 2H), 3.66 (d, J=11.2 Hz, 1H), 3.41-3.34 (m, 1H), 3.13-3.11 (m, 1H), 2.95-2.93 (m, 1H), 2.66-2.63 (m, 1H), 2.48-2.38 (m, 1H), 1.84-1.72 (m, 1H), 0.77-0.67 (m, 2H), 0.54-0.44 (m, 2H). LC-MS: [M+H]+ = 555.0 |
| 148 | 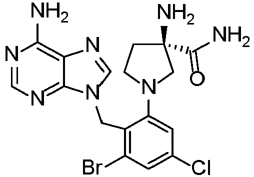 | ¹H NMR (400 MHz, Methanol-d4) δ 8.24 (s, 1H) 7.82 (s, 1H) 7.43 (d, J = 1.71 Hz, 1H) 7.28 (d, J = 1.83 Hz, 1H) 5.62 (d, J = 14.43 Hz, 1H) 5.52 (d, J = 14.43 Hz, 1H) 3.65 (d, J = 9.41 Hz, 1H) 3.34 - 3.41 (m, 1H) 3.36 (br s, 1 H) 3.13 (td, J = 8.71, 4.71 Hz, 1H) 3.00 (d, J = 9.41 Hz, 1H) 2.35 - 2.49 (m, 1H) 1.84 (ddd, J = 12.56, 7.31, 4.95 Hz, 1H). LC-MS: [M+H]+ = 464.8. |
| 149 | 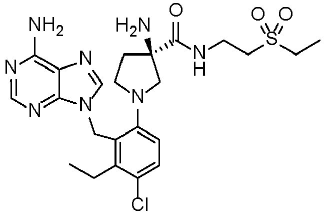 | ¹H NMR (400 MHz, Methanol-d4) δ 8.27 (s, 1H), 7.71 (s, 1H), 7.44 (d, J = 8.7 Hz, 1H), 7.27 (d, J = 8.7 Hz, 1H), 5.64 - 5.54 (m, 2H), 3.73 - 3.52 (m, 3H), 3.26 - 3.14 (m, 3H), 3.10 - 3.02 (m, 3H), 2.92 - 2.81 (m, 3H), 2.49 - 2.38 (m, 1H), 1.79 - 1.71 (m, 1H), 1.29 (t, J = 7.5 Hz, 3H), 0.91 (t, J = 7.5 Hz, 3H). LC-MS: [M+H]⁺ = 535.1 |
| 150 | 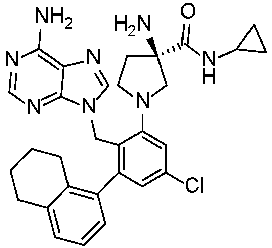 | ¹H NMR (400 MHz, Methanol-d4) δ 8.22-8.15 (m, 1H), 7.54-7.52 (m, 1H), 7.34-7.27 (m, 1H), 7.04-6.95 (m, 3H), 6.78-6.65(m, 1H), 5.72-5.50 (m, 2H), 3.83-3.77 (m, 1H), 3.57-3.49 (m, 3H), 2.76-2.67 (m, 4H), 2.37-2.25 (m, 2H), 1.72-1.29(m, 5H), 0.77-0.57 (m, 4H). LC-MS: [M+H]+ = 557.3. |
| 151 | 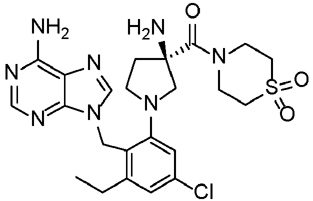 | ¹H NMR (400 MHz, Methanol-d4) δ 8.26 (s, 1H), 7.63 (s, 1H), 7.18 (d, J = 2.1 Hz, 1H), 7.08 (d, J = 2.1 Hz, 1H), 5.50 (d, J = 1.2 Hz, 2H), 4.28 - 3.91 (m, 4H), 3.69 (d, J = 9.5 Hz, 1H), 3.20 - 3.09 (m, 5H), 3.04 -2.98 (m, 1H), 2.87 (d, J = 9.5 Hz, 1H), 2.70 - 2.63 (m, 2H), 2.56 - 2.47 (m, 1H), 1.92 - 1.86 (m, 1H), 1.07 (t, J = 7.5 Hz, 3H). LC-MS: [M+H]⁺ =533.1 |
| 152 | 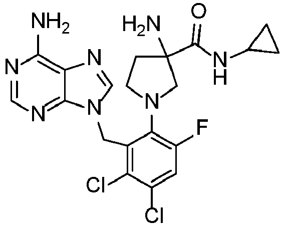 | ¹H NMR (400 MHz, Methanol-d4) 6 8.23 (s, 1H), 7.94 (s, 1H), 7.55 (d, J = 11.5 Hz, 1H), 5.83 - 5.58 (m, 2H), 3.68 (dd, J = 8.9, 1.3 Hz, 1H), 3.18 - 3.03 (m, 2H), 2.97 (d, J = 8.8 Hz, 1H), 2.67 - 2.59 (m, 1H), 2.55 - 2.43 (m, 1H), 1.73 - 1.64 (m, 1H), 0.74 - 0.65 (m, 2H), 0.52 - 0.42 (m, 2H). LC-MS: [M+H]⁺ =478.9 |
| 153 | 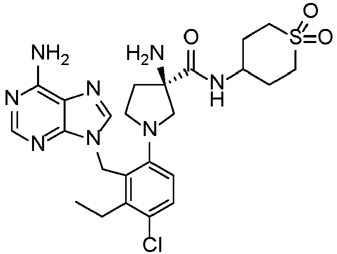 | ¹H NMR (400 MHz, Methanol-d4) δ 8.28 (s, 1H), 7.68 (s, 1H), 7.44 (d, J = 8.7 Hz, 1H), 7.27 (d, J = 8.7 Hz, 1H), 5.62 - 5.51 (m, 2H), 3.98 - 3.90 (m, 1H), 3.51 (d, J = 9.3 Hz, 1H), 3.26 - 3.20 (m, 2H), 3.10 - 2.99 (m, 3H), 2.93 - 2.78 (m, 3H), 2.49 - 2.39 (m, 1H), 2.22 - 2.02 (m, 4H), 1.78 - 1.68 (m, 1H), 0.94 (t, J = 7.5 Hz, 3H). LC-MS: [M+H]⁺ = 547.1 |
| 154 | 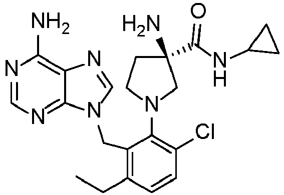 | ¹H NMR (400 MHz, Methanol-d4) δ 8.28 (s, 1H), 7.65 (s, 1H), 7.47 (d, J = 8.4 Hz, 1H), 7.26 (d, J = 8.4 Hz, 1H), 5.74-5.37 (m, 2H), 4.01-3.64 (s, 1H), 3.36-3.34 (m, 1H), 2.94-2.39 (m, 6H), 1.77-1.48 (m, 1H), 1.22-0.99 (m, 3H), 0.75-0.61 (m, 2H), 0.53-0.39 (m, 2H). LC-MS: [M+H]+ = 455.4 |
| 155 | 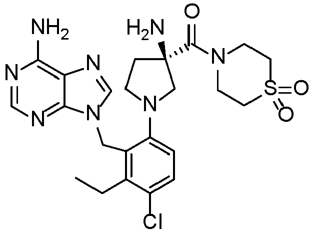 | ¹H NMR (400 MHz, Methanol-d4) δ 8.27 (s, 1H), 7.64 (s, 1H), 7.45 (d, J = 8.7 Hz, 1H), 7.25 (d, J = 8.7 Hz, 1H), 5.57 (s, 2H), 4.26 - 3.91 (m, 4H), 3.68 (d, J = 9.5 Hz, 1H), 3.17 - 3.08 (m, 5H), 3.03 - 2.94 (m, 1H), 2.89 - 2.83 (m, 3H), 2.58 - 2.49 (m, 1H), 1.92 - 1.85 (m, 1H), 0.95 (t, J = 7.6 Hz, 3H). LC-MS: [M+H]⁺ = 533.1 |
| 156 | 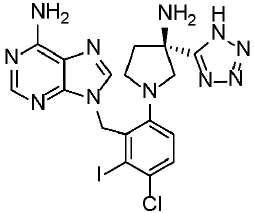 | ¹H NMR (400 MHz, Methanol-d4) δ 8.58 (s, 1H), 8.44 (s, 1H), 8.03 (s, 1H), 7.68-7.61 (m, 1H), 7.58-7.52 (m, 1H), 5.88 (s, 2H), ), 3.86-3.83 (m, 1H), 3.72-3.69 (m, 1H), 3.58-3.50 (m, 1H), 3.49-3.39 (m, 1H), 2.92-2.90 (m, 1H), 2.63-2.53 (m, 1H). LC-MS: [M+H]+ = 538.0 |
| 157 | 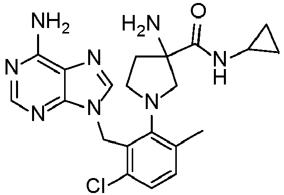 | ¹H NMR (400 MHz, Methanol-d4) δ 8.26 (s, 1H), 7.70 (s, 1H), 7.35 - 7.28 (m, 2H), 5.61 (dd, J = 77.4, 13.4 Hz, 2H), 3.68 (d, J = 8.6 Hz, 1H), 3.25 - 3.13 (m, 1H), 3.12 - 2.73 (m, 2H), 2.67 - 2.59 (m, 1H), 2.56 - 2.46 (m, 1H), 2.36 (s, 3H), 1.72 - 1.62 (m, 1H), 0.76 - 0.65 (m, 2H), 0.56 - 0.43 (m, 2H). LC-MS: [M+H]⁺ =441.0. |
| 158 | 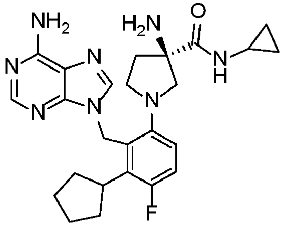 | ¹H NMR (400 MHz, Methanol-d4) δ 8.47 (s, 1H), 7.98 (s, 1H), 7.45-7.41 ( m, 1H), 7.22-7.16 (m, 1H), 5.86-5.74 (m, 2H), 3.63 (d, J = 10.4 Hz, 1H), 3.38-3.33 (m, 1H), 3.28-3.20 (m, 2H), 3.16-3.06 (m, 1H), 2.75-2.64 (m, 1H), 2.63-2.60 (m, 1H), 2.30-2.19 (m, 1H), 1.85-1.62 (m, 4H), 1.56-1.35 (m, 4H), 0.81-0.63 (m, 2H), 0.57-0.42 (m, 2H). LC-MS: [M+H]+ = 479.2 |
| 159 | 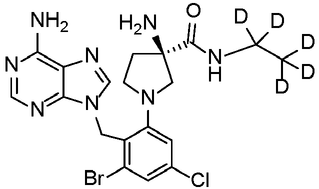 | ¹H NMR (400 MHz, Methanol-*d*₄) δ 8.25 (s, 1H), 7.79 (s, 1H), 7.41 (d, *J* = 2.1 Hz, 1H), 7.25 (d, *J* = 2.1 Hz, 1H), 5.61 (d, *J* = 14.4 Hz, 1H), 5.51 (d, *J* = 14.3 Hz, 1H), 3.63 (d, *J* = 9.3 Hz, 1H), 3.40 (q, *J* = 7.8 Hz, 1H), 3.13 (td, *J* = 8.8, 4.5 Hz, 1H), 2.92 (d, *J* = 9.3 Hz, 1H), 2.39 (dt, *J=* 12.9, 8.0 Hz, 1H), 1.76 (dt, *J* = 12.6, 6.6 Hz, 1H). LC-MS: [M+H]+ = 497.9. |
| 160 | 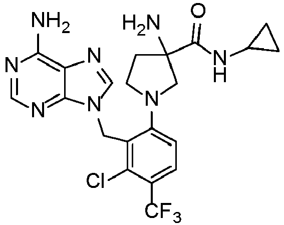 | ¹H NMR (400 MHz, Methanol-d4) δ 8.25 (s, 1H), 7.81 (s, 1H), 7.72 (d, J = 8.9 Hz, 1H), 7.17 (d, J = 8.9 Hz, 1H), 5.69 - 5.54 (m, 2H), 3.79 (d, J = 9.4 Hz, 1H), 3.67 - 3.59 (m, 1H), 3.29 - 3.24 (m, 1H), 3.02 (dd, J = 9.4, 1.2 Hz, 1H), 2.65 - 2.58 (m, 1H), 2.45 - 2.36 (m, 1H), 1.85 - 1.77 (m, 1H), 0.73 - 0.67 (m, 2H), 0.50 - 0.43 (m, 2H). LC-MS: [M+H]⁺ = 494.9. |
| 161 | 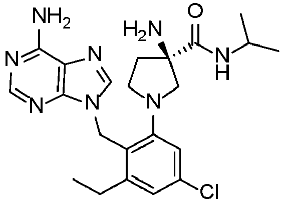 | ¹H NMR (400 MHz, Methanol-d4) δ 8.28 (s, 1H), 7.71 (s, 1H), 7.23-7.22 (d, J = 1.6 Hz, 1H), 7.11-7.09 (m, 1H), 5.57-5.48 (m, 2H), 3.97-3.93 (m, 1H), 3.59-3.57 (m, 1H), 3.29-3.27 (m, 1H), 3.06 (m, 1H), 2.87-2.85 (m, 1H), 2.69-2.65 (m, 1H), 2.44-2.43 (m, 1H), 1.75-1.74 (m, 1H), 1.15-1.12 (m, 6H), 1.10-1.06 (m, 3H). LC-MS: [M+H]+ = 457.3 |
| 162 | 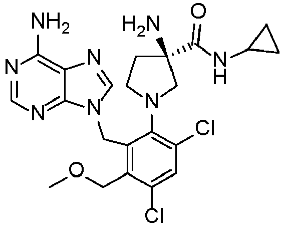 | ¹H NMR (400 MHz, Methanol-d4) δ 8.25 (s, 1H), 7.85 (s, 1H), 7.67 (s, 1H), 5.69 (d, J = 11.6 Hz, 1H), 5.47 (s, 1H), 4.78 (s, 2H), 3.75 (s, 1H), 3.33 (s, 3H), 3.30 - 3.25 (m, 1H), 2.65 - 2.55 (m, 4H), 1.56 (s, 1H), 0.77 - 0.65 (m, 2H), 0.53 - 0.43 (m, 2H). LC-MS: [M+H]+ = 505.3 |
| 163 | 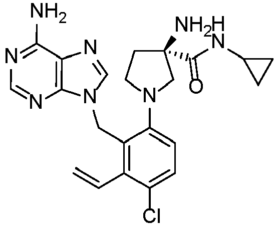 | ¹H NMR (400 MHz, Methanol-d4) δ 8.26-8.20 (m, 1H), 7.70 (s, 1H), 7.45 (d, J = 8.8 Hz, 1H), 7.27 (d, J = 8.8 Hz, 1H), 6.70-6.63 (m, 1H), 5.64-5.52 (m, 2H), 5.49-5.46 (m, 1H), 5.30-5.26 (m, 1H), 3.56 (d, J = 9.2 Hz, 1H), 3.27-3.21 (m, 1H), 3.03-3.01 (m, 1H), 2.84 (d, J = 9.2 Hz, 1H), 2.65-2.62 (m, 1H), 2.41-2.39 (m, 1H), 1.72-1.68 (m, 1H), 0.73-0.67 (m, 2H), 0.51-0.44 (m, 2H). LC-MS: [M+H]+ = 453.3 |
| 164 | 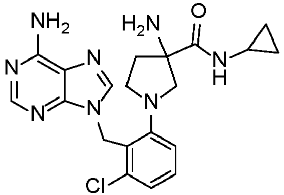 | ¹H NMR (400 MHz, Methanol-d4) δ 8.25 (s, 1H), 7.77 (s, 1H), 7.38 (t, J = 8.1 Hz, 1H), 7.28 - 7.18 (m, 2H), 5.68 - 5.53 (m, 2H), 3.59 (d, J = 9.3 Hz, 1H), 3.47 - 3.41 (m, 1H), 3.11 - 3.02 (m, 1H), 2.89 (d, J = 9.3 Hz, 1H), 2.65 - 2.58 (m, 1H), 2.45 - 2.36 (m, 1H), 1.78 - 1.70 (m, 1H), 0.74 - 0.65 (m, 2H), 0.50 - 0.43 (m, 2H). LC-MS: [M+H]⁺ = 427.0 |
| 165 | 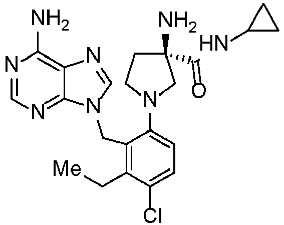 | ¹H NMR (400 MHz, Methanol-d4) δ 8.28 (s, 1H), 7.69 (s, 1H), 7.43 (d, J = 8.8 Hz, 1H), 7.28 (d, J = 8.8 Hz, 1H), 5.65-5.53 (m, 2H), 3.55 (d, J = 9.2 Hz, 1H), 3.25-3.17 (m, 1H), 3.05-3.03 (m, 1H), 2.85 (d, J = 9.2 Hz, 1H), 2.80-2.70 (m, 2H), 2.64-2.63 (m, 1H), 2.44 (s, 1H), 1.73 (s, 1H), 1.32-1.18 (m, 2H), 0.84-0.80 (m, 3H), 0.70-0.68 (m, 2H), 0.53-0.42 (m, 2H). LC-MS: [M+H]+ = 455.3 |
| 166 | 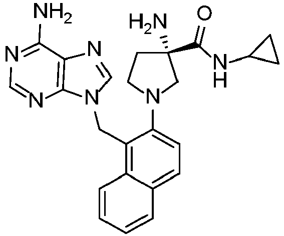 | ¹H NMR (400 MHz, Methanol-d4) δ 8.34 (s, 1H), 7.97 (d, J = 9.2 Hz, 1H), 7.93-7.85 (m, 2H), 7.63 (d, J = 9.2 Hz, 1H), 7.53 (s, 1H), 7.49-7.37 (m, 2H), 6.02-5.86 (m, 2H), 3.80-3.61 (m, 1H), 3.41-3.39 (m, 1H), 3.23-3.16 (m, 1H), 3.04-2.96 (m, 1H), 2.70-2.61 (m, 1H), 2.55-2.44 (m, 1H), 1.87-1.77 (m, 1H), 1.73-1.55 (m, 1H), 0.78-0.67 (m, 2H), 0.55-0.44 (m, 2H). LC-MS: [M+H]+ = 443.4 |
| 167 | 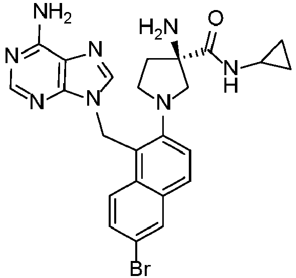 | ¹H NMR (400 MHz, Methanol-d4) δ 8.33 (s, 1H), 8.06 (d, J = 2.0 Hz, 1H), 7.93-7.83 (m, 2H), 7.65 (d, J = 9.2 Hz, 1H), 7.58 (s, 1H), 7.54 (dd, J = 2.0, 9.2 Hz, 1H), 5.96-5.84 (m, 2H), 3.75 (d, J = 9.2 Hz, 1H), 3.51-3.40 (m, 1H), 3.26-3.22 (m, 1H), 3.02 (d, J = 9.2 Hz, 1H), 2.68-2.63 (m, 1H), 2.53-2.45 (m, 1H), 1.86-1.79 (m, 1H), 0.76-0.69 (m, 2H), 0.53-0.46 (m, 1H), 0.53-0.46 (m, 1H). LC-MS: [M+H]+ = 521.1 |
| 168 | 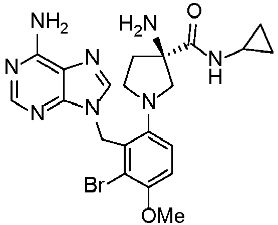 | ¹H NMR: (400MHz, MeOD) δ 8.27 (s, 1H), 7.71 (s, 1H), 7.45 (d, *J* = 8.8 Hz, 1H), 7.16 (d, *J* = 8.8 Hz, 1H), 5.76-5.72 (m, 1H), 5.65-5.60 (m, 1H), 3.90 (s, 3H), 3.46 (d, *J* = 9.2 Hz, 1H), 3.01-2.91 (m, 2H), 2.82 (d, *J* = 9.2 Hz, 1H), 2.64-2.60 (m, 1H), 2.42-2.35 (m, 1H), 1.70-1.66 (m, 1H), 0.72-0.66 (m, 2H), 0.49-0.43 (m, 2H). LC-MS [M+H]⁺= 501.4. |
| 169 | 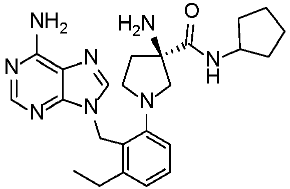 | ¹H NMR: (MeOD 400 MHz): δ = 8.24 (s, 1H), 7.56 (s, 1H), 7.21 (t, *J* = 8.0 Hz, 1H), 7.22 (d, *J* = 7.6 Hz, 1H), 7.07 (d, *J* = 7.6 Hz, 1H), 5.58-5.44 (m, 2H), 4.07-3.99 (m, 1H), 3.51-3.49 (m, 1H), 3.04-2.98 (m, 1H), 2.80 (d, *J* = 9.2 Hz, 1H), 2.68-2.60 (m, 2H), 2.45-2.35 (m, 1H), 1.92-1.81 (m, 2H), 1.73-1.47 (m, 6H), 1.41-1.29 (m, 2H), 1.03 (t, *J* = 7.6 Hz, 3H). LC-MS [M+H]⁺= 449.5. |
| 170 | 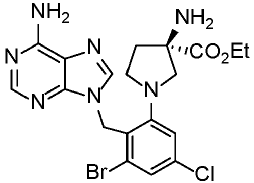 | ¹H NMR (400 MHz, DMSO-d⁶) δ ppm 1.10 (t, *J* = 7.03 Hz, 4 H) 1.72 - 1.87 (m, 1 H) 2.20 (dt, J = 12.10, 8.01 Hz, 4 H) 2.91 (br d, *J* = 9.54 Hz, 2 H) 3.04 - 3.26 (m, 5 H) 3.58 (br d, *J* = 9.41 Hz, 5 H) 3.98 - 4.15 (m, 6 H) 5.17 - 5.50 (m, 2 H) 7.15 (s, 1 H) 7.26 (br s, 2 H) 7.39 (s, 1 H) 7.72 (s, 1 H) 8.17 (s, 1 H). LC-MS [M+H]⁺= 494.0. |

### Biological Assays

The compounds of the present invention may be evaluated for their ability to inhibit MLL1 using assays described below, as well as other assays known in the art.

### MLL1 LC-MS assay

The compounds of the present disclosure were serially and separately diluted 3-fold in DMSO to obtain a total of eight or twelve concentrations. Then the test compounds at each concentration (120nL of each) were transferred by Mosquito into white Proxiplate plus 384-well microplate (PerkinElmer). Solutions (6 µL) of 60 nM wild type MLL1 four-member complex (MLL1-4C) and 5 µM SAM in the reaction buffer (20 mM Tris-HCl, pH8.0, 0.01% Tween 20, 1 mM DTT, 10 mM MgCl₂, 0.01% BSA) were added to the wells that were then incubated with the test compound for 20 min. A 6 µL solution of 20 µM of the peptide substrate H3K4me0 (histone H3[1-21]-Biotin) in the reaction buffer was added to initiate each reaction. The final components in the reaction solution include 30 nM MLL1-4C, 2.5pM SAM, and 10 µM H3K4me0 with varying concentration of the compounds. A positive control consisted of 30nM MLL1-4C, 2.5pM SAM, and 10 µM substrate in the absence of the test compound, and a negative control consisted of 2.5pM SAM, and 10 µM substrate only. Each reaction was incubated at rt for 120 min, then stopped by addition of 3 µL per of quench solution (2.5% TFA with 320 nM d4-SAH). The reaction mixture was centrifuged (Eppendorf centrifuge 5810, Rotor A-4-62) for 2 min at 2000 rmp. The SAH production from the enzymatic assays were monitored by LC-MS/MS on an API 4000 triple quadrupole mass spec with Turbolon Spray (Applied Biosystem) coupled with Prominenece UFLC(Shimazu). The level of SAH production were then normalized based on the values coming from the positive and negative controls to give percent enzyme activities. The data were then fit to a dose response equation using the program Helios (Novartis) to get the IC50 values of the test compounds.

### MLL1 Flashplate assay

To assess the compound potency in MLL1 Flashplate assay, compounds were serially diluted 3-fold in DMSO to obtain a total of twelve concentrations. Then the test compounds at each concentration (250nL of each) were transferred by Mosquito into Corning #3675 384-well plate. Solutions (15 µL) of 4.2 nM wild type MLL1 five-member complex (MLL1-5C) in the assay buffer (20 mM Tris-HCl, pH8.0, 0.01% Tween 20, 1 mM DTT, 0.01% BSA) were added to the wells. A 10 µL solution of 2.5 µM of the peptide substrate H3K4me0 (histone H3[1-21]-Biotin) and 1.25 µM ³H-SAM in the reaction buffer was then added to initiate each reaction. The final components in the reaction solution include 2.5 nM MLL1-5C, 0.5 µM ³H-SAM, and 1 µM H3K4me0 with varying concentration of the compounds. A positive control consisted of 2.5 nM MLL1-5C, 0.5µM ³H-SAM, and 1 µM substrate in the absence of the test compound, and a negative control consisted of 0.5µM ³H-SAM, and 1 µM substrate only. Each reaction was incubated at rt for 90 min, then stopped by addition of 5 µL per of quench solution (0.5 mM SAM in assay buffer).

25 µL of each reaction solution was transferred to FlashPlate streptavidin 384-well microplate (PerkinElmer). After at least 1h incubation at rt, the wells were washed three times with 0.1% Tween-20 in dH₂O using the BioTek plate washer. The plates were then read in MicroBeta (PerkinElmer). The radioactive readouts were then normalized based on the values coming from the positive and negative controls to give percent enzyme activities. The data were then fit to a dose response equation using the program Helios to get the IC50 values of the test compounds.

### MV4-11 H3K4me3 cellular ELISA assay

MV4-11 (ATCC^{®} CRL-9591^{™}) was cultured with RPMI1640 medium (Thermo Fisher Scientific, cat #11875) supplemented with 10% FBS (Thermo Fisher Scientific, cat #10099141) and 50 U/ml Penicillin-Streptomycin (Thermo Fisher Scientific, cat#10378016) in humidified incubator at 37°C, 5% CO2.

200 nL compounds dissolved in DMSO were serially diluted at 1:3 ratio for 12 points starting from 10 mM and dispensed into each wells of cell culture plates (PDL-coated ViewPlate-384 Black, FTC, PerkinElmer, # 6007710). 3,750 MV4-11 cells in 40µL culture medium were seeded into each well and treated with desired concentration for 48 hours. After treatment, wash cells with cold phosphate based buffer (PBS, pH 7.4) and aspirate the remaining PBS buffer. Crude histone lysate was extracted by 40µL 0.5M HCl for 1hour in 4°C following neutralization by adding 32µL neutralization buffer (0.5M Sodium phosphate dibasic, pH~12.5, 1.25 mM Dithiothreitol, Promega#V3151, Protease Inhibitor Cocktail,Sigma #8340) for each well. 5µL and 20µL of histone lysate was loaded to ELISA plate (384 Well Plates, PE HB, White, 6005620) for detection of total H3 and H3K4me3, respectively. Shake the plates gently in 4°C for overnight. After histone binding, plates were washed with 80µL PBS/T (PBS with 0.05% Tween-20) and blocked with 50µL 3% BSA diluted in PBS/T per well for 1 hour. Incubate wells with primary antibody for 1 hour at room temperature using anti-H3K4me3 (CST#9727) and anti-total H3 (CST#9715) diluted 1:2000 in 3% BSA. Plates were then washed with PBS/T for 3 times and incubated with horseradish peroxidase (HRP) conjugated secondary antibody (CST#7074, 1:5000) for 1hour at room temperature. The abundance of H3K4me3 and total H3 were measured by ECL substrates chemiluminesce (Pierce, #34080) on a plate reader (PerkinElmer EnVison 2104 Mutilable Reader). Percentage inhibition was calculated against DMSO control after normalization of H3K4me3 signal to H3 signal for individual samples. The data were then fit to a dose response curve using GraphPad Prism for IC50 calculation.

### MV4-11 6-day cell growth CTG (CellTiter-Glo) assay

Acute myeloid leukemia cell MV4-11 (ATCC^{®} CRL-9591^{™}) was cultured with RPMI 1640 medium (Thermo Fisher Scientific, cat #11875) supplemented with 10% FBS (Thermo Fisher Scientific, cat #10099141) in humidified incubator at 37°C, 5% CO2. To assess the effect of MLL1 inhibition on cell growth, the compound of the present invention was dissolved in DMSO and serially diluted at 1:3 for 12 points starting from 10 mM then 200 nL for the replicate of each dose per well was dispensed to Viewplate-384 Black (Perkin Elmer). Exponentially growing MV4-11 cells were seeded at the density of 300 cells per well in 40 µL to the plate, so the final compound working concentration starts from 50 µM. After 6 days, 40 µL CellTiter-Glo (Promega, cat #G7573) was added into the cell culture well and luminescence was read with Envision (Perkin Elmer) to determine the viable cells. The percentage inhibition was calculated against the samples treated with DMSO only and the data were used for dose response curve fitting in GraphPad Prism to get the IC50 of representative compound of present invention, which reflects the inhibition of MLL1 activity.

The activity of the compounds of the present invention in different assay formats and cell lines are summarized in Table 2.

**Table 2**

| Ex No. | IUPAC name | LCMS | MLL1 flash plate | ELISA | CTG |
|---|---|---|---|---|---|
| 1 | (R)-3-amino-1-(2-((6-amino-9H-purin-9-yl)methyl)-3-bromo-5-chlorophenyl)-N-cyclopropylpyrrolidine-3-carboxamide | +++ | +++ | +++ | ++ |
| 2 | (R)-3-amino-1-(2-((6-amino-9H-purin-9-yl)methyl)-5-chloro-3-ethylphenyl)-N-cyclopropylpyrrolidine-3-carboxamide | +++ | +++ | +++ | +++ |
| 3 | (R)-3-amino-1-(2-((6-amino-9H-purin-9-yl)methyl)-5-chloro-3-(2,3-dihydrobenzofuran-5-yl)phenyl)-N-cyclopropylpyrrolidine-3-carboxamide | + | + | + | + |
| 4 | (*R*)-3-amino-1-(2-((6-amino-9*H*-purin-9-yl)methyl)-3-cyano-4-fluorophenyl)-*N*-cyclopropylpyrrolidine-3-carboxamide | + | + | ++ | + |
| 5 | (*R*)-3-amino-1-(2-((6-amino-9*H*-purin-9-yl)methyl)-3-ethynyl-4-fluorophenyl)-*N*-cyclopropylpyrrolidine-3-carboxamide | ++ | N/A | +++ | +++ |
| 6 | (*R*)-3-amino-1-(2-((6-amino-9*H*-purin-9-yl)methyl)-4-fluoro-3-(2,2,2-trifluoroethyl)phenyl) -*N-*cyclopropylpyrrolidine-3-carboxamide | +++ | N/A | +++ | +++ |
| 7 | (*R*)-3-amino-1-(2-((6-amino-9*H*-purin-9-yl)methyl)-5-chloro-3-(2,2,2-trifluoroethyl)phenyl)-*N-*cyclopropylpyrrolidine-3-carboxamide | ++ | N/A | +++ | + |
| 8 | (*R*)-3-amino-1-(2-((6-amino-9*H*-purin-9-yl)methyl)-3-cyclopropyl-4-fluorophenyl)-*N*-cyclopropylpyrrolidine-3-carboxamide | ++ | N/A | +++ | +++ |
| 9 | 3-amino-1-(2-((6-amino-9*H*-purin-9-yl)methyl)-4-fluoro-3-vinylphenyl)-*N*-cyclopropylpyrrolidine-3-carboxamide | + | N/A | +++ | +++ |
| 10 | (*R*)-3-amino-1-(2-((6-amino-9*H*-purin-9-yl)methyl)-3-ethyl-4-fluorophenyl)-*N*-cyclopropylpyrrolidine-3-carboxamide | +++ | N/A | +++ | +++ |
| 11 | (*R*)-3-amino-1-(2-((6-amino-9*H*-purin-9-yl)methyl)-3-methyl-4-(trifluoromethyl)phenyl)-*N-*cyclopropylpyrrolidine-3-carboxamide | +++ | N/A | +++ | +++ |
| 12 | (*R*)-3-amino-1-(2-((6-amino-9*H*-purin-9-yl)methyl)-3-ethyl-4-(trifluoromethyl)phenyl)-*N-*cyclopropylpyrrolidine-3-carboxamide | ++ | N/A | +++ | +++ |
| 13 | (*R*)-3-amino-1-(2-((6-amino-9*H*-purin-9-yl)methyl)-3-bromo-4-fluorophenyl)-*N*-cyclopropylpyrrolidine-3-carboxamide | +++ | N/A | +++ | +++ |
| 14 | (*R*)-3-amino-1-(2-((6-amino-9*H*-purin-9-yl)methyl)-4-fluorophenyl)-*N*-cyclopropylpyrrolidine-3-carboxamide | + | + | +++ | ++ |
| 15 | (R)-3-amino-1-(4-((6-amino-9*H*-purin-9-yl)methyl)-2,2-difluorobenzo[d][1,3]dioxol-5-yl)-*N-*cyclopropylpyrrolidine-3-carboxamide | +++ | +++ | +++ | +++ |
| 16 | (*R*)-3-amino-1-(2-((6-amino-9*H*-purin-9-yl)methyl)-5-chloro-3'-cyclohexyl-[1,1'-biphenyl]-3-yl)pyrrolidine-3-carboxamide | + | + | +++ | +++ |
| 17 | (*R*)-1-(4-(3-amino-1-(2-((6-amino-9*H*-purin-9-yl)methyl)-3-ethyl-4-fluorophenyl)pyrrolidine-3-carbonyl)piperazin-1-yl)prop-2-en-1-one | +++ | +++ | +++ | +++ |
| 18 | (R)-9-(2-(3-amino-3-(1H-1 ,2,4-triazol-3-yl)pyrrolidin-1-yl)-6-bromo-4-chlorobenzyl)-9H-purin-6-amine | +++ | +++ | +++ | +++ |
| 19 | (R)-(3-amino-1-(2-((6-amino-9H-purin-9-yl)methyl)-3-bromo-5-chlorophenyl)pyrrolidin-3-yl)methanol | ++ | +++ | +++ | ++ |
| 20 | (*R*)-3-amino-1-(6-((6-amino-9*H*-purin-9-yl)methyl)-5-bromo-4-fluoro-2,3-dihydrobenzofuran-7-yl)-*N-*cyclopropylpyrrolidine-3-carboxamide | +++ | +++ | +++ | +++ |
| 21 | (*R*)-3-amino-1-(2-((6-amino-9*H*-purin-9-yl)methyl)-3-bromo-6-methoxyphenyl)-*N*-cyclopropylpyrrolidine-3-carboxamide | ++ | N/A | + | N/A |
| 22 | 9-(6-(3-amino-3-(pyridin-2-yl)pyrrolidin-1-yl)-3-fluoro-2-(trifluoromethyl)benzyl)-9*H*-purin-6-amine | ++ | ++ | +++ | +++ |
| 23 | (R)-9-(6-(3-amino-3-(pyridin-2-yl)pyrrolidin-1-yl)-3-fluoro-2-(trifluoromethyl)benzyl)-9H-purin-6-amine | +++ | +++ | +++ | +++ |
| 24 | (*R*)-9-(2-(3-amino-3-(1H-1,2,4-triazol-3-yl)pyrrolidin-1-yl)-4-chloro-6-vinylbenzyl)-9*H*-purin-6-amine | +++ | N/A | +++ | +++ |
| 25 | (*R*)-9-(2-(3-amino-3-(1*H*-1 ,2,4-triazol-3-yl)pyrrolidin-1-yl)-4-chloro-6-ethylbenzyl)-9*H*-purin-6-amine | +++ | N/A | +++ | +++ |
| 26 | (S)-9-(6-(3-amino-3-phenylpyrrolidin-1-yl)-3-fluoro-2-(trifluoromethyl)benzyl)-9H-purin-6-amine | ++ | N/A | ++ | ++ |
| 27 | (R)-9-(2-(3-amino-3-(4-(trifluoromethyl)pyridin-2-yl)pyrrolidin-1-yl)-6-bromo-4-chlorobenzyl)-9H-purin-6-amine | +++ | N/A | + | N/A |
| 28 | (R)-9-(6-(3-amino-3-(2-(pyridin-2-yl)ethyl)pyrrolidin-1-yl)-3-fluoro-2-(trifluoromethyl)benzyl)-9H-purin-6-amine | + | + | + | N/A |
| 29 | methyl (R)-3-amino-1-(2-((6-amino-9H-purin-9-yl)methyl)-5-chloro-3-cyclobutylphenyl)pyrrolidine-3-carboxylate | +++ | N/A | +++ | +++ |
| 30 | (*R*)-2-((3-amino-1-(2-((6-amino-9*H*-purin-9-yl)methyl)-3-bromo-5-chlorophenyl)pyrrolidin-3-yl)methoxy)-*N*-cyclopropylacetamide | + | N/A | + | N/A |
| 31 | 3-amino-1-(5-((6-amino-9*H*-purin-9-yl)methyl)-6-bromobenzo[*d*][1,3]dioxol-4-yl)-*N-*cyclopropylpyrrolidine-3-carboxamide | +++ | +++ | +++ | +++ |
| 32 | (*R*)-9-(2-(3-amino-3-(methoxymethyl)pyrrolidin-1-yl)-6-bromo-4-chlorobenzyl)-9H-purin-6-amine | + | ++ | + | + |
| 33 | (R)-3-amino-1-(2-((6-amino-9H-purin-9-yl)methyl)-5-chloro-3-((R)-2,2,2-trifluoro-1-hydroxyethyl)phenyl)-N-cyclopropylpyrrolidine-3-carboxamide | ++ | N/A | ++ | + |
| 34 | ethyl (*R*)-3-(3-amino-1-(2-((6-amino-9*H*-purin-9-yl)methyl)-3-bromo-5-chlorophenyl)pyrrolidin-3-yl) propanoate | + | N/A | +++ | N/A |
| 35 | (*R*)-5-(2-(3-amino-1-(2-((6-amino-9*H*-purin-9-yl)methyl)-5-chloro-3-ethylphenyl)pyrrolidin-3-yl)ethyl)-*N*-methyl-1,3,4-thiadiazol-2-amine | ++ | N/A | + | N/A |
| 36 | (R)-3-(3-amino-1-(2-((6-amino-9*H*-purin-9-yl)methyl)-3-bromo-5-chlorophenyl)pyrrolidin-3-yl)-*N-*ethylpropanamide | ++ | N/A | + | N/A |
| 37 | 3-(3-amino-1-(2-((6-amino-9*H*-purin-9-yl)methyl)-3-bromo-5-chlorophenyl)pyrrolidin-3-yl)propanamide | + | N/A | + | N/A |
| 38 | (R)-3-(3-amino-1-(2-((6-amino-9*H*-purin-9-yl)methyl)-5-chloro-3-ethylphenyl)pyrrolidin-3-yl)-*N-*ethylpropanamide | +++ | N/A | + | N/A |
| 39 | (*R*)-9-(2-(3-(2-(2*H*-tetrazol-5-yl)ethyl)-3-aminopyrrolidin-1-yl)-6-bromo-4-chlorobenzyl)-9*H-*purin-6-amine | +++ | N/A | + | N/A |
| 40 | (*R*)-9-(2-(3-(2-(2*H*-tetrazol-5-yl)ethyl)-3-aminopyrrolidin-1-yl)-4-chloro-6-ethylbenzyl)-9*H-*purin-6-amine | + | N/A | + | N/A |
| 41 | (*R*)-3-amino-1-(2-((6-amino-9*H*-purin-9-yl)methyl)-3-bromo-4-(trifluoromethyl)phenyl)-*N-*cyclopropylpyrrolidine-3-carboxamide | +++ | N/A | +++ | +++ |
| 42 | 3-amino-1-(5-((6-amino-9*H*-purin-9-yl)methyl)-6-bromo-7-chloro-2,3-dihydro-1*H*-inden-4-yl)-*N-*cyclopropylpyrrolidine-3-carboxamide | ++ | N/A | +++ | ++ |
| 43 | (*R*)-3-amino-1-(2-((6-amino-9*H*-purin-9-yl)methyl)-3-bromo-5,6-dichlorophenyl)-*N*-cyclopropylpyrrolidine-3-carboxamide | ++ | +++ | + | + |
| 44 | 3-amino-1-(2-((6-amino-9*H*-purin-9-yl)methyl)-3-bromo-4,5,6-trichlorophenyl)-*N-*cyclopropylpyrrolidine-3-carboxamide | ++ | +++ | +++ | + |
| 45 | (*R*)-3-amino-1-(2-((6-amino-9*H*-purin-9-yl)methyl)-3-((dimethylamino)methyl)-4-fluorophenyl)-*N-*cyclopropylpyrrolidine-3-carboxamide | ++ | + | +++ | +++ |
| 46 | (*R*)-3-amino-1-(2-((6-amino-9*H*-purin-9-yl)methyl)-4-fluoro-3-(piperidin-1-ylmethyl)phenyl)-*N-*cyclopropylpyrrolidine-3-carboxamide | ++ | N/A | +++ | +++ |
| 47 | (*R*)-3-amino-1-(2-((6-amino-9*H*-purin-9-yl)methyl)-3-((dimethylamino)methyl)-4-(trifluoromethyl)phenyl)-*N*-cyclopropylpyrrolidine-3-carboxamide | +++ | N/A | +++ | +++ |
| 48 | (*R*)-3-amino-1-(2-((6-amino-9*H*-purin-9-yl)methyl)-3-((cyclopropyl(methyl)amino)methyl)-4-fluorophenyl)-*N*-cyclopropylpyrrolidine-3-carboxamide | + | N/A | +++ | ++ |
| 49 | (*R*)-3-amino-1-(2-((6-amino-9*H*-purin-9-yl)methyl)-3-((dimethylamino)methyl)-4-fluorophenyl)-*N*-((1S,2R)-2-phenylcyclopropyl)pyrrolidine-3-carboxamide | +++ | N/A | +++ | +++ |
| 50 | (*R*)-3-amino-1-(2-((6-amino-9*H*-purin-9-yl)methyl)-4-fluoro-3-(((*R*)-3-fluoropyrrolidin-1-yl)methyl)phenyl)-*N*-cyclopropylpyrrolidine-3-carboxamide | +++ | N/A | +++ | +++ |
| 51 | (*R*)-3-amino-1-(2-((6-amino-9*H*-purin-9-yl)methyl)-4-fluoro-3-(morpholinomethyl)phenyl)-*N*-((*1S*,*2R*)-2-phenylcyclopropyl)pyrrolidine-3-carboxamide | +++ | +++ | +++ | +++ |
| 52 | (*R*)-3-amino-1-(2-((6-amino-9*H*-purin-9-yl)methyl)-3-((3,3-difluoroazetidin-1-yl)methyl)-4-fluorophenyl)-*N-*cyclopropylpyrrolidine-3-carboxamide | ++ | ++ | +++ | +++ |
| 53 | (*R*)-3-amino-1-(2-((6-amino-9*H*-purin-9-yl)methyl)-4-fluoro-3-((3-fluoroazetidin-1-yl)methyl)phenyl)-*N-*cyclopropylpyrrolidine-3-carboxamide | ++ | N/A | +++ | +++ |
| 54 | (*R*)-3-amino-1-(2-((6-amino-9*H*-purin-9-yl)methyl)-3-((cyclopropylamino)methyl)-4-fluorophenyl)-*N-*cyclopropylpyrrolidine-3-carboxamide | + | N/A | +++ | +++ |
| 55 | (*R*)-3-amino-1-(2-((6-amino-9*H*-purin-9-yl)methyl)-3-((4,4-difluoropiperidin-1-yl)methyl)-4-fluorophenyl)-*N*-cyclopropylpyrrolidine-3-carboxamide | ++ | N/A | +++ | +++ |
| 56 | (*R*)-3-amino-1-(2-((6-amino-9*H*-purin-9-yl)methyl)-4-fluoro-3-((4-fluoropiperidin-1-yl)methyl)phenyl)-*N-*cyclopropylpyrrolidine-3-carboxamide | ++ | N/A | +++ | +++ |
| 57 | (*R*)-3-amino-1-(2-((6-amino-9*H*-purin-9-yl)methyl)-4-fluoro-3-(((2,2,2-trifluoroethyl)amino)methyl)phenyl)-*N*-cyclopropylpyrrolidine-3-carboxamide | ++ | N/A | +++ | +++ |
| 58 | (*3R*)-1-(3-((8-oxa-3-azabicyclo[3.2.1]octan-3-yl)methyl)-2-((6-amino-9*H*-purin-9-yl)methyl)-4-fluorophenyl)-3-amino-*N*-cyclopropylpyrrolidine-3-carboxamide | ++ | N/A | +++ | +++ |
| 59 | (*R*)-3-amino-1-(2-((6-amino-9*H*-purin-9-yl)methyl)-3-((cyclopropyl(ethyl)amino)methyl)-4-fluorophenyl)-*N-*cyclopropylpyrrolidine-3-carboxamide | +++ | N/A | +++ | +++ |
| 60 | (*R*)-3-amino-1-(2-((6-amino-9*H*-purin-9-yl)methyl)-4-chloro-3-((dimethylamino)methyl)phenyl)-*N-*cyclopropylpyrrolidine-3-carboxamide | ++ | N/A | +++ | +++ |
| 61 | (*R*)-3-amino-1-(2-((6-amino-9*H*-purin-9-yl)methyl)-4-fluoro-3-(pyrrolidin-1-ylmethyl)phenyl)-*N-*cyclopropylpyrrolidine-3-carboxamide | ++ | N/A | +++ | +++ |
| 62 | (*R*)-3-amino-1-(2-((6-amino-9*H*-purin-9-yl)methyl)-3-(azetidin-1-ylmethyl)-4-(trifluoromethyl)phenyl)-*N-*cyclopropylpyrrolidine-3-carboxamide | +++ | +++ | +++ | +++ |
| 63 | (*R*)-3-amino-1-(2-((6-amino-9*H*-purin-9-yl)methyl)-3-((3,3-dimethylpyrrolidin-1-yl)methyl)-4-fluorophenyl)-*N*-cyclopropylpyrrolidine-3-carboxamide | + | N/A | +++ | +++ |
| 64 | (R)-3-amino-1-(2-((6-amino-9H-purin-9-yl)methyl)-4-fluoro-3-((3-methylazetidin-1-yl)methyl)phenyl)-N-cyclopropylpyrrolidine-3-carboxamide | ++ | N/A | +++ | +++ |
| 65 | (*R*)-3-amino-1-(2-((6-amino-9*H*-purin-9-yl)methyl)-4-fluoro-3-(morpholinomethyl)phenyl)-N-cyclopropylpyrrolidine-3-carboxamide | +++ | + | +++ | +++ |
| 66 | (*R*)-3-amino-1-(2-((6-amino-9*H*-purin-9-yl)methyl)-3-((3,3-difluoropyrrolidin-1-yl)methyl)-4-fluorophenyl)-*N*-cyclopropylpyrrolidine-3-carboxamide | +++ | N/A | +++ | +++ |
| 67 | (*R*)-3-amino-1-(2-((6-amino-9*H*-purin-9-yl)methyl)-3-((diethylamino)methyl)-4-fluorophenyl)-*N-*cyclopropylpyrrolidine-3-carboxamide | ++ | N/A | +++ | +++ |
| 68 | (*R*)-3-amino-1-(2-((6-amino-9*H*-purin-9-yl)methyl)-4-fluoro-3-(((*S*)-3-fluoropyrrolidin-1-yl)methyl)phenyl)-*N*-cyclopropylpyrrolidine-3-carboxamide | +++ | N/A | +++ | +++ |
| 69 | (*R*)-3-amino-1-(2-((6-amino-9*H*-purin-9-yl)methyl)-3-((3,3-difluoropiperidin-1-yl)methyl)-4-fluorophenyl)-*N*-cyclopropylpyrrolidine-3-carboxamide | ++ | N/A | +++ | +++ |
| 70 | (*R*)-3-amino-1-(2-((6-amino-9*H*-purin-9-yl)methyl)-3-(azetidin-1-ylmethyl)-4-fluorophenyl)-*N-*cyclopropylpyrrolidine-3-carboxamide | ++ | + | +++ | +++ |
| 71 | methyl (*R*)-(3-(3-amino-3-(cyclopropylcarbamoyl)pyrrolidin-1-yl)-2-((6-amino-9*H*-purin-9-yl)methyl)-6-fluorobenzyl)carbamate | +++ | N/A | + | N/A |
| 72 | (*R*)-3-amino-1-(2-((6-amino-9*H*-purin-9-yl)methyl)-6-chloro-3-((dimethylamino)methyl)-4-fluorophenyl)-*N-*cyclopropylpyrrolidine-3-carboxamide | + | + | + | N/A |
| 73 | (*R*)-(3-amino-1-(2-((6-amino-9*H*-purin-9-yl)methyl)-4,6-dichloro-3-ethylphenyl)pyrrolidin-3-yl)(1,1-dioxidothiomorpholino)methanone | + | N/A | +++ | +++ |
| 74 | (*R*)-3-amino-1-(2-((6-amino-9H-purin-9-yl)methyl)-4,6-dichloro-3-((dimethylamino)methyl)phenyl)-*N-*cyclopropylpyrrolidine-3-carboxamide | + | N/A | + | N/A |
| 75 | (*R*)-3-amino-1-(2-((6-amino-9*H*-purin-9-yl)methyl)-6-chloro-4-fluoro-3-(morpholinomethyl)phenyl)-*N-*cyclopropylpyrrolidine-3-carboxamide | ++ | + | + | N/A |
| 76 | (*R*)-3-amino-1-(2-((6-amino-9*H*-purin-9-yl)methyl)-4-fluoro-3-(hydroxymethyl) phenyl)-*N-*cyclopropylpyrrolidine-3-carboxamide | ++ | N/A | +++ | ++ |
| 77 | (*R*)-3-amino-1-(2-((6-amino-9*H*-purin-9-yl)methyl)-4-fluoro-3-(methoxymethyl) phenyl)-*N-*cyclopropylpyrrolidine-3-carboxamide | ++ | N/A | +++ | +++ |
| 78 | (*R*)-3-amino-1-(2-((6-amino-9*H*-purin-9-yl)methyl)-4-chloro-3-(methoxymethyl)phenyl)-*N-*cyclopropylpyrrolidine-3-carboxamide | +++ | N/A | +++ | +++ |
| 79 | (3*R*)-3-amino-1-(2-((6-amino-9*H*-purin-9-yl)methyl)-4-chloro-3-(((tetrahydrofuran-3-yl)oxy)methyl)phenyl)-*N*-cyclopropylpyrrolidine-3-carboxamide | ++ | N/A | +++ | +++ |
| 80 | (*R*)-3-amino-1-(2-((6-amino-9*H*-purin-9-yl)methyl)-4-fluoro-3-((methoxy-d3)methyl)phenyl)-*N-*cyclopropylpyrrolidine-3-carboxamide | ++ | N/A | +++ | +++ |
| 81 | (*R*)-3-amino-1-(2-((6-amino-9*H*-purin-9-yl)methyl)-4-chloro-3-((methoxy-d3)methyl)phenyl)-*N-*cyclopropylpyrrolidine-3-carboxamide | +++ | N/A | +++ | +++ |
| 82 | (*R*)-3-amino-1-(2-((6-amino-9*H*-purin-9-yl)methyl)-4-fluoro-3-((*S*)-2,2,2-trifluoro-1-hydroxyethyl)phenyl)-*N*-cyclopropylpyrrolidine-3-carboxamide | + | N/A | +++ | ++ |
| 83 | (*R*)-9-(2-(3-amino-3-(1*H*-tetrazol-5-yl)pyrrolidin-1-yl)-6-bromo-4-chlorobenzyl)-9*H*-purin-6-amine | +++ | N/A | +++ | +++ |
| 84 | (*R*)-9-((3-(3-amino-3-(2*H*-tetrazol-5-yl)pyrrolidin-1-yl)-5-chloro-3'-cyclohexyl-[1,1'-biphenyl]-2-yl)methyl)-9*H*-purin-6-amine | ++ | +++ | +++ | +++ |
| 85 | (*R*)-9-(6-(3-amino-3-(1*H*-1,2,4-triazol-3-yl)pyrrolidin-1-yl)-2,3-dichlorobenzyl)-9H-purin-6-amine | +++ | +++ | +++ | +++ |
| 86 | (*R*)-3-amino-1-(2-((6-amino-9*H*-purin-9-yl)methyl)-4-chloro-3-((difluoromethoxy)methyl)phenyl)-*N-*cyclopropylpyrrolidine-3-carboxamide | +++ | +++ | +++ | +++ |
| 87 | (*R*)-3-amino-1-(2-((6-amino-9*H*-purin-9-yl)methyl)-3-bromo-4-(trifluoromethyl)phenyl)-*N*-(2-cyanoethyl)pyrrolidine-3-carboxamide | +++ | ++ | +++ | +++ |
| 88 | (*R*)-3-amino-1-(2-((6-amino-9*H*-purin-9-yl)methyl)-4,5-dichloro-3-((dimethylamino)methyl)phenyl)-*N-*cyclopropylpyrrolidine-3-carboxamide | +++ | +++ | +++ | +++ |
| 89 | (R)-3-amino-1-(2-((6-amino-9*H*-purin-9-yl)methyl)-5,6-dichloro-3-((dimethylamino)methyl)phenyl)-*N-*cyclopropylpyrrolidine-3-carboxamide | + | N/A | + | N/A |
| 90 | (*R*)-9-(2-(3-amino-3-(1-methyl-1H-tetrazol-5-yl)pyrrolidin-1-yl)-6-bromo-4-chlorobenzyl)-9H-purin-6-amine | +++ | + | + | N/A |
| 91 | (R)-9-(2-(3-amino-3-(2-methyl-2*H*-tetrazol-5-yl)pyrrolidin-1-yl)-6-bromo-4-chlorobenzyl)-9*H*-purin-6-amine | ++ | N/A | + | N/A |
| 92 | (*R*)-3-amino-1-(4-((6-amino-9*H*-purin-9-yl)methyl)-5-ethyl-2'-fluoro-[1,1'-biphenyl]-3-yl)-*N-*cyclopropylpyrrolidine-3-carboxamide | +++ | N/A | +++ | +++ |
| 93 | (*R*)-3-amino-1-(2-((6-amino-9*H*-purin-9-yl)methyl)-3-ethyl-5-(thiophen-2-yl)phenyl)-*N-*cyclopropylpyrrolidine-3-carboxamide | +++ | +++ | +++ | +++ |
| 94 | (*R*)-3-amino-1-(2-((6-amino-9*H*-purin-9-yl)methyl)-5-(thiophen-2-yl)-3-(trifluoromethyl) phenyl)-*N-*cyclopropylpyrrolidine-3-carboxamide | +++ | N/A | +++ | +++ |
| 95 | (*R*)-3-amino-1-(2-((6-amino-9*H*-purin-9-yl)methyl)-3-ethyl-5-(pyridin-3-yl)phenyl)-*N-*cyclopropylpyrrolidine-3-carboxamide | +++ | N/A | +++ | +++ |
| 96 | (*R*)-3-amino-1-(2-((6-amino-9*H*-purin-9-yl)methyl)-3-ethyl-5-(1-methyl-1*H*-pyrazol-4-yl)phenyl)-*N-*cyclopropylpyrrolidine-3-carboxamide | +++ | +++ | ++ | + |
| 97 | 3-amino-1-(2-((6-amino-9*H*-purin-9-yl)methyl)-4-chloro-3-cyclopropylphenyl)-*N-*cyclopropylpyrrolidine-3-carboxamide | +++ | N/A | + | +++ |
| 98 | (*R*)-3-amino-1-(2-((6-amino-9*H*-purin-9-yl)methyl)-3-ethyl-5-(thiazol-4-yl)phenyl)-*N-*cyclopropylpyrrolidine-3-carboxamide | ++ | N/A | +++ | +++ |
| 99 | (*R*)-3-amino-1-(2-((6-amino-9*H*-purin-9-yl)methyl)-3-ethyl-5-(pyridin-2-yl)phenyl)-*N-*cyclopropylpyrrolidine-3-carboxamide | +++ | +++ | +++ | +++ |
| 100 | (*R*)-3-amino-1-(2-((6-amino-9*H*-purin-9-yl)methyl)-3-ethyl-5-(pyrazin-2-yl)phenyl)-*N-*cyclopropylpyrrolidine-3-carboxamide | +++ | N/A | +++ | +++ |
| 101 | (*R*)-3-amino-1-(2-((6-amino-9*H*-purin-9-yl)methyl)-5-chloro-4-cyano-3-ethylphenyl)-*N-*cyclopropylpyrrolidine-3-carboxamide | +++ | +++ | +++ | ++ |
| 102 | (*R*)-3-amino-1-(2-((6-amino-9*H*-purin-9-yl)methyl)-5-chloro-4-fluoro-3-(morpholinomethyl)phenyl)-*N-*cyclopropylpyrrolidine-3-carboxamide | +++ | +++ | +++ | +++ |
| 103 | (*R*)-3-amino-1-(2-((6-amino-9*H*-purin-9-yl)methyl)-3-chloro-4-fluoro-5-(morpholinomethyl)phenyl)-*N-*cyclopropylpyrrolidine-3-carboxamide | +++ | N/A | +++ | +++ |
| 104 | (*R*)-3-amino-1-(2-((6-amino-9*H*-purin-9-yl)methyl)-4-chloro-3-cyanophenyl)-*N*-cyclopropylpyrrolidine-3-carboxamide | +++ | N/A | +++ | +++ |
| 105 | (*R*)-3-amino-1-(2-((6-amino-9*H*-purin-9-yl)methyl)-4,5-dichloro-3-(morpholinomethyl)phenyl)-*N-*cyclopropylpyrrolidine-3-carboxamide | +++ | N/A | +++ | +++ |
| 106 | (S)-(3-amino-1-(1-((6-amino-9*H*-purin-9-yl)methyl)naphthalen-2-yl)pyrrolidin-3-yl)methanol | +++ | +++ | +++ | +++ |
| 107 | (S)-(3-amino-1-(2-((6-amino-9*H*-purin-9-yl)methyl)-3-bromo-5-chlorophenyl)pyrrolidin-3-yl)methanol | + | + | + | +++ |
| 108 | (*R*)-(3-amino-1-(2-((6-amino-9*H*-purin-9-yl)methyl)-5-chloro-3-ethylphenyl)pyrrolidin-3-yl)methanol | +++ | N/A | +++ | +++ |
| 109 | (*R*)-9-((2-(3-amino-3-(methoxymethyl)pyrrolidin-1-yl)naphthalen-1-yl)methyl)-9*H*-purin-6-amine | ++ | N/A | +++ | +++ |
| 110 | (*R*)-3-amino-1-(6-((6-amino-9*H*-purin-9-yl)methyl)-5-bromo-4-chloro-2,3-dihydrobenzofuran-7-yl)-*N-*cyclopropylpyrrolidine-3-carboxamide | +++ | +++ | +++ | +++ |
| 111 | (*R*)-3-amino-1-(6-((6-amino-9*H*-purin-9-yl)methyl)-4-fluoro-5-iodo-2,3-dihydrobenzofuran-7-yl)-*N-*cyclopropylpyrrolidine-3-carboxamide | +++ | N/A | ++ | ++ |
| 112 | (*R*)-3-amino-1-(7-((6-amino-9*H*-purin-9-yl)methyl)benzofuran-6-yl)-*N*-cyclopropylpyrrolidine-3-carboxamide | ++ | +++ | +++ | +++ |
| 113 | (*R*)-3-amino-1-(2-((6-amino-9*H*-purin-9-yl)methyl)-4-chloro-3-(ethylthio)phenyl)-*N*-cyclopropylpyrrolidine-3-carboxamide | +++ | N/A | +++ | +++ |
| 114 | (*R*)-3-amino-1-(7-((6-amino-9*H*-purin-9-yl)methyl)-2,2-dimethyl-2,3-dihydrobenzofuran-6-yl)-*N-*cyclopropylpyrrolidine-3-carboxamide | + | + | + | N/A |
| 115 | 3-amino-1-(5-((6-amino-9*H*-purin-9-yl)methyl)-6-bromo-2,3-dihydrobenzofuran-4-yl)-*N-*cyclopropylpyrrolidine-3-carboxamide | ++ | +++ | + | N/A |
| 116 | (*R*)-3-amino-1-(2-((6-amino-9*H*-purin-9-yl)methyl)-4-fluoro-3-(trifluoromethyl)phenyl)-*N-*cyclopropylpyrrolidine-3-carboxamide | +++ | +++ | +++ | +++ |
| 117 | (*R*)-3-amino-1-(2-((6-amino-9*H*-purin-9-yl)methyl)-4-fluoro-3-(trifluoromethyl)phenyl)-*N*-((1*r*,3*R*)-3-(methylthio)cyclobutyl)pyrrolidine-3-carboxamide | +++ | +++ | +++ | +++ |
| 118 | (*R*)-3-amino-1-(2-((6-amino-9*H*-purin-9-yl)methyl)-4-fluoro-3-(trifluoromethyl)phenyl)-*N*-(pyridazin-3-ylmethyl)pyrrolidine-3-carboxamide | ++ | +++ | +++ | +++ |
| 119 | (*R*)-3-amino-1-(2-((6-amino-9*H*-purin-9-yl)methyl)-4-fluoro-3-(trifluoromethyl)phenyl)-*N*-(2-cyano-2-methylpropyl)pyrrolidine-3-carboxamide | ++ | +++ | +++ | +++ |
| 120 | (*R*)-3-amino-1-(2-((6-amino-9*H*-purin-9-yl)methyl)-4-fluoro-3-(trifluoromethyl)phenyl)-*N*-((2-hydroxypyridin-3-yl)methyl)pyrrolidine-3-carboxamide | ++ | N/A | +++ | +++ |
| 121 | (*R*)-*N*-(2-(1*H*-imidazol-4-yl)ethyl)-3-amino-1-(2-((6-amino-9*H*-purin-9-yl)methyl)-4-fluoro-3-(trifluoromethyl)phenyl)pyrrolidine-3-carboxamide | +++ | N/A | +++ | +++ |
| 122 | (3*R*)-3-amino-1-(2-((6-amino-9*H*-purin-9-yl)methyl)-4-fluoro-3-(trifluoromethyl)phenyl)-*N*-((2,2-difluorocyclopropyl)methyl)pyrrolidine-3-carboxamide | +++ | N/A | +++ | +++ |
| 123 | (*R*)-3-amino-1-(2-((6-amino-9*H*-purin-9-yl)methyl)-4-fluoro-3-(trifluoromethyl)phenyl)*-N*-(3-cyclopropyl-1*H*-pyrazol-5-yl)pyrrolidine-3-carboxamide | + | +++ | +++ | +++ |
| 124 | (*R*)-3-amino-1-(2-((6-amino-9*H*-purin-9-yl)methyl)-4-fluoro-3-(trifluoromethyl)phenyl)-*N*-((*R*)-1-(tetrahydro-2*H*-pyran-4-yl)ethyl)pyrrolidine-3-carboxamide | ++ | N/A | +++ | +++ |
| 125 | (*R*)-3-amino-1-(2-((6-amino-9*H*-purin-9-yl)methyl)-4-fluoro-3-(trifluoromethyl)phenyl)-*N*-(2-cyano-2-methylpropyl)pyrrolidine-3-carboxamide | +++ | +++ | +++ | +++ |
| 126 | (3*R*)-3-amino-1-(2-((6-amino-9*H*-purin-9-yl)methyl)-4-fluoro-3-(trifluoromethyl)phenyl)-*N*-(6-oxaspiro[2.5]octan-1-yl)pyrrolidine-3-carboxamide | ++ | N/A | +++ | +++ |
| 127 | (*R*)-3-amino-1-(2-((6-amino-9*H*-purin-9-yl)methyl)-4-fluoro-3-(trifluoromethyl)phenyl)-*N*-((1*R*,2*R*)-2-(hydroxymethyl)cyclopropyl)pyrrolidine-3-carboxamide | +++ | +++ | +++ | +++ |
| 128 | (*R*)-(3-amino-1-(2-((6-amino-9*H*-purin-9-yl)methyl)-4-fluoro-3-(trifluoromethyl)phenyl)pyrrolidin-3-yl)(6,7-dihydro-[1,2,3]triazolo[1,5-a]pyrazin-5(4*H*)-yl)methanone | + | + | +++ | + |
| 129 | (3*R*)-3-amino-1-(2-((6-amino-9*H*-purin-9-yl)methyl)-4-fluoro-3-(trifluoromethyl)phenyl)-*N-*(tetrahydrofuran-3-yl)pyrrolidine-3-carboxamide | +++ | N/A | +++ | +++ |
| 130 | (3*R*)-3-amino-1-(2-((6-amino-9*H*-purin-9-yl)methyl)-4-fluoro-3-(trifluoromethyl)phenyl)-*N*-(5-methoxyspiro[2.3]hexan-1-yl)pyrrolidine-3-carboxamide | +++ | N/A | +++ | +++ |
| 131 | ((*R*)-3-amino-1-(2-((6-amino-9*H*-purin-9-yl)methyl)-4-fluoro-3-(trifluoromethyl)phenyl)pyrrolidin-3-yl)((*S*)-2-methylmorpholino)methanone | ++ | +++ | +++ | +++ |
| 132 | (*R*)-1-(3-amino-1-(2-((6-amino-9*H*-purin-9-yl)methyl)-4-fluoro-3-(trifluoromethyl)phenyl)pyrrolidine-3-carbonyl)azetidine-3-carbonitrile | ++ | +++ | +++ | +++ |
| 133 | (*R*)-3-amino-1-(2-((6-amino-9*H*-purin-9-yl)methyl)-4-fluoro-3-(trifluoromethyl)phenyl)-*N*-((2-aminopyrimidin-4-yl)methyl)pyrrolidine-3-carboxamide | ++ | ++ | +++ | +++ |
| 134 | (*R*)-(3-amino-1-(2-((6-amino-9*H*-purin-9-yl)methyl)-4-fluoro-3-(trifluoromethyl)phenyl)pyrrolidin-3-yl)(6,7-dihydroisoxazolo[4,3-c]pyridin-5(4*H*)-yl)methanone | + | +++ | +++ | +++ |
| 135 | ((*R*)-3-amino-1-(2-((6-amino-9*H*-purin-9-yl)methyl)-4-fluoro-3-(trifluoromethyl)phenyl)pyrrolidin-3-yl)((*R*)-2-methylmorpholino)methanone | ++ | +++ | +++ | +++ |
| 136 | (*R*)-2-(4-(3-amino-1-(2-((6-amino-9*H*-purin-9-yl)methyl)-4-fluoro-3-(trifluoromethyl)phenyl)pyrrolidine-3-carbonyl)piperazin-1-yl)acetonitrile | ++ | +++ | +++ | +++ |
| 137 | (*R*)-(3-amino-1-(2-((6-amino-9*H*-purin-9-yl)methyl)-4-fluoro-3-(trifluoromethyl)phenyl)pyrrolidin-3-yl)(6,7-dihydropyrazolo[1,5-a]pyrazin-5(4*H*)-yl)methanone | ++ | N/A | +++ | ++ |
| 138 | ((*R*)-3-amino-1-(2-((6-amino-9*H*-purin-9-yl)methyl)-4-fluoro-3-(trifluoromethyl)phenyl)pyrrolidin-3-yl)((R)-3-hydroxypiperidin-1-yl)methanone | ++ | +++ | +++ | +++ |
| 139 | (*R*)-3-amino-1-(2-((6-amino-9*H*-purin-9-yl)methyl)-4-fluoro-3-(trifluoromethyl)phenyl)-*N*-(3-oxo-3-(propylamino)propyl)pyrrolidine-3-carboxamide | ++ | N/A | +++ | +++ |
| 140 | (*R*)-3-amino-1-(2-((6-amino-9*H*-purin-9-yl)methyl)-4-fluoro-3-(trifluoromethyl)phenyl)-*N*-((1-ethyl-1*H-*1,2,4-triazol-5-yl)methyl)pyrrolidine-3-carboxamide | + | ++ | +++ | +++ |
| 141 | 1-((*R*)-3-amino-1-(2-((6-amino-9*H*-purin-9-yl)methyl)-4-fluoro-3-(trifluoromethyl)phenyl)pyrrolidine-3-carbonyl)pyrrolidine-3-carbonitrile | +++ | +++ | +++ | +++ |
| 142 | (S)-3-amino-1-(2-((6-amino-9H-purin-9-yl)methyl)-3-bromo-6-chlorophenyl)pyrrolidine-3-carboxylic acid | +++ | +++ | +++ | + |
| 143 | (R)-3-amino-1-(2-((6-amino-9H-purin-9-yl)methyl)-3-bromo-4,6-dichlorophenyl)-N-cyclopropylpyrrolidine-3-carboxamide | +++ | +++ | +++ | +++ |
| 144 | (R)-3-amino-1-(2-((6-amino-9H-purin-9-yl)methyl)-3,4-dichlorophenyl)-N-cyclopropylpyrrolidine-3-carboxamide | +++ | +++ | +++ | +++ |
| 145 | (R)-3-amino-1-(2-((6-amino-9H-purin-9-yl)methyl)-4-chloro-3-ethylphenyl)-N-(2-(phenylsulfonyl)ethyl)pyrrolidine-3-carboxamide | +++ | +++ | +++ | +++ |
| 146 | (R)-3-amino-1-(2-((6-amino-9H-purin-9-yl)methyl)-4-bromo-3-(trifluoromethyl)phenyl)-N-cyclopropylpyrrolidine-3-carboxamide | +++ | +++ | +++ | +++ |
| 147 | (R)-3-amino-1-(2-((6-amino-9H-purin-9-yl)methyl)-3-bromo-4-(trifluoromethoxy)phenyl)-N-cyclopropylpyrrolidine-3-carboxamide | +++ | +++ | +++ | +++ |
| 148 | (R)-3-amino-1-(2-((6-amino-9H-purin-9-yl)methyl)-3-bromo-5-chlorophenyl)pyrrolidine-3-carboxamide | +++ | +++ | +++ | +++ |
| 149 | (R)-3-amino-1-(2-((6-amino-9H-purin-9-yl)methyl)-4-chloro-3-ethylphenyl)-N-(2-(ethylsulfonyl)ethyl)pyrrolidine-3-carboxamide | +++ | +++ | +++ | +++ |
| 150 | (R)-3-amino-1-(2-((6-amino-9H-purin-9-yl)methyl)-5-chloro-3-(5,6,7,8-tetrahydronaphthalen-1-yl)phenyl)-N-cyclopropylpyrrolidine-3-carboxamide | +++ | +++ | ++ | +++ |
| 151 | (R)-(3-amino-1-(2-((6-amino-9H-purin-9-yl)methyl)-5-chloro-3-ethylphenyl)pyrrolidin-3-yl)(1,1-dioxidothiomorpholino)methanone | + | +++ | +++ | +++ |
| 152 | 3-amino-1-(2-((6-amino-9H-purin-9-yl)methyl)-3,4-dichloro-6-fluorophenyl)-N-cyclopropylpyrrolidine-3-carboxamide | + | +++ | +++ | +++ |
| 153 | (R)-3-amino-1-(2-((6-amino-9H-purin-9-yl)methyl)-4-chloro-3-ethylphenyl)-N-(1,1-dioxidotetrahydro-2H-thiopyran-4-yl)pyrrolidine-3-carboxamide | +++ | ++ | +++ | +++ |
| 154 | (R)-3-amino-1-(2-((6-amino-9H-purin-9-yl)methyl)-6-chloro-3-ethylphenyl)-N-cyclopropylpyrrolidine-3-carboxamide | +++ | + | +++ | +++ |
| 155 | (R)-(3-amino-1-(2-((6-amino-9H-purin-9-yl)methyl)-4-chloro-3-ethylphenyl)pyrrolidin-3-yl)(1,1-dioxidothiomorpholino)methanone | + | + | +++ | +++ |
| 156 | (R)-9-(6-(3-amino-3-(1 H-tetrazol-5-yl)pyrrolidin-1-yl)-3-chloro-2-iodobenzyl)-9H-purin-6-amine | ++ | N/A | +++ | +++ |
| 157 | 3-amino-1-(2-((6-amino-9H-purin-9-yl)methyl)-3-chloro-6-methylphenyl)-N-cyclopropylpyrrolidine-3-carboxamide | + | N/A | + | N/A |
| 158 | (R)-3-amino-1-(2-((6-amino-9H-purin-9-yl)methyl)-3-cyclopentyl-4-fluorophenyl)-N-cyclopropylpyrrolidine-3-carboxamide | +++ | N/A | +++ | +++ |
| 159 | (R)-3-amino-1-(2-((6-amino-9H-purin-9-yl)methyl)-3-bromo-5-chlorophenyl)-N-(ethyl-d5)pyrrolidine-3-carboxamide | +++ | N/A | +++ | ++ |
| 160 | 3-amino-1-(2-((6-amino-9H-purin-9-yl)methyl)-3-chloro-4-(trifluoromethyl)phenyl)-N-cyclopropylpyrrolidine-3-carboxamide | ++ | N/A | +++ | +++ |
| 161 | (R)-3-amino-1-(2-((6-amino-9H-purin-9-yl)methyl)-5-chloro-3-ethylphenyl)-N-isopropylpyrrolidine-3-carboxamide | +++ | N/A | +++ | +++ |
| 162 | (R)-3-amino-1-(2-((6-amino-9H-purin-9-yl)methyl)-4,6-dichloro-3-(methoxymethyl)phenyl)-N-cyclopropylpyrrolidine-3-carboxamide | ++ | N/A | +++ | ++ |
| 163 | (R)-3-amino-1-(2-((6-amino-9H-purin-9-yl)methyl)-4-chloro-3-vinylphenyl)-N-cyclopropylpyrrolidine-3-carboxamide | +++ | N/A | +++ | +++ |
| 164 | 3-amino-1-(2-((6-amino-9H-purin-9-yl)methyl)-3-chlorophenyl)-N-cyclopropylpyrrolidine-3-carboxamide | ++ | N/A | +++ | +++ |
| 165 | (R)-3-amino-1-(2-((6-amino-9H-purin-9-yl)methyl)-4-chloro-3-ethylphenyl)-N-cyclopropylpyrrolidine-3-carboxamide | ++ | N/A | +++ | +++ |
| 166 | (S)-3-amino-1-(1-((6-amino-9H-purin-9-yl)methyl)naphthalen-2-yl)-N-cyclopropylpyrrolidine-3-carboxamide | ++ | N/A | +++ | N/A |
| 167 | (R)-3-amino-1-(1-((6-amino-9H-purin-9-yl)methyl)-6-bromonaphthalen-2-yl)-N-cyclopropylpyrrolidine-3-carboxamide | +++ | N/A | +++ | N/A |
| 168 | (R)-3-amino-1-(2-((6-amino-9H-purin-9-yl)methyl)-3-bromo-4-methoxyphenyl)-N-cyclopropylpyrrolidine-3-carboxamide | ++ | N/A | +++ | +++ |
| 169 | (R)-3-amino-1-(2-((6-amino-9H-purin-9-yl)methyl)-3-ethylphenyl)-N-cyclopentylpyrrolidine-3-carboxamide | ++ | N/A | +++ | +++ |
| 170 | ethyl (R)-3-amino-1-(2-((6-amino-9H-purin-9-yl)methyl)-3-bromo-5-chlorophenyl)pyrrolidine-3-carboxylate | ++ | +++ | +++ | +++ |

| Rule: | "+++" | "++" | "+" |
|---|---|---|---|
| LCMS | <0.05 uM | 0.05-0.10 uM | >0.10 uM |
| MLL1 flash plate | <0.06 uM | 0.06-0.10 uM | >0.10 uM |
| ELISA | <1.9 uM | 1.9-3.0 uM | >3.0 uM |
| CTG MV4:11 | <2.0 uM | 2.0-3.0 uM | >3.0 uM |

It is understood that the examples and embodiments described herein are for illustrative purposes only and that various modifications or changes in light thereof will be suggested to persons skilled in the art and are to be included within the scope of the appended claims.

## Claims

1. A compound of Formula (I) 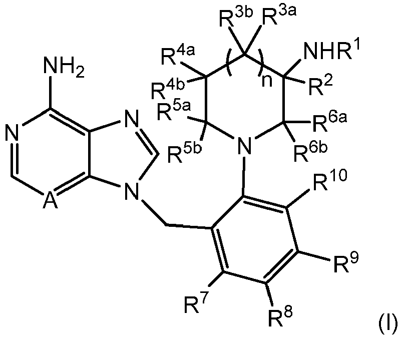 or an enantiomer, an enantiomeric mixture, or a pharmaceutically acceptable salt thereof; wherein:
A is N;
R1 is H; or
R¹ and R² together with NH forms a 5-8 membered heterocyclyl comprising 1-2 heteroatoms selected from N, O and S as ring members; wherein said 5-8 membered heterocyclyl is unsubstituted or substituted by an oxo substituent;
R² is selected from the group consisting of:
(i) -C₁₋₆ alkyl, -haloC₁₋₆ alkyl, -hydroxyC₁₋₆ alkyl, -C₁₋₆alkoxyC₁₋₆alkyl or -C₃₋₈ cycloalkoxy(C₁₋₆ alkyl);
(ii) cyano, -cyanoC₁₋₆ alkyl, -C₁₋₆ alkylthioC₁₋₆alkyl, -C₂₋₆ alkenyl, -haloC₂₋₆ alkenyl, -C₂₋₆ alkynyl, -C₁₋₄ alkyl-S-C₁₋₄alkyl, -C₁₋₄ alkylSO₂C₁₋₄alkyl, -SO₂(₁₋₄ alkyl) or -C(C₁₋₄ alkyl)=N-O(C₁₋₄ alkyl);
(iii) -C₁₋₄alkylcarbonyl, -(CR^{a}R^{b})ₚ-C(=O)-OR^{10a} or -C(=O)-(CR^{a}R^{b})_{q}R¹¹;
wherein R¹¹ is C₃₋₇ cycloalkyl, 5-6 membered heterocyclyl or 5-6 membered heteroaryl, each of which is independently unsubstituted or substituted with -C₁₋₆ alkyl or -C₁₋₆ alkoxy; and said 5-6 membered heterocyclyl or 5-6 membered heteroaryl independently comprises 1-3 heteroatoms selected from nitrogen, oxygen and sulfur;
(iv) -(CR^{a}R^{b})ᵣ-C(=O)-NR¹²R¹³ -(CR^{a}R^{b})ₛ-NR¹²R¹³, -(CR^{a}R^{b})₁₋₄-O-(CR^{a}R^{b})₁₋₄-OR^{10a} or
-(CR^{a}R^{b})₁₋₄-O-(CR^{a}R^{b})₁₋₄-C(=O)-NR¹²R¹³;
wherein R¹² is hydrogen or -C₁₋₆ alkyl;
R¹³ is hydrogen, -C₁₋₆ alkyl, -C₁₋₆alkoxyC₁₋₆alkyl, -cyanoC₁₋₆ alkyl; -C₁₋₄ alkylSO₂R^{10b} wherein R^{10b} is C₁₋₆ alkyl or phenyl; C₃₋₁₀ monocylic or bicyclic cycloalkylC₀₋₆alkyl, phenyl, 5-10 membered monocyclic or bicyclic heterocyclic ring or 5-9 membered heteroarylC₀₋₆alkyl;
said 5-10 membered monocyclic or bicyclic heterocyclic ring or 5-9 membered heteroaryl radical independently comprises 1-4 heteroatoms selected from nitrogen, oxygen and sulfur; and
said C₃₋₁₀ monocylic or bicyclic cycloalkyl, phenyl, 5-10 membered monocyclic or bicyclic heterocyclic ring or 5-9 membered heteroaryl radical are independently unsubstituted or substituted with 1-2 -C₁₋₄ alkyl, -hydroxyC₁₋₆ alkyl, -C₁₋₄ alkoxy, halo, hydroxyl, phenyl or -S(C₁₋₄ alkyl);
or R¹² and R¹³ together form a 5-10 membered monocyclic or bicyclic heterocyclic ring comprising 1-4 heteroatoms selected from nitrogen, oxygen and sulfur; and is unsubstituted or substituted with -C₁₋₄ alkyl, hydroxyl, cyano, -cyanoC₁₋₆ alkyl, -SO₂ or -C₂₋₄alkenylcarbonyl;
(v) 5-6 membered heterocyclylC₀₋₆alkyl or 5-6 membered heterocyclyl(haloC₁₋₄ alkyl) wherein each said heterocyclyl radical is unsubstituted or substituted by oxo; and wherein each said heterocyclyl radical comprises 1-3 heteroatoms selected from nitrogen, oxygen and sulfur; and
(vi) phenyl, 5-9 membered heteroarylC₀₋₆alkyl or 5-9 membered heteroaryl(haloC₁₋₄alkyl), wherein each said phenyl or heteroaryl radical is independently unsubstituted or substituted by
- C₁₋₄ alkyl, -haloC₁₋₄ alkyl, -hydroxyC₁₋₄ alkyl, -C₁₋₄ alkoxy, -haloC₁₋₄ alkoxy, halo, hydroxy, cyano, oxido, amino, -C₁₋₄ alkylamino, -C₁₋₄ dialkylamino, -aminocarbonylC₀₋₆alkyl,
- C₁₋₄alkylaminocarbonylC₀₋₆alkyl, -diC₁₋₄alkylaminocarbonylC₀₋₆alkyl or C₃₋₇ cycloalkyl;
wherein each said heteroaryl radical comprises 1-4 heteroatoms selected from nitrogen, oxygen and sulfur;
R^{3a}, R^{3b}, R^{4a}, R^{4b}, R^{5a}, R^{5b}, R^{6a} and R^{6b} are independently hydrogen, halo, cyano, hydroxyl,
-C₁₋₆ alkyl, -haloC₁₋₆ alkyl, -hydroxyC₁₋₆ alkyl, -C₁₋₆ alkoxy, -C₁₋₆alkoxyC₁₋₆alkyl, aryl,-C(=O)-OR¹⁴ or -(CR^{a}R^{b})ₛ-C(=O)-NR¹⁵R¹⁶; or
R^{3a} and R^{3b}, R^{4a} and R^{4b}, R^{5a} and R^{5b} or R^{6a} and R^{6b} forms an oxo substituent;
R⁷, R⁸, R⁹ and R¹⁰ are independently hydrogen, halo, -C₁₋₄ alkyl, -haloC₁₋₆ alkyl,-hydroxyC₁₋₆ alkyl, cyano, -cyanoC₁₋₆ alkyl, -C₂₋₆ alkenyl, -C₂₋₆ alkynyl, -C₁₋₆ alkylthio,-(CR^{a}R^{b})₁₋₄-NR¹⁷R¹⁸,
-(CR^{a}R^{b})₁₋₄NR¹⁷-C(O)-OR¹⁸, -(CR^{a}R^{b})₁₋₄-OR¹⁹, C₃₋₈ cycloalkyl, phenyl or 5-6 membered heteroaryl comprising 1-3 heteroatoms selected from nitrogen, oxygen and sulfur; wherein said C₃₋₈ cycloalkyl, phenyl or 5-6 membered heteroaryl is independently substituted with 1-2 R²⁰; and provided at least one of R⁷, R⁸, R⁹ and R¹⁰ is not hydrogen;
alternatively, R⁷ and R⁸, R⁸ and R⁹, and R⁹ together with the phenyl ring to which they are attached form a 9-10 membered benzo-fused carbocycle or benzo-fused heterocycle comprising 1-3 heteroatoms selected from nitrogen, oxygen and sulfur; wherein said benzo-fused carbocycle or benzo-fused heterocycle is independently unsubstituted or substituted with 1-2 halo or C₁₋₄ alkyl;
R^{a}, R^{b}, R^{10a}, R¹⁴, R¹⁵, R¹⁶ and R¹⁷ are independently hydrogen or -C₁₋₄ alkyl;
R¹⁸ is hydrogen, -C₁₋₄ alkyl, -haloC₁₋₆ alkyl or -C₃₋₆ cycloalkyl;
alternatively, R¹⁷ and R¹⁸ together with N in the -NR¹⁷R¹⁸ moiety form a 4-10 membered monocyclic or bicyclic heterocyclic ring comprising 1-3 heteroatoms selected from nitrogen, oxygen and sulfur; wherein said 4-10 membered monocyclic or bicyclic heterocyclic ring is unsubstituted or substituted with 1-2 halo or C₁₋₄ alkyl;
R¹⁹ is hydrogen, -C₁₋₄ alkyl, -haloC₁₋₆ alkyl, -C₃₋₆ cycloalkyl, phenyl, 5-6 membered heterocyclyl or 5-6 membered heteroaryl; wherein said 5-6 membered heterocyclyl or 5-6 membered heteroaryl independently comprises 1-3 heteroatoms selected from nitrogen, oxygen and sulfur;
R²⁰ is -C₁₋₄ alkyl, -halo or -C₃₋₆ cycloalkyl; or two R²⁰ together form a 5-6 membered ring comprising 0-2 heteroatoms selected from nitrogen, oxygen and sulfur;
n is 0 or 1; and
p, q, r and s are independently 0, 1, 2, 3 or 4.

2. The compound of Formula (I) in claim 1, wherein said compound is a compound of Formula (II): 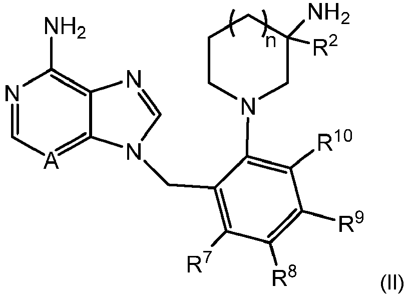 or an enantiomer, an enantiomeric mixture, or a pharmaceutically acceptable salt thereof; wherein:
R² is selected from the group consisting of:
(i) -hydroxyC₁₋₆ alkyl, -C₁₋₆alkoxyC₁₋₆alkyl or -(CR^{a}R^{b})ₚ-C(=O)-OR^{10a};
(ii) -(CR^{a}R^{b})ᵣ-C(=O)-NR¹²R¹³ or-(CR^{a}R^{b})₁₋₄-O-(CR^{a}R^{b})₁₋₄-C(=O)-NR¹²R¹³; wherein R¹² is hydrogen or -C₁₋₆ alkyl;
R¹³ is hydrogen, -C₁₋₆ alkyl, -cyanoC₁₋₆ alkyl; -C₁₋₄ alkylSO₂R^{10b} wherein R^{10b} is-C₁₋₆ alkyl or phenyl; C₃₋₁₀ monocylic or bicyclic cycloalkylC₀₋₆alkyl, phenyl, 5-10 membered monocyclic or bicyclic heterocyclic ring or 5-9 membered heteroarylC₀₋₆alkyl; wherein said 5-10 membered monocyclic or bicyclic heterocyclic ring or 5-9 membered heteroaryl radical independently comprises 1-4 heteroatoms selected from nitrogen, oxygen and sulfur; and
said C₃₋₁₀ monocylic or bicyclic cycloalkyl, phenyl, 5-10 membered monocyclic or bicyclic heterocyclic ring or 5-9 membered heteroaryl radical are independently unsubstituted or substituted with 1-2 -C₁₋₄ alkyl,-hydroxyC₁₋₆ alkyl, -C₁₋₄ alkoxy, halo, hydroxyl, phenyl or -S(C₁₋₄ alkyl); or
R¹² and R¹³ together form a 5-10 membered monocyclic or bicyclic heterocyclic ring comprising 1-4 heteroatoms selected from nitrogen, oxygen and sulfur; and is unsubstituted or substituted with -C₁₋₄ alkyl, hydroxyl, cyano, -cyanoC₁₋₆ alkyl, -SO₂ or -C₂₋₄alkenylcarbonyl; and
(iii) phenyl or 5-9 membered heteroarylC₀₋₆alkyl; wherein said heteroaryl radical is unsubstituted or substituted by -C₁₋₄ alkyl, -haloC₁₋₄ alkyl, amino, -C₁₋₄ alkylamino or -C₁₋₄ dialkylamino; and said heteroaryl radical comprises 1-4 heteroatoms selected from nitrogen, oxygen and sulfur;
R⁷, R⁸, R⁹ and R¹⁰ are independently hydrogen, halo, -C₁₋₄ alkyl, -haloC₁₋₆ alkyl,-hydroxyC₁₋₆ alkyl, cyano, -cyanoC₁₋₆ alkyl, -C₂₋₆ alkenyl, -C₂₋₆ alkynyl, -C₁₋₆ alkylthio,-(CR^{a}R^{b})₁₋₄-NR¹⁷R¹⁸,
-(CR^{a}R^{b})₁₋₄NR¹⁷-C(O)-OR¹⁸, -(CR^{a}R^{b})₁₋₄-OR¹⁹, -C₃₋₈ cycloalkyl, phenyl or 5-6 membered heteroaryl comprising 1-4 heteroatoms selected from nitrogen, oxygen and sulfur; and
wherein said C₃₋₈ cycloalkyl, phenyl or 5-6 membered heteroaryl is independently substituted with 1-2 R²⁰; and
provided at least one of R⁷, R⁸, R⁹ and R¹⁰ is not hydrogen;
alternatively, R⁷ and R⁸, R⁸ and R⁹, R⁹ and R¹⁰ together with the phenyl ring to which they are attached form a 9-10 membered benzo-fused carbocycle or benzo-fused heterocycle comprising 1-3 heteroatoms selected from nitrogen, oxygen and sulfur; wherein said benzo-fused carbocycle or benzo-fused heterocycle is independently unsubstituted or substituted with 1-2 halo or -C₁₋₄ alkyl;
R^{a}, R^{b}, R^{10a} and R¹⁷ are independently hydrogen or -C₁₋₄ alkyl;
R¹⁸ is hydrogen, -C₁₋₄ alkyl, -haloC₁₋₆ alkyl or -C₃₋₆ cycloalkyl;
alternatively, R¹⁷ and R¹⁸ together with N in the -NR¹⁷R¹⁸ moiety form a 4-10 membered monocyclic or bicyclic heterocyclic ring comprising 1-3 heteroatoms selected from nitrogen, oxygen and sulfur; wherein said heterocyclic ring is unsubstituted or substituted with 1-2 halo or -C₁₋₄ alkyl;
R¹⁹ is hydrogen, -C₁₋₄ alkyl, -haloC₁₋₆ alkyl, -C₃₋₆ cycloalkyl, phenyl, 5-6 membered heterocyclyl or 5-6 membered heteroaryl; wherein said 5-6 membered heterocyclyl or 5-6 membered heteroaryl independently comprises 1-3 heteroatoms selected from nitrogen, oxygen and sulfur;
R²⁰ is -C₁₋₄ alkyl, halo or -C₃₋₆ cycloalkyl; or two R²⁰ together form a 5-6 membered ring comprising 0-2 heteroatoms selected from nitrogen, oxygen and sulfur;
n is 0 or 1; and
p, q, r, and sare independently 0, 1, 2, 3 or 4.

3. The compound of Formula (II) in claim 2, wherein said compound is a compound of Formula (III): 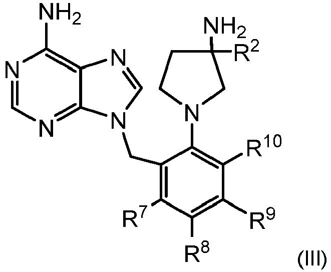 or an enantiomer, an enantiomeric mixture, or a pharmaceutically acceptable salt thereof.

4. The compound of Formula (III) in claim 3, wherein said compound is a compound of Formula (IV): 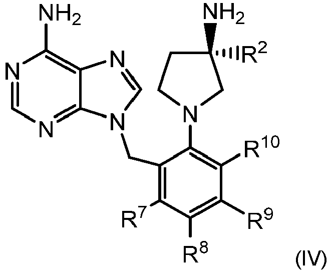 or an enantiomer, an enantiomeric mixture, or a pharmaceutically acceptable salt thereof.

5. The compound of any one of claims 1-4 or an enantiomer, an enantiomeric mixture, or a pharmaceutically acceptable salt thereof; wherein R² is

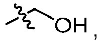

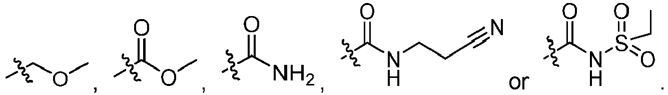

6. The compound of any one of claims 1-4 or an enantiomer, an enantiomeric mixture, or a pharmaceutically acceptable salt thereof; wherein R² is,

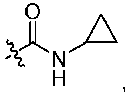

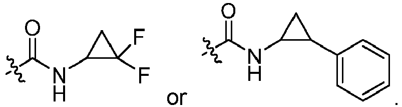

7. The compound of any one of claims 1-4 or an enantiomer, an enantiomeric mixture, or a pharmaceutically acceptable salt thereof; wherein R² is

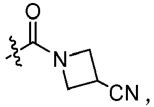

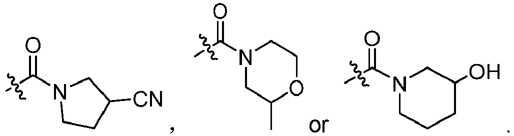

8. The compound of any one of claims 1-4 or an enantiomer, an enantiomeric mixture, or a pharmaceutically acceptable salt thereof; wherein R² is 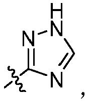 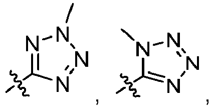 1H-tetrazolyl, 2H-tetrazoly, pyridyl, trifluoromethylpyridyl or phenyl.

9. The compound of any one of claims 1-4 or an enantiomer, an enantiomeric mixture, or a pharmaceutically acceptable salt thereof; wherein said compound is a compound of Formula (V):

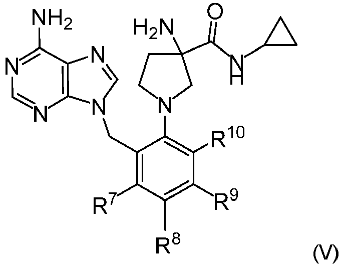

10. The compound of any one of claims 1-9 or a pharmaceutically acceptable salt thereof; wherein at least two of R⁷, R⁸, R⁹ and R¹⁰ are not hydrogen.

11. The compound of claim 10 or a pharmaceutically acceptable salt thereof;
wherein R⁷, R⁸, R⁹ and R¹⁰ are independently hydrogen, halo, -C₁₋₄alkyl, -haloC₁₋₆ alkyl, -hydroxyC₁₋₆ alkyl, -C₂₋₆ alkenyl, -C₃₋₈ cycloalkyl, -(CR^{a}R^{b})₁₋₄-NR¹⁷R¹⁸, or-(CR^{a}R^{b})₁₋₄-OR¹⁹; provided at least two of R⁷, R⁸, R⁹ and R¹⁰ are not hydrogen;
R^{a}, R^{b} and R¹⁷ are independently hydrogen or -C₁₋₄ alkyl;
R¹⁸ is hydrogen, -C₁₋₄ alkyl, -haloC₁₋₆ alkyl or -C₃₋₆ cycloalkyl;
alternatively, R¹⁷ and R¹⁸ together with N in the -NR¹⁷R¹⁸ moiety form a 4-10 membered monocyclic or bicyclic heterocyclic ring comprising 1-3 heteroatoms selected from nitrogen, oxygen and sulfur; wherein said heterocyclic ring is unsubstituted or substituted with 1-2 halo or -C₁₋₄ alkyl; and
R¹⁹ is hydrogen, -C₁₋₄ alkyl, -haloC₁₋₆ alkyl, -C₃₋₆ cycloalkyl, phenyl, 5-6 membered heterocyclyl or 5-6 membered heteroaryl; wherein said 5-6 membered heterocyclyl or 5-6 membered heteroaryl independently comprises 1-3 heteroatoms selected from nitrogen, oxygen and sulfur.

12. The compound of any one of claims 1-4 or an enantiomer, an enantiomeric mixture, or a pharmaceutically acceptable salt thereof; wherein said compound is a compound of Formula (V): 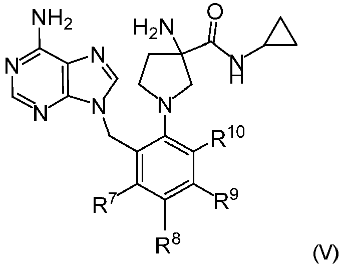 wherein R⁷, R⁸, R⁹ and R¹⁰ are independently hydrogen, halo, -haloC₁₋₆ alkyl, provided at least two of R⁷, R⁸, R⁹ and R¹⁰ are not hydrogen.

13. The compound according to claim 1 or an enantiomer, an enantiomeric mixture, or a pharmaceutically acceptable salt thereof; wherein said compound is selected from a compoundbelow, or a pharmaceutically acceptable salt thereof, (R)-3-amino-1-(2-((6-amino-9H-purin-9-yl)methyl)-3-bromo-5-chlorophenyl)-N-cyclopropylpyrrolidine-3-carboxamide, (R)-3-amino-1-(2-((6-amino-9H-purin-9-yl)methyl)-5-chloro-3-ethylphenyl)-N-cyclopropylpyrrolidine-3-carboxamide, (R)-3-amino-1-(2-((6-amino-9H-purin-9-yl)methyl)-5-chloro-3-(2,3-dihydrobenzofuran-5-yl)phenyl)-N-cyclopropylpyrrolidine-3-carboxamide, (R)-3-amino-1-(2-((6-amino-9H-purin-9-yl)methyl)-3-cyano-4-fluorophenyl)-N-cyclopropylpyrrolidine-3-carboxamide, (R)-3-amino-1-(2-((6-amino-9H-purin-9-yl)methyl)-3-ethynyl-4-fluorophenyl)-N-cyclopropylpyrrolidine-3-carboxamide, (R)-3-amino-1-(2-((6-amino-9H-purin-9-yl)methyl)-4-fluoro-3-(2,2,2-trifluoroethyl)phenyl) -N-cyclopropylpyrrolidine-3-carboxamide, (R)-3-amino-1-(2-((6-amino-9H-purin-9-yl)methyl)-5-chloro-3-(2,2,2-trifluoroethyl)phenyl)-N-cyclopropylpyrrolidine-3-carboxamide, (R)-3-amino-1-(2-((6-amino-9H-purin-9-yl)methyl)-3-cyclopropyl-4-fluorophenyl)-N-cyclopropylpyrrolidine-3-carboxamide, 3-amino-1-(2-((6-amino-9H-purin-9-yl)methyl)-4-fluoro-3-vinylphenyl)-N-cyclopropylpyrrolidine-3-carboxamide, (R)-3-amino-1-(2-((6-amino-9H-purin-9-yl)methyl)-3-ethyl-4-fluorophenyl)-N-cyclopropylpyrrolidine-3-carboxamide, (R)-3-amino-1-(2-((6-amino-9H-purin-9-yl)methyl)-3-methyl-4-(trifluoromethyl)phenyl)-N-cyclopropylpyrrolidine-3-carboxamide, (R)-3-amino-1-(2-((6-amino-9H-purin-9-yl)methyl)-3-ethyl-4-(trifluoromethyl)phenyl)-N-cyclopropylpyrrolidine-3-carboxamide, (R)-3-amino-1-(2-((6-amino-9H-purin-9-yl)methyl)-3-bromo-4-fluorophenyl)-N-cyclopropylpyrrolidine-3-carboxamide, (R)-3-amino-1-(2-((6-amino-9H-purin-9-yl)methyl)-4-fluorophenyl)-N-cyclopropylpyrrolidine-3-carboxamide, (R)-3-amino-1-(4-((6-amino-9H-purin-9-yl)methyl)-2,2-difluorobenzo[d][1,3]dioxol-5-yl)-N-cyclopropylpyrrolidine-3-carboxamide, (R)-3-amino-1-(2-((6-amino-9H-purin-9-yl)methyl)-5-chloro-3'-cyclohexyl-[1,1'-biphenyl]-3-yl)pyrrolidine-3-carboxamide, (R)-1-(4-(3-amino-1-(2-((6-amino-9H-purin-9-yl)methyl)-3-ethyl-4-fluorophenyl)pyrrolidine-3-carbonyl)piperazin-1-yl)prop-2-en-1-one, (R)-9-(2-(3-amino-3-(1H-1,2,4-triazol-3-yl)pyrrolidin-1-yl)-6-bromo-4-chlorobenzyl)-9H-purin-6-amine, (R)-(3-amino-1-(2-((6-amino-9H-purin-9-yl)methyl)-3-bromo-5-chlorophenyl)pyrrolidin-3-yl)methanol, (R)-3-amino-1-(6-((6-amino-9H-purin-9-yl)methyl)-5-bromo-4-fluoro-2,3-dihydrobenzofuran-7-yl)-N-cyclopropylpyrrolidine-3-carboxamide, (R)-3-amino-1-(2-((6-amino-9H-purin-9-yl)methyl)-3-bromo-6-methoxyphenyl )-N-cyclopropylpyrrolidine-3-carboxamide, 9-(6-(3-amino-3-(pyridin-2-yl)pyrrolidin-1-yl)-3-fluoro-2-(trifluoromethyl)benzyl)-9H-purin-6-amine, (R)-9-(6-(3-amino-3-(pyridin-2-yl)pyrrolidin-1-yl)-3-fluoro-2-(trifluoromethyl)benzyl)-9H-purin-6-amine, (R)-9-(2-(3-amino-3-(1H-1,2,4-triazol-3-yl)pyrrolidin-1-yl)-4-chloro-6-vinyl benzyl)-9H-purin-6-amine, (R)-9-(2-(3-amino-3-(1H-1,2,4-triazol-3-yl)pyrrolidin-1-yl)-4-chloro-6-ethylbenzyl)-9H-purin-6-amine, (S)-9-(6-(3-amino-3-phenylpyrrolidin-1-yl)-3-fluoro-2-(trifluoromethyl)benzyl)-9H-purin-6-amine, (R)-9-(2-(3-amino-3-(4-(trifluoromethyl)pyridin-2-yl)pyrrolidin-1-yl)-6-bromo-4-chlorobenzyl)-9H-purin-6-amine, (R)-9-(6-(3-amino-3-(2-(pyridin-2-yl)ethyl)pyrrolidin-1-yl)-3-fluoro-2-(trifluoromethyl)benzyl)-9H-purin-6-amine, methyl (R)-3-amino-1-(2-((6-amino-9H-purin-9-yl)methyl)-5-chloro-3-cyclobutylphenyl)pyrrolidine-3-carboxylate, (R)-2-((3-amino-1-(2-((6-amino-9H-purin-9-yl)methyl)-3-bromo-5-chlorophenyl)pyrrolidin-3-yl)methoxy)-N-cyclopropylacetamide, 3-amino-1-(5-((6-amino-9H-purin-9-yl)methyl)-6-bromobenzo[d][1,3]dioxol-4-yl)-N-cyclopropylpyrrolidine-3-carboxamide, (R)-9-(2-(3-amino-3-(methoxymethyl)pyrrolidin-1-yl)-6-bromo-4-chlorobenzyl)-9H-purin-6-amine, (R)-3-amino-1-(2-((6-amino-9H-purin-9-yl)methyl)-5-chloro-3-((R)-2,2,2-trifluoro-1-hydroxyethyl)phenyl)-N-cyclopropylpyrrolidine-3-carboxamide, ethyl (R)-3-(3-amino-1-(2-((6-amino-9H-purin-9-yl)methyl)-3-bromo-5-chlorophenyl)pyrrolidin-3-yl) propanoate, (R)-5-(2-(3-amino-1-(2-((6-amino-9H-purin-9-yl)methyl)-5-chloro-3-ethylphenyl)pyrrolidin-3-yl)ethyl)-N-methyl-1,3,4-thiadiazol-2-amine, (R)-3-(3-amino-1-(2-((6-amino-9H-purin-9-yl)methyl)-3-bromo-5-chlorophenyl)pyrrolidin-3-yl)-N-ethylpropanamide, 3-(3-amino-1-(2-((6-amino-9H-purin-9-yl)methyl)-3-bromo-5-chlorophenyl)pyrrolidin-3-yl)propanamide, (R)-3-(3-amino-1-(2-((6-amino-9H-purin-9-yl)methyl)-5-chloro-3-ethylphenyl)pyrrolidin-3-yl)-N-ethylpropanamide, (R)-9-(2-(3-(2-(2H-tetrazol-5-yl)ethyl)-3-aminopyrrolidin-1-yl)-6-bromo-4-chlorobenzyl)-9H-purin-6-amine, (R)-9-(2-(3-(2-(2H-tetrazol-5-yl)ethyl)-3-aminopyrrolidin-1-yl)-4-chloro-6-ethylbenzyl)-9H-purin-6-amine, (R)-3-amino-1-(2-((6-amino-9H-purin-9-yl)methyl)-3-bromo-4-(trifluoromethyl)phenyl)-N-cyclopropylpyrrolidine-3-carboxamide, 3-amino-1-(5-((6-amino-9H-purin-9-yl)methyl)-6-bromo-7-chloro-2,3-dihydro-1H-inden-4-yl)-N-cyclopropylpyrrolidine-3-carboxamide, (R)-3-amino-1-(2-((6-amino-9H-purin-9-yl)methyl)-3-bromo-5,6-dichlorophenyl)-N-cyclopropylpyrrolidine-3-carboxamide, 3-amino-1-(2-((6-amino-9H-purin-9-yl)methyl)-3-bromo-4,5,6-trichlorophenyl)-N-cyclopropylpyrrolidine-3-carboxamide, (R)-3-amino-1-(2-((6-amino-9H-purin-9-yl)methyl)-3-((dimethylamino)methyl)-4-fluorophenyl)-N-cyclopropylpyrrolidine-3-carboxamide, (R)-3-amino-1-(2-((6-amino-9H-purin-9-yl)methyl)-4-fluoro-3-(piperidin-1-ylmethyl)phenyl)-N-cyclopropylpyrrolidine-3-carboxamide, (R)-3-amino-1-(2-((6-amino-9H-purin-9-yl)methyl)-3-((dimethylamino)methyl)-4-(trifluoromethyl)phenyl)-N-cyclopropylpyrrolidine-3-carboxamide, (R)-3-amino-1-(2-((6-amino-9H-purin-9-yl)methyl)-3-((cyclopropyl(methyl)amino)methyl)-4-fluorophenyl)-N-cyclopropylpyrrolidine-3-carboxamide, (R)-3-amino-1-(2-((6-amino-9H-purin-9-yl)methyl)-3-((dimethylamino)methyl)-4-fluorophenyl)-N-((1S,2R)-2-phenylcyclopropyl)pyrrolidine-3-carboxamide, (R)-3-amino-1-(2-((6-amino-9H-purin-9-yl)methyl)-4-fluoro-3-(((R)-3-fluoropyrrolidin-1-yl)methyl)phenyl)-N-cyclopropylpyrrolidine-3-carboxamide, (R)-3-amino-1-(2-((6-amino-9H-purin-9-yl)methyl)-4-fluoro-3-(morpholinomethyl)phenyl)-N-((1S,2R)-2-phenylcyclopropyl)pyrrolidine-3-carboxamide, (R)-3-amino-1-(2-((6-amino-9H-purin-9-yl)methyl)-3-((3,3-difluoroazetidin-1-yl)methyl)-4-fluorophenyl)-N-cyclopropylpyrrolidine-3-carboxamide, (R)-3-amino-1-(2-((6-amino-9H-purin-9-yl)methyl)-4-fluoro-3-((3-fluoroazetidin-1-yl)methyl)phenyl)-N-cyclopropylpyrrolidine-3-carboxamide, (R)-3-amino-1-(2-((6-amino-9H-purin-9-yl)methyl)-3-((cyclopropylamino)methyl)-4-fluorophenyl)-N-cyclopropylpyrrolidine-3-carboxamide, (R)-3-amino-1-(2-((6-amino-9H-purin-9-yl)methyl)-3-((4,4-difluoropiperidin-1-yl)methyl)-4-fluorophenyl)-N-cyclopropylpyrrolidine-3-carboxamide, (R)-3-amino-1-(2-((6-amino-9H-purin-9-yl)methyl)-4-fluoro-3-((4-fluoropiperidin-1-yl)methyl)phenyl)-N-cyclopropylpyrrolidine-3-carboxamide, (R)-3-amino-1-(2-((6-amino-9H-purin-9-yl)methyl)-4-fluoro-3-(((2,2,2-trifluoroethyl)amino)methyl)phenyl)-N-cyclopropylpyrrolidine-3-carboxamide, (3R)-1-(3-((8-oxa-3-azabicyclo[3.2.1]octan-3-yl)methyl)-2-((6-amino-9H-purin-9-yl)methyl)-4-fluorophenyl)-3-amino-N-cyclopropylpyrrolidine-3-carboxamide, (R)-3-amino-1-(2-((6-amino-9H-purin-9-yl)methyl)-3-((cyclopropyl(ethyl)amino)methyl)-4-fluorophenyl)-N-cyclopropylpyrrolidine-3-carboxamide, (R)-3-amino-1-(2-((6-amino-9H-purin-9-yl)methyl)-4-chloro-3-((dimethylamino)methyl)phenyl)-N-cyclopropylpyrrolidine-3-carboxamide, (R)-3-amino-1-(2-((6-amino-9H-purin-9-yl)methyl)-4-fluoro-3-(pyrrolidin-1-ylmethyl)phenyl)-N-cyclopropylpyrrolidine-3-carboxamide, (R)-3-amino-1-(2-((6-amino-9H-purin-9-yl)methyl)-3-(azetidin-1-ylmethyl)-4-(trifluoromethyl)phenyl)-N-cyclopropylpyrrolidine-3-carboxamide, (R)-3-amino-1-(2-((6-amino-9H-purin-9-yl)methyl)-3-((3,3-dimethylpyrrolidin-1-yl)methyl)-4-fluorophenyl)-N-cyclopropylpyrrolidine-3-carboxamide, (R)-3-amino-1-(2-((6-amino-9H-purin-9-yl)methyl)-4-fluoro-3-((3-methylazetidin-1-yl)methyl)phenyl)-N-cyclopropylpyrrolidine-3-carboxamide, (R)-3-amino-1-(2-((6-amino-9H-purin-9-yl)methyl)-4-fluoro-3-(morpholinomethyl)phenyl)-N-cyclopropylpyrrolidine-3-carboxamide, (R)-3-amino-1-(2-((6-amino-9H-purin-9-yl)methyl)-3-((3,3-difluoropyrrolidin-1-yl)methyl)-4-fluorophenyl)-N-cyclopropylpyrrolidine-3-carboxamide, (R)-3-amino-1-(2-((6-amino-9H-purin-9-yl)methyl)-3-((diethylamino)methyl)-4-fluorophenyl)-N-cyclopropylpyrrolidine-3-carboxamide, (R)-3-amino-1-(2-((6-amino-9H-purin-9-yl)methyl)-4-fluoro-3-(((S)-3-fluoropyrrolidin-1-yl)methyl)phenyl)-N-cyclopropylpyrrolidine-3-carboxamide, (R)-3-amino-1-(2-((6-amino-9H-purin-9-yl)methyl)-3-((3,3-difluoropiperidin-1-yl)methyl)-4-fluorophenyl)-N-cyclopropylpyrrolidine-3-carboxamide, (R)-3-amino-1-(2-((6-amino-9H-purin-9-yl)methyl)-3-(azetidin-1-ylmethyl)-4-fluorophenyl)-N-cyclopropylpyrrolidine-3-carboxamide, methyl (R)-(3-(3-amino-3-(cyclopropylcarbamoyl)pyrrolidin-1-yl)-2-((6-amino-9H-purin-9-yl)methyl)-6-fluorobenzyl)carbamate, (R)-3-amino-1-(2-((6-amino-9H-purin-9-yl)methyl)-6-chloro-3-((dimethylamino)methyl)-4-fluorophenyl)-N-cyclopropylpyrrolidine-3-carboxamide, (R)-(3-amino-1-(2-((6-amino-9H-purin-9-yl)methyl)-4,6-dichloro-3-ethylphenyl)pyrrolidin-3-yl)(1,1-dioxidothiomorpholino)methanone, (R)-3-amino-1-(2-((6-amino-9H-purin-9-yl)methyl)-4,6-dichloro-3-((dimethylamino)methyl)phenyl)-N-cyclopropylpyrrolidine-3-carboxamide, (R)-3-amino-1-(2-((6-amino-9H-purin-9-yl)methyl)-6-chloro-4-fluoro-3-(morpholinomethyl)phenyl)-N-cyclopropylpyrrolidine-3-carboxamide, (R)-3-amino-1-(2-((6-amino-9H-purin-9-yl)methyl)-4-fluoro-3-(hydroxymethyl) phenyl)-N-cyclopropylpyrrolidine-3-carboxamide, (R)-3-amino-1-(2-((6-amino-9H-purin-9-yl)methyl)-4-fluoro-3-(methoxymethyl) phenyl)-N-cyclopropylpyrrolidine-3-carboxamide , (R)-3-amino-1-(2-((6-amino-9H-purin-9-yl)methyl)-4-chloro-3-(methoxymethyl)phenyl)-N-cyclopropylpyrrolidine-3-carboxamide, (3R)-3-amino-1-(2-((6-amino-9H-purin-9-yl)methyl)-4-chloro-3-(((tetrahydrofuran-3-yl)oxy)methyl)phenyl)-N-cyclopropylpyrrolidine-3-carboxamide, (R)-3-amino-1-(2-((6-amino-9H-purin-9-yl)methyl)-4-fluoro-3-((methoxy-d3)methyl)phenyl)-N-cyclopropylpyrrolidine-3-carboxamide, (R)-3-amino-1-(2-((6-amino-9H-purin-9-yl)methyl)-4-chloro-3-((methoxy-d3)methyl)phenyl)-N-cyclopropylpyrrolidine-3-carboxamide, (R)-3-amino-1-(2-((6-amino-9H-purin-9-yl)methyl)-4-fluoro-3-((S)-2,2,2-trifluoro-1-hydroxyethyl)phenyl)-N-cyclopropylpyrrolidine-3-carboxamide , (R)-9-(2-(3-amino-3-(1H-tetrazol-5-yl)pyrrolidin-1-yl)-6-bromo-4-chlorobenzyl)-9H-purin-6-amine, (R)-9-((3-(3-amino-3-(2H-tetrazol-5-yl)pyrrolidin-1-yl)-5-chloro-3'-cyclohexyl-[1,1'-biphenyl]-2-yl)methyl)-9H-purin-6-amine, (R)-9-(6-(3-amino-3-(1H-1,2,4-triazol-3-yl)pyrrolidin-1-yl)-2,3-dichlorobenzyl)-9H-purin-6-amine, (R)-3-amino-1-(2-((6-amino-9H-purin-9-yl)methyl)-4-chloro-3-((difluoromethoxy)methyl)phenyl)-N-cyclopropylpyrrolidine-3-carboxamide, (R)-3-amino-1-(2-((6-amino-9H-purin-9-yl)methyl)-3-bromo-4-(trifluoromethyl)phenyl)-N-(2-cyanoethyl)pyrrolidine-3-carboxamide, (R)-3-amino-1-(2-((6-amino-9H-purin-9-yl)methyl)-4,5-dichloro-3-((dimethylamino)methyl)phenyl)-N-cyclopropylpyrrolidine-3-carboxamide, (R)-3-amino-1-(2-((6-amino-9H-purin-9-yl)methyl)-5,6-dichloro-3-((dimethylamino)methyl)phenyl)-N-cyclopropylpyrrolidine-3-carboxamide, (R)-9-(2-(3-amino-3-(1-methyl-1H-tetrazol-5-yl)pyrrolidin-1-yl)-6-bromo-4-chlorobenzyl)-9H-purin-6-amine, (R)-9-(2-(3-amino-3-(2-methyl-2H-tetrazol-5-yl)pyrrolidin-1-yl)-6-bromo-4-chlorobenzyl)-9H-purin-6-amine, (R)-3-amino-1-(4-((6-amino-9H-purin-9-yl)methyl)-5-ethyl-2'-fluoro-[1,1'-biphenyl]-3-yl)-N-cyclopropylpyrrolidine-3-carboxamide, (R)-3-amino-1-(2-((6-amino-9H-purin-9-yl)methyl)-3-ethyl-5-(thiophen-2-yl)phenyl)-N-cyclopropylpyrrolidine-3-carboxamide, (R)-3-amino-1-(2-((6-amino-9H-purin-9-yl)methyl)-5-(thiophen-2-yl)-3-(trifluoromethyl) phenyl)-N-cyclopropylpyrrolidine-3-carboxamide, (R)-3-amino-1-(2-((6-amino-9H-purin-9-yl)methyl)-3-ethyl-5-(pyridin-3-yl)phenyl)-N-cyclopropylpyrrolidine-3-carboxamide, (R)-3-amino-1-(2-((6-amino-9H-purin-9-yl)methyl)-3-ethyl-5-(1-methyl-1H-pyrazol-4-yl)phenyl)-N-cyclopropylpyrrolidine-3-carboxamide, 3-amino-1-(2-((6-amino-9H-purin-9-yl)methyl)-4-chloro-3-cyclopropylphenyl)-N-cyclopropylpyrrolidine-3-carboxamide, (R)-3-amino-1-(2-((6-amino-9H-purin-9-yl)methyl)-3-ethyl-5-(thiazol-4-yl)phenyl)-N-cyclopropylpyrrolidine-3-carboxamide, (R)-3-amino-1-(2-((6-amino-9H-purin-9-yl)methyl)-3-ethyl-5-(pyridin-2-yl)phenyl)-N-cyclopropylpyrrolidine-3-carboxamide, (R)-3-amino-1-(2-((6-amino-9H-purin-9-yl)methyl)-3-ethyl-5-(pyrazin-2-yl)phenyl)-N-cyclopropylpyrrolidine-3-carboxamide, (R)-3-amino-1-(2-((6-amino-9H-purin-9-yl)methyl)-5-chloro-4-cyano-3-ethylphenyl)-N-cyclopropylpyrrolidine-3-carboxamide, (R)-3-amino-1-(2-((6-amino-9H-purin-9-yl)methyl)-5-chloro-4-fluoro-3-(morpholinomethyl)phenyl)-N-cyclopropylpyrrolidine-3-carboxamide , (R)-3-amino-1-(2-((6-amino-9H-purin-9-yl)methyl)-3-chloro-4-fluoro-5-(morpholinomethyl)phenyl)-N-cyclopropylpyrrolidine-3-carboxamide , (R)-3-amino-1-(2-((6-amino-9H-purin-9-yl)methyl)-4-chloro-3-cyanophenyl)-N-cyclopropylpyrrolidine-3-carboxamide, (R)-3-amino-1-(2-((6-amino-9H-purin-9-yl)methyl)-4,5-dichloro-3-(morpholinomethyl)phenyl)-N-cyclopropylpyrrolidine-3-carboxamide, (S)-(3-amino-1-(1-((6-amino-9H-purin-9-yl)methyl)naphthalen-2-yl)pyrrolidin-3-yl)methanol, (S)-(3-amino-1-(2-((6-amino-9H-purin-9-yl)methyl)-3-bromo-5-chlorophenyl)pyrrolidin-3-yl)methanol, (R)-(3-amino-1-(2-((6-amino-9H-purin-9-yl)methyl)-5-chloro-3-ethylphenyl)pyrrolidin-3-yl)methanol, (R)-9-((2-(3-amino-3-(methoxymethyl)pyrrolidin-1-yl)naphthalen-1-yl)methyl)-9H-purin-6-amine , (R)-3-amino-1-(6-((6-amino-9H-purin-9-yl)methyl)-5-bromo-4-chloro-2,3-dihydrobenzofuran-7-yl)-N-cyclopropylpyrrolidine-3-carboxamide, (R)-3-amino-1-(6-((6-amino-9H-purin-9-yl)methyl)-4-fluoro-5-iodo-2,3-dihydrobenzofuran-7-yl)-N-cyclopropylpyrrolidine-3-carboxamide, (R)-3-amino-1-(7-((6-amino-9H-purin-9-yl)methyl)benzofuran-6-yl)-N-cyclopropylpyrrolidine-3-carboxamide, (R)-3-amino-1-(2-((6-amino-9H-purin-9-yl)methyl)-4-chloro-3-(ethylthio)phenyl)-N-cyclopropylpyrrolidine-3-carboxamide, (R)-3-amino-1-(7-((6-amino-9H-purin-9-yl)methyl)-2,2-dimethyl-2,3-dihydrobenzofuran-6-yl)-N-cyclopropylpyrrolidine-3-carboxamide, 3-amino-1-(5-((6-amino-9H-purin-9-yl)methyl)-6-bromo-2,3-dihydrobenzofuran-4-yl)-N-cyclopropylpyrrolidine-3-carboxamide, (R)-3-amino-1-(2-((6-amino-9H-purin-9-yl)methyl)-4-fluoro-3-(trifluoromethyl)phenyl)-N-cyclopropylpyrrolidine-3-carboxamide, (R)-3-amino-1-(2-((6-amino-9H-purin-9-yl)methyl)-4-fluoro-3-(trifluoromethyl)phenyl)-N-((1r,3R)-3-(methylthio)cyclobutyl)pyrrolidine-3-carboxamide, (R)-3-amino-1-(2-((6-amino-9H-purin-9-yl)methyl)-4-fluoro-3-(trifluoromethyl)phenyl)-N-(pyridazin-3-ylmethyl)pyrrolidine-3-carboxamide, (R)-3-amino-1-(2-((6-amino-9H-purin-9-yl)methyl)-4-fluoro-3-(trifluoromethyl)phenyl)-N-(2-cyano-2-methylpropyl)pyrrolidine-3-carboxamide, (R)-3-amino-1-(2-((6-amino-9H-purin-9-yl)methyl)-4-fluoro-3-(trifluoromethyl)phenyl)-N-((2-hydroxypyridin-3-yl)methyl)pyrrolidine-3-carboxamide, (R)-N-(2-(1H-imidazol-4-yl)ethyl)-3-amino-1-(2-((6-amino-9H-purin-9-yl)methyl)-4-fluoro-3-(trifluoromethyl)phenyl)pyrrolidine-3-carboxamide, (3R)-3-amino-1-(2-((6-amino-9H-purin-9-yl)methyl)-4-fluoro-3-(trifluoromethyl)phenyl)-N-((2,2-difluorocyclopropyl)methyl)pyrrolidine-3-carboxamide, (R)-3-amino-1-(2-((6-amino-9H-purin-9-yl)methyl)-4-fluoro-3-(trifluoromethyl)phenyl)-N-(3-cyclopropyl-1H-pyrazol-5-yl)pyrrolidine-3-carboxamide, (R)-3-amino-1-(2-((6-amino-9H-purin-9-yl)methyl)-4-fluoro-3-(trifluoromethyl)phenyl)-N-((R)-1-(tetrahydro-2H-pyran-4-yl)ethyl)pyrrolidine-3-carboxamide, (R)-3-amino-1-(2-((6-amino-9H-purin-9-yl)methyl)-4-fluoro-3-(trifluoromethyl)phenyl)-N-(2-cyano-2-methylpropyl)pyrrolidine-3-carboxamide, (3R)-3-amino-1-(2-((6-amino-9H-purin-9-yl)methyl)-4-fluoro-3-(trifluoromethyl)phenyl)-N-(6-oxaspiro[2.5]octan-1-yl)pyrrolidine-3-carboxamide, (R)-3-amino-1-(2-((6-amino-9H-purin-9-yl)methyl)-4-fluoro-3-(trifluoromethyl)phenyl)-N-((1R,2R)-2-(hydroxymethyl)cyclopropyl)pyrrolidine-3-carboxamide, (R)-(3-amino-1-(2-((6-amino-9H-purin-9-yl)methyl)-4-fluoro-3-(trifluoromethyl)phenyl)pyrrolidin-3-yl)(6,7-dihydro-[1,2,3]triazolo[1,5-a]pyrazin-5(4H)-yl)methanone, (3R)-3-amino-1-(2-((6-amino-9H-purin-9-yl)methyl)-4-fluoro-3-(trifluoromethyl)phenyl)-N-(tetrahydrofuran-3-yl)pyrrolidine-3-carboxamide, (3R)-3-amino-1-(2-((6-amino-9H-purin-9-yl)methyl)-4-fluoro-3-(trifluoromethyl)phenyl)-N-(5-methoxyspiro[2.3]hexan-1-yl)pyrrolidine-3-carboxamide, ((R)-3-amino-1-(2-((6-amino-9H-purin-9-yl)methyl)-4-fluoro-3-(trifluoromethyl)phenyl)pyrrolidin-3-yl)((S)-2-methylmorpholino)methanone, (R)-1-(3-amino-1-(2-((6-amino-9H-purin-9-yl)methyl)-4-fluoro-3-(trifluoromethyl)phenyl)pyrrolidine-3-carbonyl)azetidine-3-carbonitrile, (R)-3-amino-1-(2-((6-amino-9H-purin-9-yl)methyl)-4-fluoro-3-(trifluoromethyl)phenyl)-N-((2-aminopyrimidin-4-yl)methyl)pyrrolidine-3-carboxamide, (R)-(3-amino-1-(2-((6-amino-9H-purin-9-yl)methyl)-4-fluoro-3-(trifluoromethyl)phenyl)pyrrolidin-3-yl)(6,7-dihydroisoxazolo[4,3-c]pyridin-5(4H)-yl)methanone, ((R)-3-amino-1-(2-((6-amino-9H-purin-9-yl)methyl)-4-fluoro-3-(trifluoromethyl)phenyl)pyrrolidin-3-yl)((R)-2-methylmorpholino)methanone, (R)-2-(4-(3-amino-1-(2-((6-amino-9H-purin-9-yl)methyl)-4-fluoro-3-(trifluoromethyl)phenyl)pyrrolidine-3-carbonyl)piperazin-1-yl)acetonitrile, (R)-(3-amino-1-(2-((6-amino-9H-purin-9-yl)methyl)-4-fluoro-3-(trifluoromethyl)phenyl)pyrrolidin-3-yl)(6,7-dihydropyrazolo[1,5-a]pyrazin-5(4H)-yl)methanone, ((R)-3-amino-1-(2-((6-amino-9H-purin-9-yl)methyl)-4-fluoro-3-(trifluoromethyl)phenyl)pyrrolidin-3-yl)((R)-3-hydroxypiperidin-1-yl)methanone, (R)-3-amino-1-(2-((6-amino-9H-purin-9-yl)methyl)-4-fluoro-3-(trifluoromethyl)phenyl)-N-(3-oxo-3-(propylamino)propyl)pyrrolidine-3-carboxamide, (R)-3-amino-1-(2-((6-amino-9H-purin-9-yl)methyl)-4-fluoro-3-(trifluoromethyl)phenyl)-N-((1-ethyl-1H-1,2,4-triazol-5-yl)methyl)pyrrolidine-3-carboxamide, 1-((R)-3-amino-1-(2-((6-amino-9H-purin-9-yl)methyl)-4-fluoro-3-(trifluoromethyl)phenyl)pyrrolidine-3-carbonyl)pyrrolidine-3-carbonitrile, (S)-3-amino-1-(2-((6-amino-9H-purin-9-yl)methyl)-3-bromo-6-chlorophenyl)pyrrolidine-3-carboxylic acid, (R)-3-amino-1-(2-((6-amino-9H-purin-9-yl)methyl)-3-bromo-4,6-dichlorophenyl)-N-cyclopropylpyrrolidine-3-carboxamide, (R)-3-amino-1-(2-((6-amino-9H-purin-9-yl)methyl)-3,4-dichlorophenyl)-N-cyclopropylpyrrolidine-3-carboxamide, (R)-3-amino-1-(2-((6-amino-9H-purin-9-yl)methyl)-4-chloro-3-ethylphenyl)-N-(2-(phenylsulfonyl)ethyl)pyrrolidine-3-carboxamide, (R)-3-amino-1-(2-((6-amino-9H-purin-9-yl)methyl)-4-bromo-3-(trifluoromethyl)phenyl)-N-cyclopropylpyrrolidine-3-carboxamide, (R)-3-amino-1-(2-((6-amino-9H-purin-9-yl)methyl)-3-bromo-4-(trifluoromethoxy)phenyl)-N-cyclopropylpyrrolidine-3-carboxamide, (R)-3-amino-1-(2-((6-amino-9H-purin-9-yl)methyl)-3-bromo-5-chlorophenyl)pyrrolidine-3-carboxamide, (R)-3-amino-1-(2-((6-amino-9H-purin-9-yl)methyl)-4-chloro-3-ethylphenyl)-N-(2-(ethylsulfonyl)ethyl)pyrrolidine-3-carboxamide, (R)-3-amino-1-(2-((6-amino-9H-purin-9-yl)methyl)-5-chloro-3-(5,6,7,8-tetrahydronaphthalen-1-yl)phenyl)-N-cyclopropylpyrrolidine-3-carboxamide, (R)-(3-amino-1-(2-((6-amino-9H-purin-9-yl)methyl)-5-chloro-3-ethylphenyl)pyrrolidin-3-yl)(1,1-dioxidothiomorpholino)methanone, 3-amino-1-(2-((6-amino-9H-purin-9-yl)methyl)-3,4-dichloro-6-fluorophenyl)-N-cyclopropylpyrrolidine-3-carboxamide, (R)-3-amino-1-(2-((6-amino-9H-purin-9-yl)methyl)-4-chloro-3-ethylphenyl)-N-(1,1-dioxidotetrahydro-2H-thiopyran-4-yl)pyrrolidine-3-carboxamide, (R)-3-amino-1-(2-((6-amino-9H-purin-9-yl)methyl)-6-chloro-3-ethylphenyl)-N-cyclopropylpyrrolidine-3-carboxamide, (R)-(3-amino-1-(2-((6-amino-9H-purin-9-yl)methyl)-4-chloro-3-ethylphenyl)pyrrolidin-3-yl)(1,1-dioxidothiomorpholino)methanone, (R)-9-(6-(3-amino-3-(1H-tetrazol-5-yl)pyrrolidin-1-yl)-3-chloro-2-iodobenzyl)-9H-purin-6-amine, 3-amino-1-(2-((6-amino-9H-purin-9-yl)methyl)-3-chloro-6-methylphenyl)-N-cyclopropylpyrrolidine-3-carboxamide, (R)-3-amino-1-(2-((6-amino-9H-purin-9-yl)methyl)-3-cyclopentyl-4-fluorophenyl)-N-cyclopropylpyrrolidine-3-carboxamide, (R)-3-amino-1-(2-((6-amino-9H-purin-9-yl)methyl)-3-bromo-5-chlorophenyl)-N-(ethyl-d5)pyrrolidine-3-carboxamide, 3-amino-1-(2-((6-amino-9H-purin-9-yl)methyl)-3-chloro-4-(trifluoromethyl)phenyl)-N-cyclopropylpyrrolidine-3-carboxamide, (R)-3-amino-1-(2-((6-amino-9H-purin-9-yl)methyl)-5-chloro-3-ethylphenyl)-N-isopropylpyrrolidine-3-carboxamide, (R)-3-amino-1-(2-((6-amino-9H-purin-9-yl)methyl)-4,6-dichloro-3-(methoxymethyl)phenyl)-N-cyclopropylpyrrolidine-3-carboxamide, (R)-3-amino-1-(2-((6-amino-9H-purin-9-yl)methyl)-4-chloro-3-vinylphenyl)-N-cyclopropylpyrrolidine-3-carboxamide, 3-amino-1-(2-((6-amino-9H-purin-9-yl)methyl)-3-chlorophenyl)-N-cyclopropylpyrrolidine-3-carboxamide, (R)-3-amino-1-(2-((6-amino-9H-purin-9-yl)methyl)-4-chloro-3-ethylphenyl)-N-cyclopropylpyrrolidine-3-carboxamide, (S)-3-amino-1-(1-((6-amino-9H-purin-9-yl)methyl)naphthalen-2-yl)-N-cyclopropylpyrrolidine-3-carboxamide, (R)-3-amino-1-(1-((6-amino-9H-purin-9-yl)methyl)-6-bromonaphthalen-2-yl)-N-cyclopropylpyrrolidine-3-carboxamide, (R)-3-amino-1-(2-((6-amino-9H-purin-9-yl)methyl)-3-bromo-4-methoxyphenyl)-N-cyclopropylpyrrolidine-3-carboxamide, (R)-3-amino-1-(2-((6-amino-9H-purin-9-yl)methyl)-3-ethylphenyl)-N-cyclopentylpyrrolidine-3-carboxamide, ethyl (R)-3-amino-1-(2-((6-amino-9H-purin-9-yl)methyl)-3-bromo-5-chlorophenyl)pyrrolidine-3-carboxylate.

14. A pharmaceutical composition comprising a compound according to any one of claims 1-13 or a pharmaceutically acceptable salt thereof, and one or more pharmaceutically acceptable carrier.

15. A combination comprising a compound according to any one of claims 1-13 or a pharmaceutically acceptable salt thereof, and one or more therapeutically active agent.

16. The combination according to claim 15, wherein said one or more therapeutically active agent is an anti-cancer agent, an analgesic or an antiinflammatory agent.

17. A compound according to any one of claims 1-13 or a pharmaceutically acceptable salt thereof, for use in a method for treating a disease or condition that benefit from or is treatable by inhibition of mixed lineage leukemia 1 (MLL1).

18. A compound for use according to claim 17, wherein said disease or condition that benefit from or is treatable by inhibition of MLL1 is selected from solid tumors, leukemia, myeloma, lymphoma and hypertension.

19. A compound for use according to claim 17, wherein said disease or condition that benefit from or is treatable by inhibition of MLL1 is breast cancer, cervical cancer, skin cancer, ovarian cancer, gastric cancer, prostate cancer, pancreatic cancer, lung cancer, hepatocellular carcinoma, head and neck cancer, peripheral nerve sheath tumor, osteosarcoma, multiple myeloma, neuroblastoma, leukemia, non-Hodgkin's lymphoma or pulmonary arterial hypertension.

20. A compound according to any one of claims 1-13 and optionally in combination with a second therapeutic agent, for use in the manufacture of a medicament for a disease or condition that benefit from or is treatable by inhibition of mixed lineage leukemia 1 (MLL1).

## Patentansprüche

1. Verbindung der Formel (I) 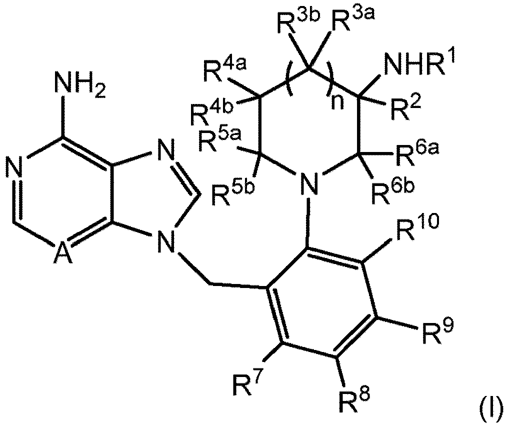 oder ein Enantiomer, ein Enantiomerengemisch oder ein pharmazeutisch akzeptables Salz davon, wobei:
A N ist;
R¹ H ist; oder
R¹ und R² zusammen mit NH einen 5-8-gliedrigen Heterocyclylrest bilden, der 1-2 Heteroatome, ausgewählt aus N, O und S, als Ringglieder enthält; wobei der 5-8-gliedrige Heterocyclylrest unsubstituiert oder mit einem Oxosubstituenten substituiert ist;
R² ausgewählt ist aus der Gruppe bestehend aus:
(i) -C₁₋₆-Alkyl, -Halo-C₁₋₆-Alkyl, -Hydroxy-C₁₋₆-Alkyl, -C₁₋₆-Alkoxy-C₁₋₆-Alkyl oder-C₃₋₈-Cycloalkoxy(C₁₋₆-Alkyl);
(ii) Cyano, -Cyano-C₁₋₆-Alkyl, -C₁₋₆-Alkylthio-c₁₋₆-Alkyl, -C₂₋₆-Alkenyl, -Halo-C₂₋₆-Alkenyl,-C₂₋₆-Alkinyl, -C₁₋₄-Alkyl-S-C₁₋₄-alkyl, -C₁₋₄-AlkylSO₂C₁₋₄-alkyl, -SO₂(₁₋₄-Alkyl) oder -C(C₁₋₄-Alkyl)=N-O(C₁₋₄-Alkyl);
(iii) -C₁₋₄-Alkylcarbonyl, -(CR^{a}R^{b})ₚ-C(=O)-OR^{10a} oder -C(=O)-(CR^{a}R^{b})_{q}R¹¹;
wobei R¹¹ C3-7-Cycloalkyl, 5-6-gliedriges Heterocyclyl oder 5-6-gliedriges Heteroaryl ist, die jeweils unabhängig voneinander unsubstituiert oder mit -C₁₋₆-Alkyl oder -C₁₋₆-Alkoxy substituiert sind; und das 5-6-gliedrige Heterocyclyl oder 5-6-gliedrige Heteroaryl unabhängig voneinander 1-3 Heteroatome, ausgewählt aus Stickstoff, Sauerstoff und Schwefel, umfasst;
(iv) -(CR^{a}R^{b})ᵣ-C(=O)-NR¹²R¹³ -(CR^{a}R^{b})ₛ-NR¹²R¹³, -(CR^{a}R^{b})₁₋₄-O-(CR^{a}R^{b}) ₁₋₄-OR^{10a}
oder
- (CR^{a}R^{b}) ₁₋₄-O-(CR^{a}R^{b}) ₁₋₄-C(=O)-NR¹²R¹³;
wobei R¹² Wasserstoff oder -C₁₋₆-Alkyl ist;
R¹³ Wasserstoff, C₁₋₆-Alkyl, -C₁₋₆AlkoxyC₁₋₆-Alkyl, -CyanoC₁₋₆-Alkyl;
-C₁₋₄AlkylSO₂R^{10b} ist, wobei R10b C₁₋₆-Alkyl oder -Phenyl ist; C₃₋₁₀ monozyklisches oder bizyklisches C₀₋₆-Alkyl, Phenyl ist, ein 5-10-gliedrige monozyklischer oder bizyklischer heterozyklischer Ring oder 5-9-gliedriges HeteroarylC₀₋₆-Alkyl ist;
der 5-10-gliedrige monozyklische oder bizyklische heterozyklische Ring oder der 5-9-gliedrige Heteroarylrest unabhängig 1-4 Heteroatome aufweisen, die ausgewählt sind aus Stickstoff, Sauerstoff und Schwefel; und
das monozyklische oder bizyklische C₃₋₁₀-Cycloalkyl , der 5-10-gliedrige monozyklische oder bizyklische heterozyklische Ring oder der 5-9-gliedrige Heteroarylrest unabhängig mit 1-2-C₁₋₃-Alkyl, -HydroxyC₁₋₆-Alkyl,-C₁₋₄-Alkoxy, Halo, Hydroxyl, Phenyl oder -S(C₁₋₄-Alkyl) unsubstituiert oder substituiert sind;
oder R¹² und R¹³ zusammen einen monozyklischen oder bizyklischen heterozyklischen Ring bilden, der 1-4 Heteroatome aufweist, die ausgewählt sind aus Stickstoff, Sauerstoff und Schwefel; und mit -C₁₋₄-Alkyl, Hydroxy, Cyano,
- CyanoC₁₋₆-Alkyl, -SO₂ oder -C₂₋₄-Alkenylcarbonyl substituiert ist;
(v) 5-6-gliedriges HeterocyklylC₀₋₆Alkyl oder 5-6-gliedriges Heterocyklyl(haloC₁₋₄-Alkyl) ist, wobei jeder Heterocyclylrest unsubstituiert oder durch Oxo substituiert ist; und wobei jeder Heterocyclylrest 1-3 Heteroatome umfasst, die ausgewählt sind aus Stickstoff, Sauerstoff und Schwefel; und
(vi) Phenyl, 5-9-gliedrigem HeteroarylC₀₋₆-Alkyl oder 5-9-gliedrigem Heteroaryl/haloC₁₋₄Alkyl), wobei jeder Phenyl- oder Heteroarylrest unabhängig unsubstituiert oder substituiert ist durch
-C₁₋₄-Akyl, -HaloC₁₋₄-Alkyl, -HydroxyC₁₋₄-Alkyl, -C₁₋₄-Alkoxy, -HaloC₁₋₄-Alkoxy, Halo, Hydroxy, Cyano, Oxido, Amino, -C₁₋₄-Alkylamino, -C₁₋₄-Dialkylamino,-AminocarbonylC₀₋₆-Alkyl, -C₁₋₄-AlkylaminocarbonylC₀₋₆-Alkyl, -BiC₁₋₄ AlkylaminocabonylC₀₋₆-Alkyl oder C³⁻⁷-Cycloalkyl;
wobei jeder der Heteroarylreste 1-4- Heteroatome umfasst, die ausgewählt sind aus Stickstoff, Sauerstoff und Schwefel;
R^{3a}, R^{3b}, R^{4a}, R^{4b}, R^{5a}, R^{5b}, R^{6a} und R^{6b} unabhängig Wasserstoff, Halo, Cyano, Hydroxyl sind,
-C₁₋₆-Akyl, -HaloC₁₋₆-Alkyl, -HydroxyC₁₋₆-Alkyl, -C₁₋₆-Alkoxy, -C₁₋₆AlkoxyC₁₋₆Alkyl, Aryl, -C(=O)-OR¹⁴ oder -(CR^{a}R^{b})s-C(=O-NR¹⁵R¹⁶ sind; oder
R^{3a}, R^{3b}, R^{4a}, R^{4b}, R^{5a}, R^{5b}, R^{6a} und R^{6b} einen Oxo-Substituenten bilden;
R⁷, R⁸, R⁹ und R¹⁰ unabhängig Stickstoff, Halo, -C₁₋₄-Alkyl, -Halo-C₁₋₆-Alkyl,
-Hydroxy-C₁₋₆-Alkyl, Cyano, -Cyano-C₁₋₆-Alkyl, -C₂₋₆-Alkenyl, ,-C₂₋₆-Alkinyl, -C₁₋₆-Alkylthio,-(CR^{a}R^{b})₁₋₄-NR¹⁷R¹⁸ sind;
-(CR^{a}R^{b})₁₋₄NR¹⁷-C(O)-OR¹⁸, -(CR^{a}R^{b})₁₋₄NR¹⁷-C(O)-OR¹⁹, -C₃₋₈-Cycloalkoxyl, Phenyl oder 5-6-gliedriges Heteroaryl mit 1-3 Heteroatomen ist, die ausgewählt sind aus Stickstoff, Sauerstoff und Schwefel; wobei das C₃₋₈-Cycloalkoxyl, Phenyl oder 5-6-gliedrige Heteroaryl unabhängig mit 1-2 R²⁰ substituiert werden und vorausgesetzt, R⁷ und/oder R⁸ und/oder R⁹ und/oder R¹⁰ nicht Stickstoff ist;
alternativ R⁷, R⁸ und R⁹, und R⁹ zusammen mit dem Phenylring, an den sie angeheftet sind, einen 9-10-gliedrigen Benzo-Fusionierten Kohlenstoffring oder Benzo-fusionierten Heteroring bilden, der 1-3 Heteroatome aufweist, die ausgewählt sind aus Stickstoff, Sauerstoff und Schwefel;
wobei der Benzo-Fusionierte Kohlenstoffring oder Benzo-fusionierte Heteroring unabhängig oder abhängig mit 1-2-Halo oder C₁₋₄-Alkyl substituiert sind;
R^{a}, R^{b}, R^{10a}, R¹⁴, R¹⁵, R¹⁶ und R¹⁷ unabhängig Wasserstoff oder -C₁₋₄-Alkyl sind;
R¹⁸ Wasserstoff, -C₁₋₄-Alkyl, -Halo-C₁₋₆-Alkyl oder-C₃₋₆-Cycloalkoxyl ist;
alternativ R¹⁷ und R¹⁸ zusammen mit dem N in dem -NR¹⁷R¹⁸ -Rest einen 4-10-gliedrigen monozyklischen oder bizyklischen heterozyklischen Ring bilden, der 1-3 Heteroatome aufweist, die ausgewählt sind aus Stickstoff, Sauerstoff und Schwefel; wobei der 4-10-gliedrige monozyklische oder bizyklische heterozyklische Ring mit 1-2-Halo oder C₁₋₄-Alkyl substituiert oder unsubstituiert ist;
R¹⁹ Wasserstoff, -C₁₋₄-Alkyl, -Halo-C₁₋₆-Alkyl, -C₃₋₆-Cycloalkoxyl, Phenyl, 5-6-gliedriges Heterocyklyl oder 5-6-gliedriges Heteroaryl ist; wobei das 5-6-gliedrige Heterocyklyl oder 5-6-gliedrige Heteroaryl unabhängig 1-3 Heteroatome aufweist, die ausgewählt sind aus Stickstoff, Sauerstoff und Schwefel;
R²⁰ -C₁₋₄-Alkyl, -Halo oder -C₃₋₆-Cycloalkyl ist; oder zwei R20 zusammen einen 5-6-gliedrigen Ring bilden, der 0-2 Heteroatme aufweist, die ausgewählt sind aus Stickstoff, Sauerstoff und Schwefel;
n 0 oder 1 ist; und
p, q, r und s unabhängig 0, 1, 2, 3 oder 4 sind.

2. Verbindung der Formel (I) nach Anspruch 1, wobei die Verbindung eine Verbindung der Formel (II) 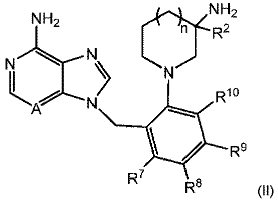 oder ein Enantiomer, ein Enantiomerengemisch oder ein pharmazeutisch akzeptables Salz davon ist, wobei:
R² ausgewählt ist aus der Gruppe bestehend aus:
(i) -Hydroxy-C₁₋₆-Alkyl, -C₁₋₆-Alkoxy-C₁₋₆-Alkyl oder -(CR^{a}R^{b})ₚ-C(=O)-OR^{10a};
(ii) -(CR^{a}R^{b})ᵣ-C(=O)-NR¹²R¹³ -(CR^{a}R^{b})₁₋₄-O-(CR^{a}R^{b}) ₁₋₄-C(=O)-NR¹²R¹³; wobei
R¹² Wasserstoff oder -C₁₋₆-Alkyl ist;
R¹³ Wasserstoff, -C₁₋₆-Alkyl, -Cyano-C₁₋₆-Alkyl, -C₁₋₄AlkylSO₂R^{10b} ist, wobei R10b C₁₋₆-Alkyl oder -Phenyl ist; C₃₋₁₀ monozyklisches oder bizyklisches C₀₋₆-Alkyl, Phenyl ist, ein 5-10-gliedrige monozyklischer oder bizyklischer heterozyklischer Ring oder 5-9-gliedriges HeteroarylC₀₋₆-Alkyl ist; wobei der 5-10-gliedrige monozyklische oder bizyklische heterozyklische Ring oder der 5-9-gliedrige Heteroarylrest unabhängig 1-4 Heteroatome aufweisen, die ausgewählt sind aus Stickstoff, Sauerstoff und Schwefel; und
das monozyklische oder bizyklische C₃₋₁₀-Cycloalkyl , der 5-10-gliedrige monozyklische oder bizyklische heterozyklische Ring oder der 5-9-gliedrige Heteroarylrest unabhängig mit 1-2-C₁₋₃-Alkyl, -HydroxyC₁₋₆-Alkyl,-C₁₋₄-Alkoxy, Halo, Hydroxyl, Phenyl oder -S(C₁₋₄-Alkyl) unsubstituiert oder substituiert sind;
oder R¹² und R¹³ zusammen einen monozyklischen oder bizyklischen heterozyklischen Ring bilden, der 1-4 Heteroatome aufweist, die ausgewählt sind aus Stickstoff, Sauerstoff und Schwefel; und mit -C₁₋₄-Alkyl, Hydroxy, Cyano, -CyanoC₁₋₆-Alkyl, -SO₂ oder -C₂₋₄-Alkenylcarbonyl substituiert ist; und
(iii) Phenyl oder das 5-9-gliedrige HeteroarylC₀₋₆-Alkyl, wobei der Heteroarylrest substituiert oder unsubstituiert ist mit -C₁₋₄-Alkyl, -Halo-C₁₋₆-Alkyl, Amino, -C₁₋₄-Alkylamino, -C₁₋₄-Dialkylamino und der Heteroarylrest 1-4 Heteroatome aufweist, die ausgewählt sind aus Stickstoff, Sauerstoff und Schwefel;
R⁷, R⁸, R⁹ und R¹⁰ unabhängig Wasserstoff, Halo, -C₁₋₄-Alkyl, -Halo-C₁₋₆-Alkyl, -Hydroxy-C₁₋₆-Alkyl, Cyano, -Cyano-C₁₋₆-Alkyl, -C₂₋₆-Alkenyl, ,-C₂₋₆-Alkinyl, -C₁₋₆-Alkylthio,-(CR^{a}R^{b})₁₋₄-NR¹⁷R¹⁸ sind,
-(CR^{a}R^{b})₁₋₄NR¹⁷-C(O)-OR¹⁸, -(CR^{a}R^{b})₁₋₄NR¹⁷-C(O)-OR¹⁹, -C₃₋₈-Cycloalkoxyl, Phenyl oder 5-6-gliedriges Heteroaryl mit 1-3 Heteroatomen ist, die ausgewählt sind aus Stickstoff, Sauerstoff und Schwefel; und
wobei das C₃₋₈ Cylcoalkyl, Phenyl oder das 5-6-gliedrige Heteroaryl unabhängig mit 1-2 R²⁰ substituiert sind; und
vorausgesetzt, dass R⁷ und/oder R⁸ und/oder R⁹ und/oder R¹⁰ nicht Stickstoff ist;
alternativ R⁷, R⁸ und R⁹, und/oder R⁹ zusammen mit dem Phenylring, an den sie angeheftet sind, einen 9-10-gliedrigen Benzo-Fusionierten Kohlenstoffring oder Benzo-fusionierten Heteroring bilden, der 1-3 Heteroatome aufweist, die ausgewählt sind aus Stickstoff, Sauerstoff und Schwefel; wobei der Benzo-Fusionierte Kohlenstoffring oder Benzo-fusionierte Heteroring unabhängig oder abhängig mit 1-2-Halo oder -C₁₋₄-Alkyl substituiert sind;
R^{a}, R^{b}, R^{10a}, R¹⁴, R¹⁵, R¹⁶ und R¹⁷ unabhängig Wasserstoff oder -C₁₋₄-Alkyl sind;
R¹⁸ Wasserstoff, -C₁₋₄-Alkyl, -Halo-C₁₋₆-Alkyl oder-C₃₋₆-Cycloalkoxyl ist;
alternativ R¹⁷ und R¹⁸ zusammen mit dem N in dem -NR¹⁷R¹⁸ -Rest einen 4-10-gliedrigen monozyklischen oder bizyklischen heterozyklische Ring bilden, der 1-3 Heteroatome aufweist, die ausgewählt sind aus Stickstoff, Sauerstoff und Schwefel;
wobei der heterozyklische Ring mit 1-2-Halo oder C₁₋₄-Alkyl substituiert oder unsubstituiert ist;
R¹⁹ Wasserstoff, -C₁₋₄-Alkyl, -Halo-C₁₋₆-Alkyl, -C₃₋₆-Cycloalkoxyl, Phenyl, 5-6-gliedriges Heterocyklyl oder 5-6-gliedriges Heteroaryl ist; wobei das 5-6-gliedrige Heterocyklyl oder 5-6-gliedrige Heteroaryl unabhängig 1-3 Heteroatome aufweist, die ausgewählt sind aus Stickstoff, Sauerstoff und Schwefel;
R²⁰ -C₁₋₄-Alkyl, -Halo oder -C₃₋₆-Cycloalkyl ist; oder zwei R20 zusammen einen 5-6-gliedrigen Ring bilden, der 0-2 Heteroatme aufweist, die ausgewählt sind aus Stickstoff, Sauerstoff und Schwefel;
n 0 oder 1 ist; und
p, q, r und s unabhängig 0, 1, 2, 3 oder 4 sind.

3. Verbindung der Formel (II) in Anspruch 2, wobei die Verbindung eine Verbindung der Formel (III): 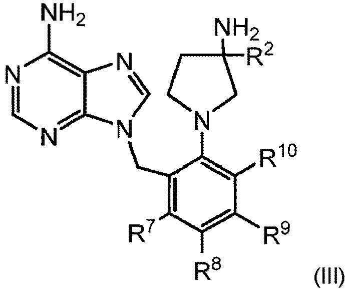 oder ein Enantiomer, ein Enantiomerengemisch oder ein pharmazeutisch akzeptables Salz davon ist.

4. Verbindung der Formel (III) in Anspruch 3, wobei die Verbindung eine Verbindung der Formel (IV): 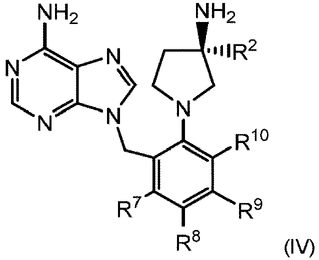 oder ein Enantiomer, ein Enantiomerengemisch oder ein pharmazeutisch akzeptables Salz davon ist.

5. Verbindung nach einem der Ansprüche 1-4 oder ein Enantiomer, ein Enantiomerengemisch oder ein pharmazeutisch akzeptables Salz davon, wobei R² 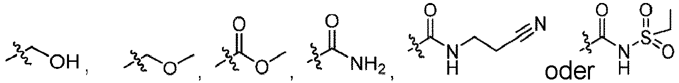 ist.

6. Verbindung nach einem der Ansprüche 1-4 oder ein Enantiomer, ein Enantiomerengemisch oder ein pharmazeutisch akzeptables Salz davon, wobei R² 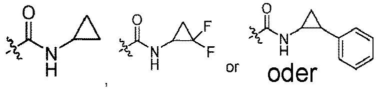 ist.

7. Verbindung nach einem der Ansprüche 1-4 oder ein Enantiomer, ein Enantiomerengemisch oder ein pharmazeutisch akzeptables Salz davon, wobei R² 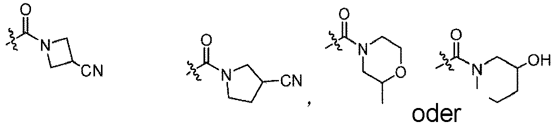 ist.

8. Verbindung nach einem der Ansprüche 1-4 oder ein Enantiomer, ein Enantiomerengemisch oder ein pharmazeutisch akzeptables Salz davon, wobei R² 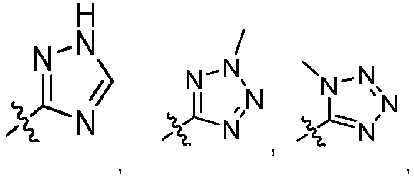 1H-Tetrazolyl, 2H-Tetrazolyl, Pyridil, Trifluormethylpyridyl oder Phenyl ist.

9. Verbindung nach einem der Ansprüche 1-4 oder ein Enantiomer, ein Enantiomerengemisch oder ein pharmazeutisch akzeptables Salz davon, wobei die Verbindung eine Verbindung der Formel (V) ist:

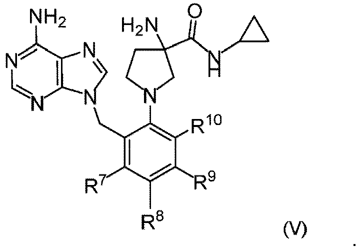

10. Verbindung nach einem der Ansprüche 1-9 oder ein pharmazeutisch akzeptables Salz davon; wobei mindestens zwei von R⁷, R⁸, R⁹ und R¹⁰ nicht Wasserstoff sind.

11. Verbindung nach Anspruch 10 oder ein pharmazeutisch akzeptables Salz davon; wobei R⁷, R⁸, R⁹ und R10 unabhängig Wasserstoff, Halo, -C₁₋₄-Alkyl, -Halo-_{C1}-₆-Alkyl, -Hydroxy-C₁₋₆-Alkyl, -C₂₋₆-Alkenyl, C₃₋₈-Cycloalkyl, ,-(CR^{a}R^{b})₁₋₄-NR¹⁷R¹⁸ oder -(CR^{a}R^{b})₁₋₄-OR¹⁹ sind, vorausgesetzt, dass mindestens zwei von R⁷, R⁸, R⁹ und R¹⁰ nicht Wasserstoff sind;
R^{a}, R^{b} und R¹⁷ unabhängig Wasserstoff oder -C₁₋₄-Alkyl sind;
R¹⁸ Wasserstoff, -C₁₋₄-Alkyl, -Halo-C₁₋₆-Alkyl oder-C₃₋₆-Cycloalkoxyl ist;
alternativ R¹⁷ und R¹⁸ zusammen mit dem N in dem -NR¹⁷R¹⁸ -Rest einen 4-10-gliedrigen monozyklischen oder bizyklischen heterozyklischen Ring bilden, der 1-3 Heteroatome aufweist, die ausgewählt sind aus Stickstoff, Sauerstoff und Schwefel;
wobei der heterozyklische Ring mit 1-2-Halo oder C₁₋₄-Alkyl substituiert oder unsubstituiert ist; und
R¹⁹ Wasserstoff, -C₁₋₄-Alkyl, -Halo-C₁₋₆-Alkyl, -C₃₋₆-Cycloalkoxyl, Phenyl, 5-6-gliedriges Heterocyklyl oder 5-6-gliedriges Heteroaryl ist; wobei das 5-6-gliedrige Heterocyklyl oder 5-6-gliedrige Heteroaryl unabhängig 1-3 Heteroatome aufweist, die ausgewählt sind aus Stickstoff, Sauerstoff und Schwefel.

12. Verbindung nach einem der Ansprüche 1-4 oder ein Enantiomer, ein Enantiomerengemisch oder ein pharmazeutisch akzeptables Salz davon, wobei die Verbindung eine Verbindung der Formel (V) ist: 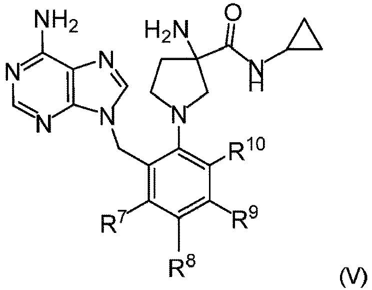 wobei R⁷, R⁸, R⁹ und R¹⁰ unabhängig Wasserstoff, Halo, -C₁₋₆-Alkyl, -Halo-_{C1-6}-Alkyl sind, vorausgesetzt, dass mindestens zwei von R⁷, R⁸, R⁹ und R¹⁰ nicht Wasserstoff sind.

13. Verbindung nach Anspruch 1 oder ein Enantiomer, ein Enantiomerengemisch oder ein pharmazeutisch akzeptables Salz davon, wobei die Verbindung ausgewählt ist aus einer der untenstehenden Verbindungen oder eines pharmazeutisch akzeptablen Salzes davon, (R)-3-Amino-1-(2-((6-amino-9H-purin-9-yl)methyl)-3-brom-5-chlorphenyl)-N-cyclopropylpyrrolidin-3-carboxamid, (R)-3-Amino-1-(2-((6-amino-9H-purin-9-yl)methyl)-5-chlor-3-ethylphenyl)-N-cyclopropylpyrrolidin-3-carboxamid, (R)-3-Amino-1-(2-((6-amino-9H-purin-9-yl)methyl)-5-chlor-3-(2,3-dihydrobenzofuran-5-yl)phenyl)-N-cyclopropylpyrrolidin-3-carboxamid, (R)-3-Amino-1-(2-((6-Amino-9H-purin-9-yl)methyl)-3-cyano-4-fluorphenyl)-N-cyclopropylpyrrolidin-3-carboxamide, (R)-3-Amino-1-(2-((6-Amino-9H-purin-9-yl)methyl)-3-ethinyl-4-fluorphenyl)-N-cyclopropylpyrrolidin-3-carboxamid,(R)-3-Amino-1-(2-((6-Amino-9H-purin-9-yl)methyl)-4-fluoro-3-(2,2,2-trifluoroethyl)phenyl)-N-cyclopropylpyrrolidin-3-carboxamid, (R)-3-Amino-1-(2-((6-amino-9H-purin-9-yl)methyl)-5-chlor-3-(2,2,2-trifluorethyl)phenyl)-N-cyclopropylpyrrolidin-3-carboxamid, (R)-3-Amino-1-(2-((6-amino-9H-purin-9-yl)methyl)-3-cyclopropyl-4-fluorphenyl)-N-cyclopropylpyrrolidin-3-carboxamid, 3-Amino-1-(2-((6-amino-9H-purin-9-yl)methyl)-4-fluor-3-vinylphenyl)-N-cyclopropylpyrrolidin-3-carboxamid,(R)-3-Amino-1-(2-((6-amino-9H-purin-9-yl)methyl)-3-ethyl-4-fluorphenyl)-N-cyclopropylpyrrolidin-3-carboxamide, (R)-3-Amino-1-(2-((6-amino-9H-purin-9-yl)methyl)-3-methyl-4-(trifluormethyl)phenyl)-N-cyclopropylpyrrolidin-3-carboxamide, (R)-3-Amino-1-(2-((6-amino-9H-purin-9-yl)methyl)-3-ethyl-4-(trifluormethyl)phenyl)-N-cyclopropylpyrrolidin-3-carboxamid, (R)-3-Amino-1-(2-((6-amino-9H-purin-9-yl)methyl)-3-brom-4-fluorphenyl)-N-cyclopropylpyrrolidin-3-carboxamide, (R)-3-Amino-1-(2-((6-amino-9H-purin-9-yl)methyl)-4-fluorphenyl)-N-cyclopropylpyrrolidin-3-carboxamid, (R)-3-Amino-1-(4-((6-amino-9H-purin-9-yl)methyl)-2, 2-Difluorbenzo[d][1,3]dioxol-5-yl)-N-cyclopropylpyrrolidin-3-carboxamid, (R)-3-Amino-1-(2-((6-amino-9H-purin-9-yl)methyl)-5-chlor-3'-cyclohexyl-[1, 1'-biphenyl]-3-yl)pyrrolidine-3-carboxamide, (R)-1-(4-(3-amino-1-(2-((6-amino-9H-purin-9-yl)methyl)-3-ethyl-4-Fluorphenyl)pyrrolidin-3-carbonyl)piperazin-1-yl)prop-2-en-1-on, (R)-9-(2-(3-Amino-3-(1H-1,2,4-triazol-3-yl)pyrrolidin-1-yl)-6-brom-4-chlorbenzyl)-9H-purin-6-amin, (R)-(3-amino-1-(2-((6-amino-9H-purin-9-yl)methyl)-3-bromo-5-chlorophenyl)pyrrolidin-3-yl)methanol, (R)-3-amino-1-(6-((6-amino-9H-purin-9-yl)methyl)-5-bromo-4-fluoro-2, 3-Dihydrobenzofuran-7-yl)-N-cyclopropylpyrrolidin-3-carboxamid, (R)-3-Amino-1-(2-((6-amino-9H-purin-9-yl)methyl)-3-brom-6-methoxyphenyl )-N-cyclopropylpyrrolidin-3-carboxamid, 9-(6-(3-Amino-3-(pyridin-2-yl)pyrrolidin-1-yl)-3-fluor-2-(trifluormethyl)benzyl)-9H-purin-6-amin, (R)-9-(6-(3-Amino-3-(pyridi n-2-yl)pyrrolidin-1-yl)-3-fluor-2-(trifluormethyl)benzyl)-9H-purin-6-amin, (R)-9-(2-(3-amino-3-(1H-1,2,4-triazol-3-yl)pyrrolidin-1-yl)-4-chloro-6-vinylbenzyl)-9H-purin-6-amine, (R)-9-(2-(3-amino-3-(1H-1,2,4-Triazol-3-yl)pyrrolidin-1-yl)-4-chlor-6-ethylbenzyl)-9H-purin-6-amin, (S)-9-(6-(3-Amino-3-phenylpyrrolidin-1-yl)-3-fluor-2-(trifluormethyl)benzyl)-9H-purin-6-amin, (R)-9-(2-(3-Amino-3-(4-(trifluormethyl) pyridin-2-yl)pyrrolidin-1-yl)-6-brom-4-chlorbenzyl)-9H-purin-6-amin, (R)-9-(6-(3-Amino-3-(2-(pyri din-2-yl)ethyl) pyrrolidin-1-yl)-3-fluor-2-(trifluormethyl)benzyl)-9H-purin-6-amin, Methyl(R)-3-amino-1-(2-((6-amino-9H-purin-9-yl)methyl)-5-chlor-3-cyclobutylphenyl)pyrrolidin-3-carboxylat, (R)-2-((3-Amino-1-(2-((6-amino-9H-purin-9-yl)methyl)-3-brom-5-chlorphenyl)pyrrolidin-3- yl)methoxy)-N-cyclopropylacetamid,3-Amino-1-(5-((6-amino-9H-purin-9-yl)methyl)-6-brombenzo[ d][1, 3]dioxol-4-yl)-N-cyclopropylpyrrolidin-3-carboxamide,(R)-9-(2-(3-Amino-3-(methoxymethyl)pyrrolidin-1-yl)-6-brom-4-chlorbenzyl)-9H-purin-6-amin, (R)-3-Amino-1-(2-((6-Amino-9H-purin-9-yl)methyl)-5-chlor-3-((R)-2,2,2-trifluor-1-hydroxyethyl)phenyl)-N-cyclopropylpyrrolidin-3-carboxamide, Ethyl-(R)-3-(3-amino-1-(2-((6-amino-9H-purin-9-yl)methyl)-3-brom-5-chlorphenyl)pyrrolidin-3-yl)propanoat, (R)-5-(2-(3-amino-1-(2-((6-amino-9H-purin-9-yl)methyl)-5-chloro-3-ethylphenyl)pyrrolidin-3-yl)ethyl)-N-methyl-1,3, 4-Thiadiazol-2-amin,(R)-3-(3-Amino-1-(2-((6-amino-9H-purin-9-yl)methyl)-3-brom-5-chlorphenyl)pyrrolidin-3-yl)-N-ethylpropanamid, 3-(3-Amino-1-(2-((6-amino-9H-purin-9-yl)methyl)-3-brom-5-chlorphenyl)pyrrolidin-3-yl)propanamid, (R)-3-(3-Amino-1-(2-((6-amino-9H-purin-9-yl)methyl)-5-chlor-3-ethylphenyl)pyrrolidin-3-yl)-N-ethylpropanamid, (R)-9-(2-(3-(2- (2H-tetrazol-5-yl)ethyl)-3-aminopyrrolidin-1-yl)-6-bromo-4-chlorobenzyl)-9H-purin-6-amin, (R)-9-(2-(3-(2-(2H-Tetrazol-5-yl)ethyl)-3-aminopyrrolidin-1-yl)-4-chlor-6-ethylbenzyl)-9H-Purin-6-amin,(R)-3-Amino-1-(2-((6-amino-9H-purin-9-yl)methyl)-3- brom-4-(trifluormethyl)phenyl)-N-cyclopropylpyrrolidin-3-carboxamide, 3-Amino-1-(5-((6-amino-9H-purin-9-yl)methyl)-6-brom-7-chloro-2,3-dihydro-1H-inden-4-yl)-N-cyclopropylpyrrolidin-3-carboxamid,(R)-3-Amino-1-(2-((6-amino-9H-purin-9-yl)methyl)-3-bromo-5,6-dichlorphenyl)-N-cyclopropylpyrrolidin-3-carboxamid, 3-Amino-1-(2-((6-amino-9H-purin-9-yl)methyl)-3-brom-4,5,6-trichlorphenyl)-N-cyclopropylpyrrolidin-3-carboxamid, (R)-3-Amino-1-(2-((6-amino-9H-purin-9- yl)methyl)-3-((dimethylamino)methyl)-4-fluorphenyl)-N-cyclopropylpyrrolidin-3-carboxamid, (R)-3-Amino-1-(2-((6-Amino-9H-purin-9-yl)methyl)-4-fluor-3-(piperidin-1-ylmethyl)phenyl)-N-cyclopropylpyrrolidin-3-carboxamide, (R)-3-Amino-1-(2-((6- amino-9H-purin-9-yl)methyl)-3-((dimethylamino)methyl)-4-(trifluormethyl)phenyl)-N-cyclopropylpyrrolidin-3-carboxamide, (R)-3-Amino-1-(2-((6-Amino-9H-purin-9- yl)methyl)-3-((Cyclopropyl(methyl)am ino)methyl)-4-fluorphenyl)-N-cyclopropylpyrrolidin-3-carboxamide, (R)-3-Amino-1-(2-((6-amino-9H-purin-9- yl)methyl)-3-((dimethylamino)methyl)-4-fluorphenyl)-N-((1S,2R)-2- phenylcyclopropyl)pyrrolidin-3-carboxamid, (R)-3-Amino-1-(2-((6-Amino-9H-purin- 9-yl)methyl)-4-fluor-3-(((R)-3-fluorpyrrolidin-1-yl)methyl)phenyl)-N-cyclopropylpyrrolidin-3-carboxamide, (R)-3-Amino-1-(2-((6-amino-9H-purin-9- yl)methyl)-4-fluor-3-(morpholinomethyl)phenyl)-N-((1S,2R)-2- phenylcyclopropyl)pyrrolidin-3-carboxamid, (R)-3-Amino-1-(2-((6-Amino-9H-purin-9-yl)methyl)-3-((3,3-difluorazetidin-1-yl)methyl)-4-fluorphenyl)-N-cyclopropylpyrrolidin-3-carboxamide, (R)-3-Amino-1-(2-((6-amino-9H-purin-9-yl)methyl)-4-fluor-3-((3-fluorazetidin-1-yl)methyl)phenyl)-N-cyclopropylpyrrolidin-3-Carboxamid, (R)-3-Amino-1-(2-((6-amino-9H-purin-9-yl)methyl)-3-((cyclopropylamino)methyl)-4-fluorphenyl)-N-cyclopropylpyrrolidin-3-carboxamid, (R)-3-Amino-1 -(2-((6-Amino-9H-purin-9-yl)methyl)-3-((4,4-difluoropiperidin-1 - yl)methyl)-4-fluorphenyl)-N-cyclopropylpyrrolidin-3-carboxamid, (R)-3-Amino-1-(2-((6-amino-9H-purin-9-yl)methyl)-4-fluor-3-((4-fluoropiperidin-1-yl)methyl)phenyl)-N-cyclopropylpyrrolidin-3-carboxamide, (R)-3-Amino-1-(2-((6-amino-9H-purin-9-yl)methyl)-4-fluor-3-(((2,2,2-trifluorethyl)amino)methyl)phenyl)-N-cyclopropylpyrrolidin-3-carboxamid, (3R)-1-(3-((8-oxa-3-azabicyclo[3. 2. 1]octan-3-yl)methyl)-2-((6-amino-9H-purin-9-yl)methyl)-4-fluorphenyl)-3-amino-N-cyclopropylpyrrolidin-3-carboxamide, (R)-3-Amino-1-(2-((6-amino-9H-purin-9-yl)methyl)-3-((cyclopropyl( ethyl)amino)methyl)-4-fluorphenyl)-N-cyclopropylpyrrolidin-3-carboxamide, (R)-3-Amino-1-(2-((6-amino-9H-purin-9-yl)methyl)-4-chlor-3-((dimethylamino)methyl)phenyl)-N-cyclopropylpyrrolidin-3-carboxamid, (R)-3-Amino-1-(2-((6-Amino-9H-purin-9-yl)methyl)-4-fluor-3-(pyrrolidin-1-ylmethyl)phenyl)-N-cyclopropylpyrrolidin-3-carboxamide, (R)-3-Amino-1-(2-((6-amino-9H-purin-9-yl)methyl)-3-( azetidin-1-ylmethyl)-4-(trifluormethyl)phenyl)-N-cyclopropylpyrrolidin-3-carboxamid, (R)-3-Amino-1-(2-((6-Amino-9H-purin-9-yl)methyl)-3-((3,3-dimethylpyrrolidin-1-yl)methyl)-4-fluorphenyl)-N-cyclopropylpyrrolidin-3-carboxamide, (R)-3-Amino-1-(2-((6-amino-9H-purin-9-yl)methyl)-4-fluor-3-((3-methylazetidin-1-yl)methyl)phenyl)-N-cyclopropylpyrrolidin-3-carboxamid, (R)-3-Amino-1-(2-((6-Amino-9H-purin-9-yl)methyl)-4-fluor-3-(morpholinomethyl)phenyl)-N-cyclopropylpyrrolidin-3-carboxamide, (R)-3-Amino-1-(2-((6-Amino-9H-purin-9-yl)methyl)-3-((3, 3-Difluorpyrrolidin-1-yl)methyl)-4-fluorphenyl)-N-cyclopropylpyrrolidin-3-carboxamide, (R)-3-Amino-1-(2-((6-amino- 9H-purin-9-yl)methyl)-3-((diethylamino)methyl)-4-fluorphenyl)-N-cyclopropylpyrrolidin-3-carboxamide, (R)-3-Amino-1-(2-((6-Amino-9H-purin-9- yl)methyl)-4-fluor-3-(((S)-3-fluorpyrrolidin-1-yl)methyl)phenyl)-N- cyclopropylpyrrolidin-3-carboxamide, (R)-3-Amino-1-(2-((6-Amino-9H-purin-9- yl)methyl)-3-((3, 3-Difluorpiperidin-1-yl)methyl)-4-fluorphenyl)-N-cyclopropylpyrrolidin-3-carboxamide, (R)-3-Amino-1-(2-((6-amino-9H-purin-9- yl)methyl)-3-(azetidin-1-ylmethyl)-4-fluorphenyl)-N-cyclopropylpyrrolidin-3-carboxamid, (R)-(3-(3-Amino-3-(cyclopropylcarbamoyl)pyrrolidin-1-yl)-2-((6-amino-9H-purin-9-yl)methyl)-6-fluorbenzyl)carbamat, (R)-3-Amino-1-(2-((6-amino-9H-purin-9-yl)methyl)-6-chlor-3-((dimethylamino)methyl)-4-fluorphenyl)-N-cyclopropylpyrrolidin-3-carboxamide, (R)-(3-Amino-1-(2-((6-amino-9H-purin-9-yl)methyl)-4, 6-Dichlor-3-ethylphenyl)pyrrolidin-3-yl)(1,1-Dioxidothiomorpholino)methanon, (R)-3-Amino-1-(2-((6-amino-9H-purin-9-yl)methyl)-4,6-dichlor-3-((dimethylamino)methyl)phenyl)-N-cyclopropylpyrrolidin-3- carboxamid, (R)-3-Amino-1-(2-((6-Amino-9H-purin-9-yl)methyl)-6-chlor-4-fluor-3-(morpholinomethyl)phenyl)-N-cyclopropylpyrrolidin-3-carboxamide, (R)-3-Amino-1-(2-((6-amino-9H-purin-9-yl)methyl)-4-fluor-3-(hydroxymethyl)phenyl)-N-cyclopropylpyrrolidin-3-carboxamide, (R)-3-Amino-1-(2-((6-amino-9H-purin-9-yl)methyl)-4-fluor-3-(methoxymethyl)phenyl)-N-cyclopropylpyrrolidin-3-carboxamid (R)-3-Amino-1-(2-((6-amino-9H-purin-9-yl)methyl)-4-chlor-3-(methoxymethyl)phenyl)-N-cyclopropylpyrrolidin-3-carboxamide, (3R)-3-Amino-1-(2-((6-amino-9H-purin-9-yl)methyl)-4-chlor-3-(((tetrahydrofuran-3-yl)oxy)methyl)phenyl)-N-cyclopropylpyrrolidin-3-carboxamid, (R)-3-Amino-1-(2-((6-amino-9H-purin-9-yl)methyl)-4-fluor-3-((methoxy-d3)methyl)phenyl)-N-cyclopropylpyrrolidin-3-carboxamide, (R)-3-Amino-1-(2-((6-amino-9H-purin-9-yl)methyl)-4-chlor-3-((methoxy-d3)methyl)phenyl)-N-cyclopropylpyrrolidin-3-carboxamid, (R)-3-Amino-1-(2-((6-amino-9H-purin-9-yl)methyl)-4-fluor-3-((S)-2,2,2-trifluor-1-hydroxyethyl)phenyl)-N-cyclopropylpyrrolidin-3-carboxamide , (R)-9-(2-(3-amino-3-(1H-tetrazol-5-yl)pyrrolidin-1-yl)-6-bromo-4-chlorobenzyl)-9H-purin-6-amine, (R)-9-((3-(3-amino-3-(2H-tetrazol-5-yl)pyrrolidin-1-yl)-5-chloro-3'-cyclohexyl-[1,1'-biphenyl]-2-yl)methyl)-9H-purin-6-amine, (R)-9-(6-(3-Amino-3-(1H-1,2,4-triazol-3-yl)pyrrolidin-1-yl)-2,3-dichlorbenzyl)-9H-purin-6-amin, (R)-3-Amino-1-(2-((6-amino-9H-purin-9-yl)methyl)-4-chlor-3-((difluormethoxy)methyl)phenyl)-N-cyclopropylpyrrolidin-3-carboxamide, (R)-3-Amino-1-(2-((6-amino-9H-purin-9-yl)methyl)-3-brom-4-(trifluormethyl)phenyl)-N-(2-cyanoethyl)pyrrolidin-3-carboxamid, (R)-3-Amino-1-(2-((6-amino-9H-purin-9-yl)methyl)-4,5-dichlor-3-((dimethylamino)methyl)phenyl)-N-cyclopropylpyrrolidin-3-carboxamid, (R)-3-Amino-1-(2-((6-amino-9H-purin-9-yl)methyl)-5, 6-Dichlor-3-((dimethylamino)methyl)phenyl)-N-cyclopropylpyrrolidin-3-carboxamide, (R)-9-(2-(3-Amino-3-(1-methyl-1H-tetrazol-5-yl)pyrrolidin-1-yl)-6-brom-4-chlorbenzyl)-9H-purin- 6-amin, (R)-9-(2-(3-amino-3-(2-methyl-2H-tetrazol-5-yl)pyrrolidin-1-yl)-6-bromo-4-chlorobenzyl)-9H-purin-6-amine, (R)-3-amino-1-(4-((6-amino-9H-purin-9-yl)methyl)-5- ethyl-2'-fluoro-[1, 1'-Biphenyl]-3-yl)-N-cyclopropylpyrrolidin-3-carboxamid, (R)-3-Amino-1-(2-((6-amino-9H-purin-9-yl)methyl)-3-ethyl-5-(thiophen-2-yl)phenyl)-N-cyclopropylpyrrolidin-3-carboxamide, (R)-3-Amino-1-(2-((6-Amino-9H-purin-9- yl)methyl)-5-(thiophen-2-yl)-3-(trifluormethyl)phenyl)-N-cyclopropylpyrrolidin-3-carboxamid, (R)-3-Amino-1-(2-((6-Amino-9H-purin-9-yl)methyl)-3-ethyl-5-(pyridin-3-yl)phenyl)-N-cyclopropylpyrrolidin-3-carboxamide, (R)-3-Amino-1-(2-((6-amino-9H-purin-9-yl)methyl)-3-ethyl-5-(1-methyl-1H-pyrazol-4-yl)phenyl)-N-cyclopropylpyrrolidin-3-carboxamide, 3-Amino-1-(2-((6-amino-9H-purin-9-yl)methyl)-4-chlor-3-cyclopropylphenyl)-N-cyclopropylpyrrolidin-3-carboxamid, (R)-3-Amino-1-(2-((6-amino-9H-purin-9-yl)methyl)-3-ethyl-5-(thiazol-4-yl)phenyl)-N-cyclopropylpyrrolidin-3-carboxamide, (R)-3-Amino-1-(2-((6-Amino-9H-purin-9-yl)methyl)-3-ethyl-5-(pyridin-2-yl)phenyl)-N-cyclopropylpyrrolidin-3-carboxamid, (R)-3-Amino-1-(2-((6-amino-9H-purin-9-yl)methyl)-3-ethyl-5-(pyrazin-2-yl)phenyl)-N-cyclopropylpyrrolidin-3-carboxamide, (R)-3-amino-1-(2-((6-amino-9H-purin-9-yl)methyl)-5-chloro-4-cyano-3-ethylphenyl)-N-cyclopropylpyrrolidine-3-carboxamide, (R)-3-amino-1-(2-((6-amino-9H-purin-9-yl)methyl)-5-chloro-4-fluoro-3-(Morpholinomethyl)phenyl)-N-cyclopropylpyrrolidin-3-carboxamide, (R)-3-Amino-1-(2-((6-amino-9H-purin-9-yl)methyl)-3-chlor-4-fluor-5-(morpholinomethyl)phenyl)-N-cyclopropylpyrrolidin-3-carboxamide (R)-3-Amino-1-(2-((6-amino-9H-purin-9-yl)methyl)-4-chlor-3-cyanophenyl)-N-cyclopropylpyrrolidin-3-carboxamid, (R)-3-amino-1-(2-((6-amino-9H-purin-9-yl)methyl)-4,5-dichloro-3-(Morpholinomethyl)phenyl)-N-cyclopropylpyrrolidin-3-carboxamid, (S)-(3-Amino-1-(1-((6-amino-9H-purin-9-yl)methyl)naphthalen-2-yl)pyrrolidin-3-yl)methanol, (S)-(3-Amino-1-(2-((6-amino-9H-purin-9-yl)methyl)-3-brom-5-chlorphenyl)pyrrolidin-3-yl)methanol, (R)-(3-Amino-1-(2-((6-amino-9H-purin-9-yl)methyl)-5-chlor-3-ethylphenyl)pyrrolidin-3-yl)methanol, (R)-9-((2-(3-Amino-3-(methoxymethyl)pyrrolidin-1-yl)naphthalin-1-yl)methyl)-9H-Purin-6-amin (R)-3-Amino-1-(6-((6-amino-9H-purin-9-yl)methyl)-5-brom-4-chlor-2, 3-Dihydrobenzofuran-7-yl)-N-cyclopropylpyrrolidin-3-carboxamide, (R)-3-Amino-1-(6-((6-amino-9H-purin-9- yl)methyl)-4-fluor-5-lode-2, 3-Dihydrobenzofuran-7-yl)-N-Cyclopropylpyrrolidin-3-carboxamid, (R)-3-Amino-1-(7-((6-amino-9H-purin-9-yl)methyl)benzofuran-6-yl)-N-Cyclopropylpyrrolidin-3-carboxamide, (R)-3-Amino-1-(2-((6-Amino-9H-purin-9- yl)methyl)-4-chlor-3-(ethylthio)phenyl)-N-cyclopropylpyrrolidin-3-carboxamide, (R)-3-Amino-1-(7-((6-Amino-9H-purin-9-yl)methyl)-2, 2-Dimethyl-2,3-dihydrobenzofuran-6-yl)-N-cyclopropylpyrrolidin-3-carboxamide, 3-Amino-1-(5-((6-amino-9H-purin-9-yl)methyl)-6-bromo-2, 3-Dihydrobenzofuran-4-yl)-N-cyclopropylpyrrolidin-3-carboxamid, (R)-3-Amino-1-(2-((6-Amino-9H-purin-9-yl)methyl)-4-fluor-3-(trifluormethyl)phenyl)-N-cyclopropylpyrrolidin-3-carboxamide, (R)-3-Amino-1-(2-((6-amino-9H-purin-9-yl)methyl)-4-fluor-3-(trifluormethyl)phenyl)-N-((1r,3R)-3-methylthio)cyclobutyl)pyrrolidin-3-carboxamid, (R)-3-Amino-1-(2-((6-Amino-9H-purin-9-yl)methyl)-4-fluor-3-(trifluormethyl)phenyl)-N-(pyridazin-3- ylmethyl)pyrrolidin-3-carboxamid, (R)-3-Amino-1-(2-((6-amino-9H-purin-9- yl)methyl)-4-fluor-3-(trifluormethyl)phenyl)-N-(2-cyano-2-methylpropyl)pyrrolidin-3-carboxamid, (R)-3-Amino-1-(2-((6-amino-9H-purin-9-yl)methyl)-4-fluor-3-(trifluormethyl)phenyl)-N-((2-hydroxypyridin-3-yl)methyl)pyrrolidin-3-carboxamid, (R)-N-(2-(1H-Imidazol-4-yl)ethyl)-3-amino-1-(2-((6-Amino-9H-purin-9-yl)methyl)-4-fluor-3-(trifluormethyl)phenyl)pyrrolidin-3-carboxamide, (3R)-3-Amino-1-(2-((6-Amino-9H-purin-9-yl)methyl)-4-fluor-3-(trifluormethyl)phenyl)-N-((2,2-Difluorcyclopropyl)methyl)pyrrolidin-3-carboxamid, (R)-3-Amino-1-(2-((6-Amino-9H-purin-9-yl)methyl)-4-fluor-3-(trifluormethyl)phenyl)-N-(3-cyclopropyl-1H-pyrazol-5-yl)pyrrolidin-3-carboxamid, (R)-3-Amino-1-(2-((6-Amino-9H-purin-9-yl)methyl)-4-fluor-3-(trifluormethyl)phenyl)-N-( (R)-1-(Tetrahydro-2H-pyran-4-yl)ethyl)pyrrolidin-3-carboxamid, (R)-3-Amino-1-(2-((6-amino-9H-purin-9-yl)methyl)-4-fluor-3-(trifluormethyl)phenyl)-N-(2-cyano-2-methylpropyl)pyrrolidin-3-carboxamid, (3R)-3-Amino-1-(2-((6-amino-9H-purin-9-yl)methyl)-4-fluor-3-(trifluormethyl)phenyl)-N-(6-Oxaspiro[2.S]octan-1-yl)pyrrolidin-3-carboxamid, (R)-3-Amino-1-(2-((6-amino-9)¬ purin-9-yl)methyl)-4-fluor-3-(trifluormethyl)phenyl)-N-((1R, 2R)-2-(Hydroxymethyl)cyclopropyl)pyrrolidin-3-carboxamid, (R)-(3-Amino-1-(2-((6-amino-9H-purin-9-yl)methyl)-4-fluor-3-(trifluormethyl)phenyl) pyrrolidin-3-yl)(6, 7-Dihydro-[1,2,3]triazolo[1,5-a]pyrazin-5(4H)-yl)methanon, (3R)-3-Amino-1-(2-((6-amino-9H-purin-9-yl)methyl)-4-fluor-3-(trifluormethyl)phenyl)-N-(tetrahydrofuran-3- yl)pyrrolidin-3-carboxamid, (3R)-3-Amino-1-(2-((6-amino-9H-purin-9-yl)methyl)-4-fluor-3-(trifluormethyl)phenyl)-N-(5-methoxyspiro[2. 3]hexan-1-yl)pyrrolidin-3- carboxamid, ((R)-3-Amino-1-(2-((6-amino-9H-purin-9-yl)methyl)-4-fluor-3-(trifluormethyl)phenyl)pyrrolidin-3-yl)((S)-2-methylmorpholino)methanon, (R)-1-(3-Amino-1-(2-((6-amino-9H-purin-9-yl)methyl)-4-fluor-3-(trifluormethyl)phenyl) pyrrolidin-3-carbonyl)azetidin-3-carbonitril, (R)-3-Amino-1-(2-((6-Amino-9H-purin-9-yl)methyl)-4-fluor-3-(trifluormethyl)phenyl)-N-((2-Aminopyrimidin-4-yl)methyl)pyrrolidin-3-carboxamid, (R)-(3-Amino-1-(2-((6-amino- 9H-purin-9-yl)methyl)-4-fluor-3-(trifluormethyl)phenyl)pyrrolidin-3-yl)(6,7-dihydroisoxazolo[4,3-c]pyridin-5(4H)-yl)methanon, ((R)-3-Amino-1-(2-((6-amino-9H¬ purin-9-yl)methyl)-4-fluor-3-(trifluormethyl)phenyl)pyrrolidin-3-yl)((R)-2-methylmorpholino)methanon, (R)-2-(4-(3-Amino-1-(2-((6-amino-9H-purin-9- yl)methyl)-4-fluor-3-(trifluormethyl)phenyl)pyrrolidin-3-carbonyl)piperazin-1- yl)acetonitril, (R)-(3-Amino-1-(2-((6-amino-9H-purin-9-yl)methyl)-4-fluor-3-(trifluormethyl)phenyl)pyrrolidin-3-yl)(6, 7-Dihydropyrazolo[1,5-a]pyrazin-5(4H)¬ yl)methanon, ((R)-3-Amino-1 -(2-((6-amino-9H-purin-9-yl)methyl)-4-fluor-3-(trifluormethyl)phenyl)pyrrolidin-3-yl)((R)-3-hydroxypiperidin-1-yl)methanon, (R)-3-Amino-1-(2-((6-amino-9H-purin-9-yl)methyl)-4-fluoro-3-(trifluoromethyl)phenyl)-N-(3-oxo-3-(propylamino)propyl)pyrrolidin-3-carboxamid, (R)-3-Amino-1-(2-((6-Amino-9H-purin-9-yl)methyl)-4-fluor-3-(trifluormethyl)phenyl)-N-((1-Ethyl-1H-1,2,4-triazol-5-yl)methyl)pyrrolidin-3-carboxamid, 1-((R)-3-Amino-1-(2-((6-amino-9H-purin-9- yl)methyl)-4-fluor-3-(trifluormethyl)phenyl)pyrrolidin-3-carbonyl)pyrrolidin-3- carbonitril, (S)-3-amino-1-(2-((6-amino-9H-purin-9-yl)methyl)-3-bromo-6-chlorophenyl)pyrrolidine-3-carboxylic acid, (R)-3-amino-1-(2-((6-amino-9H-purin-9- yl)methyl)-3-bromo-4, 6-Dichlorphenyl)-N-cyclopropylpyrrolidin-3-carboxamid, (R)-3-Amino-1-(2-((6-amino-9H-purin-9-yl)methyl)-3,4-dichlorphenyl)-N-cyclopropylpyrrolidin-3-carboxamide, (R)-3-Amino-1-(2-((6-Amino-9H-purin-9- yl)methyl)-4-chlor-3-ethylphenyl)-N-(2-(phenylsulfonyl)ethyl)pyrrolidin-3-carboxamid, (R)-3-Amino-1-(2-((6-Amino-9H-purin-9-yl)methyl)-4-brom-3-(trifluormethyl)phenyl)-N-cyclopropylpyrrolidin-3-carboxamid, (R)-3-Amino-1-(2-((6-amino-9H-purin-9-yl)methyl)-3-brom-4-(trifluormethoxy)phenyl)-N-cyclopropylpyrrolidin-3-carboxamide, (R)-3-Amino-1-(2-((6-amino-9H-purin-9-yl)methyl)-3-brom-5-chlorphenyl)pyrrolidin-3-carboxamide, (R)-3-Amino-1-(2-((6-amino-9H-purin-9-yl)methyl)-4-chlor-3-ethylphenyl)-N-(2-(ethylsulfonyl)ethyl)pyrrolidin-3-carboxamid, (R)-3-Amino-1-(2-((6-amino-9H-purin- 9-yl)methyl)-5-chlor-3-(5,6,7,8-tetrahydronaphthalin-1-yl)phenyl)-N-cyclopropylpyrrolidin-3-carboxamide, (R)-(3-Amino-1-(2-((6-amino-9H-purin-9-yl)methyl)-5-chlor-3-ethylphenyl)pyrrolidin-3-yl)(1,1 -dioxidothiomorpholino)methanon, 3-Amino-1-(2-((6-amino-9H-purin-9-yl)methyl)-3, 4-Dichlor-6-fluorphenyl)-N-cyclopropylpyrrolidin-3-carboxamid, (R)-3-Amino-1-(2-((6-amino-9H-purin-9-yl)methyl)-4-chlor-3-ethylphenyl)-N-(1, 1-Dioxidotetrahydro-2H-thiopyran-4-yl)pyrrolidin-3-carboxamid, (R)-3-Amino-1-(2-((6-amino-9H-purin-9- yl)methyl)-6-chlor-3-ethylphenyl)-N-cyclopropylpyrrolidin-3-carboxamid, (R)-(3-Amino-1-(2-((6-amino-9H-purin-9-yl)methyl)-4-chlor-3-ethylphenyl)pyrrolidin-3- yl)(1,1-dioxidothiomorpholino)methanon, (R)-9-(6-(3-Amino-3-(1H-tetrazol-5- yl)pyrrolidin-1-yl)-3-chlor-2-iodobenzyl)-9H-purin-6-amin, 3-Amino-1-(2-((6-amino- 9H-purin-9-yl)methyl)-3-chlor-6-methylphenyl)-N-cyclopropylpyrrolidin-3- carboxamid, (R)-3-Amino-1-(2-((6-Amino-9H-purin-9-yl)methyl)-3-cyclopentyl-4-fluorphenyl)-N-cyclopropylpyrrolidin-3-carboxamid, (R)-3-Amino-1-(2-((6-Amino-9H-purin-9-yl)methyl)-3-brom-5-chlorphenyl)-N-(ethyl-d5)pyrrolidin-3- carboxamid,3-Amino-1-(2-((6-Amino-9H-purin-9-yl)methyl)-3-chlor-4-(trifluormethyl)phenyl)-N-cyclopropylpyrrolidin-3-carboxamide, (R)-3-Amino-1-(2-((6-amino-9H-purin-9-yl)methyl)-5-chlor-3-ethylphenyl)-N-isopropylpyrrolidin-3-carboxamid, (R)-3-Amino-1-(2-((6-amino-9H-purin-9-yl)methyl)-4, 6-Dichlor-3-(methoxymethyl)phenyl)-N-cyclopropylpyrrolidin-3-carboxamid, (R)-3-Amino-1-(2-((6-amino-9H-purin-9-yl)methyl)-4-chlor-3-vinylphenyl)-N-cyclopropylpyrrolidin-3- carboxamid, 3-Amino-1-(2-((6-amino-9H-purin-9-yl)methyl)-3-chlorphenyl)-N-cyclopropylpyrrolidin-3-carboxam ide, (R)-3-Amino-1-(2-((6-Amino-9H-purin-9-yl)methyl)-4-chlor-3-ethylphenyl)-N-cyclopropylpyrrolidin-3-carboxamide, (S)-3-Amino-1-(1-((6-Amino-9H-purin-9-yl)methyl)naphthalen-2-yl)-N-cyclopropylpyrrolidin-3-carboxamide, (R)-3-Amino-1-(1-((6-Amino-9H-purin-9-yl)methyl)-6-bromnaphthalin-2-yl)-N-cyclopropylpyrrolidin-3-carboxamide, (R)-3-Amino-1-(2-((6-amino-9H-purin-9-yl)methyl)-3-brom-4-methoxyphenyl)-N-cyclopropylpyrrolidin-3-carboxamid, (R)-3-Amino-1-(2-((6-Amino-9H-purin-9-yl)methyl)-3-ethylphenyl)-N-cyclopentylpyrrolidin-3-carboxamid, Ethyl (R)-3-Amino-1-(2-((6-Amino-9H-purin-9-yl)methyl)-3-brom-5-chlorphenyl)pyrrolidin-3-carboxylat.

14. Pharmazeutische Zusammensetzung, aufweisend eine Verbindung nach einem der Ansprüche 1-13 oder ein pharmazeutisch akzeptables Salz davon, und einen oder mehrere pharmazeutisch akzeptable Träger.

15. Kombination, aufweisend eine Verbindung nach einem der Ansprüche 1-13 oder ein pharmazeutisch akzeptables Salz davon, und einen oder mehrere therapeutische Wirkstoffe.

16. Kombination nach Anspruch 15, wobei der eine oder die mehreren therapeutische(n) Wirkstoffe ein Krebsmittel, ein Analgetikum oder ein Entzündungshemmer sind.

17. Verbindung nach einem der Ansprüche 1-13 oder ein pharmazeutisch akzeptables Salz davon zur Verwendung in einem Verfahren zum Behandeln einer Krankheit oder eines Leidens, die von dieser profitieren oder behandelbar sind durch Inhibition einer Leukämie mit gemischter Abstammung.

18. Verbindung zur Verwendung nach Anspruch 17, wobei die Krankheit oder das Leiden, die von der Inhibition von MLL1 profitieren oder durch diese behandelbar sind, ausgewählt sind aus soliden Tumoren, Leukämie, Myelom, Lymphom, und Hypertonie.

19. Verbindung zur Verwendung nach Anspruch 17, wobei die Krankheit oder das Leiden, die von der Inhibition von MLL1 profitieren oder durch diese behandelbar sind Brustkrebs, Gebärmutterhalskrebs, Hautkrebs, Eierstockkrebs, Magenkrebs, Prostatakrebs, Bauchspeicheldrüsenkrebs, Lungenkrebs, hepatozelluläres Karzinom, Kopf- und Halskrebs, Tumor der peripheren Nervenscheide, Osteosarkom, multiples Myelom, Neuroblastom, Leukämie, Non-Hodgkin-Lymphom oder pulmonale arterielle Hypertonie sind.

20. Verbindung nach einem der Ansprüche 1-13 und optional kombiniert mit einem zweiten therapeutischen Wirkstoff, zur Verwendung bei der Herstellung eines Medikaments für eine Krankheit oder ein Leiden, die von der Inhibition einer Leukämie mit gemischter Abstammung (MLL1) profitieren.

## Revendications

1. Composé de Formule (I) 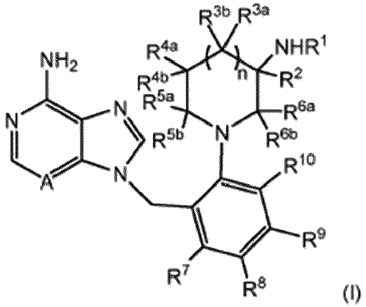 ou un énantiomère, un mélange énantiomérique ou un sel de qualité pharmaceutique dudit composé ; dans lequel :
A est N ;
R¹ est H ; ou
R¹ et R² conjointement avec NH forment un hétérocyclyle à 5-8 chaînons comprenant 1-2 hétéroatomes choisis parmi N, O et S en tant que chaînons du cycle ; ledit hétérocyclyle à 5-8 chaînons étant non substitué ou substitué par un substituant oxo ;
R² est choisi dans le groupe constitué par :
(i) -alkyle C₁₋₆, -haloalkyle C₁₋₆, -hydroxyalkyle C₁₋₆, -alcoxy C₁₋₆-alkyle C₁₋₆ ou-cycloalcoxy C₃₋₈(alkyle C₁₋₆) ;
(ii) cyano, -cyanoalkyle C₁₋₆, -alkylthio C₁₋₆-alkyle C₁₋₆, -alcényle C₂₋₆,-haloalcényle C₂₋₆, -alcynyle C₂₋₆, -alkyle C₁₋₄-S-alkyle C₁₋₄, -alkyle C₁₋₄-SO₂-alkyle C₁₋₄, -SO₂(alkyle C₁₋₄) ou -C(alkyle C₁₋₄)=N-O(alkyle C₁₋₄) ;
(iii) -alkyl C₁₋₄-carbonyle, -(CR^{a}R^{b})ₚ-C(=O)-OR^{10a} ou -C(=O)-(CR^{a}R^{b})_{q}-R¹¹ ;
dans lesquels R¹¹ est cycloalkyle C₃₋₇, hétérocyclyle à 5-6 chaînons ou hétéroaryle à 5-6 chaînons, dont chacun est indépendamment non substitué ou substitué avec -alkyle C₁₋₆ ou -alcoxy C₁₋₆ ; et ledit hétérocyclyle à 5-6 chaînons ou hétéroaryle à 5-6 chaînons comprend indépendamment 1-3 hétéroatomes choisis parmi l'azote, l'oxygène et le soufre ;
(iv) -(CR^{a}R^{b})ᵣ-C(=O)-NR¹²R¹³, -(CR^{a}R^{b})ₛ-NR¹²R¹³, -(CR^{a}R^{b})₁₋₄-O-(CR^{a}R^{b})₁₋₄-OR^{10a} ou
-(CR^{a}R^{b})₁₋₄-O-(CR^{a}R^{b})₁₋₄-C(=O)-NR¹²R¹³ ;
dans lesquels R¹² est l'hydrogène ou -alkyle C₁₋₆ ;
R¹³ est l'hydrogène, -alkyle C₁₋₆, -alcoxy C₁₋₆-alkyle C₁₋₆, -cyanoalkyle C₁₋₆ ; -alkyl C₁₋₄-SO₂R^{10b} où R^{10b} est alkyle C₁₋₆ ou phényle ; cycloalkyle monocyclique ou bicyclique C₃₋₁₀-alkyle C₀₋₆, phényle, un cycle hétérocyclique monocyclique ou bicyclique à 5-10 chaînons ou hétéroaryle à 5-9 chaînons-alkyle C₀₋₆ ;
ledit cycle hétérocyclique monocyclique ou bicyclique à 5-10 chaînons ou radical hétéroaryle à 5-9 chaînons comprend indépendamment 1-4 hétéroatomes choisis parmi l'azote, l'oxygène et le soufre ; et
lesdits cycloalkyle monocyclique ou bicyclique C₃₋₁₀, phényle, cycle hétérocyclique monocyclique ou bicyclique à 5-10 chaînons ou radical hétéroaryle à 5-9 chaînons sont indépendamment non substitués ou substitués avec 1-2 -alkyle C₁₋₄, -hydroxyalkyle C₁₋₆,
-alcoxy C₁₋₄, halo, hydroxyle, phényle ou -S(alkyle C₁₋₄) ;
ou R¹² et R¹³ forment ensemble un cycle hétérocyclique monocyclique ou bicyclique à 5-10 chaînons comprenant 1-4 hétéroatomes choisis parmi l'azote, l'oxygène et le soufre ; et étant non substitué ou substitué avec -alkyl C₁₋₄, hydroxyle, cyano, -cyanoalkyle C₁₋₆, -SO₂ ou -alcényle C₂₋₄-carbonyle ;
(v) hétérocyclyle à 5-6 chaînons-alkyle C₀₋₆ ou hétérocyclyle à 5-6 chaînons(haloalkyle C₁₋₄), dans lesquels chaque radical hétérocyclyle est non substitué ou substitué par oxo ; et dans lesquels chaque radical hétérocyclyle comprend 1-3 hétéroatomes choisis parmi l'azote, l'oxygène et le soufre ; et
(vi) phényle, hétéroaryle à 5-9 chaînons-alkyle C₀₋₆ ou hétéroaryle à 5-9 chaînons(haloalkyle C₁₋₄), dans lesquels chaque phényle ou radical hétéroaryle est indépendamment non substitué ou substitué par -alkyle C₁₋₄, -haloalkyle C₁₋₄,-hydroxyalkyle C₁₋₄, -alcoxy C₁₋₄, -haloalcoxy C₁₋₄, halo, hydroxy, cyano, oxydo, amino, -alkyle C₁₋₄-amino, -dialkyle C₁₋₄-amino, -aminocarbonyl-alkyl C₀₋₆, -alkyle C₁₋₄-aminocarbonyl-alkyle C₀₋₆, -dialkyle C₁₋₄-aminocarbonyl-alkyle C₀₋₆ ou cycloalkyle C₃₋₇ ; dans lesquels chaque radical hétéroaryle comprend 1-4 hétéroatomes choisis parmi l'azote, l'oxygène et le soufre ;
R^{3a}, R^{3b}, R^{4a}, R^{4b}, R^{5a}, R^{5b}, R^{6a} et R^{6b} sont indépendamment l'hydrogène, halo, cyano, hydroxyle, -alkyle C₁₋₆, -haloalkyle C₁₋₆, -hydroxyalkyle C₁₋₆, -alcoxy C₁₋₆,-alcoxy C₁₋₆-alkyle C₁₋₆, aryle, -C(=O)-OR¹⁴ ou -(CR^{a}R^{b})ₛ-C(=O)-NR¹⁵R¹⁶ ; ou
R^{3a} et R^{3b}, R^{4a} et R^{4b}, R^{5a} et R^{5b} ou R^{6a} et R^{6b} forment un substituant oxo ;
R⁷, R⁸, R⁹ et R¹⁰ sont indépendamment l'hydrogène, halo, -alkyle C₁₋₄, -haloalkyle C₁₋₆, -hydroxyalkyle C₁₋₆, cyano, -cyanoalkyle C₁₋₆, -alcényle C₂₋₆, -alcynyle C₂₋₆,-alkylthio C₁₋₆, -(CR^{a}R^{b})₁₋₄-NR¹⁷R¹⁸, -(CR^{a}R^{b})₁₋₄-NR¹⁷-C(O)-OR¹⁸, -(CR^{a}R^{b})₁₋₄-OR¹⁹, cycloalkyle C₃₋₈, phényle ou hétéroaryle à 5-6 chaînons comprenant 1-3 hétéroatomes choisis parmi l'azote, l'oxygène et le soufre, ledit cycloalkyle C₃₋₈, phényle ou hétéroaryle à 5-6 chaînons étant indépendamment substitué avec 1-2 R²⁰ ; et à condition qu'au moins un parmi R⁷, R⁸, R⁹ et R¹⁰ ne soit pas hydrogène ;
en variante, R⁷ et R⁸, R⁸ et R⁹, et R⁹ conjointement avec le cycle phényle auquel ils sont liés forment un carbocycle benzo-fusionné ou hétérocycle benzo-fusionné à 9-10 chaînons comprenant 1-3 hétéroatomes choisis parmi l'azote, l'oxygène et le soufre ; lesdits carbocycle benzo-fusionné ou hétérocycle benzo-fusionné étant indépendamment non substitués ou substitués avec 1-2 halo ou alkyle C₁₋₄ ;
R^{a}, R^{b}, R^{10a}, R¹⁴, R¹⁵, R¹⁶ et R¹⁷ sont indépendamment l'hydrogène ou -alkyle C₁₋4 ;
R¹⁸ est l'hydrogène, -alkyle C₁₋₄, -haloalkyle C₁₋₆ ou -cycloalkyle C₃₋₆ ;
en variante, R¹⁷ et R¹⁸ conjointement avec N dans la fraction -NR¹⁷R¹⁸ forment un cycle hétérocyclique monocyclique ou bicyclique à 4-10 chaînons comprenant 1-3 hétéroatomes choisis parmi l'azote, l'oxygène et le soufre ; ledit cycle hétérocyclique monocyclique ou bicyclique à 4-10 chaînons étant non substitué ou substitué avec 1-2 halo ou alkyle C₁₋₄ ;
R¹⁹ est l'hydrogène, -alkyle C₁₋₄, -haloalkyle C₁₋₆, -cycloalkyle C₃₋₆, phényle, hétérocyclyle à 5-6 chaînons ou hétéroaryle à 5-6 chaînons ; ledit hétérocyclyle à 5-6 chaînons ou hétéroaryle à 5-6 chaînons comprenant indépendamment 1-3 hétéroatomes choisis parmi l'azote, l'oxygène et le soufre ;
R²⁰ est -alkyle C₁₋₄, -halo ou -cycloalkyle C₃₋₆ ; ou deux R²⁰ forment ensemble un cycle à 5-6 chaînons comprenant 0-2 hétéroatomes choisis parmi l'azote, l'oxygène et le soufre ;
n est 0 ou 1 ; et
p, q, r et s sont indépendamment 0, 1, 2, 3 ou 4.

2. Le composé de Formule (I) dans la revendication 1, dans lequel ledit composé est un composé de Formule (II) : 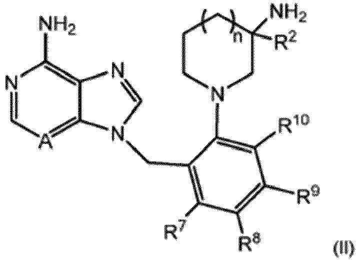 ou un énantiomère, un mélange énantiomérique ou un sel de qualité pharmaceutique dudit composé ; dans lequel :
R² est choisi dans le groupe constitué par :
(i) -hydroxyalkyle C₁₋₆, -alcoxy C₁₋₆-alkyle C₁₋₆ ou -(CR^{a}R^{b})ₚ-C(=O)-OR^{10a} ;
(ii) -(CR^{a}R^{b})ᵣ-C(=O)-NR¹²R¹³ ou (CR^{a}R^{b})₁₋₄-O-(CR^{a}R^{b})₁₋₄-C(=O)-NR¹²R¹³ ; dans lesquels
R¹² est l'hydrogène ou -alkyle C₁₋₆ ;
R¹³ est l'hydrogène, -alkyle C₁₋₆, -cyanoalkyle C₁₋₆ ; -alkyl C₁₋₄-SO₂R^{10b} où R^{10b} est alkyle C₁₋₆ ou phényle ; cycloalkyle monocyclique ou bicyclique C₃₋₁₀-alkyle C₀₋₆, phényle, un cycle hétérocyclique monocyclique ou bicyclique à 5-10 chaînons ou hétéroaryle à 5-9 chaînons-alkyle C₀₋₆ ; ledit cycle hétérocyclique monocyclique ou bicyclique à 5-10 chaînons ou radical hétéroaryle à 5-9 chaînons comprenant indépendamment1-4 hétéroatomes choisis parmi l'azote, l'oxygène et le soufre ; et
lesdits cycloalkyle monocyclique ou bicyclique C₃₋₁₀, phényle, cycle hétérocyclique monocyclique ou bicyclique à 5-10 chaînons ou radical hétéroaryle à 5-9 chaînons sont indépendamment non substitués ou substitués avec 1-2 -alkyle C₁₋₄, -hydroxyalkyle C₁₋₆, alcoxy C₁₋₄, halo, hydroxyle, phényle ou -S(alkyle C₁₋₄) ; ou
R¹² et R¹³ forment ensemble un cycle hétérocyclique monocyclique ou bicyclique à 5-10 chaînons comprenant 1-4 hétéroatomes choisis parmi l'azote, l'oxygène et le soufre ; et étant non substitué ou substitué avec -alkyl C₁₋₄, hydroxyle, cyano, -cyanoalkyle C₁₋₆, SO₂ ou -alcényle C₂₋₄-carbonyle ; et
(iii) phényle ou hétéroaryle à 5-9 chaînons-alkyle C₀₋₆ ; ledit radical hétéroaryle étant non substitué ou substitué par -alkyle C₁₋₄, -haloalkyle C₁₋₄, amino, -alkyle C₁₋₄-amino ou -dialkyle C₁₋₄-amino ; et ledit radical hétéroaryle comprend 1-4 hétéroatomes choisis parmi l'azote, l'oxygène et le soufre ;
R⁷, R⁸, R⁹ et R¹⁰ sont indépendamment l'hydrogène, halo, -alkyle C₁₋₄, -haloalkyle C₁₋₆, -hydroxyalkyle C₁₋₆, cyano, -cyanoalkyle C₁₋₆, alcényle C₂₋₆, alcynyle C₂₋₆,-alkylthio C₁₋₆, -(CR^{a}R^{b})₁₋₄-NR¹⁷R¹⁸, -(CR^{a}R^{b})₁₋₄-NR¹⁷-C(O)-OR¹⁸, -(CR^{a}R^{b})₁₋₄-OR¹⁹,-cycloalkyle C₃₋₈, phényle ou hétéroaryle à 5-6 chaînons comprenant 1-4 hétéroatomes choisis parmi l'azote, l'oxygène et le soufre ; et
ledit cycloalkyle C₃₋₈, phényle ou hétéroaryle à 5-6 chaînons étant indépendamment substitué avec 1-2 R²⁰ ; et
à condition qu'au moins un parmi R⁷, R⁸, R⁹ et R¹⁰ ne soit pas hydrogène ;
en variante, R⁷ et R⁸, R⁸ et R⁹, R⁹ et R¹⁰ conjointement avec le cycle phényle auquel ils sont liés forment un carbocycle benzo-fusionné ou hétérocycle benzo-fusionné à 9-10 chaînons comprenant 1-3 hétéroatomes choisis parmi l'azote, l'oxygène et le soufre ; lesdits carbocycle benzo-fusionné ou hétérocycle benzo-fusionné étant indépendamment non substitués ou substitués avec 1-2 halo ou-alkyle C₁₋₄ ;
R^{a}, R^{b}, R^{10a} et R¹⁷ sont indépendamment l'hydrogène ou -alkyle C₁₋₄ ;
R¹⁸ est l'hydrogène, -alkyle C₁₋₄, -haloalkyle C₁₋₆ ou -cycloalkyle C₃₋₆ ;
en variante, R¹⁷ et R¹⁸ conjointement avec N dans la fraction -NR¹⁷R¹⁸ forment un cycle hétérocyclique monocyclique ou bicyclique à 4-10 chaînons comprenant 1-3 hétéroatomes choisis parmi l'azote, l'oxygène et le soufre ; ledit cycle hétérocyclique étant non substitué ou substitué avec 1-2 halo ou -alkyle C₁₋₄ ;
R¹⁹ est l'hydrogène, -alkyle C₁₋₄, -haloalkyle C₁₋₆, -cycloalkyle C₃₋₆, phényle, hétérocyclyle à 5-6 chaînons ou hétéroaryle à 5-6 chaînons ; ledit hétérocyclyle à 5-6 chaînons ou hétéroaryle à 5-6 chaînons comprenant indépendamment 1-3 hétéroatomes choisis parmi l'azote, l'oxygène et le soufre ;
R²⁰ est -alkyle C₁₋₄, halo ou -cycloalkyle C₃₋₆ ; ou deux R²⁰ forment ensemble un cycle à 5-6 chaînons comprenant 0-2 hétéroatomes choisis parmi l'azote, l'oxygène et le soufre ;
n est 0 ou 1 ; et
p, q, r et s sont indépendamment 0, 1, 2, 3 ou 4.

3. Le composé de Formule (II) dans la revendication 2, dans lequel ledit composé est un composé de Formule (III) : 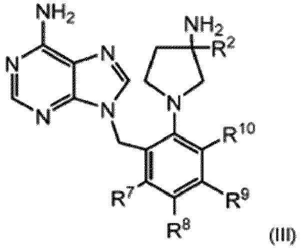 ou un énantiomère, un mélange énantiomérique ou un sel de qualité pharmaceutique dudit composé.

4. Le composé de Formule (III) dans la revendication 3, dans lequel ledit composé est un composé de Formule (IV) : 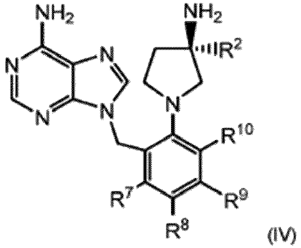 ou un énantiomère, un mélange énantiomérique ou un sel de qualité pharmaceutique dudit composé.

5. Le composé de l'une quelconque des revendications 1-4 ou un énantiomère, un mélange énantiomérique ou un sel de qualité pharmaceutique dudit composé ; dans lequel R² est 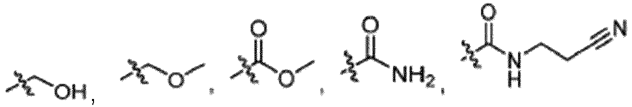 ou

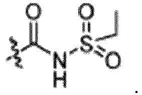

6. Le composé de l'une quelconque des revendications 1-4 ou un énantiomère, un mélange énantiomérique ou un sel de qualité pharmaceutique dudit composé ; dans lequel R² est

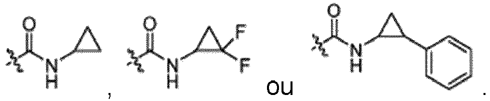

7. Le composé de l'une quelconque des revendications 1-4 ou un énantiomère, un mélange énantiomérique ou un sel de qualité pharmaceutique dudit composé ; dans lequel R² est

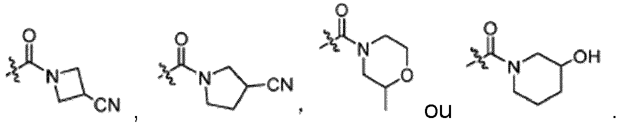

8. Le composé de l'une quelconque des revendications 1-4 ou un énantiomère, un mélange énantiomérique ou un sel de qualité pharmaceutique dudit composé ; dans lequel R² est 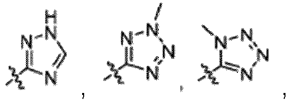 1H-tétrazolyle, 2H-tétrazolyle, pyridyle, trifluorométhylpyridyle ou phényle.

9. Le composé de l'une quelconque des revendications 1-4 ou un énantiomère, un mélange énantiomérique ou un sel de qualité pharmaceutique dudit composé ; dans lequel ledit composé est un composé de Formule (V) :

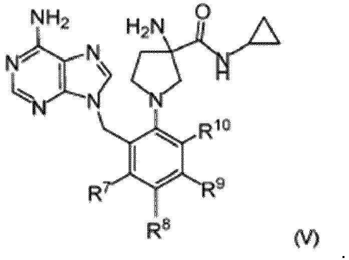

10. Le composé de l'une quelconque des revendications 1-9 ou un sel de qualité pharmaceutique dudit composé ; dans lequel au moins deux parmi R⁷, R⁸, R⁹ et R¹⁰ ne sont pas hydrogène.

11. Le composé de la revendication 10 ou un sel de qualité pharmaceutique dudit composé ; dans lequel R⁷, R⁸, R⁹ et R¹⁰ sont indépendamment l'hydrogène, halo,-alkyle C₁₋₄, -haloalkyle C₁₋₆, -hydroxyalkyle C₁₋₆, -alcényle C₂₋₆, -cycloalkyle C₃₋₈,-(CR^{a}R^{b})₁₋₄-NR¹⁷R¹⁸ ou -(CR^{a}R^{b})₁₋₄-OR¹⁹, à condition qu'au moins deux parmi R⁷, R⁸, R⁹ et R¹⁰ ne soient pas hydrogène ;
R^{a}, R^{b} et R¹⁷ sont indépendamment l'hydrogène ou -alkyle C₁₋₄ ;
R¹⁸ est l'hydrogène, -alkyle C₁₋₄, -haloalkyle C₁₋₆ ou -cycloalkyle C₃₋₆ ;
en variante, R¹⁷ et R¹⁸ conjointement avec N dans la fraction -NR¹⁷R¹⁸ forment un cycle hétérocyclique monocyclique ou bicyclique à 4-10 chaînons comprenant 1-3 hétéroatomes choisis parmi l'azote, l'oxygène et le soufre ; ledit cycle hétérocyclique étant non substitué ou substitué avec 1-2 halo ou -alkyle C₁₋₄ ; et
R¹⁹ est l'hydrogène, -alkyle C₁₋₄, -haloalkyle C₁₋₆, -cycloalkyle C₃₋₆, phényle, hétérocyclyle à 5-6 chaînons ou hétéroaryle à 5-6 chaînons ; ledit hétérocyclyle à 5-6 chaînons ou hétéroaryle à 5-6 chaînons comprenant indépendamment 1-3 hétéroatomes choisis parmi l'azote, l'oxygène et le soufre.

12. Le composé de l'une quelconque des revendications 1-4 ou un énantiomère, un mélange énantiomérique ou un sel de qualité pharmaceutique dudit composé ; dans lequel ledit composé est un composé de Formule (V) : 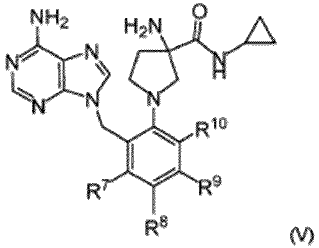 dans laquelle R⁷, R⁸, R⁹ et R¹⁰ sont indépendamment l'hydrogène, halo,-haloalkyle C₁₋₆, à condition qu'au moins deux parmi R⁷, R⁸, R⁹ et R¹⁰ ne soient pas hydrogène.

13. Le composé selon la revendication 1 ou un énantiomère, un mélange énantiomérique ou un sel de qualité pharmaceutique dudit composé ; dans lequel ledit composé est choisi parmi l'un des composés ci-dessous, ou un sel de qualité pharmaceutique desdits composés :
(R)-3-amino-1-(2-((6-amino-9H-purin-9-yl)méthyl)-3-bromo-5-chlorophényl)-N-cyclopropylpyrrolidine-3-carboxamide, (R)-3-amino-1-(2-((6-amino-9H-purin-9-yl)méthyl)-5-chloro-3-éthylphényl)-N-cyclopropylpyrrolidine-3-carboxamide, (R)-3-amino-1-(2-((6-amino-9H-purin-9-yl)méthyl)-5-chloro-3-(2,3-dihydrobenzofuran-5-yl)phényl)-N-cyclopropylpyrrolidine-3-carboxamide, (R)-3-amino-1-(2-((6-amino-9H-purin-9-yl)méthyl)-3-cyano-4-fluorophényl)-N-cyclopropylpyrrolidine-3-carboxam ide, (R)-3-amino-1-(2-((6-amino-9H-purin-9-yl)méthyl)-3-éthynyl-4-fluorophényl)-N-cyclopropylpyrrolidine-3-carboxamide, (R)-3-amino-1-(2-((6-amino-9H-purin-9-yl)méthyl)-4-fluoro-3-(2,2,2-trifluoroéthyl)phényl)-N-cyclopropylpyrrolidine-3-carboxamide, (R)-3-amino-1-(2-((6-amino-9H-purin-9-yl)méthyl)-5-chloro-3-(2,2,2-trifluoroéthyl)phényl)-N-cyclopropylpyrrolidine-3-carboxamide, (R)-3-amino-1-(2-((6-am ino-9H-purin-9-yl)méthyl)-3-cyclopropyl-4-fluorophényl)-N-cyclopropylpyrrolidine-3-carboxamide, 3-amino-1-(2-((6-amino-9H-purin-9-yl)méthyl)-4-fluoro-3-vinylphényl)-N-cyclopropylpyrrolidine-3-carboxamide, (R)-3-amino-1-(2-((6-amino-9H-purin-9-yl)méthyl)-3-éthyl-4-fluorophényl)-N-cyclopropylpyrrolidine-3-carboxam ide, (R)-3-amino-1-(2-((6-amino-9H-purin-9-yl)méthyl)-3-méthyl-4-(trifluorométhyl)phényl)-N-cyclopropylpyrrolidine-3-carboxamide, (R)-3-amino-1-(2-((6-amino-9H-purin-9-yl)méthyl)-3-éthyl-4-(trifluorométhyl)phényl)-N-cyclopropylpyrrolidine-3-carboxamide, (R)-3-amino-1-(2-((6-amino-9H-purin-9-yl)méthyl)-3-bromo-4-fluorophényl)-N-cyclopropylpyrrolidine-3-carboxamide, (R)-3-amino-1-(2-((6-amino-9H-purin-9-yl)méthyl)-4-fluorophényl)-N-cyclopropylpyrrolidine-3-carboxamide, (R)-3-amino-1-(4-((6-amino-9H-purin-9-yl)méthyl)-2,2-difluorobenzo[d][1,3]dioxol-5-yl)-N-cyclopropylpyrrolidine-3-carboxamide, (R)-3-amino-1-(2-((6-amino-9H-purin-9-yl)méthyl)-5-chloro-3'-cyclohexyl-[1,1'-biphényl]-3-yl)pyrrolidine-3-carboxamide, (R)-1-(4-(3-amino-1-(2-((6-amino-9H-purin-9-yl)méthyl)-3-éthyl-4-fluorophényl)pyrrolidine-3-carbonyl)piperazin-1-yl)prop-2-en-1-one, (R)-9-(2-(3-amino-3-(1H-1,2,4-triazol-3-yl)pyrrolidin-1-yl)-6-bromo-4-chlorobenzyl)-9H-purin-6-amine, (R)-(3-amino-1-(2-((6-amino-9H-purin-9-yl)méthyl)-3-bromo-5-chlorophényl)pyrrolidin-3-yl)methanol, (R)-3-amino-1-(6-((6-amino-9H-purin-9-yl)méthyl)-5-bromo-4-fluoro-2,3-dihydrobenzofuran-7-yl)-N-cyclopropylpyrrolidine-3-carboxamide, (R)-3-amino-1-(2-((6-amino-9H-purin-9-yl)méthyl)-3-bromo-6-méthoxyphényl)-N-cyclopropylpyrrolidine-3-carboxamide, 9-(6-(3-amino-3-(pyridin-2-yl)pyrrolidin-1-yl)-3-fluoro-2-(trifluorométhyl)benzyl)-9H-purin-6-amine, (R)-9-(6-(3-amino-3-(pyridin-2-yl)pyrrolidin-1-yl)-3-fluoro-2-(trifluorométhyl)benzyl)-9H-purin-6-amine, (R)-9-(2-(3-amino-3-(1H-1,2,4-triazol-3-yl)pyrrolidin-1-yl)-4-chloro-6-vinylbenzyl)-9H-purin-6-amine, (R)-9-(2-(3-amino-3-(1H-1,2,4-triazol-3-yl)pyrrolidin-1-yl)-4-chloro-6-éthylbenzyl)-9H-purin-6-amine, (S)-9-(6-(3-amino-3-phénylpyrrolidin-1-yl)-3-fluoro-2-(trifluorométhyl)benzyl)-9H-purin-6-amine, (R)-9-(2-(3-amino-3-(4-(trifluorométhyl)pyridin-2-yl)pyrrolidin-1-yl)-6-bromo-4-chlorobenzyl)-9H-purin-6-amine, (R)-9-(6-(3-amino-3-(2-(pyridin-2-yl)éthyl)pyrrolidin-1-yl)-3-fluoro-2-(trifluorométhyl)benzyl)-9H-purin-6-amine, (R)-3-amino-1-(2-((6-amino-9H-purin-9-yl)méthyl)-5-chloro-3-cyclobutylphényl)pyrrolidine-3-carboxylate de méthyle, (R)-2-((3-amino-1-(2-((6-amino-9H-purin-9-yl)méthyl)-3-bromo-5-chlorophényl)pyrrolidin-3-yl)méthoxy)-N-cyclopropylacétamide, 3-amino-1-(5-((6-amino-9H-purin-9-yl)méthyl)-6-bromobenzo[d][1,3]dioxol-4-yl)-N-cyclopropylpyrrolidine-3-carboxamide, (R)-9-(2-(3-amino-3-(méthoxyméthyl)pyrrolidin-1-yl)-6-bromo-4-chlorobenzyl)-9H-purin-6-amine, (R)-3-amino-1-(2-((6-amino-9H-purin-9-yl)méthyl)-5-chloro-3-((R)-2,2,2-trifluoro-1-hydroxyéthyl)phényl)-N-cyclopropylpyrrolidine-3-carboxamide, (R)-3-(3-amino-1-(2-((6-amino-9H-purin-9-yl)méthyl)-3-bromo-5-chlorophényl)pyrrolidin-3-yl)propanoate d'éthyle, (R)-5-(2-(3-amino-1-(2-((6-amino-9H-purin-9-yl)méthyl)-5-chloro-3-éthylphényl)pyrrolidin-3-yl)éthyl)-N-méthyl-1,3,4-thiadiazol-2-amine, (R)-3-(3-amino-1-(2-((6-amino-9H-purin-9-yl)méthyl)-3-bromo-5-chlorophényl)pyrrolidin-3-yl)-N-éthylpropanamide, 3-(3-amino-1-(2-((6-amino-9H-purin-9-yl)méthyl)-3-bromo-5-chlorophényl)pyrrolidin-3-yl)propanamide, (R)-3-(3-amino-1-(2-((6-amino-9H-purin-9-yl)méthyl)-5-chloro-3-éthylphényl)pyrrolidin-3-yl)-N-éthylpropanamide, (R)-9-(2-(3-(2-(2H-tétrazol-5-yl)éthyl)-3-aminopyrrolidin-1-yl)-6-bromo-4-chlorobenzyl)-9H-purin-6-amine, (R)-9-(2-(3-(2-(2H-tétrazol-5-yl)éthyl)-3-aminopyrrolidin-1-yl)-4-chloro-6-éthylbenzyl)-9H-purin-6-amine, (R)-3-amino-1-(2-((6-amino-9H-purin-9-yl)méthyl)-3-bromo-4-(trifluorométhyl)phényl)-N-cyclopropylpyrrolidine-3-carboxamide, 3-amino-1-(5-((6-amino-9H-purin-9-yl)méthyl)-6-bromo-7-chloro-2,3-dihydro-1H-inden-4-yl)-N-cyclopropylpyrrolidine-3-carboxamide, (R)-3-amino-1-(2-((6-amino-9H-purin-9-yl)méthyl)-3-bromo-5,6-dichlorophényl)-N-cyclopropylpyrrolidine-3-carboxamide, 3-amino-1-(2-((6-amino-9H-purin-9-yl)méthyl)-3-bromo-4,5,6-trichlorophényl)-N-cyclopropylpyrrolidine-3-carboxamide, (R)-3-amino-1-(2-((6-amino-9H-purin-9-yl)méthyl)-3-((diméthylamino)méthyl)-4-fluorophényl)-N-cyclopropylpyrrolidine-3-carboxamide, (R)-3-amino-1-(2-((6-amino-9H-purin-9-yl)méthyl)-4-fluoro-3-(pipéridin-1-ylméthyl)phényl)-N-cyclopropylpyrrolidine-3-carboxamide, (R)-3-amino-1-(2-((6-amino-9H-purin-9-yl)méthyl)-3-((diméthylamino)méthyl)-4-(trifluorométhyl)phényl)-N-cyclopropylpyrrolidine-3-carboxamide, (R)-3-amino-1-(2-((6-amino-9H-purin-9-yl)méthyl)-3-((cyclopropyl(méthyl)amino)méthyl)-4-fluorophényl)-N-cyclopropylpyrrolidine-3-carboxamide, (R)-3-amino-1-(2-((6-amino-9H-purin-9-yl)méthyl)-3-((diméthylamino)méthyl)-4-fluorophényl)-N-((1S,2R)-2-phénylcyclopropyl)pyrrolidine-3-carboxamide, (R)-3-amino-1-(2-((6-amino-9H-purin-9-yl)méthyl)-4-fluoro-3-(((R)-3-fluoropyrrolidin-1-yl)méthyl)phényl)-N-cyclopropylpyrrolidine-3-carboxamide, (R)-3-amino-1-(2-((6-amino-9H-purin-9-yl)méthyl)-4-fluoro-3-(morpholinométhyl)phényl)-N-((1S,2R)-2-phénylcyclopropyl)pyrrolidine-3-carboxamide, (R)-3-amino-1-(2-((6-amino-9H-purin-9-yl)méthyl)-3-((3,3-difluoroazétidin-1-yl)méthyl)-4-fluorophényl)-N-cyclopropylpyrrolidine-3-carboxamide, (R)-3-amino-1-(2-((6-amino-9H-purin-9-yl)méthyl)-4-fluoro-3-((3-fluoroazétidin-1-yl)méthyl)phényl)-N-cyclopropylpyrrolidine-3-carboxamide, (R)-3-amino-1-(2-((6-amino-9H-purin-9-yl)méthyl)-3-((cyclopropylamino)méthyl)-4-fluorophényl)-N-cyclopropylpyrrolidine-3-carboxamide, (R)-3-amino-1-(2-((6-amino-9H-purin-9-yl)méthyl)-3-((4,4-difluoropipéridin-1-yl)méthyl)-4-fluorophényl)-N-cyclopropylpyrrolidine-3-carboxamide, (R)-3-amino-1-(2-((6-amino-9H-purin-9-yl)méthyl)-4-fluoro-3-((4-fluoropipéridin-1-yl)méthyl)phényl)-N-cyclopropylpyrrolidine-3-carboxamide, (R)-3-amino-1-(2-((6-amino-9H-purin-9-yl)méthyl)-4-fluoro-3-(((2,2,2-trifluoroéthyl)amino)méthyl)phényl)-N-cyclopropylpyrrolidine-3-carboxamide, (3R)-1-(3-((8-oxa-3-azabicyclo[3.2.1]octan-3-yl)méthyl)-2-((6-amino-9H-purin-9-yl)méthyl)-4-fluorophényl)-3-amino-N-cyclopropylpyrrolidine-3-carboxamide, (R)-3-amino-1-(2-((6-amino-9H-purin-9-yl)méthyl)-3-((cyclopropyl(éthyl)amino)méthyl)-4-fluorophényl)-N-cyclopropylpyrrolidine-3-carboxamide, (R)-3-amino-1-(2-((6-amino-9H-purin-9-yl)méthyl)-4-chloro-3-((diméthylamino)méthyl)phényl)-N-cyclopropylpyrrolidine-3-carboxamide, (R)-3-amino-1-(2-((6-amino-9H-purin-9-yl)méthyl)-4-fluoro-3-(pyrrolidin-1-ylméthyl)phényl)-N-cyclopropylpyrrolidine-3-carboxamide, (R)-3-amino-1-(2-((6-amino-9H-purin-9-yl)méthyl)-3-(azétidin-1-ylméthyl)-4-(trifluorométhyl)phényl)-N-cyclopropylpyrrolidine-3-carboxamide, (R)-3-amino-1-(2-((6-amino-9H-purin-9-yl)méthyl)-3-((3,3-diméthylpyrrolidin-1-yl)méthyl)-4-fluorophényl)-N-cyclopropylpyrrolidine-3-carboxamide, (R)-3-amino-1-(2-((6-amino-9H-purin-9-yl)méthyl)-4-fluoro-3-((3-méthylazétidin-1-yl)méthyl)phényl)-N-cyclopropylpyrrolidine-3-carboxamide, (R)-3-amino-1-(2-((6-amino-9H-purin-9-yl)méthyl)-4-fluoro-3-(morpholinométhyl)phényl)-N-cyclopropylpyrrolidine-3-carboxamide, (R)-3-amino-1-(2-((6-amino-9H-purin-9-yl)méthyl)-3-((3,3-difluoropyrrolidin-1-yl)méthyl)-4-fluorophényl)-N-cyclopropylpyrrolidine-3-carboxamide, (R)-3-amino-1-(2-((6-amino-9H-purin-9-yl)méthyl)-3-((diéthylamino)méthyl)-4-fluorophényl)-N-cyclopropylpyrrolidine-3-carboxamide, (R)-3-amino-1-(2-((6-amino-9H-purin-9-yl)méthyl)-4-fluoro-3-(((S)-3-fluoropyrrolidin-1-yl)méthyl)phényl)-N-cyclopropylpyrrolidine-3-carboxamide, (R)-3-amino-1-(2-((6-amino-9H-purin-9-yl)méthyl)-3-((3,3-difluoropipéridin-1-yl)méthyl)-4-fluorophényl)-N-cyclopropylpyrrolidine-3-carboxamide, (R)-3-amino-1-(2-((6-amino-9H-purin-9-yl)méthyl)-3-(azétidin-1-ylméthyl)-4-fluorophényl)-N-cyclopropylpyrrolidine-3-carboxamide, (R)-(3-(3-amino-3-(cyclopropylcarbamoyl)pyrrolidin-1-yl)-2-((6-amino-9H-purin-9-yl)méthyl)-6-fluorobenzyl)carbamate de méthyle, (R)-3-amino-1-(2-((6-amino-9H-purin-9-yl)méthyl)-6-chloro-3-((diméthylamino)méthyl)-4-fluorophényl)-N-cyclopropylpyrrolidine-3-carboxamide, (R)-(3-amino-1-(2-((6-amino-9H-purin-9-yl)méthyl)-4,6-dichloro-3-éthylphényl)pyrrolidin-3-yl)(1, 1 - dioxydothiomorpholino)méthanone, (R)-3-amino-1-(2-((6-amino-9H-purin-9-yl)méthyl)-4,6-dichloro-3-((diméthylamino)méthyl)phényl)-N-cyclopropylpyrrolidine-3-carboxamide, (R)-3-amino-1-(2-((6-amino-9H-purin-9-yl)méthyl)-6-chloro-4-fluoro-3-(morpholinométhyl)phényl)-N-cyclopropylpyrrolidine-3-carboxamide, (R)-3-amino-1-(2-((6-amino-9H-purin-9-yl)méthyl)-4-fluoro-3-(hydroxyméthyl)phényl)-N-cyclopropylpyrrolidine-3-carboxamide, (R)-3-amino-1-(2-((6-amino-9H-purin-9-yl)méthyl)-4-fluoro-3-(méthoxyméthyl)phényl)-N-cyclopropylpyrrolidine-3-carboxamide, (R)-3-amino-1-(2-((6-amino-9H-purin-9-yl)méthyl)-4-chloro-3-(méthoxyméthyl)phényl)-N-cyclopropylpyrrolidine-3-carboxamide, (3R)-3-amino-1-(2-((6-amino-9H-purin-9-yl)méthyl)-4-chloro-3-(((tétrahydrofuran-3-yl)oxy)méthyl)phényl)-N-cyclopropylpyrrolidine-3-carboxamide, (R)-3-amino-1-(2-((6-amino-9H-purin-9-yl)méthyl)-4-fluoro-3-((méthoxy-d3)méthyl)phényl)-N-cyclopropylpyrrolidine-3-carboxamide, (R)-3-amino-1-(2-((6-amino-9H-purin-9-yl)méthyl)-4-chloro-3-((méthoxy-d3)méthyl)phényl)-N-cyclopropylpyrrolidine-3-carboxamide, (R)-3-amino-1-(2-((6-amino-9H-purin-9-yl)méthyl)-4-fluoro-3-((S)-2,2,2-trifluoro-1-hydroxyéthyl)phényl)-N-cyclopropylpyrrolidine-3-carboxamide, (R)-9-(2-(3-amino-3-(1H-tétrazol-5-yl)pyrrolidin-1-yl)-6-bromo-4-chlorobenzyl)-9H-purin-6-amine, (R)-9-((3-(3-amino-3-(2H-tétrazol-5-yl)pyrrolidin-1-yl)-5-chloro-3'-cyclohexyl-[1,1'-biphényl]-2-yl)méthyl)-9H-purin-6-amine, (R)-9-(6-(3-amino-3-(1H-1,2,4-triazol-3-yl)pyrrolidin-1-yl)-2,3-dichlorobenzyl)-9H-purin-6-amine, (R)-3-amino-1-(2-((6-amino-9H-purin-9-yl)méthyl)-4-chloro-3-((difluorométhoxy)méthyl)phényl)-N-cyclopropylpyrrolidine-3-carboxamide, (R)-3-amino-1-(2-((6-amino-9H-purin-9-yl)méthyl)-3-bromo-4-(trifluorométhyl)phényl)-N-(2-cyanoéthyl)pyrrolidine-3-carboxamide, (R)-3-amino-1-(2-((6-amino-9H-purin-9-yl)méthyl)-4,5-dichloro-3-((diméthylamino)méthyl)phényl)-N-cyclopropylpyrrolidine-3-carboxamide, (R)-3-amino-1-(2-((6-amino-9H-purin-9-yl)méthyl)-5,6-dichloro-3-((diméthylamino)méthyl)phényl)-N-cyclopropylpyrrolidine-3-carboxamide, (R)-9-(2-(3-amino-3-(1-méthyl-1H-tétrazol-5-yl)pyrrolidin-1-yl)-6-bromo-4-chlorobenzyl)-9H-purin-6-amine, (R)-9-(2-(3-amino-3-(2-méthyl-2H-tétrazol-5-yl)pyrrolidin-1-yl)-6-bromo-4-chlorobenzyl)-9H-purin-6-amine, (R)-3-amino-1-(4-((6-amino-9H-purin-9-yl)méthyl)-5-éthyl-2'-fluoro-[1,1'-biphényl]-3-yl)-N-cyclopropylpyrrolidine-3-carboxamide, (R)-3-amino-1-(2-((6-amino-9H-purin-9-yl)méthyl)-3-éthyl-5-(thiophen-2-yl)phényl)-N-cyclopropylpyrrolidine-3-carboxamide, (R)-3-amino-1-(2-((6-amino-9H-purin-9-yl)méthyl)-5-(thiophen-2-yl)-3-(trifluorométhyl)phényl)-N-cyclopropylpyrrolidine-3-carboxamide, (R)-3-amino-1-(2-((6-amino-9H-purin-9-yl)méthyl)-3-éthyl-5-(pyridin-3-yl)phényl)-N-cyclopropylpyrrolidine-3-carboxamide, (R)-3-amino-1-(2-((6-amino-9H-purin-9-yl)méthyl)-3-éthyl-5-(1-méthyl-1H-pyrazol-4-yl)phényl)-N-cyclopropylpyrrolidine-3-carboxamide, 3-amino-1-(2-((6-amino-9H-purin-9-yl)méthyl)-4-chloro-3-cyclopropylphényl)-N-cyclopropylpyrrolidine-3-carboxamide, (R)-3-amino-1-(2-((6-amino-9H-purin-9-yl)méthyl)-3-éthyl-5-(thiazol-4-yl)phényl)-N-cyclopropylpyrrolidine-3-carboxamide, (R)-3-amino-1-(2-((6-amino-9H-purin-9-yl)méthyl)-3-éthyl-5-(pyridin-2-yl)phényl)-N-cyclopropylpyrrolidine-3-carboxamide, (R)-3-amino-1-(2-((6-amino-9H-purin-9-yl)méthyl)-3-éthyl-5-(pyrazin-2-yl)phényl)-N-cyclopropylpyrrolidine-3-carboxamide, (R)-3-amino-1-(2-((6-amino-9H-purin-9-yl)méthyl)-5-chloro-4-cyano-3-éthylphényl)-N-cyclopropylpyrrolidine-3-carboxamide, (R)-3-amino-1-(2-((6-amino-9H-purin-9-yl)méthyl)-5-chloro-4-fluoro-3-(morpholinométhyl)phényl)-N-cyclopropylpyrrolidine-3-carboxamide, (R)-3-amino-1-(2-((6-amino-9H-purin-9-yl)méthyl)-3-chloro-4-fluoro-5-(morpholinométhyl)phényl)-N-cyclopropylpyrrolidine-3-carboxamide, (R)-3-amino-1-(2-((6-amino-9H-purin-9-yl)méthyl)-4-chloro-3-cyanophényl)-N-cyclopropylpyrrolidine-3-carboxamide, (R)-3-amino-1-(2-((6-amino-9H-purin-9-yl)méthyl)-4,5-dichloro-3-(morpholinométhyl)phényl)-N-cyclopropylpyrrolidine-3-carboxamide, (S)-(3-amino-1-(1-((6-amino-9H-purin-9-yl)méthyl)naphthalen-2-yl)pyrrolidin-3-yl)méthanol, (S)-(3-amino-1-(2-((6-amino-9H-purin-9-yl)méthyl)-3-bromo-5-chlorophényl)pyrrolidin-3-yl)méthanol, (R)-(3-amino-1-(2-((6-amino-9H-purin-9-yl)méthyl)-5-chloro-3-éthylphényl)pyrrolidin-3-yl)méthanol, (R)-9-((2-(3-amino-3-(méthoxyméthyl)pyrrolidin-1-yl)naphthalen-1-yl)méthyl)-9H-purin-6-amine, (R)-3-amino-1-(6-((6-amino-9H-purin-9-yl)méthyl)-5-bromo-4-chloro-2,3-dihydrobenzofuran-7-yl)-N-cyclopropylpyrrolidine-3-carboxamide, (R)-3-amino-1-(6-((6-amino-9H-purin-9-yl)méthyl)-4-fluoro-5-iodo-2,3-dihydrobenzofuran-7-yl)-N-cyclopropylpyrrolidine-3-carboxamide, (R)-3-amino-1-(7-((6-amino-9H-purin-9-yl)méthyl)benzofuran-6-yl)-N-cyclopropylpyrrolidine-3-carboxamide, (R)-3-amino-1-(2-((6-amino-9H-purin-9-yl)méthyl)-4-chloro-3-(éthylthio)phényl)-N-cyclopropylpyrrolidine-3-carboxamide, (R)-3-amino-1-(7-((6-amino-9H-purin-9-yl)méthyl)-2,2-diméthyl-2,3-dihydrobenzofuran-6-yl)-N-cyclopropylpyrrolidine-3-carboxamide, 3-amino-1-(5-((6-amino-9H-purin-9-yl)méthyl)-6-bromo-2,3-dihydrobenzofuran-4-yl)-N-cyclopropylpyrrolidine-3-carboxamide, (R)-3-amino-1-(2-((6-amino-9H-purin-9-yl)méthyl)-4-fluoro-3-(trifluorométhyl)phényl)-N-cyclopropylpyrrolidine-3-carboxamide, (R)-3-amino-1-(2-((6-amino-9H-purin-9-yl)méthyl)-4-fluoro-3-(trifluorométhyl)phényl)-N-((1r,3R)-3-(méthylthio)cyclobutyl)pyrrolidine-3-carboxamide, (R)-3-amino-1-(2-((6-amino-9H-purin-9-yl)méthyl)-4-fluoro-3-(trifluorométhyl)phényl)-N-(pyridazin-3-ylméthyl)pyrrolidine-3-carboxamide, (R)-3-amino-1-(2-((6-amino-9H-purin-9-yl)méthyl)-4-fluoro-3-(trifluorométhyl)phényl)-N-(2-cyano-2-méthylpropyl)pyrrolidine-3-carboxamide, (R)-3-amino-1-(2-((6-amino-9H-purin-9-yl)méthyl)-4-fluoro-3-(trifluorométhyl)phényl)-N-((2-hydroxypyridin-3-yl)méthyl)pyrrolidine-3-carboxam ide, (R)-N-(2-(1H-imidazol-4-yl)éthyl)-3-amino-1-(2-((6-amino-9H-purin-9-yl)méthyl)-4-fluoro-3-(trifluorométhyl)phényl)pyrrolidine-3-carboxamide, (3R)-3-amino-1-(2-((6-amino-9H-purin-9-yl)méthyl)-4-fluoro-3-(trifluorométhyl)phényl)-N-((2,2-difluorocyclopropyl)méthyl)pyrrolidine-3-carboxamide, (R)-3-amino-1-(2-((6-amino-9H-purin-9-yl)méthyl)-4-fluoro-3-(trifluorométhyl)phényl)-N-(3-cyclopropyl-1H-pyrazol-5-yl)pyrrolidine-3-carboxamide, (R)-3-amino-1-(2-((6-amino-9H-purin-9-yl)méthyl)-4-fluoro-3-(trifluorométhyl)phényl)-N-((R)-1-(tétrahydro-2H-pyran-4-yl)éthyl)pyrrolidine-3-carboxamide, (R)-3-amino-1-(2-((6-amino-9H-purin-9-yl)méthyl)-4-fluoro-3-(trifluorométhyl)phényl)-N-(2-cyano-2-méthylpropyl)pyrrolidine-3-carboxamide, (3R)-3-amino-1-(2-((6-amino-9H-purin-9-yl)méthyl)-4-fluoro-3-(trifluorométhyl)phényl)-N-(6-oxaspiro[2.5]octan-1-yl)pyrrolidine-3-carboxamide, (R)-3-amino-1-(2-((6-amino-9H-purin-9-yl)méthyl)-4-fluoro-3-(trifluorométhyl)phényl)-N-((1R,2R)-2-(hydroxyméthyl)cyclopropyl)pyrrolidine-3-carboxamide, (R)-(3-amino-1-(2-((6-amino-9H-purin-9-yl)méthyl)-4-fluoro-3-(trifluorométhyl)phényl)pyrrolidin-3-yl)(6,7-dihydro-[1,2,3]triazolo[1,5-a]pyrazin-5(4H)-yl)méthanone, (3R)-3-amino-1-(2-((6-amino-9H-purin-9-yl)méthyl)-4-fluoro-3-(trifluorométhyl)phényl)-N-(tétrahydrofuran-3-yl)pyrrolidine-3-carboxamide, (3R)-3-amino-1-(2-((6-amino-9H-purin-9-yl)méthyl)-4-fluoro-3-(trifluorométhyl)phényl)-N-(5-méthoxyspiro[2.3]hexan-1-yl)pyrrolidine-3-carboxamide, ((R)-3-amino-1-(2-((6-amino-9H-purin-9-yl)méthyl)-4-fluoro-3-(trifluorométhyl)phényl)pyrrolidin-3-yl)((S)-2-méthylmorpholino)méthanone, (R)-1-(3-amino-1-(2-((6-amino-9H-purin-9-yl)méthyl)-4-fluoro-3-(trifluorométhyl)phényl)pyrrolidine-3-carbonyl)azétidine-3-carbonitrile, (R)-3-amino-1-(2-((6-amino-9H-purin-9-yl)méthyl)-4-fluoro-3-(trifluorométhyl)phényl)-N-((2-aminopyrimidin-4-yl)méthyl)pyrrolidine-3-carboxamide, (R)-(3-amino-1-(2-((6-amino-9H-purin-9-yl)méthyl)-4-fluoro-3-(trifluorométhyl)phényl)pyrrolidin-3-yl)(6,7-dihydroisoxazolo[4,3-c]pyridin-5(4H)-yl)méthanone, ((R)-3-amino-1-(2-((6-amino-9H-purin-9-yl)méthyl)-4-fluoro-3-(trifluorométhyl)phényl)pyrrolidin-3-yl)((R)-2-méthylmorpholino)méthanone, (R)-2-(4-(3-amino-1-(2-((6-amino-9H-purin-9-yl)méthyl)-4-fluoro-3-(trifluorométhyl)phényl)pyrrolidine-3-carbonyl)piperazin-1-yl)acétonitrile, (R)-(3-amino-1-(2-((6-amino-9H-purin-9-yl)méthyl)-4-fluoro-3-(trifluorométhyl)phényl)pyrrolidin-3-yl)(6,7-dihydropyrazolo[1,5-a]pyrazin-5(4H)-yl)méthanone, ((R)-3-amino-1-(2-((6-amino-9H-purin-9-yl)méthyl)-4-fluoro-3-(trifluorométhyl)phényl)pyrrolidin-3-yl)((R)-3-hydroxypipéridin-1-yl)méthanone, (R)-3-amino-1 -(2-((6-amino-9H-purin-9-yl)méthyl)-4-fluoro-3-(trifluorométhyl)phényl)-N-(3-oxo-3-(propylamino)propyl)pyrrolidine-3-carboxamide, (R)-3-amino-1-(2-((6-amino-9H-purin-9-yl)méthyl)-4-fluoro-3-(trifluorométhyl)phényl)-N-((1-éthyl-1H-1,2,4-triazol-5-yl)méthyl)pyrrolidine-3-carboxamide, 1-((R)-3-amino-1-(2-((6-amino-9H-purin-9-yl)méthyl)-4-fluoro-3-(trifluorométhyl)phényl)pyrrolidine-3-carbonyl)pyrrolidine-3-carbonitrile, acide (S)-3-amino-1-(2-((6-amino-9H-purin-9-yl)méthyl)-3-bromo-6-chlorophényl)pyrrolidine-3-carboxylique, (R)-3-amino-1-(2-((6-amino-9H-purin-9-yl)méthyl)-3-bromo-4,6-dichlorophényl)-N-cyclopropylpyrrolidine-3-carboxamide, (R)-3-amino-1-(2-((6-amino-9H-purin-9-yl)méthyl)-3,4-dichlorophényl)-N-cyclopropylpyrrolidine-3-carboxamide, (R)-3-amino-1-(2-((6-amino-9H-purin-9-yl)méthyl)-4-chloro-3-éthylphényl)-N-(2-(phénylsulfonyl)éthyl)pyrrolidine-3-carboxamide, (R)-3-amino-1-(2-((6-amino-9H-purin-9-yl)méthyl)-4-bromo-3-(trifluorométhyl)phényl)-N-cyclopropylpyrrolidine-3-carboxamide, (R)-3-amino-1-(2-((6-amino-9H-purin-9-yl)méthyl)-3-bromo-4-(trifluorométhoxy)phényl)-N-cyclopropylpyrrolidine-3-carboxamide, (R)-3-amino-1-(2-((6-amino-9H-purin-9-yl)méthyl)-3-bromo-5-chlorophényl)pyrrolidine-3-carboxamide, (R)-3-amino-1-(2-((6-amino-9H-purin-9-yl)méthyl)-4-chloro-3-éthylphényl)-N-(2-(éthylsulfonyl)éthyl)pyrrolidine-3-carboxamide, (R)-3-amino-1-(2-((6-amino-9H-purin-9-yl)méthyl)-5-chloro-3-(5,6,7,8-tétrahydronaphthalen-1-yl)phényl)-N-cyclopropylpyrrolidine-3-carboxamide, (R)-(3-amino-1-(2-((6-amino-9H-purin-9-yl)méthyl)-5-chloro-3-éthylphényl)pyrrolidin-3-yl)(1,1 - dioxydothiomorpholino)méthanone, 3-amino-1-(2-((6-amino-9H-purin-9-yl)méthyl)-3,4-dichloro-6-fluorophényl)-N-cyclopropylpyrrolidine-3-carboxamide, (R)-3-amino-1-(2-((6-amino-9H-purin-9-yl)méthyl)-4-chloro-3-éthylphényl)-N-(1,1-dioxydotétrahydro-2H-thiopyran-4-yl)pyrrolidine-3-carboxamide, (R)-3-amino-1-(2-((6-amino-9H-purin-9-yl)méthyl)-6-chloro-3-éthylphényl)-N-cyclopropylpyrrolidine-3-carboxamide, (R)-(3-amino-1-(2-((6-amino-9H-purin-9-yl)méthyl)-4-chloro-3-éthylphényl)pyrrolidin-3-yl)(1,1-dioxydothiomorpholino)méthanone, (R)-9-(6-(3-amino-3-(1H-tétrazol-5-yl)pyrrolidin-1-yl)-3-chloro-2-iodobenzyl)-9H-purin-6-amine, 3-amino-1-(2-((6-amino-9H-purin-9-yl)méthyl)-3-chloro-6-méthylphényl)-N-cyclopropylpyrrolidine-3-carboxamide, (R)-3-amino-1-(2-((6-amino-9H-purin-9-yl)méthyl)-3-cyclopentyl-4-fluorophényl)-N-cyclopropylpyrrolidine-3-carboxamide, (R)-3-amino-1-(2-((6-amino-9H-purin-9-yl)méthyl)-3-bromo-5-chlorophényl)-N-(éthyl-d5)pyrrolidine-3-carboxam ide, 3-amino-1-(2-((6-amino-9H-purin-9-yl)méthyl)-3-chloro-4-(trifluorométhyl)phényl)-N-cyclopropylpyrrolidine-3-carboxamide, (R)-3-amino-1-(2-((6-amino-9H-purin-9-yl)méthyl)-5-chloro-3-éthylphényl)-N-isopropylpyrrolidine-3-carboxamide, (R)-3-amino-1-(2-((6-amino-9H-purin-9-yl)méthyl)-4,6-dichloro-3-(méthoxyméthyl)phényl)-N-cyclopropylpyrrolidine-3-carboxamide, (R)-3-amino-1-(2-((6-amino-9H-purin-9-yl)méthyl)-4-chloro-3-vinylphényl)-N-cyclopropylpyrrolidine-3-carboxam ide, 3-amino-1-(2-((6-amino-9H-purin-9-yl)méthyl)-3-chlorophényl)-N-cyclopropylpyrrolidine-3-carboxamide, (R)-3-amino-1-(2-((6-amino-9H-purin-9-yl)méthyl)-4-chloro-3-éthylphényl)-N-cyclopropylpyrrolidine-3-carboxamide, (S)-3-amino-1-(1-((6-amino-9H-purin-9-yl)méthyl)naphthalen-2-yl)-N-cyclopropylpyrrolidine-3-carboxamide, (R)-3-amino-1-(1-((6-amino-9H-purin-9-yl)méthyl)-6-bromonaphthalen-2-yl)-N-cyclopropylpyrrolidine-3-carboxamide, (R)-3-amino-1-(2-((6-amino-9H-purin-9-yl)méthyl)-3-bromo-4-méthoxyphényl)-N-cyclopropylpyrrolidine-3-carboxamide, (R)-3-amino-1-(2-((6-amino-9H-purin-9-yl)méthyl)-3-éthylphényl)-N-cyclopentylpyrrolidine-3-carboxamide, (R)-3-amino-1-(2-((6-amino-9H-purin-9-yl)méthyl)-3-bromo-5-chlorophényl)pyrrolidine-3-carboxylate d'éthyle.

14. Composition pharmaceutique comprenant un composé selon l'une quelconque des revendications 1-13 ou un sel de qualité pharmaceutique dudit composé, et un ou plusieurs vecteurs de qualité pharmaceutique.

15. Combinaison comprenant un composé selon l'une quelconque des revendications 1-13 ou un sel de qualité pharmaceutique dudit composé, et un ou plusieurs agents à activité thérapeutique.

16. La combinaison selon la revendication 15, dans laquelle ledit ou lesdits agents à activité thérapeutique comprennent un agent anticancer, un antalgique ou un agent antiinflammatoire.

17. Un composé selon l'une quelconque des revendications 1-13 ou un sel de qualité pharmaceutique dudit composé, à être utilisé dans un procédé permettant de traiter une maladie ou affection pouvant bénéficier de, ou être traitée par, l'inhibition de la leucémie 1 de lignée mixte (LLM 1).

18. Un composé à être utilisé selon la revendication 17, dans lequel ladite maladie ou affection pouvant bénéficier de, ou être traitée par, l'inhibition de la LLM 1 est choisie parmi les tumeurs solides, la leucémie, le myélome, le lymphome et l'hypertension.

19. Un composé à être utilisé selon la revendication 17, dans lequel ladite maladie ou affection pouvant bénéficier de, ou être traitée par, l'inhibition de la LLM 1 comprend le cancer du sein, le cancer du col de l'utérus, le cancer de la peau, le cancer de l'ovaire, le cancer de l'estomac, le cancer de la prostate, le cancer du pancréas, le cancer du poumon, le carcinome hépatocellulaire, le cancer de la tête et du cou, la tumeur de la gaine du nerf périphérique, l'ostéosarcome, le myélome multiple, le neuroblastome, la leucémie, le lymphome non hodgkinien ou l'hypertension artérielle pulmonaire.

20. Un composé selon l'une quelconque des revendications 1-13 et éventuellement en combinaison avec un second agent thérapeutique, à être utilisé dans l'élaboration d'un médicament destiné à une maladie ou affection pouvant bénéficier de, ou être traitée par, l'inhibition de la leucémie 1 de lignée mixte (LLM 1).
